(19) Europäisches Patentamt

European Patent Office

Office européen des brevets

(11) **EP 3 899 030 B1**

(12) **EUROPEAN PATENT SPECIFICATION**

(45) Date of publication and mention
of the grant of the patent:
**07.05.2025 Bulletin 2025/19**

(21) Application number: **19842446.7**

(22) Date of filing: **16.12.2019**

(51) International Patent Classification (IPC):
***C12Q 1/6806*** (2018.01)      ***C12Q 1/6886*** (2018.01)

(52) Cooperative Patent Classification (CPC):
(C-Sets available)
**C12Q 1/6806; C12Q 1/6886;** C12Q 2600/156
(Cont.)

(86) International application number:
**PCT/US2019/066544**

(87) International publication number:
**WO 2020/131699 (25.06.2020 Gazette 2020/26)**

(54) **METHODS FOR ANALYSIS OF CIRCULATING CELLS**

VERFAHREN ZUR ANALYSE VON ZIRKULIERENDEN ZELLEN

PROCÉDÉS D'ANALYSE DE CELLULES CIRCULANTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.12.2018 US 201862780724 P
28.01.2019 US 201962797518 P**

(43) Date of publication of application:
**27.10.2021 Bulletin 2021/43**

(73) Proprietor: **Natera, Inc.
San Carlos, CA 94070 (US)**

(72) Inventors:
• **RIVERS, Elizabeth
San Carlos, CA 94070 (US)**
• **BREVNOV, Maxim
San Carlos, CA 94070 (US)**

(74) Representative: **Marks & Clerk LLP
15 Fetter Lane
London EC4A 1BW (GB)**

(56) References cited:
**WO-A1-2010/075459      US-A1- 2016 186 253**

• **SHYAMALA MAHESWARAN ET AL: "Detection of mutations in EGFR in circulating lung-cancer cells", INTERNET CITATION, 24 July 2008 (2008-07-24), pages 1 - 15, XP002794106, ISSN: 1533-4406, Retrieved from the Internet <URL:https://www.nejm.org/doi/pdf/10.1056/ NEJMoa0800668?articleTools=true> [retrieved on 20190916]**
• **THOMPSON JEFFREY C. ET AL: "Detection of Therapeutically Targetable Driver and Resistance Mutations in Lung Cancer Patients by Next-Generation Sequencing of Cell-Free Circulating Tumor DNA", CLINICAL CANCER RESEARCH, vol. 22, no. 23, 1 December 2016 (2016-12-01), US, pages 5772 - 5782, XP093036296, ISSN: 1078-0432, Retrieved from the Internet <URL:https://watermark.silverchair. com/5772.pdf? token=AQECAHi208BE49Ooan9kkhW_Er cy7Dm3ZL_9Cf3qfKAc485ysgAAAtlwggLOBgkq hkiG9w0BBwagggK_MIICuwlBADCCArQGCSqG SIb3DQEHATAeBglghkgBZQMEAS4wEQQMbRx aHole_fB0V0VhAgEQgIIChT9iGn7Yj_3s-6rlzoB4 z2NEmXNxJnlZJN5t3rFkiyrDUUOrjrP5EEe5zwb q2RZz37pTslYK1C79ThVf5DzEQS7fUdiJJb4> DOI: 10.1158/1078-0432.CCR-16-1231**

EP 3 899 030 B1

- SHYAMALA MAHESWARAN ET AL: "Detection of mutations in EGFR in circulating lung-cancer cells", INTERNET CITATION, 24 July 2008 (2008-07-24), pages 1 - 15, XP002794106, ISSN: 1533-4406, Retrieved from the Internet <URL:https://www.nejm.org/doi/pdf/10.1056/NEJMoa0800668?articleTools=true> [retrieved on 20190916]
- THOMPSON JEFFREY C. ET AL: "Detection of Therapeutically Targetable Driver and Resistance Mutations in Lung Cancer Patients by Next-Generation Sequencing of Cell-Free Circulating Tumor DNA", CLINICAL CANCER RESEARCH, vol. 22, no. 23, 1 December 2016 (2016-12-01), US, pages 5772 - 5782, XP093036296, ISSN: 1078-0432, Retrieved from the Internet <URL:https://watermark.silverchair.com/5772.pdf?token=AQECAHi208BE49Ooan9kkhW_Ercy7Dm3ZL_9Cf3qfKAc485ysgAAAtlwggLOBgkqhkiG9w0BBwagggK_MIICuwIBADCCArQGCSqGSIb3DQEHATAeBglghkgBZQMEAS4wEQQMbRxaHole_fB0V0VhAgEQglIChT9iGn7Yj_3s-6rlzoB4z2NEmXNxJnlZJN5t3rFkiyrDUUOrjrP5EEe5zwbq2RZz37pTsIYK1C79ThVf5DzEQS7fUdiJJb4> DOI: 10.1158/1078-0432.CCR-16-1231
- YIN XIUJU ET AL: "Identification of a de novo fetal variant in osteogenesis imperfecta by targeted sequencing-based noninvasive prenatal testing", JOURNAL OF HUMAN GENETICS, SPRINGER SINGAPORE, SINGAPORE, vol. 63, no. 11, 21 August 2018 (2018-08-21), pages 1129 - 1137, XP036619243, ISSN: 1434-5161, [retrieved on 20180821], DOI: 10.1038/S10038-018-0489-9

(52)  Cooperative Patent Classification (CPC): (Cont.)

     C-Sets
     C12Q 1/6806, C12Q 2535/122, C12Q 2537/165

**Description**

**CROSS REFERENCE TO RELATED APPLICATIONS**

This application claims priority to U.S. Provisional Application No. 62/780,724 filed December 17, 2018 and U.S. Provisional Application No. 62/797,518 filed January 28, 2019.

**BACKGROUND**

There is currently a great interest in using liquid biopsy in place of the more invasive tissue biopsy techniques for prenatal diagnostics, diagnostics of diseases and conditions, and testing and monitoring disease development and treatment responses. For example, liquid biopsy of pregnant mother provides diagnostics and tests of the fetus that are noninvasive and avoid the risks associated with performing invasive biopsy on the fetus or amniotic fluid. In the context of diagnosing and monitoring cancer development and treatment response, liquid biopsy may also avoid performing invasive biopsy of tumor tissue, which carries risk of potentially contributing to metastasis or surgical complications. Liquid biopsy based methods may also be more sensitive than imaging-based detection strategies that are not sufficiently sensitive to detect relapse or metastasis in an early stage.

Liquid biopsy is based on taking blood or urine sample from patients and isolating cell-free DNA and circulating cells that are shed from the fetus, the tumor, or other organs of interest. Analyzing cell-free DNA and circulating cells have provided powerful and noninvasive diagnostic methods. However, analyzing cell-free DNA will not provide the same amount of information as analyzing whole genome DNA directly from cells and/or tissues because cell-free DNA is fragmented and does not cover the whole genome. On the other hand, analyzing circulating cells is challenged by difficulties in characterizing and analyzing very small cell samples. Accordingly, to take full advantage of liquid biopsy based methods, a great need remains for methods to isolate and analyze samples containing only a few cells or a single cell.

**SUMMARY**

Disclosed herein are methods for analyzing and characterizing samples of circulating cells that may contain only a few circulating cells or a single circulating cell. In a first aspect, there is provided a method for monitoring and detection of early relapse or metastasis of a tumor in a cancer patient, wherein the method comprises the steps of:

(a) selecting one or more patient-specific mutations based on somatic mutations identified in a tumor sample of a patient who has been diagnosed with a cancer;
(b) longitudinally collecting one or more blood samples from the patient after the patient has been treated with surgery, first-line chemotherapy, and/or adjuvant therapy;
(c) generating a set of amplicons from cellular DNA isolated from one or more circulating cells that are suspected to be circulating tumor cells and are obtained from a blood sample of the patient, wherein the set of amplicons are generated by multiplex amplification of genomic loci encompassing the patient-specific mutations associated with cancer;
(d) sequencing the set of amplicons by next-generation sequencing and determining the origin of the one or more circulating cells based on the presence of one or more patient-specific mutations in the set of amplicons, wherein the detection of one or more circulating tumor cells comprising the one or more patient-specific mutations is indicative of early relapse or metastasis of the cancer.

In a second aspect of the present disclosure, there is provided a method for monitoring and detection of early relapse or metastasis of a tumor in a cancer patient, wherein the method comprises the steps of:

a) selecting one or more patient-specific mutations based on somatic mutations identified in a tumor sample of a patient who has been diagnosed with a cancer;
b) longitudinally collecting one or more blood samples from the patient after the patient has been treated with surgery, first-line chemotherapy, and/or adjuvant therapy;
c) generating a first set of amplicons from cell-free DNA isolated from a blood sample of the patient, wherein the first set of amplicons are generated by multiplex amplification of genomic loci encompassing the patient-specific mutations associated with cancer;
d) sequencing the first set of amplicons by next-generation sequencing and detecting the presence of one or more patient-specific mutations in the set of amplicons, wherein the presence of one or more patient-specific mutations is indicative of early relapse or metastasis of cancer;
e) generating a second set of amplicons from cellular DNA isolated from one or more circulating cells obtained from a blood sample of the patient, wherein the second set of amplicons are generated by multiplex amplification of genomic loci encompaissing the patient-specific mutations associated with cancer;

f) sequencing the second set of amplicons by next-generation sequencing and determining the origin of the one or more circulating cells based on the presence of one or more patient-specific mutations in the set of amplicons and by using the cell-free DNA sequences as reference to determine the origin of the cellular DNA and circulating cells, wherein the presence of one or more patient-specific mutations is indicative that the one or more circulating cells are tumor cells; and

g) identifying one or more additional mutations associated with cancer from the sequences of the one or more circulating cells determined to be tumor cells.

A method of analyzing and characterizing circulating cells suspected to be of fetal origin from a blood sample of a mother pregnant with a fetus or cell lines, may comprise the steps of a) generating a first set of amplicons from cellular DNA isolated from one or more circulating cells suspected to be of fetal origin obtained from the blood sample or cell lines and a second set of amplicons from cell-free DNA obtained from a plasma fraction of the blood sample or mixed DNA from child's cell line and mother's cell line, wherein the first set of amplicons and the second set of amplicons are obtained by performing a multiplex amplification reaction of a plurality of single nucleotide polymorphism (SNP) loci; b) genotyping the first set of amplicons and the second set of amplicons by next-generation sequencing; and c) determining that the one or more circulating cells are one or more maternal cells, one or more fetal cells, or a mixture of maternal cells and fetal cells based on the genotypes of the first set of amplicons and the second set of amplicons.

The disclosed methods may further comprise generating a third set of amplicons from maternal cellular DNA isolated from one or more maternal cells obtained from a buffy coat fraction of the blood sample, and genotyping the third set of amplicons by next-generation sequencing to determine maternal genotype.

The disclosed methods may further comprise obtaining the circulating cells suspected to be of fetal origin, the plasma faction comprising the cell-free DNA, and the buffy coat fraction comprising the maternal cells, from the blood sample.

Determining that the one or more circulating cells are one or more maternal cells, one or more fetal cells, or a mixture of maternal cells and fetal cells may involve calculating a maternal concordance and a fetal concordance between the cell-free DNA obtained from the plasma fraction (or known DNA mix of child and mom) and the cellular DNA obtained from the circulating cells suspected to be of fetal origin (or child cell line), and a calculation of a fetal mixture fraction (or child fraction).

In one example, the first set of amplicons and the second set of amplicons are obtained by performing a multiplex amplification reaction of at least 1,000 SNP loci.

The disclosed methods may comprise estimating the fetal fraction of the mixture of maternal and fetal cells (or the mixture of mom's and child's cell lines) by measuring an amount of the SNP loci.

In one example, the methods disclosed herein further comprise determining allele ratios at the SNPs.

Estimating the fetal fraction may use the allele ratios.

In one example, the methods disclosed herein comprise separating multiple circulating cells into individual reaction volumes, isolating cellular DNA in each reaction volume, and attaching a sample barcode to the cellular DNA isolated in each reaction volume.

Cellular DNA may be isolated from a single circulating cell suspected to be a fetal cell.

A method may further comprise performing non-invasive prenatal testing using the genotypes of one or more circulating cells determined to be one or more fetal cells.

A method may further comprise detecting a copy number variation or aneuploidy of a target chromosome or chromosome segment of interest in the one or more circulating cells determined to be one or more fetal cells.

The target chromosome or chromosome segment of interest may be chromosome 13, 18, 21, the sex chromosomes, and/or chromosome segments thereof.

The disclosed methods may further comprise detecting a microdeletion in the one or more circulating cell determined to be one or more pure fetal cells.

The microdeletion may be 22q11.2 deletion associated with DiGeorge syndrome, microdeletion associated with Prader-Willi syndrome, microdeletion associated with Angelman syndrom, 1p36 deletion, and/or microdeletion associated with the Cri-du-chat syndrome.

In one example, the present disclosure provides a method for monitoring and detection of early relapse or metastasis of a tumor in a cancer patient, by obtaining one or more circulating cells determined to originate from the tumor, wherein the method comprises the steps of:

a) selecting one or more patient-specific mutations based on mutations identified in a tumor sample of a patient who has been diagnosed with a cancer;

b) longitudinally collecting one or more blood samples from the patient after the patient has been treated with surgery, first-line chemotherapy, and/or adjuvant therapy;

c) generating a first set of amplicons from cellular DNA isolated from one or more circulating cells suspected to be circulating tumor cells obtained from the blood samples obtained from the cancer patient, and a second set of amplicons from cell free DNA, wherein the tumor cells and the normal cells are obtained from each of the blood samples or fractions thereof from the cancer patient, wherein the first set of amplicons and the second set of amplicons are obtained by performing a multiplex amplification reaction of the patient-specific mutations associated with cancer;

d) sequencing the first set of amplicons and the second set of amplicons by next-generation sequencing;

e) determining the origin of the one or more circulating cells based on the sequences of the first set of amplicons, wherein the sequences of the second set of amplicons are used as reference, wherein detection of one or more patient-specific mutations from first set of amplicons generated from the circulating cell determined to originate from the tumor is indicative of early relapse or metastasis of the cancer.

In one example, the disclosure herein may provide a method comprising the steps of:

a) longitudinally collecting one or more blood samples from a cancer patient after the patient has been treated with surgery, first-line chemotherapy, and/or adjuvant therapy;

b) generating a first set of amplicons from cellular DNA isolated from one or more circulating cells suspected to be circulating tumor cells obtained from the blood samples obtained from the cancer patient, and a second set of amplicons from cell free DNA, wherein the tumor cells and the normal cells are obtained from each of the blood samples or fractions thereof from the cancer patient, wherein the first set of amplicons and the second set of amplicons are obtained by performing a multiplex amplification reaction of the patient-specific mutations associated with cancer;

c) sequencing the first set of amplicons and the second set of amplicons by next-generation sequencing;

d) determining the origin of the one or more circulating cells based on the sequences of the first set of amplicons, wherein the sequences of the second set of amplicons are used as reference, wherein detection of one or more patient-specific mutations from first set of amplicons generated from the circulating cell determined to originate from the tumor is indicative of early relapse or metastasis of the cancer.

In some examples, the present disclosure may provide a compound for use in the treatment of cancer, wherein the compound is known to be effective in treating the cancer having the one or more patient-specific mutations detected from the blood samples.

The improved methods provided herein allows for accurate analysis and characterization of samples containing very few CTCs. The number of CTCs in the sample analyzed by the present methods may be less than 100 cells, less than 75 cells, less than 50 cells, less than 25 cells, less than 20 cells, less than 15 cells, less than 10 cells, less than 5 cells, or a single cell. By using the methods provided herein, accurate genomic information may be obtained from a single circulating tumor cell, 2 CTCs, 3 CTCs, 4 CTCs, 5 CTCs, 6 CTCs, 7 CTCs, 8 CTCs, 9 CTCs, or 10 CTCs.

In some examples, the method comprises monitoring and detecting early relapse or metastasis of a tumor in a cancer patient by determining the identity of a circulating cell suspected to be a circulating tumor cell based on pre-determined patient-specific mutations. In some examples, the patient specific mutations comprise single nucleotide variants (SNV), copy number variants (CNV), indels, deletions, or gene fusions associated with cancer. In some examples, the presence of at least 2 patient-specific mutations associated with cancer is indicative that the one or more circulating cells are tumor cells. In some examples, the presence of at least 8 patient-specific mutations associated with cancer is indicative that the one or more circulating cells are tumor cells. In some examples, the presence at least 16 patient-specific mutations associated with cancer is indicative that the one or more circulating cells are tumor cells. In some examples, the presence of at least 50 patient-specific mutations associated with cancer is indicative that the one or more circulating cells are tumor cells. In some examples, the presence of at least 100 patient-specific mutations associated with cancer is indicative that the one or more circulating cells are tumor cells. In some examples, the presence of at least 100 to about 1000 patient-specific mutations associated with cancer is indicative that the one or more circulating cells are tumor cells. In some examples, the presence of at least 100 to about 1000 patient-specific mutations associated with cancer is indicative that the one or more circulating cells are tumor cells.

In one teaching, multiplex amplification is performed to obtain amplicons that encompasses the patient specific mutations associated with cancer. In some examples, the first set of amplicons and the second set of amplicons are obtained by performing a multiplex amplification reaction of 50 to 1000 patient-specific mutations associated with cancer.

In one teaching, the method for monitoring and detection of early relapse or metastasis of a tumor in a cancer patient may comprise separating multiple circulating cells into individual reaction volumes, isolating cellular DNA in each reaction volume, and attaching a sample barcode to the cellular DNA isolated in each reaction volume. In some examples, the cellular DNA is isolated from a single circulating cell suspected to be a tumor cell.

In some examples, the method further comprises performing non-invasive cancer testing using the genotypes of one or more circulating cells determined to be one or more tumor cells.

In another teaching, a method of analyzing and characterizing circulating cells suspected to be donor cells from a blood sample of a transplant recipient comprises the steps of a) generating a first set of amplicons from cellular DNA isolated from one or more circulating cells suspected to be donor cells obtained from the blood sample and a second set of amplicons from cell-free DNA obtained from a plasma fraction of the blood sample, wherein the first set of amplicons and the second set of amplicons are obtained by performing a multiplex amplification reaction of a plurality of single nucleotide polymorphism (SNP) loci; b) genotyping the first set of amplicons and the second set of amplicons by next-generation sequencing; c) determining that the one or more circulating cells are one or more donor cells, one or more recipient cells, or a mixture of donor cells and recipient cells based on the genotypes of the first set of amplicons and the second set of amplicons.

## BRIEF DESCRIPTION OF THE DRAWINGS

Figure 1 shows the workflow of an example method for obtaining and analyzing circulating cells.

Figure 2 shows the workflow of an example method for confirming the identity of individual circulating cells.

Figure 3 shows the hetrate plot of the genetic data obtained in Example 2. Figure 3A is the hetrate plot of sample 1F containing 1 fetal cell. Figure 3B is the hetrate plot of sample 2F containing 2 fetal cells. Figure 3C is the hetrate plot of sample 4F containing 4 fetal cells. Figure 3D is the hetrate plot of sample 10F containing 10 fetal cells. Figure 3E is the hetrate plot of sample 20F containing 20 fetal cells.

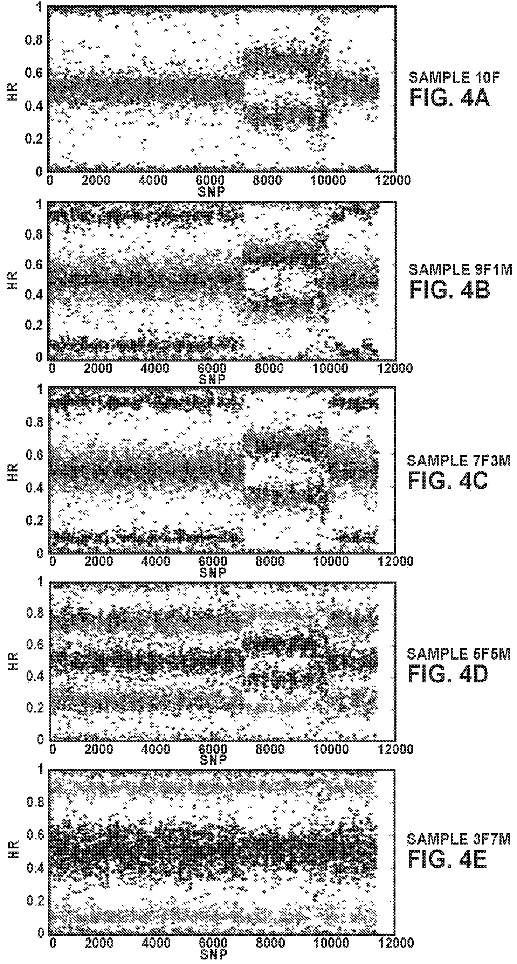

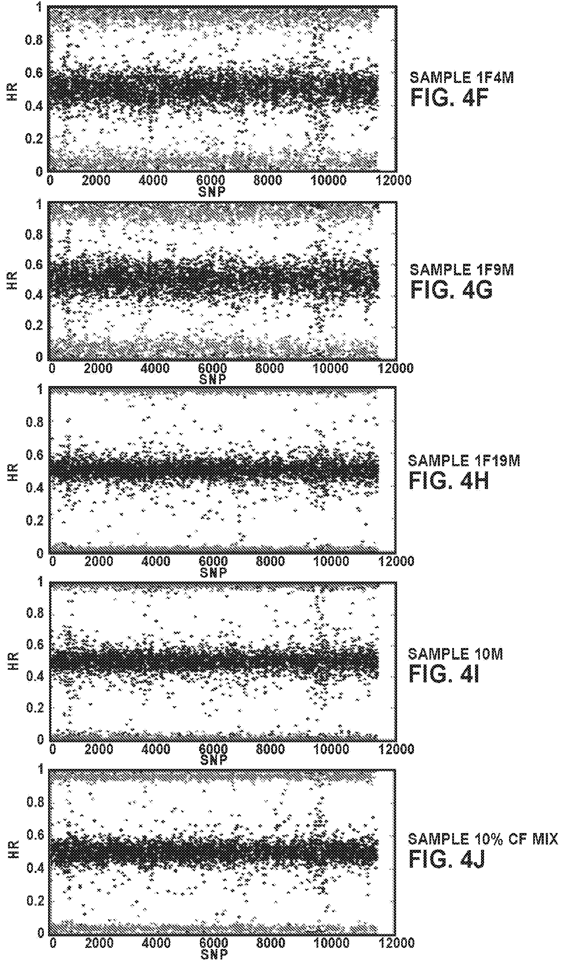

Figure 4 shows the hetrate plot of the genetic data obtained in Example 3. Figure 4A is the hetrate plot of sample 10F containing 10 fetal cells. Figure 4B is the hetrate plot of sample 9F1M containing 9 fetal cells and 1 maternal cell. Figure 4C is the hetrate plot of sample 7F3M containing 7 fetal cells and 3 maternal cells. Figure 4D is the hetrate plot of sample 5F5M containing 5 fetal cells and 5 maternal cells. Figure 4E is the hetrate plot of sample 3F7M containing 3 fetal cells and 7 maternal cells. Figure 4F is the hetrate plot of sample 1F4M containing 1 fetal cell and 4 maternal cells. Figure 4G is the hetrate plot of sample 1F9M containing 1 fetal cell and 9 maternal cells. Figure 4H is the hetrate plot of sample 1F19M containing 1 fetal cell and 19 maternal cells. Figure 4I is the hetrate plot of sample 10M containing 10 maternal cells. Figure 4J is the hetrate plot of sample 10% CF Mix containing 10% of fetal DNA and 90% of maternal DNA.

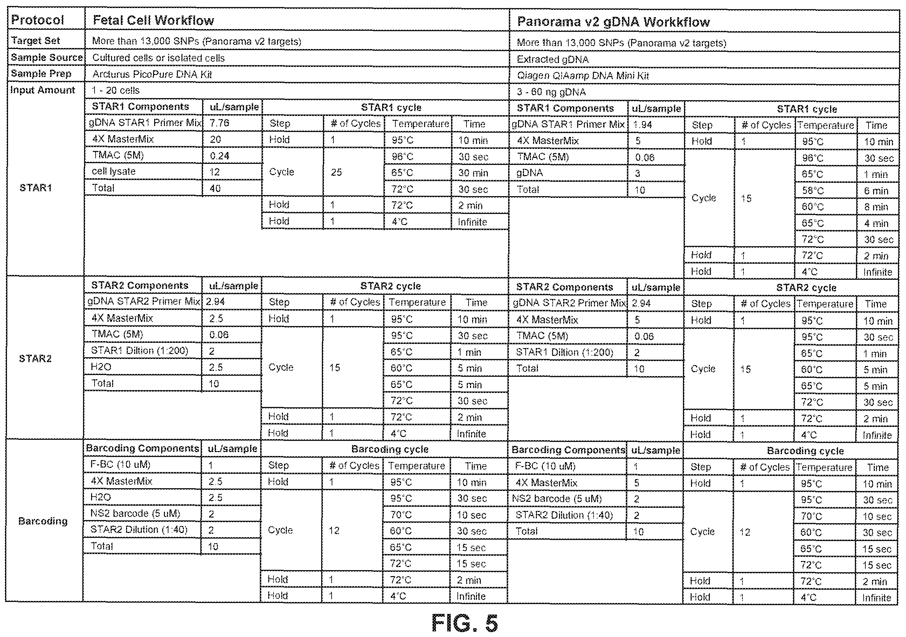

Figure 5 shows exemplary workflow and conditions for the multiplex PCR amplification reactions according to Example 1.

The presently disclosed embodiments will be further explained with reference to the attached drawings, wherein like structures are referred to by like numerals throughout the several views. The drawings shown are not necessarily to scale, with emphasis instead generally being placed upon illustrating the principles of the presently disclosed embodiments.

The above-identified figures are provided by way of representation and not limitation.

Examples which do not fall under the scope of the claims appended hereto are provided as background information for understanding the present disclosure.

## DETAILED DESCRIPTION

### 1. Overview

The present disclosure provides methods and compositions for characterization and genomic analysis of circulating cells. In particular, the present disclosure provides methods for confirmation of the identity of isolated single circulating cells and analyzing genomic data from single cells, which is unprecedented in the literature.

The isolation and genomic analysis of single cells have been gaining more and more interests by clinical researchers and industrial investigators. Single-cell genomics would provide genetic information at the cellular level, and the genotyping profiles and genomic information obtained from isolated single cells have great potential applications in clinical diagnosis, risk prognosis, drug development and disease treatment. However, developing methods for acquiring genomic information from single cells have been hampered by substantial challenges posed by analyzing data from samples containing a small number of cells, let alone single cell samples. The disclosure herein provides both biochemical and analytical methods for accurately acquiring and analyzing genomic information from single cell samples.

In particular, methods for accurately acquiring and analyzing genomic information from single cells would greatly impact diagnostics based on liquid biopsy. Liquid biopsy refers to the sampling of non-solid biological tissue, most commonly of blood, but also saliva, urine, cerebrospinal fluid and other body fluids. For example, liquid biopsies of pregnant mothers may be used to obtain samples containing both cell-free DNA (cfDNA) and circulating fetal cells originating from the fetus that enable noninvasive diagnostics of the fetus. Intact circulating fetal cells (CFCs) can enter maternal blood circulation, and thus, allow analysis of fetal genetic material to provide Non-Invasive Prenatal Genetic Diagnosis (NIPGD or NPD). However, CFCs are highly diluted among billions of maternal blood cells. Another example is the use of liquid biopsy of cancer patients to diagnose or monitor a tumor without performing a biopsy of the tumor itself. Liquid biopsy of cancer patients enables isolation of circulating tumor cells (CTCs) and circulating tumor DNA (ctDNA) to diagnose and monitor the tumor, and test the responsiveness of the tumor to treatments. The CTCs are shed into the bloodstream of the cancer patient from primary and metastatic lesions, but even in patients with advanced metastatic disease, the CTCs are proportionally rare in the context of the patient's blood cells. Thus, accurate diagnostics of tumors based on liquid biopsy relies on being able to accurately isolated and analyze cell sample containing only a few cells or a single cell. In addition, liquid biopsy may also be used to monitor a transplanted organ by isolating and analyzing circulating cells originating from the transplanted organ.

Accordingly, taking full advantage of liquid biopsy based diagnostics relies on the ability of the diagnostic methods to accurately isolate and analyze CRCs such as circulating fetal cells (CFCs) and circulating tumor cells (CTCs). For example, in the context of using CFCs to diagnose the fetus of a pregnant mother, analysis of a single circulating cell from a blood sample of the pregnant mother relies on that the isolated single circulating cell or cells is/are confirmed to originate from the fetus to provide useful information. Similarly, analysis of a single circulating cell from a cancer patient relies on that the isolated single circulating cell or cells is/are confirmed to originate from the tumor to provide useful information. In addition, circulating rare cell (CRC) based diagnostics also depends on the ability to analyze a sample comprising only a few cells or even only a single cell.

The present disclosure meets this need by providing methods that can accurately analyze isolated circulating single cells and characterize them qualitatively as well as quantitatively. The disclosure herein provides a SNP-based next generation sequencing (NGS) method to analyze isolated circulating single cells sample, which can identify the type of the cells, estimate the purity of their DNAs and measure the quality of the sample. For example, the methods of the present disclosure may be used to confirm that an isolated cell suspected to be a fetal cell is a pure isolated single fetal cell, a pure maternal cell, or a mixture of fetal and maternal cells. The methods of the present disclosure may be used to confirm that an isolated cell sample suspected to be a circulating tumor cells is an isolated single circulating tumor cell, or a normal cell from the patient's blood.

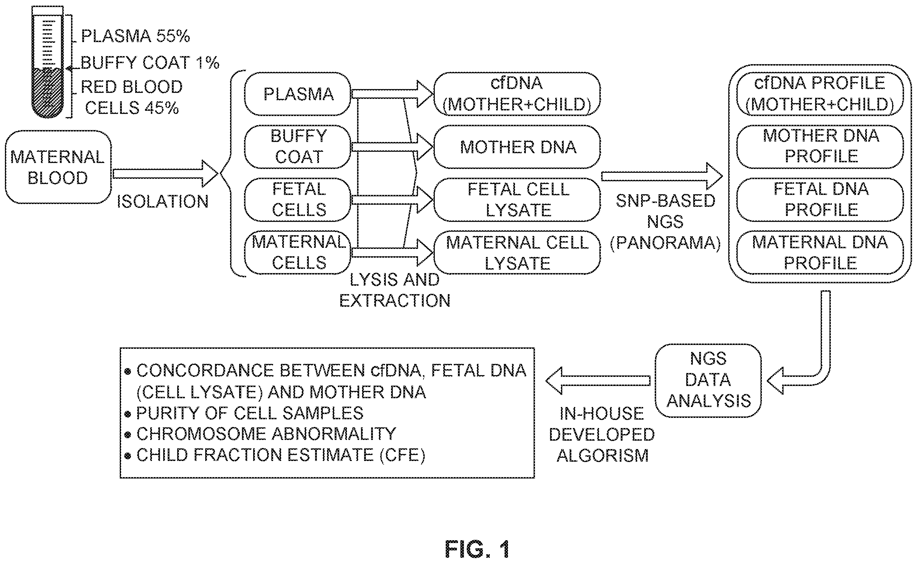

By way of example, Figure 1 provides an outline of the workflow of an illustrative embodiment of the present disclosure for analysis of circulating fetal cells isolated from a maternal blood sample. Accordingly, the workflow outlined in Figure 1 may also be adapted to embody analysis of circulating tumor cells or any other circulating rare cell isolated from a subject. In one illustrative embodiment, the methods of the present disclosure as outlined in Figure 1 and described in further detail below comprises the steps of:

   1. collecting a blood sample from a mother pregnant with a fetus;
   2. isolation of a plasma fraction and/or a buffy coat fraction from the blood sample;
   3. isolation of fetal cells and maternal cells from the blood sample;
   4. extraction of (a) cell-free DNA (cfDNA) from the plasma, and optionally (b) genomic DNA from the buffy coat fraction;
   5. lysis of the isolated (a) fetal cells and (b) maternal cells;
   6. preparation of cfDNA library for sequencing;
   7. perform massively-multiplexed PCR (mmPCR) (a) directly on the cell lysates, (b) on the mother's DNA, and (c) on the cfDNA library;
   8. append DNA barcodes to the amplified DNA samples to label each sample;
   9. preparation of DNA sample pools for sequencing;
   10. run samples through Next Generation Sequencing (NGS) protocols;
   11. perform (a) NGS data pre-processing, and (b) NGS data analysis; and
   12. interpretation of the results for the NGS data processing and analysis.

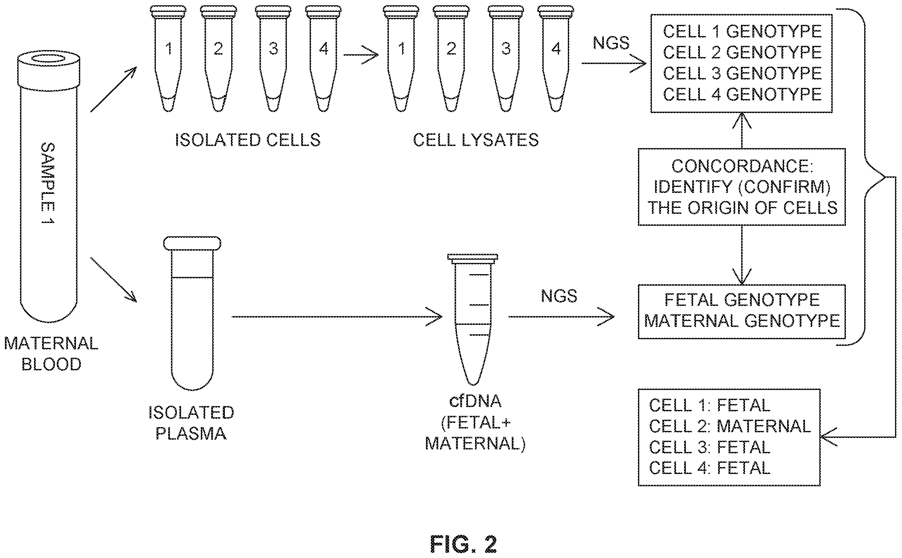

The results of the above outlined method may provide information on the concordance between cfDNA and the fetal DNA (cell lysate) and mother DNA to enable determination of the purity of the cell samples. As illustrated in Figure 2 and the Examples herein, the methods of the present disclosure may be used to confirm that the analyzed DNA sample was derived from a single circulating cell. By way of example, the Examples provided herein showed that by following the workflow outlined in Figures 1 and 2 and using a cfDNA as reference, the method can determine the origin of the cell DNA from an unknown sample and the composition of the sample. For example, in case of fetal/maternal cells isolated from the blood of a pregnant woman, the method could determine if the sample is a pure fetal or pure maternal cell (Figure 2 and the Examples).

Moreover, the data can reveal chromosomal abnormalities in the circulating cells, and if the sample was determined to be a mixture of maternal and fetal cells, the fetal fraction can be determined. In another teaching, the method provided herein may be used to determine the purity of a circulating tumor cell sample, and analysis of the genomic information from the circulating tumor cell. In some examples, the methods provided herein may allow determination that a circulating cell suspected to originate from a transplanted organ is a pure isolated cell originating from the transplanted organ.

## 2. Method of Analyzing Single Cells to Confirm their Identity

The disclosure herein provides a data analysis method that enables analysis of genomic information from a sample with very few cells and even a single cell sample. In particular, the present disclosure provides a method that can determine the identity of an isolated single circulating cell from a blood sample and analyze the genomic information of the single circulating cell. By way of example, as explained further below and in the working examples herein, the present disclosure shows how to confirm the identity of a circulating fetal cell from a blood sample from the pregnant mother, and then analyze the genomic information from the confirmed pure fetal cell sample. The following described method can be adapted for confirming the identity of any cell sample by using corresponding cell-free DNA as a reference.

By way of example and as illustrated by the Examples of the present disclosure, there is provided a method of analyzing and characterizing circulating cells suspected to be of fetal origin from a blood sample of a mother pregnant with a fetus, may comprise a) generating a first set of amplicons from cellular DNA isolated from one or more cell lines or one or more circulating cells suspected to be of fetal origin obtained from the blood sample and a second set of amplicons from cell-free DNA obtained from a plasma fraction of the blood sample or from a DNA mixture obtained from child's and mom's DNA, wherein the first set of amplicons and the second set of amplicons are obtained by performing a multiplex amplification reaction of a plurality of single nucleotide polymorphism (SNP) loci; b) genotyping the first set of amplicons and the second set of amplicons by next-generation sequencing; and c) determining that the one or more circulating cells are one or more maternal cells, one or more fetal cells, or a mixture of maternal cells and fetal cells based on the genotypes of the first set of amplicons and the second set of amplicons. In an example, the methods disclosed herein further comprise generating a third set of amplicons from maternal cellular DNA isolated from one or more maternal cells obtained from a buffy coat fraction of the blood sample or from mom's cell line, and genotyping the third set of amplicons by next-generation sequencing to determine maternal genotype.

In an example, determining that the one or more circulating cells are one or more maternal cells, one or more fetal cells, or a mixture of maternal cells and fetal cells involves calculating a maternal concordance and a fetal concordance between the cell-free DNA obtained from the plasma fraction or DNA mixture of the child's and mom's and the cellular DNA obtained from the circulating cells suspected to be of fetal origin, and a calculation of a fetal mixture fraction.

The first step for calculating all metrics below is to analyze the cfDNA sample allele ratios (AR) and classify them as Maternal Genotype/Fetal Genotype = RR/RR, RR/RM, RM/*, MM/MM, or MM/MR. Allele ratios are calculated by the following manner: Reference counts / Total counts for a given SNP. In some embodiments, the basic genotyper for cfDNA classifies SNPs by AR. The algorithm for this calculation is adjustable according to different applications. In an exemplary but *non-limiting* embodiment, the basic genotyper for cfDNA classifies SNPs by AR in the following manner:

RR/RR: AR $\in$ (0.99,1.0]
RR/RM: AR E (0.8,0.99]
RM/*: AR E [0.2,0.8]
MM/MR: AR E [0.01,0.2)
MM/MM: AR E [0,0.01)

Alternatively, in some examples, the genotype of the cfDNA could be classified by using the PANORAMA® genotyper.

Second, SNPs in the unknown sample are classified as homozygous-R (HoR), homozygous-M (HoM) or heterozygous (Het) based on allele ratios (AR). The algorithm for this calculation is adjustable according to different applications. In an exemplary example, SNPs in the unknown sample are classified as homozygous-R (HoR), homozygous-M (HoM) or heterozygous (Het) allele ratios (AR) in the following manner:

HoR: AR E [0.9,1]
Het: AR E (0.1,0.9)
HoM: AR E [0,0.1]

Third, by using these SNP genotypes/classifications, the following metrics that may be used to classify the unknown sample were computed:

***Maternal concordance*** - fraction of homozygous SNPs (RR/RR or MM/MM) in the cfDNA sample that have allele ratios within the expected ranges (HoR and HoM, respectively) in the unknown sample.

$$Maternal\ Concordance = \frac{N(\ (RR/RR\ \&\ HoR)\ |\ (MM/MM\ \&\ HoM)\ )}{N(RR/RR\ |\ MM/MM)}$$

If maternal concordance is high (> around 85%) then the unknown sample is likely from the same mother as the cfDNA sample.

*Fetal concordance* - fraction of fetal band SNPs (RR/RM or MM/MR) in the cfDNA sample that has allele ratios in the expected range (Het) in the unknown sample.

$$Fetal\ Concordance = \frac{N(\ (RR/RM\ \&\ Het)\ |\ (MM/MR\ \&\ Het)\ )}{N(RR/RM\ |\ MM/MR)}$$

If fetal concordance is high (> around 85%) then the unknown sample likely contains at least some fetal DNA.

*Fetal mixture fraction* - this is an estimation of the contribution from fetal DNA in the unknown sample. It is 1 minus the separation of the fetal RR/RM and MM/MR bands in the unknown sample. If the sample is pure maternal cells, then the separation is close to 1 and the fetal mixture fraction is 0. If the sample is pure fetal cells, then the separation is close to 0 and the fetal fraction is close to 1. Mixtures have something in between these two values.

$$Fetal\ Mixture\ Fraction = 1 - (Mean\ AR(RR/RM\ \&\ Het) - Mean\ AR(MM/MR\ \&\ Het))$$

In one illustrative example, the unknown sample is classified according to Table 1 below by using the calculated maternal concordance, fetal concordance, and fetal mixture fraction. According to the illustrative example shown in Table 1, a pure fetal sample is defined as having a fetal mixture fraction of more than 95% and the fetal and maternal concordance of more than 85%. In some examples, the sample is a pure fetal sample when the fetal mixture fraction is more than 75%, 80%, 85%, 90%, 95%, or 98%, and the maternal and fetal concordance is more than 75%, 80%, 85%, 90%, or 95%. In one particular example, the sample is a pure fetal sample when the fetal mixture fraction is more than 95%, and the fetal concordance is more than 85%. The sample may be a pure maternal sample when the maternal concordance is above 75%, 80%, 85%, 90%, or 95%, and the fetal mixture fraction is less than 15%, 10%, 5%, or 2%. In one particular example, the sample is a pure maternal cell when the maternal concordance is more than 85%, and the fetal mixture fraction is less than 5%. Note that certain combinations are impossible (such as 100% fetal concordance and 0% fetal mixture fraction) or highly unlikely (such as high fetal concordance and low maternal concordance) and as such are excluded from the classification table.

Table 1: Illustrative example of sample classification based on maternal and fetal concordance, and fetal mixture fraction:

| Maternal Concordance | Fetal Concordance | Fetal Mixture Fraction | Sample Classification |
|---|---|---|---|
| < 85% | | | Non-concordant sample |
| > 85% | < 85% | < 5% | Pure maternal sample |
| > 85% | > 85% | 5-95% | Mixture sample |
| > 85% | > 85% | > 95% | Pure fetal sample |

This method was tested in the examples herein on the Pano® V2 target set for chromosomes 13, 18 and 21, is contemplated to be useful for any target set of SNPs (provided sufficient size and minor allele frequency). For example, the SPECTRUM® target set may be a useful genotyper for the methods of the present disclosure.

## 3. Isolation of Circulating Cells

Circulating fetal cells (CFCs), circulating tumor cells (CTCs) and circulating cells from transplanted organ are proportionally rare in liquid biopsy samples. For example, the fetal cell concentration in maternal blood depends on the stage of pregnancy and the condition of the fetus, but estimates range from one to forty fetal cells in every milliliter of maternal blood, or less than one fetal cell per 100,000 maternal nucleated cells. Current techniques are able to isolate small quantities of fetal cells from the mother's blood, although it is difficult to enrich the fetal cells to purity in any quantity.

Numerous approaches to enrich for circulating rare cells have been developed based on properties that distinguish them from the surrounding normal cells in the context of circulating tumor cells or maternal cells in the context of circulating fetal cells.

These distinguishing properties may be physical properties such as size, density, electric charges, and deformability, which would allow enrichment based on using methods such as filtration through special filters, microscopy in combination with micropipetting, microfluidics systems, centrifugation, dielectrophoresis, or flow cytometry.

Alternatively, the distinguishing properties may be biological properties such as cell surface protein expression or viability, which would allow labeling of the circulating cells to distinguish them from normal or maternal cells. For example, antibodies may be used to detect specific cell surface marker expressed by the circulating cells and not the surrounding cells, and then the circulating cells may be isolated by using flow cytometry, magnetic beads, plating on solid support coated with agents binding antibodies to isolated the cells with bound antibodies. For example, circulating tumor cells have been isolated by using antibodies against the epithelial cell adhesion molecule (EpCAM). *See* Alix-Panabieres and Pantel, Clin. Chem., 59(1): 110-118 (2013). Alternatively, the circulating cells may be enriched by negative selection by using an antibody recognizing an antigen on the normal cells and not on the circulating cells, and subsequently removing the antibody labeled cells. Another negative selection method is the selective lysis of erythrocytes.

## 4. Patient Populations

As used herein "subject," "patient," or "individual" refers to any subject, patient, or individual, and the terms are used interchangeably herein. In this regard, the terms "subject," "patient," and "individual" includes mammals, and, in particular humans. When used in conjunction with "in need thereof," the term "subject," "patient," or "individual" intends any subject, patient, or individual having or at risk for a specified symptom or disorder.

The disclosed methods and compositions can be used to diagnose, monitor or treat a range of subjects, including human and non-human animals, including mammals, as well as immature and mature animals, including human children and adults suffering from a range of symptoms or disorders. The human subject to be diagnosed or treated can be an embryo, a fetus, an infant, toddler, school-aged child, teenager, young adult, adult, or elderly patient.

By way of example, the subject to be diagnosed or monitored is a fetus by isolating and analyzing circulating fetal cells obtained from a blood sample of the mother. In some examples, the methods disclosed herein may be used for paternity tests of a fetus. In some examples, the methods disclosed herein may be used to determine copy number variations at a chromosome or chromosome segment of interest in the fetus of a pregnant mother. In some examples, the methods disclosed herein may be used to determine copy number variations at chromsomes 13, 18, 21, the sex chromosomes and/or chromosome segments thereof. In some examples, the methods disclosed herein may be used to detect a microdeletion in the chromosome of the fetus. In some examples, the microdeletion detected in the fetus is selected from the 22q11.2 deletion associated with DiGeorge syndrome, the microdeletion associated with Prader-Willi syndrome, the microdeletion associated with Angelman syndrom, the 1p36 deletion, and the microdeletion associated with the Cri-du-chat syndrome. In some examples, the methods disclosed herein may be used to detect single nucleotide variations in the fetus of a pregnant mother.

In some examples, the methods disclosed herein may be used to diagnose or monitor a cancer patient. In some examples, the methods disclosed herein can be used to detect copy number variation in the tumor tissue by isolating and detecting circulating tumor cells from a blood sample of the patient. In some examples, the methods herein may be used to detect one or more patient-specific mutations associated with a cancer in the cancer patient. In some examples, the methods disclosed herein may be used to detect at least 2 patient-specific mutations associated with a cancer in the cancer patient's tumor. In some examples, the methods disclosed herein may be used to detect mutations associated with early relapse or metastasis of the cancer patient's tumor. In some examples, the methods disclosed herein may be used to monitor the cancer patient's response to treatment.

By way of example, the methods disclosed herein may be used to monitor a recipient of a transplanted organ by isolating and analyzing circulating cells originating from the transplanted organ. In some examples, the methods disclosed herein may be used to diagnose graft-host disease in a recipient of a transplanted organ. In some embodiments, the methods disclosed herein may be used to monitor an organ recipient's response to immune suppressive treatment.

### 5. Obtaining Genotype Data

#### 5.1 Sources of genetic data

Any relevant individual's genetic data can be obtained from the following: the individual's bulk diploid tissue, one or more diploid cells from the individual, one or more haploid cells from the individual, one or more blastomers from the target individual, extra-cellular genetic material found on the individual, extra-cellular genetic material from the individual found in maternal blood or the blood of a cancer patient, cells from the individual found in maternal blood, one or more embryos created from (a) gamete(s) from the related individual, one or more blastomers taken from such an embryo, extra-cellular genetic material found on the related individual, genetic material known to have originated from the related individual, and combinations thereof. In illustrative examples, methods provided herein are used to analyze free DNA originating from the genome of a target sample from a target cell, such as fetal cell or tumor cell. By way of example, the methods disclosed herein may further comprise obtaining the circulating cells suspected to be of fetal origin, the plasma faction comprising the cell-free DNA, and the buffy coat fraction comprising the maternal cells, from the blood sample. In an example, the methods disclosed herein further comprise obtaining the circulating cells suspected to be of tumor cells, the plasm faction comprising the cell-free DNA, and the buffy coat fraction comprising the normal cells, from the blood sample.

The term "cell-free DNA" as used herein refers to DNA that is available for analysis without requiring the step of lysing cells. Cell-free DNA can be found in blood or other bodily fluids. Cell-free DNA may be obtained from a variety of tissues. Such tissues may be tissues that are in liquid form such as blood, lymph, ascites fluid, cerebral spinal fluid, and the like. Cell-free DNA may be from a variety of cellular sources. In some cases, the cell-free DNA will be comprised of DNA derived from fetal cells. The cell-free DNA may be a mixture of DNA derived from target cells and non-target cells. In the case of the analysis of DNA for fetal aneuploidies, in some examplesthe cell-free DNA may be obtained from the blood of the pregnant woman, wherein the cell-free DNA comprises a mixture of maternally derived cell-free DNA and fetally derived cell-free DNA. In other examples, the cell-free DNA may be derived from a cancerous tumor cell. The cell-free DNA may comprise a mixture of cell-free DNA derived from the tumor cell and cell-free DNA derived from non-tumor cells elsewhere in the body.

A sample analyzed in methods of the present disclosure, in certain illustrative examples, is a blood sample, or a fraction thereof. Methods provided herein, in certain examples, are specially adapted for amplifying DNA fragments, especially tumor DNA fragments that are found in circulating tumor DNA (ctDNA). Such fragments are typically about 160 nucleotides in length.

It is known in the art that cell-free nucleic acid (cfNA), e.g. cfDNA, can be released into the circulation via various forms of cell death such as apoptosis, necrosis, autophagy and necroptosis. The cfDNA, is fragmented and the size distribution of the fragments varies from 150-350 bp to > 10000 bp. (see Kalnina et al. World J Gastroenterol. 2015 Nov 7; 21(41): 11636-11653). For example, the size distributions of plasma DNA fragments in hepatocellular carcinoma (HCC) patients spanned a range of 100-220 bp in length with a peak in count frequency at about 166bp and the highest tumor DNA concentration in fragments of 150-180 bp in length (see: Jiang et al. Proc Natl Acad Sci USA 112: E1317-E1325).

In an illustrative example the circulating tumor DNA (ctDNA) is isolated from blood using EDTA-2Na tube after removal of cellular debris and platelets by centrifugation. The plasma samples can be stored at -80oC until the DNA is extracted using, for example, QIAamp DNA Mini Kit (Qiagen, Hilden, Germany), (e.g. Hamakawa et al., Br J Cancer. 2015; 112:352-356). Hamakava et al. reported median concentration of extracted cell free DNA of all samples 43.1 ng per ml plasma (range 9.5-1338 ng ml/) and a mutant fraction range of 0.001-77.8%, with a median of 0.90%.

Genetic data, e.g., DNA sequence data, can be obtained from a mixture of DNA comprising DNA derived from one or more target cells and DNA derived from one or more non-target cells. The target cells and non-target cells differ with respect to one another at the genomic level as by virtue of other criteria. The term "derived" is used to indicate that the cells are the ultimate source of the DNA. Thus, for example, cell-free DNA obtained from maternal blood of pregnant woman is derived from cells from the placenta of the fetus, which are typically genetically identical to the fetus itself, and the mother's cells. The method employs a set of patients.

*5.2 Amplification (e.g. PCR) Reaction Mixtures:*

In some examples, obtaining genotype data involves sequencing (such as shotgun sequencing or single molecule sequencing or other next generation sequencing techniques), a SNP array to detect polymorphic loci, or multiplex PCR. In some examples, the genotyping involves use of a SNP array to detect polymorphic loci, such as at least 100; 200; 500; 750; 1,000; 2,000; 5,000; 7,500; 10,000; 20,000; 25,000; 30,000; 40,000; 50,000; 75,000; or 100,000 different polymorphic loci. In some examples, the genotyping involves the use of multiplex PCR. In some examples, the method involves contacting the sample in a fraction with a library of primers that simultaneously hybridize to at least 100; 200; 500; 750; 1,000; 2,000; 5,000; 7,500; 10,000; 13,000, 15,000; 20,000; 25,000; 30,000; 40,000; 50,000; 75,000; or 100,000 different polymorphic loci (such as SNPs) to produce a reaction mixture; and subjecting the reaction mixture to primer extension reaction conditions to produce amplified products that are measured with a high throughput sequencer to produce sequencing data. In some examples, RNA (such as mRNA) is sequenced. Since mRNA contains only exons, sequencing mRNA allows alleles to be determined for polymorphic loci (such as SNPs) over a large distance in the genome, such as a few megabases.

In one example, the multiplexed targeted PCR is performed directly on the lysed cell sample. In some examples, the cells are lysed by Proteinase K digestion. In some examples, the cells are lysed by Proteinase K digestion in a saline solution comprising potassium chloride (KCl), magnesium chloride ($MgCl_2$), and Tris-hydrogenchloride. In some examples, to prepare the cells for PCR amplification, the cells are lysed by Proteinase K digestion in a saline solution without potassium chloride (KCl), magnesium chloride ($MgCl_2$), and Tris-hydrogenchloride. Further details can be found in the Working Example section.

In some examples, a sample barcode is attached to the DNA during the generating amplicons step.

Methods of the present disclosure, in certain examples, include forming an amplification reaction mixture. The reaction mixture typically is formed by combining a polymerase, nucleotide triphosphates, nucleic acid fragments from a nucleic acid library generated from the sample, a series of forward target-specific outer primers and a first strand reverse outer universal primer. Another illustrative example is a reaction mixture that includes forward target-specific inner primers instead of the forward target-specific outer primers and amplicons from a first PCR reaction using the outer primers, instead of nucleic acid fragments from the nucleic acid library. The reaction mixtures provided herein, themselves forming in illustrative examples, a separate teaching of the present disclosure. In illustrative examples, the reaction mixtures are PCR reaction mixtures. PCR reaction mixtures typically include magnesium.

In some examples, the reaction mixture includes ethylenediaminetetraacetic acid (EDTA), magnesium, tetramethyl ammonium chloride (TMAC), or any combination thereof. In some examples, the concentration of TMAC is between 20 and 70 mM, inclusive. While not meant to be bound to any particular theory, it is believed that TMAC binds to DNA, stabilizes duplexes, increases primer specificity, and/or equalizes the melting temperatures of different primers. In some examples, TMAC increases the uniformity in the amount of amplified products for the different targets. In some examples, the concentration of magnesium (such as magnesium from magnesium chloride) is between 1 and 8 mM.

The large number of primers used for multiplex PCR of a large number of targets may chelate a lot of the magnesium (2 phosphates in the primers chelate 1 magnesium). For example, if enough primers are used such that the concentration of phosphate from the primers is ~9 mM, then the primers may reduce the effective magnesium concentration by ~4.5 mM. In some examples, EDTA is used to decrease the amount of magnesium available as a cofactor for the polymerase since high concentrations of magnesium can result in PCR errors, such as amplification of non-target loci. In some examples, the concentration of EDTA reduces the amount of available magnesium to between 1 and 5 mM (such as between 3 and 5 mM).

In some examples, the pH is between 7.5 and 8.5, such as between 7.5 and 8, 8 and 8.3, or 8.3 and 8.5, inclusive. In some examples, Tris is used at, for example, a concentration of between 10 and 100 mM, such as between 10 and 25 mM, 25 and 50 mM, 50 and 75 mM, or 25 and 75 mM, inclusive. In some examples, any of these concentrations of Tris are used at a pH between 7.5 and 8.5. In some examples, a combination of KCl and (NH4)2SO4 is used, such as between 50 and 150 mM KCl and between 10 and 90 mM (NH4)2SO4, inclusive. In some examples, the concentration of KCl is between 0 and 30 mM, between 50 and 100 mM, or between 100 and 150 mM, inclusive. In some examples, the concentration of (NH4)2SO4 is between 10 and 50 mM, 50 and 90 mM, 10 and 20 mM, 20 and 40 mM, 40 and 60 mM, or 60 and 80 mM (NH4)2SO4, inclusive. In some examples, the ammonium [NH4+] concentration is between 0 and 160 mM, such as between 0 to 50, 50 to 100, or 100 to 160 mM, inclusive. In some examples, the sum of the potassium and ammonium concentration ([K+] + [NH4+]) is between 0 and 160 mM, such as between 0 to 25, 25 to 50, 50 to 150, 50 to 75, 75 to 100, 100 to 125, or 125 to 160 mM, inclusive. An exemplary buffer with [K+] + [NH4+] = 120 mM is 20 mM KCl and 50 mM (NH4)2SO4. In some examples, the buffer includes 25 to 75 mM Tris, pH 7.2 to 8, 0 to 50 mM KCl, 10 to 80 mM ammonium sulfate, and 3 to 6 mM magnesium, inclusive. In some examples, the buffer includes 25 to 75 mM Tris pH 7 to 8.5, 3 to 6 mM MgCl2, 10 to 50 mM KCl, and 20 to 80 mM (NH4)2SO4, inclusive. In some examples, 100 to 200 Units/mL of polymerase are used. In some examples, 100 mM KCl, 50 mM (NH4)2SO4, 3 mM MgCl2, 7.5 nM of each primer in the library, 50 mM TMAC, and 7 μl DNA template in a 20 μl final volume at pH 8.1 is used.

In some examples, a crowding agent is used, such as polyethylene glycol (PEG, such as PEG 8,000) or glycerol. In some examples, the amount of PEG (such as PEG 8,000) is between 0.1 to 20%, such as between 0.5 to 15%, 1 to 10%, 2 to 8%, or 4 to 8%, inclusive. In some examples, the amount of glycerol is between 0.1 to 20%, such as between 0.5 to 15%, 1 to 10%, 2 to 8%, or 4 to 8%, inclusive. In some examples, a crowding agent allows either a low polymerase concentration and/or a shorter annealing time to be used. In some examples, a crowding agent improves the uniformity of the DOR and/or reduces dropouts (undetected alleles). In some examples, a polymerase with proof-reading activity, a polymerase without (or with negligible) proof-reading activity, or a mixture of a polymerase with proof-reading activity and a polymerase without (or with negligible) proof-reading activity is used. In some examples, a hot start polymerase, a non-hot start polymerase, or a mixture of a hot start polymerase and a non-hot start polymerase is used. In some examples, a HotStarTaq DNA polymerase is used (see, for example, QIAGEN catalog No. 203203). In some examples, AmpliTaq Gold® DNA Polymerase is used. In some examples a PrimeSTAR GXL DNA polymerase, a high fidelity polymerase that provides efficient PCR amplification when there is excess template in the reaction mixture, and when amplifying long products, is used (Takara Clontech, Mountain View, CA). In some examples, KAPA Taq DNA Polymerase or KAPA Taq HotStart DNA Polymerase is used; they are based on the single-subunit, wild-type Taq DNA polymerase of the thermophilic bacterium Thermus aquaticus. KAPA Taq and KAPA Taq HotStart DNA Polymerase have 5'-3' polymerase and 5'-3' exonuclease activities, but no 3' to 5' exonuclease (proofreading) activity (see, for example, KAPA BIOSYSTEMS catalog No. BK1000). In some examples, Pfu DNA polymerase is used; it is a highly thermostable DNA polymerase from the hyperthermophilic archaeum Pyrococcus furiosus. The enzyme catalyzes the template-dependent polymerization of nucleotides into duplex DNA in the 5'→3' direction. Pfu DNA Polymerase also exhibits 3'→5' exonuclease (proofreading) activity that enables the polymerase to correct nucleotide incorporation errors. It has no 5'→3' exonuclease activity (see, for example, Thermo Scientific Catalog No. EP0501). In some examples, Klentaq1 is used; it is a Klenow-fragment analog of Taq DNA polymerase, it has no exonuclease or endonuclease activity (see, for example, DNA POLYMERASE TECHNOLOGY, Inc., St. Louis, Missouri, catalog No. 100). In some examples, the polymerase is a PHUSION DNA polymerase, such as PHUSION High Fidelity DNA polymerase (M0530S, New England BioLabs, Inc.) or PHUSION Hot Start Flex DNA polymerase (M0535S, New England BioLabs, Inc.). In some examples, the polymerase is a Q5® DNA Polymerase, such as Q5® High-Fidelity DNA Polymerase (M0491S, New England BioLabs, Inc.) or Q5® Hot Start High-Fidelity DNA Polymerase (M0493S, New England BioLabs, Inc.). In some examples, the polymerase is a T4 DNA polymerase (M0203S, New England BioLabs, Inc.).

In some examples, between 5 and 600 Units/mL (Units per 1 mL of reaction volume) of polymerase is used, such as between 5 to 100, 100 to 200, 200 to 300, 300 to 400, 400 to 500, or 500 to 600 Units/mL, inclusive.

*5.3 PCR Methods*

In some examples, hot-start PCR is used to reduce or prevent polymerization prior to PCR thermocycling. Exemplary hot-start PCR methods include initial inhibition of the DNA polymerase, or physical separation of reaction components reaction until the reaction mixture reaches the higher temperatures. In some examples, slow release of magnesium is used. DNA polymerase requires magnesium ions for activity, so the magnesium is chemically separated from the reaction by binding to a chemical compound, and is released into the solution only at high temperature. In some examples, non-covalent binding of an inhibitor is used. In this method a peptide, antibody, or aptamer are non-covalently bound to the enzyme at low temperature and inhibit its activity. After incubation at elevated temperature, the inhibitor is released and the reaction starts. In some examples, a cold-sensitive Taq polymerase is used, such as a modified DNA polymerase with almost no activity at low temperature. In some examples, chemical modification is used. In this method, a molecule is covalently bound to the side chain of an amino acid in the active site of the DNA polymerase. The molecule is released from the enzyme by incubation of the reaction mixture at elevated temperature. Once the molecule is released, the enzyme is activated.

In some examples, the amount to template nucleic acids (such as an RNA or DNA sample) is between 20 and 5,000 ng, such as between 20 to 200, 200 to 400, 400 to 600, 600 to 1,000; 1,000 to 1,500; or 2,000 to 3,000 ng, inclusive.

In some examples, a QIAGEN Multiplex PCR Kit is used (QIAGEN catalog No. 206143). For 100 x 50 μl multiplex PCR reactions, the kit includes 2x QIAGEN Multiplex PCR Master Mix (providing a final concentration of 3 mM MgCl2, 3 x 0.85 ml), 5x Q-Solution (1 x 2.0 ml), and RNase-Free Water (2 x 1.7 ml). The QIAGEN Multiplex PCR Master Mix (MM) contains a combination of KCl and (NH4)2SO4 as well as the PCR additive, Factor MP, which increases the local concentration of primers at the template. Factor MP stabilizes specifically bound primers, allowing efficient primer extension by HotStarTaq DNA Polymerase. HotStarTaq DNA Polymerase is a modified form of Taq DNA polymerase and has no polymerase activity at ambient temperatures. In some examples, HotStarTaq DNA Polymerase is activated by a 15-minute incubation at 95°C which can be incorporated into any existing thermal-cycler program.

In some examples, 1x QIAGEN MM final concentration (the recommended concentration), 7.5 nM of each primer in the library, 50 mM TMAC, and 7 μl DNA template in a 20 μl final volume is used. In some examples, the PCR thermocycling conditions include 95°C for 10 minutes (hot start); 20 cycles of 96°C for 30 seconds; 65°C for 15 minutes; and 72°C for 30 seconds; followed by 72°C for 2 minutes (final extension); and then a 4°C hold.

In some examples, 2x QIAGEN MM final concentration (twice the recommended concentration), 2 nM of each primer in the library, 70 mM TMAC, and 7 μl DNA template in a 20 μl total volume is used. In some examples, up to 4 mM EDTA is also included. In some examples, the PCR thermocycling conditions include 95°C for 10 minutes (hot start); 25 cycles of 96°C for 30 seconds; 65°C for 20, 25, 30, 45, 60, 120, or 180 minutes; and optionally 72°C for 30 seconds); followed by 72°C for 2 minutes (final extension); and then a 4°C hold.

Another exemplary set of conditions includes a semi-nested PCR approach. The first PCR reaction uses 20 μl a reaction volume with 2x QIAGEN MM final concentration, 1.875 nM of each primer in the library (outer forward and reverse primers), and DNA template.

Thermocycling parameters include 95°C for 10 minutes; 25 cycles of 96°C for 30 seconds, 65°C for 1 minute, 58°C for 6 minutes, 60°C for 8 minutes, 65°C for 4 minutes, and 72°C for 30 seconds; and then 72°C for 2 minutes, and then a 4°C hold. Next, 2 μl of the resulting product, diluted 1:200, is used as input in a second PCR reaction. This reaction uses a 10 μl reaction volume with 1x QIAGEN MM final concentration, 20 nM of each inner forward primer, and 1 μM of reverse primer tag. Thermocycling parameters include 95°C for 10 minutes; 15 cycles of 95°C for 30 seconds, 65°C for 1 minute, 60°C for 5 minutes, 65°C for 5 minutes, and 72°C for 30 seconds; and then 72°C for 2 minutes, and then a 4°C hold. The annealing temperature can optionally be higher than the melting temperatures of some or all of the primers, as discussed herein (see U.S. Patent Application No. 14/918,544, filed Oct. 20, 2015).

The melting temperature (Tm) is the temperature at which one-half (50%) of a DNA duplex of an oligonucleotide (such as a primer) and its perfect complement dissociates and becomes single strand DNA. The annealing temperature (TA) is the temperature one runs the PCR protocol at. For prior methods, it is usually 5°C below the lowest Tm of the primers used, thus close to all possible duplexes are formed (such that essentially all the primer molecules bind the template nucleic acid). While this is highly efficient, at lower temperatures there are more unspecific reactions bound to occur. One consequence of having too low a TA is that primers may anneal to sequences other than the true target, as internal single-base mismatches or partial annealing may be tolerated. In some examples of the present disclosure, the TA is higher than Tm, wherein only a small fraction of the targets has a primer annealed (such as only ~1-5%) at a given moment. If these get extended, they are removed from the equilibrium of annealing and dissociating primers and target (as extension increases Tm quickly to above 70°C), and a new ~1-5% of targets has primers. Thus, by giving the reaction a long time for annealing, one can get ~100% of the targets copied per cycle.

In various examples, the annealing temperature is between 1, 2, 3, 4, 5, 6, 7, 8, 9, 10, 11, 12, 13 °C and 2, 3, 4, 5, 6, 7, 8, 9, 10, 11, 12, 13, or 15 °C on the high end of the range, greater than the melting temperature (such as the empirically measured or calculated Tm) of at least 25, 50, 60, 70, 75, 80, 90, 95, or 100% of the non-identical primers. In various examples, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the melting temperature (such as the empirically measured or calculated Tm) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers. In various examples, the annealing temperature is between 1 and 15 °C (such as between 1 to 10, 1 to 5, 1 to 3, 3 to 5, 3 to 8, 5 to 10, 5 to 8, 8 to 10, 10 to 12, or 12 to 15 °C, inclusive) greater than the melting temperature (such as the empirically measured or calculated Tm) of at least 25%, 50%, 60%, 70%, 75%, 80%, 90%, 95%, or all of the non-identical primers, and the length of the annealing step (per PCR cycle) is between 5 and 180 minutes, such as 15 and 120 minutes, 15 and 60 minutes, 15 and 45 minutes, or 20 and 60 minutes, inclusive.

Exemplary Multiplex PCR Methods

In various examples, long annealing times (as discussed herein and exemplified in Example 12) and/or low primer concentrations are used. In fact, in certain examples, limiting primer concentrations and/or conditions are used. In various examples, the length of the annealing step is between 15, 20, 25, 30, 35, 40, 45, or 60 minutes on the low end of the range and 20, 25, 30, 35, 40, 45, 60, 120, or 180 minutes on the high end of the range. In various examples, the length of the annealing step (per PCR cycle) is between 30 and 180 minutes. For example, the annealing step can be between 30 and 60 minutes and the concentration of each primer can be less than 20, 15, 10, or 5 nM. In other examples the primer concentration is 1, 2, 3, 4, 5, 6, 7, 8, 9, 10, 15, 20, or 25 nM on the low end of the range, and 2, 3, 4, 5, 6, 7, 8, 9, 10, 15, 20, 25, and 50 on the high end of the range.

At high level of multiplexing, the solution may become viscous due to the large amount of primers in solution. If the solution is too viscous, one can reduce the primer concentration to an amount that is still sufficient for the primers to bind the template DNA. In various examples, between 1,000 and 100,000 different primers are used and the concentration of each primer is less than 20 nM, such as less than 10 nM or between 1 and 10 nM, inclusive.

*5.4 Exemplary Multiplex PCR Methods*

In one teaching, the present disclosure features methods of amplifying target loci in a nucleic acid sample that involve (i) contacting the nucleic acid sample with a library of primers that simultaneously hybridize to least 1,000; 2,000; 5,000; 7,500; 10,000; 15,000; 19,000; 20,000; 25,000; 27,000; 28,000; 30,000; 40,000; 50,000; 75,000; or 100,000 different target loci to produce a reaction mixture; and (ii) subjecting the reaction mixture to primer extension reaction conditions (such as PCR conditions) to produce amplified products that include target amplicons. In some examples, the method also includes determining the presence or absence of at least one target amplicon (such as at least 50, 60, 70, 80, 90, 95, 96, 97, 98, 99, or 99.5% of the target amplicons). In some examples, the method also includes determining the sequence of at least one target amplicon (such as at least 50, 60, 70, 80, 90, 95, 96, 97, 98, 99, or 99.5% of the target amplicons). In some examples, at least 50, 60, 70, 80, 90, 95, 96, 97, 98, 99, or 99.5% of the target loci are amplified. In some examples, at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; or 100,000 different target loci are amplified at least 5, 10, 20, 40, 50, 60, 80, 100, 120, 150, 200, 300, or 400-fold. In some examples, at least 50, 60, 70, 80, 90, 95, 96, 97, 98, 99, 99.5, or 100% of the target loci are amplified at least 5, 10, 20, 40, 50, 60, 80, 100, 120, 150, 200, 300, or 400-fold. In various examples, less than 60, 50, 40, 30, 20, 10, 5, 4, 3, 2, 1, 0.5, 0.25, 0.1, or 0.05% of the amplified products are primer dimers. In some examples, the method involves multiplex PCR and sequencing (such as high throughput sequencing).

In various examples, long annealing times and/or low primer concentrations are used. In various examples, the length of the annealing step is greater than 3, 5, 8, 10, 15, 20, 30, 45, 60, 75, 90, 120, 150, or 180 minutes. In various examples, the length of the annealing step (per PCR cycle) is between 5 and 180 minutes, such as 5 to 60, 10 to 60, 5 to 30, or 10 to 30 minutes, inclusive. In various examples, the length of the annealing step is greater than 5 minutes (such greater than 10, or 15 minutes), and the concentration of each primer is less than 20 nM. In various examples, the length of the annealing step is greater than 5 minutes (such greater than 10, or 15 minutes), and the concentration of each primer is between 1 to 20 nM, or 1 to 10 nM, inclusive. In various examples, the length of the annealing step is greater than 20 minutes (such as greater than 30, 45, 60, or 90 minutes), and the concentration of each primer is less than 1 nM.

At high level of multiplexing, the solution may become viscous due to the large amount of primers in solution. If the solution is too viscous, one can reduce the primer concentration to an amount that is still sufficient for the primers to bind the template DNA. In various examples, less than 60,000 different primers are used and the concentration of each primer is less than 20 nM, such as less than 10 nM or between 1 and 10 nM, inclusive. In various examples, more than 60,000 different primers (such as between 60,000 and 120,000 different primers) are used and the concentration of each primer is less than 10 nM, such as less than 5 nM or between 1 and 10 nM, inclusive.

It was discovered that the annealing temperature can optionally be higher than the melting temperatures of some or all of the primers (in contrast to other methods that use an annealing temperature below the melting temperatures of the primers). The melting temperature ($T_m$) is the temperature at which one-half (50%) of a DNA duplex of an oligonucleotide (such as a primer) and its perfect complement dissociates and becomes single strand DNA. The annealing temperature ($T_A$) is the temperature one runs the PCR protocol at. For prior methods, it is usually 5 C below the lowest $T_m$ of the primers used, thus close to all possible duplexes are formed (such that essentially all the primer molecules bind the template nucleic acid). While this is highly efficient, at lower temperatures there are more unspecific reactions bound to occur. One consequence of having too low a $T_A$ is that primers may anneal to sequences other than the true target, as internal single-base mismatches or partial annealing may be tolerated. In some examples of the present disclosure, the $T_A$ is higher than ($T_m$), where at a given moment only a small fraction of the targets have a primer annealed (such as only ~1-5%). If these get extended, they are removed from the equilibrium of annealing and dissociating primers and target (as extension increases $T_m$ quickly to above 70 C), and a new ~1-5% of targets has primers. Thus, by giving the reaction long time for annealing, one can get ~100% of the targets copied per cycle. Thus, the most stable molecule pairs (those with perfect DNA pairing between the primer and the template DNA) are preferentially extended to produce the correct target amplicons. For example, the same experiment was performed with 57°C as the annealing temperature and with 63°C as the annealing temperature with primers that had a melting temperature below 63°C. When the annealing temperature was 57°C, the percent of mapped reads for the amplified PCR products was as low as 50% (with ~ 50% of the amplified products being primer-dimer). When the annealing temperature was 63°C, the percentage of amplified products that were primer dimer dropped to ~2%.

In various examples, the annealing temperature is at least 1, 2, 3, 4, 5, 6, 7, 8, 9, 10, 11, 12, 13, or 15 °C greater than the melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers. In some examples, the annealing temperature is at least 1, 2, 3, 4, 5, 6, 7, 8, 9, 10, 11, 12, 13, or 15 °C greater than the melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers, and the length of the annealing step (per PCR cycle) is greater than 1, 3, 5, 8, 10, 15, 20, 30, 45, 60, 75, 90, 120, 150, or 180 minutes.

In various examples, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers. In various examples, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers, and the length of the annealing step (per PCR cycle) is between 5 and 180 minutes, such as 5 to 60, 10 to 60, 5 to 30, or 10 to 30 minutes, inclusive.

In some examples, the annealing temperature is at least 1, 2, 3, 4, 5, 6, 7, 8, 9, 10, 11, 12, 13, or 15 °C greater than the highest melting temperature (such as the empirically measured or calculated $T_m$) of the primers. In some examples, the annealing temperature is at least 1, 2, 3, 4, 5, 6, 7, 8, 9, 10, 11, 12, 13, or 15 °C greater than the highest melting temperature (such as the empirically measured or calculated $T_m$) of the primers, and the length of the annealing step (per PCR cycle) is greater than 1, 3, 5, 8, 10, 15, 20, 30, 45, 60, 75, 90, 120, 150, or 180 minutes

In some examples, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the highest melting temperature (such as the empirically measured or calculated $T_m$) of the primers. In some examples, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the highest melting temperature (such as the empirically measured or calculated $T_m$) of the primers, and the length of the annealing step (per PCR cycle) is between 5 and 180 minutes, such as 5 to 60, 10 to 60, 5 to 30, or 10 to 30 minutes, inclusive.

In some examples, the annealing temperature is at least 1, 2, 3, 4, 5, 6, 7, 8, 9, 10, 11, 12, 13, or 15 °C greater than the average melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers. In some examples, the annealing temperature is at least 1, 2, 3, 4, 5, 6, 7, 8, 9, 10, 11, 12, 13, or 15 °C greater than the average melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers, and the length of the annealing step (per PCR cycle) is greater than 1, 3, 5, 8, 10, 15, 20, 30, 45, 60, 75, 90, 120, 150, or 180 minutes.

In some examples, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the average melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers. In some examples, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the average melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers, and the length of the annealing step (per PCR cycle) is between 5 and 180 minutes, such as 5 to 60, 10 to 60, 5 to 30, or 10 to 30 minutes, inclusive.

In some examples, the annealing temperature is between 50 to 70°C, such as between 55 to 60, 60 to 65, or 65 to 70°C, inclusive. In some examples, the annealing temperature is between 50 to 70°C, such as between 55 to 60, 60 to 65, or 65 to 70°C, inclusive, and either (I) the length of the annealing step (per PCR cycle) is greater than 3, 5, 8, 10, 15, 20, 30, 45, 60, 75, 90, 120, 150, or 180 minutes or (ii) the length of the annealing step (per PCR cycle) is between 5 and 180 minutes, such as 5 to 60, 10 to 60, 5 to 30, or 10 to 30 minutes, inclusive.

In some examples, one or more of the following conditions are used for empirical measurement of $T_m$ or are assumed for calculation of $T_m$: temperature: of 60.0 °C, primer concentration of 100 nM, and/or salt concentration of 100 mM. In some examples, other conditions are used, such as the conditions that will be used for multiplex PCR with the library. In some examples, 100 mM KCl, 50 mM (NH4)2SO4, 3 mM MgCl2, 7.5 nM of each primer, and 50 mM TMAC, at pH 8.1 is used. In some embodiments, the $T_m$ is calculated using the Primer3 program (libprimer3 release 2.2.3) using the built-in SantaLucia parameters (the world wide web at primer3.sourceforge.net). In some examples, the calculated melting temperature for a primer is the temperature at which half of the primers molecules are expected to be annealed. As discussed above, even at a temperature higher than the calculated melting temperature, a percentage of primers will be annealed, and therefore PCR extension is possible. In some examples, the empirically measured Tm (the actual Tm) is determined by using a thermostatted cell in a UV spectrophotometer. In some examples, temperature is plotted vs. absorbance, generating an S-shaped curve with two plateaus. The absorbance reading halfway between the plateaus corresponds to Tm.

In some examples, the absorbance at 260 nm is measured as a function of temperature on an ultrospec 2100 pr UV/visible spectrophotometer (Amersham Biosciences) (see, *e.g.,* Takiya et al., "An empirical approach for thermal stability (Tm) prediction of PNA/DNA duplexes," Nucleic Acids Symp Ser (Oxf); (48):131-2, 2004). In some examples, absorbance at 260 nm is measured by decreasing the temperature in steps of 2 °C per minute from 95 to 20 °C. In some examples, a primer and its perfect complement (such as 2 μM of each paired oligomer) are mixed and then annealing is performed by heating the sample to 95 °C, keeping it there for 5 minutes, followed by cooling to room temperature during 30 minutes, and keeping the samples at 95 °C for at least 60 minutes. In some examples, melting temperature is determined by analyzing the data using SWIFT Tm software. In some examplesof any of the methods of the present disclosure, the method includes empirically measuring or calculating (such as calculating with a computer) the melting temperature for at least 50, 80, 90, 92, 94, 96, 98, 99, or 100% of the primers in the library either before or after the primers are used for PCR amplification of target loci.

In some examples, the library comprises a microarray. In some embodiments, the library does not comprise a microarray.

In some examples, most or all of the primers are extended to form amplified products. Having all the primers consumed in the PCR reaction increases the uniformity of amplification of the different target loci since the same or similar number of primer molecules are converted to target amplicons for each target loci. In some embodiment, at least 80, 90, 92, 94, 96, 98, 99, or 100% of the primer molecules are extended to form amplified products. In some examples, for at least 80, 90, 92, 94, 96, 98, 99, or 100% of target loci, at least 80, 90, 92, 94, 96, 98, 99, or 100% of the primer molecules to that target loci are extended to form amplified products. In some examples, multiple cycles are performed until this percentage of the primers are consumed. In some examples, multiple cycles are performed until all or substantially all of the primers are consumed. If desired, a higher percentage of the primers can be consumed by decreasing the initial primer concentration and/or increasing the number of PCR cycles that are performed.

In some examples, the PCR methods may be performed with microliter reaction volumes, for which it can be harder to achieve specific PCR amplification (due to the lower local concentration of the template nucleic acids) compared to nanoliter or picoliter reaction volumes used in microfluidics applications. In some examples, the reaction volume is between 1 and 60 µL, such as between 5 and 50 µL, 10 and 50 µL, 10 and 20 µL, 20 and 30 µL, 30 and 40 µL, or 40 to 50 µL, inclusive.

In an examples, a method disclosed herein uses highly efficient highly multiplexed targeted PCR to amplify DNA followed by high throughput sequencing to determine the allele frequencies at each target locus. The ability to multiplex more than about 50 or 100 PCR primers in one reaction volume in a way that most of the resulting sequence reads map to targeted loci is novel and non-obvious. One technique that allows highly multiplexed targeted PCR to perform in a highly efficient manner involves designing primers that are unlikely to hybridize with one another. The PCR probes, typically referred to as primers, are selected by creating a thermodynamic model of potentially adverse interactions between at least 300; at least 500; at least 750; at least 1,000; at least 2,000; at least 5,000; at least 7,500; at least 10,000; at least 20,000; at least 25,000; at least 30,000; at least 40,000; at least 50,000; at least 75,000; or at least 100,000 potential primer pairs, or unintended interactions between primers and sample DNA, and then using the model to eliminate designs that are incompatible with other the designs in the pool. Another technique that allows highly multiplexed targeted PCR to perform in a highly efficient manner is using a partial or full nesting approach to the targeted PCR. Using one or a combination of these approaches allows multiplexing of at least 300, at least 800, at least 1,200, at least 4,000 or at least 10,000 primers in a single pool with the resulting amplified DNA comprising a majority of DNA molecules that, when sequenced, will map to targeted loci. Using one or a combination of these approaches allows multiplexing of a large number of primers in a single pool with the resulting amplified DNA comprising greater than 50%, greater than 60%, greater than 67%, greater than 80%, greater than 90%, greater than 95%, greater than 96%, greater than 97%, greater than 98%, greater than 99%, or greater than 99.5% DNA molecules that map to targeted loci.

In some examples the detection of the target genetic material may be done in a multiplexed fashion. The number of genetic target sequences that may be run in parallel can range from one to ten, ten to one hundred, one hundred to one thousand, one thousand to ten thousand, ten thousand to one hundred thousand, one hundred thousand to one million, or one million to ten million. Prior attempts to multiplex more than 100 primers per pool have resulted in significant problems with unwanted side reactions such as primer-dimer formation.

*5.5 Targeted PCR*

In some examples, PCR can be used to target specific locations of the genome. In plasma samples, the original DNA is highly fragmented (typically less than 500 bp, with an average length less than 200 bp). In PCR, both forward and reverse primers anneal to the same fragment to enable amplification. Therefore, if the fragments are short, the PCR assays must amplify relatively short regions as well. Like MIPS, if the polymorphic positions are too close the polymerase binding site, it could result in biases in the amplification from different alleles. Currently, PCR primers that target polymorphic regions, such as those containing SNPs, are typically designed such that the 3' end of the primer will hybridize to the base immediately adjacent to the polymorphic base or bases. In an example of the present disclosure, the 3' ends of both the forward and reverse PCR primers are designed to hybridize to bases that are one or a few positions away from the variant positions (polymorphic sites) of the targeted allele. The number of bases between the polymorphic site (SNP or otherwise) and the base to which the 3' end of the primer is designed to hybridize may be one base, it may be two bases, it may be three bases, it may be four bases, it may be five bases, it may be six bases, it may be seven to ten bases, it may be eleven to fifteen bases, or it may be sixteen to twenty bases. The forward and reverse primers may be designed to hybridize a different number of bases away from the polymorphic site.

PCR assay can be generated in large numbers, however, the interactions between different PCR assays makes it difficult to multiplex them beyond about one hundred assays. Various complex molecular approaches can be used to increase the level of multiplexing, but it may still be limited to fewer than 100, perhaps 200, or possibly 500 assays per reaction. Samples with large quantities of DNA can be split among multiple sub-reactions and then recombined before sequencing. For samples where either the overall sample or some subpopulation of DNA molecules is limited, splitting the sample would introduce statistical noise. In an example, a small or limited quantity of DNA may refer to an amount below 10 pg, between 10 and 100 pg, between 100 pg and 1 ng, between 1 and 10 ng, or between 10 and 100 ng. Note that while this method is particularly useful on small amounts of DNA where other methods that involve splitting into multiple pools can cause significant problems related to introduced stochastic noise, this method still provides the benefit of minimizing bias when it is run on samples of any quantity of DNA. In these situations, a universal pre-amplification step may be used to increase the overall sample quantity. Ideally, this pre-amplification step should not appreciably alter the allelic distributions.

In an example, a method of the present disclosure can generate PCR products that are specific to a large number of targeted loci, specifically 1,000 to 5,000 loci, 5,000 to 10,000 loci or more than 10,000 loci, for genotyping by sequencing or some other genotyping method, from limited samples such as single cells or DNA from body fluids. Currently, performing multiplex PCR reactions of more than 5 to 10 targets presents a major challenge and is often hindered by primer side products, such as primer dimers, and other artifacts. When detecting target sequences using microarrays with hybridization probes, primer dimers and other artifacts may be ignored, as these are not detected. However, when using sequencing as a method of detection, the vast majority of the sequencing reads would sequence such artifacts and not the desired target sequences in a sample. Methods described in the prior art used to multiplex more than 50 or 100 reactions in one reaction volume followed by sequencing will typically result in more than 20%, and often more than 50%, in many cases more than 80% and in some cases more than 90% off-target sequence reads.

In general, to perform targeted sequencing of multiple (n) targets of a sample (greater than 50, greater than 100, greater than 500, or greater than 1,000), one can split the sample into a number of parallel reactions that amplify one individual target. This has been performed in PCR multiwell plates or can be done in commercial platforms such as the FLUIDIGM ACCESS ARRAY (48 reactions per sample in microfluidic chips) or DROPLET PCR by RAIN DANCE TECHNOLOGY (100s to a few thousands of targets). Unfortunately, these split-and-pool methods are problematic for samples with a limited amount of DNA, as there are often not enough copies of the genome to ensure that there is one copy of each region of the genome in each well. This is an especially severe problem when polymorphic loci are targeted, and the relative proportions of the alleles at the polymorphic loci are needed, as the stochastic noise introduced by the splitting and pooling will cause very poorly accurate measurements of the proportions of the alleles that were present in the original sample of DNA. Described here is a method to effectively and efficiently amplify many PCR reactions that is applicable to cases where only a limited amount of DNA is available. In an example, the method may be applied for analysis of single cells, body fluids, mixtures of DNA such as the free floating DNA found in plasma, biopsies, environmental and/or forensic samples.

In an example, the targeted sequencing may involve one, a plurality, or all of the following steps. a) Generate and amplify a library with adaptor sequences on both ends of DNA fragments. b) Divide into multiple reactions after library amplification. c) Generate and optionally amplify a library with adaptor sequences on both ends of DNA fragments. d) Perform 1000- to 10,000-plex amplification of selected targets using one target specific "Forward" primer per target and one tag specific primer. e) Perform a second amplification from this product using "Reverse" target specific primers and one (or more) primer specific to a universal tag that was introduced as part of the target specific forward primers in the first round. f) Perform a 1000-plex preamplification of selected target for a limited number of cycles. g) Divide the product into multiple aliquots and amplify subpools of targets in individual reactions (for example, 50 to 500-plex, though this can be used all the way down to singleplex. h) Pool products of parallel subpools reactions. i) During these amplifications primers may carry sequencing compatible tags (partial or full length) such that the products can be sequenced.

*5.6 Highly Multiplexed PCR*

Disclosed herein are methods that permit the targeted amplification of over a hundred to tens of thousands of target sequences (e.g., SNP loci) from a nucleic acid sample such as genomic DNA obtained from plasma. The amplified sample may be relatively free of primer dimer products and have low allelic bias at target loci. If during or after amplification the products are appended with sequencing compatible adaptors, analysis of these products can be performed by sequencing.

Performing a highly multiplexed PCR amplification using methods known in the art results in the generation of primer dimer products that are in excess of the desired amplification products and not suitable for sequencing. These can be reduced empirically by eliminating primers that form these products, or by performing *in silico* selection of primers. However, the larger the number of assays, the more difficult this problem becomes.

One solution is to split the 5000-plex reaction into several lower-plexed amplifications, *e.g.* one hundred 50-plex or fifty 100-plex reactions, or to use microfluidics or even to split the sample into individual PCR reactions. However, if the sample DNA is limited, such as in non-invasive prenatal diagnostics from pregnancy plasma, dividing the sample between multiple reactions should be avoided as this will result in bottlenecking.

Described herein are methods to first globally amplify the plasma DNA of a sample and then divide the sample up into multiple multiplexed target enrichment reactions with more moderate numbers of target sequences per reaction. In an example, a method of the present disclosure can be used for preferentially enriching a DNA mixture at a plurality of loci, the method comprising one or more of the following steps: generating and amplifying a library from a mixture of DNA where the molecules in the library have adaptor sequences ligated on both ends of the DNA fragments, dividing the amplified library into multiple reactions, performing a first round of multiplex amplification of selected targets using one target specific "forward" primer per target and one or a plurality of adaptor specific universal "reverse" primers. In an example, a method of the present disclosure further includes performing a second amplification using "reverse" target specific primers and one or a plurality of primers specific to a universal tag that was introduced as part of the target specific forward primers in the first round. In an example, the method may involve a fully nested, hemi-nested, semi-nested, one sided fully nested, one sided hemi-nested, or one sided semi-nested PCR approach. In an example, a method of the present disclosure is used for preferentially enriching a DNA mixture at a plurality of loci, the method comprising performing a multiplex preamplification of selected targets for a limited number of cycles, dividing the product into multiple aliquots and amplifying subpools of targets in individual reactions, and pooling products of parallel subpools reactions. Note that this approach could be used to perform targeted amplification in a manner that would result in low levels of allelic bias for 50-500 loci, for 500 to 5,000 loci, for 5,000 to 50,000 loci, or even for 50,000 to 500,000 loci. In an example, the primers carry partial or full length sequencing compatible tags.

The workflow may entail (1) extracting DNA such as plasma DNA, (2) preparing fragment library with universal adaptors on both ends of fragments, (3) amplifying the library using universal primers specific to the adaptors, (4) dividing the amplified sample "library" into multiple aliquots, (5) performing multiplex (e.g. about 100-plex, 1,000, or 10,000-plex with one target specific primer per target and a tag-specific primer) amplifications on aliquots, (6) pooling aliquots of one sample, (7) barcoding the sample, (8) mixing the samples and adjusting the concentration, (9) sequencing the sample. The workflow may comprise multiple sub-steps that contain one of the listed steps (e.g. step (2) of preparing the library step could entail three enzymatic steps (blunt ending, dA tailing and adaptor ligation) and three purification steps). Steps of the workflow may be combined, divided up or performed in different order (*e.g.* bar coding and pooling of samples).

It is important to note that the amplification of a library can be performed in such a way that it is biased to amplify short fragments more efficiently. In this manner it is possible to preferentially amplify shorter sequences, e.g. mononucleosomal DNA fragments as the cell free fetal DNA (of placental origin) found in the circulation of pregnant women. Note that PCR assays can have the tags, for example sequencing tags, (usually a truncated form of 15-25 bases). After multiplexing, PCR multiplexes of a sample are pooled and then the tags are completed (including bar coding) by a tag-specific PCR (could also be done by ligation). Also, the full sequencing tags can be added in the same reaction as the multiplexing. In the first cycles targets may be amplified with the target specific primers, subsequently the tag-specific primers take over to complete the SQ-adaptor sequence. The PCR primers may carry no tags. The sequencing tags may be appended to the amplification products by ligation.

In an example, highly multiplex PCR followed by evaluation of amplified material by clonal sequencing may be used for various applications such as the detection of fetal aneuploidy. Whereas traditional multiplex PCRs evaluate up to fifty loci simultaneously, the approach described herein may be used to enable simultaneous evaluation of more than 50 loci simultaneously, more than 100 loci simultaneously, more than 500 loci simultaneously, more than 1,000 loci simultaneously, more than 5,000 loci simultaneously, more than 10,000 loci simultaneously, more than 50,000 loci simultaneously, and more than 100,000 loci simultaneously. Experiments have shown that up to, including and more than 10,000 distinct loci can be evaluated simultaneously, in a single reaction, with sufficiently good efficiency and specificity to make non-invasive prenatal aneuploidy diagnoses and/or copy number calls with high accuracy. Assays may be combined in a single reaction with the entirety of a sample such as a cfDNA sample isolated from plasma, a fraction thereof, or a further processed derivative of the cfDNA sample. The sample (e.g., cfDNA or derivative) may also be split into multiple parallel multiplex reactions. The optimum sample splitting and multiplex is determined by trading off various performance specifications. Due to the limited amount of material, splitting the sample into multiple fractions can introduce sampling noise, handling time, and increase the possibility of error. Conversely, higher multiplexing can result in greater amounts of spurious amplification and greater inequalities in amplification both of which can reduce test performance.

Two crucial related considerations in the application of the methods described herein are the limited amount of original sample (e.g., plasma) and the number of original molecules in that material from which allele frequency or other measurements are obtained. If the number of original molecules falls below a certain level, random sampling noise becomes significant, and can affect the accuracy of the test. Typically, data of sufficient quality for making non-invasive prenatal aneuploidy diagnoses can be obtained if measurements are made on a sample comprising the equivalent of 500-1000 original molecules per target locus. There are a number of ways of increasing the number of distinct measurements, for example increasing the sample volume. Each manipulation applied to the sample also potentially results in losses of material. It is essential to characterize losses incurred by various manipulations and avoid, or as necessary improve yield of certain manipulations to avoid losses that could degrade performance of the test.

In an example, it is possible to mitigate potential losses in subsequent steps by amplifying all or a fraction of the original sample (*e.g.*, cfDNA sample). Various methods are available to amplify all of the genetic material in a sample, increasing the amount available for downstream procedures. In an example, ligation mediated PCR (LM-PCR) DNA fragments are amplified by PCR after ligation of either one distinct adaptors, two distinct adapters, or many distinct adaptors. In an example, multiple displacement amplification (MDA) phi-29 polymerase is used to amplify all DNA isothermally. In DOP-PCR and variations, random priming is used to amplify the original material DNA. Each method has certain characteristics such as uniformity of amplification across all represented regions of the genome, efficiency of capture and amplification of original DNA, and amplification performance as a function of the length of the fragment.

In an example LM-PCR may be used with a single heteroduplexed adaptor having a 3-prime tyrosine. The heteroduplexed adaptor enables the use of a single adaptor molecule that may be converted to two distinct sequences on 5-prime and 3-prime ends of the original DNA fragment during the first round of PCR. In an example, it is possible to fractionate the amplified library by size separations, or products such as AMPURE, TASS or other similar methods. Prior to ligation, sample DNA may be blunt ended, and then a single adenosine base is added to the 3-prime end. Prior to ligation the DNA may be cleaved using a restriction enzyme or some other cleavage method. During ligation the 3-prime adenosine of the sample fragments and the complementary 3-prime tyrosine overhang of adaptor can enhance ligation efficiency. The extension step of the PCR amplification may be limited from a time standpoint to reduce amplification from fragments longer than about 200 bp, about 300 bp, about 400 bp, about 500 bp or about 1,000 bp. A number of reactions were run using conditions as specified by commercially available kits; the resulted in successful ligation of fewer than 10% of sample DNA molecules. A series of optimizations of the reaction conditions for this improved ligation to approximately 70%.

In an example, a method disclosed herein uses highly efficient highly multiplexed targeted PCR of at least 1000 target loci followed by next generation sequencing to obtain genotype data. In some examples, the multiplexed targeted PCR is performed on at least 1000, 2000, 3000, 4000, 5000, 6000, 7000, 8000, 9000, 10000, 11000, 12000, 13000, 14000, 15000, 20000, 25000, 30000, 35000, 40000, 45000, or 50000 target loci. In one example, the multiplexed targeted PCR is performed on 13000 target loci. In some examples, the target loci are single nucleotide polymorphisms (SNPs).

*5.7 Mini-PCR*

The following Mini-PCR method is desirable for samples containing short nucleic acids, digested nucleic acids, or fragmented nucleic acids, such as cfDNA. Traditional PCR assay design results in significant losses of distinct fetal molecules, but losses can be greatly reduced by designing very short PCR assays, termed mini-PCR assays. Fetal cfDNA in maternal serum is highly fragmented and the fragment sizes are distributed in approximately a Gaussian fashion with a mean of 160 bp, a standard deviation of 15 bp, a minimum size of about 100 bp, and a maximum size of about 220 bp. The distribution of fragment starts and end positions with respect to the targeted polymorphisms, while not necessarily random, vary widely among individual targets and among all targets collectively and the polymorphic site of one particular target locus may occupy any position from the start to the end among the various fragments originating from that locus. Note that the term mini-PCR may equally well refer to normal PCR with no additional restrictions or limitations.

During PCR, amplification will only occur from template DNA fragments comprising both forward and reverse primer sites. Because fetal cfDNA fragments are short, the likelihood of both primer sites being present the likelihood of a fetal fragment of length L comprising both the forward and reverse primers sites is ratio of the length of the amplicon to the length of the fragment. Under ideal conditions, assays in which the amplicon is 45, 50, 55, 60, 65, or 70 bp will successfully amplify from 72%, 69%, 66%, 63%, 59%, or 56%, respectively, of available template fragment molecules. The amplicon length is the distance between the 5-prime ends of the forward and reverse priming sites. Amplicon length that is shorter than typically used by those known in the art may result in more efficient measurements of the desired polymorphic loci by only requiring short sequence reads. In an example, a substantial fraction of the amplicons should be less than 100 bp, less than 90 bp, less than 80 bp, less than 70 bp, less than 65 bp, less than 60 bp, less than 55 bp, less than 50 bp, or less than 45 bp.

Note that in methods known in the prior art, short assays such as those described herein are usually avoided because they are not required and they impose considerable constraint on primer design by limiting primer length, annealing characteristics, and the distance between the forward and reverse primer.

Also note that there is the potential for biased amplification if the 3-prime end of the either primer is within roughly 1-6 bases of the polymorphic site. This single base difference at the site of initial polymerase binding can result in preferential amplification of one allele, which can alter observed allele frequencies and degrade performance. All of these constraints make it very challenging to identify primers that will amplify a particular locus successfully and furthermore, to design large sets of primers that are compatible in the same multiplex reaction. In an example, the 3' end of the inner forward and reverse primers are designed to hybridize to a region of DNA upstream from the polymorphic site, and separated from the polymorphic site by a small number of bases. Ideally, the number of bases may be between 6 and 10 bases, but may equally well be between 4 and 15 bases, between three and 20 bases, between two and 30 bases, or between 1 and 60 bases, and achieve substantially the same end.

Multiplex PCR may involve a single round of PCR in which all targets are amplified or it may involve one round of PCR followed by one or more rounds of nested PCR or some variant of nested PCR. Nested PCR consists of a subsequent round or rounds of PCR amplification using one or more new primers that bind internally, by at least one base pair, to the primers used in a previous round. Nested PCR reduces the number of spurious amplification targets by amplifying, in subsequent reactions, only those amplification products from the previous one that have the correct internal sequence. Reducing spurious amplification targets improves the number of useful measurements that can be obtained, especially in sequencing. Nested PCR typically entails designing primers completely internal to the previous primer binding sites, necessarily increasing the minimum DNA segment size required for amplification. For samples such as plasma cfDNA, in which the DNA is highly fragmented, the larger assay size reduces the number of distinct cfDNA molecules from which a measurement can be obtained. In an example, to offset this effect, one may use a partial nesting approach where one or both of the second round primers overlap the first binding sites extending internally some number of bases to achieve additional specificity while minimally increasing in the total assay size.

In an example, a multiplex pool of PCR assays are designed to amplify potentially heterozygous SNP or other polymorphic or non-polymorphic loci on one or more chromosomes and these assays are used in a single reaction to amplify DNA. The number of PCR assays may be between 50 and 200 PCR assays, between 200 and 1,000 PCR assays, between 1,000 and 5,000 PCR assays, or between 5,000 and 20,000 PCR assays (50 to 200-plex, 200 to 1,000-plex, 1,000 to 5,000-plex, 5,000 to 20,000-plex, more than 20,000-plex respectively). In an example, a multiplex pool of about 10,000 PCR assays (10,000-plex) are designed to amplify potentially heterozygous SNP loci on chromosomes X, Y, 13, 18, and 21 and 1 or 2 and these assays are used in a single reaction to amplify cfDNA obtained from a material plasma sample, chorion villus samples, amniocentesis samples, single or a small number of cells, other bodily fluids or tissues, cancers, or other genetic matter. The SNP frequencies of each locus may be determined by clonal or some other method of sequencing of the amplicons. Statistical analysis of the allele frequency distributions or ratios of all assays may be used to determine if the sample contains a trisomy of one or more of the chromosomes included in the test. In another example the original cfDNA samples is split into two samples and parallel 5,000-plex assays are performed. In another example, the original cfDNA samples is split into n samples and parallel (~10,000/n)-plex assays are performed where n is between 2 and 12, or between 12 and 24, or between 24 and 48, or between 48 and 96. Data is collected and analyzed in a similar manner to that already described. Note that this method is equally well applicable to detecting translocations, deletions, duplications, and other chromosomal abnormalities.

In an example, tails with no homology to the target genome may also be added to the 3-prime or 5-prime end of any of the primers. These tails facilitate subsequent manipulations, procedures, or measurements. In an example, the tail sequence can be the same for the forward and reverse target specific primers. In an example, different tails may be used for the forward and reverse target specific primers. In an example, a plurality of different tails may be used for different loci or sets of loci. Certain tails may be shared among all loci or among subsets of loci. For example, using forward and reverse tails corresponding to forward and reverse sequences required by any of the current sequencing platforms can enable direct sequencing following amplification. In an example, the tails can be used as common priming sites among all amplified targets that can be used to add other useful sequences. In some examples, the inner primers may contain a region that is designed to hybridize either upstream or downstream of the targeted locus (e.g, a polymorphic locus). In some examples, the primers may contain a molecular barcode. In some examples, the primer may contain a universal priming sequence designed to allow PCR amplification.

In an example, a 10,000-plex PCR assay pool is created such that forward and reverse primers have tails corresponding to the required forward and reverse sequences required by a high throughput sequencing instrument (often referred to as a massively parallel sequencing instrument) such as the HISEQ, GAIIX, or MYSEQ available from ILLUMINA. In addition, included 5-prime to the sequencing tails is an additional sequence that can be used as a priming site in a subsequent PCR to add nucleotide barcode sequences to the amplicons, enabling multiplex sequencing of multiple samples in a single lane of the high throughput sequencing instrument.

In an example, a 10,000-plex PCR assay pool is created such that reverse primers have tails corresponding to the required reverse sequences required by a high throughput sequencing instrument. After amplification with the first 10,000-plex assay, a subsequent PCR amplification may be performed using an another 10,000-plex pool having partly nested forward primers (e.g. 6-bases nested) for all targets and a reverse primer corresponding to the reverse sequencing tail included in the first round. This subsequent round of partly nested amplification with just one target specific primer and a universal primer limits the required size of the assay, reducing sampling noise, but greatly reduces the number of spurious amplicons. The sequencing tags can be added to appended ligation adaptors and/or as part of PCR probes, such that the tag is part of the final amplicon.

Tumor fraction affects performance of the test. There are a number of ways to enrich the tumor fraction of the DNA found in patient plasma. Tumor fraction can be increased by the previously described LM-PCR method already discussed as well as by a targeted removal of long fragments. In an example, prior to multiplex PCR amplification of the target loci, an additional multiplex PCR reaction may be carried out to selectively remove long and largely maternal fragments corresponding to the loci targeted in the subsequent multiplex PCR. Additional primers are designed to anneal a site a greater distance from the polymorphism than is expected to be present among cell free fetal DNA fragments. These primers may be used in a one cycle multiplex PCR reaction prior to multiplex PCR of the target polymorphic loci. These distal primers are tagged with a molecule or moiety that can allow selective recognition of the tagged pieces of DNA. In an example, these molecules of DNA may be covalently modified with a biotin molecule that allows removal of newly formed double stranded DNA comprising these primers after one cycle of PCR. Double stranded DNA formed during that first round is likely maternal in origin. Removal of the hybrid material may be accomplished by using magnetic streptavidin beads. There are other methods of tagging that may work equally well. In an example, size selection methods may be used to enrich the sample for shorter strands of DNA; for example, those less than about 800 bp, less than about 500 bp, or less than about 300 bp. Amplification of short fragments can then proceed as usual.

The mini-PCR method described in this disclosure enables highly multiplexed amplification and analysis of hundreds to thousands or even millions of loci in a single reaction, from a single sample. At the same, the detection of the amplified DNA can be multiplexed; tens to hundreds of samples can be multiplexed in one sequencing lane by using barcoding PCR. This multiplexed detection has been successfully tested up to 49-plex, and a much higher degree of multiplexing is possible. In effect, this allows hundreds of samples to be genotyped at thousands of SNPs in a single sequencing run. For these samples, the method allows determination of genotype and heterozygosity rate and simultaneously determination of copy number, both of which may be used for the purpose of aneuploidy detection. It may be used as part of a method for mutation dosage. This method may be used for any amount of DNA or RNA, and the targeted regions may be SNPs, other polymorphic regions, non-polymorphic regions, and combinations thereof.

In some examples, ligation mediated universal-PCR amplification of fragmented DNA may be used. The ligation mediated universal-PCR amplification can be used to amplify plasma DNA, which can then be divided into multiple parallel reactions. It may also be used to preferentially amplify short fragments, thereby enriching tumor fraction. In some examples the addition of tags to the fragments by ligation can enable detection of shorter fragments, use of shorter target sequence specific portions of the primers and/or annealing at higher temperatures which reduces unspecific reactions.

The methods described herein may be used for a number of purposes where there is a target set of DNA that is mixed with an amount of contaminating DNA. In some examples, the target DNA and the contaminating DNA may be from individuals who are genetically related. For example, genetic abnormalities in a fetus (target) may be detected from maternal plasma which contains fetal (target) DNA and also maternal (contaminating) DNA; the abnormalities include whole chromosome abnormalities (e.g. aneuploidy) partial chromosome abnormalities (e.g. deletions, duplications, inversions, translocations), polynucleotide polymorphisms (e.g. STRs), single nucleotide polymorphisms, and/or other genetic abnormalities or differences. In some examples, the target and contaminating DNA may be from the same individual, but where the target and contaminating DNA are different by one or more mutations, for example in the case of cancer. (see e.g. H. Mamon et al. Preferential Amplification of Apoptotic DNA from Plasma: Potential for Enhancing Detection of Minor DNA Alterations in Circulating DNA. Clinical Chemistry 54:9 (2008). In some examples, the DNA may be found in cell culture (apoptotic) supernatant. In some examples, it is possible to induce apoptosis in biological samples (e.g., blood) for subsequent library preparation, amplification and/or sequencing. A number of enabling workflows and protocols to achieve this end are presented elsewhere in this disclosure.

In some examples, the target DNA may originate from single cells, from samples of DNA consisting of less than one copy of the target genome, from low amounts of DNA, from DNA from mixed origin (e.g. cancer patient plasma and tumors: mix between healthy and cancer DNA, transplantation etc.), from other body fluids, from cell cultures, from culture supernatants, from forensic samples of DNA, from ancient samples of DNA (e.g. insects trapped in amber), from other samples of DNA, and combinations thereof.

In some examples, a short amplicon size may be used. Short amplicon sizes are especially suited for fragmented DNA (see e.g. A. Sikora, et sl. Detection of increased amounts of cell-free fetal DNA with short PCR amplicons. Clin Chem. 2010 Jan;56(1):136-8.)

The use of short amplicon sizes may result in some significant benefits. Short amplicon sizes may result in optimized amplification efficiency. Short amplicon sizes typically produce shorter products, therefore there is less chance for nonspecific priming. Shorter products can be clustered more densely on sequencing flow cell, as the clusters will be smaller. Note that the methods described herein may work equally well for longer PCR amplicons. Amplicon length may be increased if necessary, for example, when sequencing larger sequence stretches. Experiments with 146-plex targeted amplification with assays of 100 bp to 200 bp length as first step in a nested-PCR protocol were run on single cells and on genomic DNA with positive results.

In some examples, the methods described herein may be used to amplify and/or detect SNPs, copy number, nucleotide methylation, mRNA levels, other types of RNA expression levels, other genetic and/or epigenetic features. The mini-PCR methods described herein may be used along with next-generation sequencing; it may be used with other downstream methods such as microarrays, counting by digital PCR, real-time PCR, Mass-spectrometry analysis etc.

In some examples, the mini-PCR amplification methods described herein may be used as part of a method for accurate quantification of minority populations. It may be used for absolute quantification using spike calibrators. It may be used for mutation / minor allele quantification through very deep sequencing, and may be run in a highly multiplexed fashion. It may be used for standard paternity and identity testing of relatives or ancestors, in human, animals, plants or other creatures. It may be used for forensic testing. It may be used for rapid genotyping and copy number analysis (CN), on any kind of material, e.g. amniotic fluid and CVS, sperm, product of conception (POC). It may be used for single cell analysis, such as genotyping on samples biopsied from embryos. It may be used for rapid embryo analysis (within less than one, one, or two days of biopsy) by targeted sequencing using min-PCR.

In some examples, the mini-PCR amplification methods can be used for tumor analysis: tumor biopsies are often a mixture of healthy and tumor cells. Targeted PCR allows deep sequencing of SNPs and loci with close to no background sequences. It may be used for copy number and loss of heterozygosity analysis on tumor DNA. Said tumor DNA may be present in many different body fluids or tissues of tumor patients. It may be used for detection of tumor recurrence, and/or tumor screening. It may be used for quality control testing of seeds. It may be used for breeding, or fishing purposes. Note that any of these methods could equally well be used targeting non-polymorphic loci for the purpose of ploidy calling.

Some literature describing some of the fundamental methods that underlie the methods disclosed herein include: (1) Wang HY, Luo M, Tereshchenko IV, Frikker DM, Cui X, Li JY, Hu G, Chu Y, Azaro MA, Lin Y, Shen L, Yang Q, Kambouris ME, Gao R, Shih W, Li H. Genome Res. 2005 Feb;15(2):276-83. Department of Molecular Genetics, Microbiology and Immunology/The Cancer Institute of New Jersey, Robert Wood Johnson Medical School, New Brunswick, New Jersey 08903, USA. (2) High-throughput genotyping of single nucleotide polymorphisms with high sensitivity. Li H, Wang HY, Cui X, Luo M, Hu G, Greenawalt DM, Tereshchenko IV, Li JY, Chu Y, Gao R. Methods Mol Biol. 2007;396 - PubMed PMID: 18025699. (3) A method comprising multiplexing of an average of 9 assays for sequencing is described in: Nested Patch PCR enables highly multiplexed mutation discovery in candidate genes. Varley KE, Mitra RD. Genome Res. 2008 Nov;18(11):1844-50. Epub 2008 Oct 10. Note that the methods disclosed herein allow multiplexing of orders of magnitude more than in the above references.

### 6. Methods for Analyzing Genomic Information from Circulating Fetal Cells for Non-Invasive Prenatal Testing.

In one teaching, the method may further comprise performing non-invasive prenatal testing using the genotypes of one or more circulating cells determined to be one or more fetal cells.

In an example, the method may further comprise detecting a copy number variation or aneuploidy of a target chromosome or chromosome segment of interest in the one or more circulating cells determined to be one or more fetal cells.

Ploidy Calling, also "Chromosome Copy Number Calling," or "Copy Number Calling" (CNC), refers to the act of determining the quantity and chromosomal identity of one or more chromosomes present in a cell. Aneuploidy refers to the state where the wrong number of chromosomes are present in a cell. In the case of a somatic human cell it refers to the case where a cell does not contain 22 pairs of autosomal chromosomes and one pair of sex chromosomes. In the case of a human gamete, it refers to the case where a cell does not contain one of each of the 23 chromosomes. In the case of a single chromosome, it refers to the case where more or less than two homologous but non-identical chromosomes are present, and where each of the two chromosomes originate from a different parent. Ploidy State refers to the quantity and chromosomal identity of one or more chromosomes in a cell.

CNVs are often assigned to one of two main categories, based on the length of the affected sequence. The first category includes copy number polymorphisms (CNPs), which are common in the general population, occurring with an overall frequency of greater than 1%. CNPs are typically small (most are less than 10 kilobases in length), and they are often enriched for genes that encode proteins important in drug detoxification and immunity. A subset of these CNPs is highly variable with respect to copy number. As a result, different human chromosomes can have a wide range of copy numbers (e.g., 2, 3, 4, 5, etc.) for a particular set of genes. CNPs associated with immune response genes have recently been associated with susceptibility to complex genetic diseases, including psoriasis, Crohn's disease, and glomerulonephritis.

The second class of CNVs includes relatively rare variants that are much longer than CNPs, ranging in size from hundreds of thousands of base pairs to over 1 million base pairs in length. In some cases, these CNVs may have arisen during production of the sperm or egg that gave rise to a particular individual, or they may have been passed down for only a few generations within a family. These large and rare structural variants have been observed disproportionately in subjects with mental retardation, developmental delay, schizophrenia, and autism. Their appearance in such subjects has led to speculation that large and rare CNVs may be more important in neurocognitive diseases than other forms of inherited mutations, including single nucleotide substitutions.

Gene copy number can be altered in cancer cells. For instance, duplication of Chrlp is common in breast cancer, and the EGFR copy number can be higher than normal in non-small cell lung cancer. Further target genes for diagnosing cancer is provided in section 9 herein. Cancer is one of the leading causes of death; thus, early diagnosis and treatment of cancer is important, since it can improve the patient's outcome (such as by increasing the probability of remission and the duration of remission). Early diagnosis can also allow the patient to undergo fewer or less drastic treatment alternatives. Many of the current treatments that destroy cancerous cells also affect normal cells, resulting in a variety of possible side-effects, such as nausea, vomiting, low blood cell counts, increased risk of infection, hair loss, and ulcers in mucous membranes. Thus, early detection of cancer is desirable since it can reduce the amount and/or number of treatments (such as chemotherapeutic agents or radiation) needed to eliminate the cancer.

Copy number variation has also been associated with severe mental and physical handicaps, and idiopathic learning disability. Non-invasive prenatal testing (NIPT) using cell-free DNA (cfDNA) can be used to detect abnormalities, such as fetal trisomies 13, 18, and 21, triploidy, and sex chromosome aneuploidies. Accordingly, in an embodiment, the target chromosome or chromosome segment of interest is chromosome 13, 18, 21, the sex chromosomes, and/or chromosome segments thereof.

Subchromosomal microdeletions, which can also result in severe mental and physical handicaps, are more challenging to detect due to their smaller size. Eight of the microdeletion syndromes have an aggregate incidence of more than 1 in 1000, making them nearly as common as fetal autosomal trisomies. Accordingly, in an embodiment, the methods disclosed herein further comprise detecting a microdeletion in the one or more circulating cell determined to be one or more pure fetal cells. In an embodiment, the microdeletion is 22q11.2 deletion associated with DiGeorge syndrome, microdeletion associated with Prader-Willi syndrome, microdeletion associated with Angelman syndrom, 1p36 deletion, and/or

**EP 3 899 030 B1**

microdeletion associated with the Cri-du-chat syndrome.

In addition, a higher copy number of CCL3L1 has been associated with lower susceptibility to HIV infection, and a low copy number of FCGR3B (the CD16 cell surface immunoglobulin receptor) can increase susceptibility to systemic lupus erythematosus and similar inflammatory autoimmune disorders.

Methods for measuring chromosome copy number in fetal cells based on counting the number of DNA sequence-based reads that map to a given chromosome or chromosome segment are conveniently referred to as "counting methods", or "quantitative methods" for analyzing chromosome copy number or chromosome segment copy number. Examples of such methods can be found, among other places, in published patent application US2013/0172211 A1 U.S. Pat. No. 8,008,018; U.S. Pat. No. 8,467,976 B2; US published patent application US 2012/0003637 A1. Such methods typically involve creation of a reference value (cut-off value) for the number of DNA sequence reads mapping to a specific chromosome, where in a number of reads in excess of the value is indicative of a specific genetic abnormality.

Accordingly, the methods for analyzing and characterizing a cell sample suspected to be of fetal origin may be combined with methods and systems for determining the copy number, or detecting aneuploidy of a chromosome or chromosome segment of interest in the cell of interest confirmed to be a fetal cell. These methods for determining copy number or detecting aneuploidy are performed using the chromosome or chromosome segment of interest to set a bias model, that is to set test parameters, using samples analyzed in the same parallel analysis, that are identified as diploid samples with high confidence, for the analysis of aneuploidy for the same chromosome or chromosome segment of interest of other sample(s) in the set of on-test samples. Confidence refers to the statistical likelihood that the called SNP, allele, set of alleles, ploidy call, or determined number of chromosome segment copies correctly represents the real genetic state of the individual. Such methods of determining copy number variation and aneuploidy from fetal DNA is described in U.S. Patent Publication 2018/0173846.

The amount of each locus detected by sequencing preparations of DNA obtained from target and non-target cells can vary from locus to locus for reasons other than the starting quantity of the locus in the initial sample material prior to preparation for sequencing, e.g. prior to an amplification step such as PCR. Variables such as PCR primer binding efficiency, amplicon length, GC content, and the like can cause variations in the representation of individual loci in a preparation for sequencing or during sequencing. Factors such as these can result in a locus specific bias causing the overrepresentation or underrepresentation of one locus to another. In addition, bias can result from sample-specific inconsistencies. For example, due to a pipetting error or other measurement error during physical processing of the samples, one sample can have more DNA than another sample in the set of samples. In illustrative examples, these sample-specific biases are taken into account by sample-specific parameters. These specific examples of sample specific parameters are disclosed in U.S. Patent Publication 2018/0173846.

Independent of the specific method used to produce the genetic information, the amount of genetic sequence information from each locus is dependent upon the relative quantity of the copy numbers of the loci in the original sample. Loci that are believed to be on the same chromosomal segment, or in some examples the same chromosome, are presumed to have the same starting amount. Thus, for example, the multiple loci present on chromosome 21 in the genome of the target cell (or the genome of the non-target cell) are presumed to be present in approximately equal amounts in the genomic DNA. Thus differences in the amount of observed genetic information between loci on the same chromosome are the result of locus specific bias. For example, if SNP1 and SNP2 are located on the same chromosome and assumed to have the same copy number on the same chromosome, and SNP1 is found to have depth of read of 0.1% and SNP2 is found to have a depth of read of 0.4%, this may be explained by a quantifiable locus specific bias favoring the production of DNA sequence from SNP2 over SNP1. This bias may be additionally normalized by virtue of considering the distribution of possible sampling outcomes for the two different SNPs. Thus, methods of the present disclosure, analyze bias and provide a bias model, as discussed more fully in U.S. Patent Publication 2018/0173846.

In certain examples of the present disclosure, one or two maximum likelihood methods are used. As disclosed above, the first maximum likelihood method can be used to identify diploid samples in the set of samples and to determine a first probability that the other samples in the set of samples are aneuploidy. Accordingly, in certain examples, one or more or all of the chromosome(s) or chromosome segment(s) of interest are determined to be disomic using a first maximum likelihood method. Such methods are further described and illustrated in U.S. Patent Publication 2018/0173846.

In another example, the number of copies of the chromosome or chromosome segment of interest, is determined by creating a plurality or set of $2^{nd}$ ploidy hypotheses, wherein each $2^{nd}$ ploidy hypothesis is associated with a specific copy number of the chromosome or chromosome segment of interest in the target cell. The models are then used to test how well the genetic data from each patient fits each $2^{nd}$ hypothesis. The goodness of fit for each $2^{nd}$ hypothesis is determined. A second probability value is calculated for each second hypothesis wherein the second probability value indicates the likelihood that the genome of the target cell has the number of chromosomes or chromosome segments that is specified by the second hypothesis. Thus by selecting the $2^{nd}$ hypothesis with the maximum likelihood, one may determine the copy number for the chromosome or chromosome segment in the genome of the target cell. Such first and second hypothesis can be considered in combination to increase the confidence of the aneuploidy determination, as discussed more fully in U.S. Patent Publication 2018/0173846.

24

In one example of the present disclosure, where the method used to determine the ploidy state of a fetus, the method further includes taking into account the fraction of fetal DNA in the sample. In one example of the present disclosure, the method involves calculating the percent of DNA in a sample that is fetal or placental in origin. In one example of the present disclosure, the threshold for calling aneuploidy is adaptively normalized based on the calculated percent fetal DNA. In some examples, the method for estimating the percentage of DNA that is of fetal origin in a mixture of DNA, comprises obtaining a mixed sample that contains genetic material from the mother, and genetic material from the fetus, obtaining a genetic sample from the father of the fetus, measuring the DNA in the mixed sample, measuring the DNA in the father sample, and calculating the percentage of DNA that is of fetal origin in the mixed sample using the DNA measurements of the mixed sample, and of the father sample.

In one teaching, the methods described herein may enable confirmation that the sample is a mixture of fetal and maternal cells when the cell sample contained both true fetal and true maternal cells, and by using the cell-free DNA (cfDNA) sample as a reference, the fetal fraction may be estimated. As described in U.S. Appl. 2018/0298439, genotype data of maternal DNA, mixture of fetal and maternal DNA, and an estimated fraction of fetal DNA allows determination of paternity of the fetus. Genotype data acquired of a mixture of mother and child cfDNA, and a mixture of fetal and maternal DNA may be used to determine the fetal fraction of the sample to allow paternity testing.

Some examples may be used in combination with the PARENTAL SUPPORT™ (PS) method, examples of which are described in U.S. application Ser. No. 11/603,406 (US Publication No.: 20070184467), U.S. application Ser. No. 12/076,348 (US Publication No.: 20080243398), U.S. application Ser. No. 13/110,685, PCT Application PCT/US09/52730 (PCT Publication No.: WO/2010/017214), PCT Application No. PCT/US10/050824 (PCT Publication No.: WO/2011/041485), PCT Application No. PCT/US2011/037018 (PCT Publication No.: WO/2011/146632), and PCT Application No. PCT/US2011/61506. PARENTAL SUPPORT™ is an informatics based approach that can be used to analyze genetic data. In some examples, the methods disclosed herein may be considered as part of the PARENTAL SUPPORT™ method. In some examples, The PARENTAL SUPPORT™ method is a collection of methods that may be used to determine the genetic data of a target individual, with high accuracy, of one or a small number of cells from that individual, or of a mixture of DNA consisting of DNA from the target individual and DNA from one or a plurality of other individuals, specifically to determine disease-related alleles, other alleles of interest, the ploidy state of one or a plurality of chromosomes in the target individual, and or the extent of relationship of another individual to the target individual. PARENTAL SUPPORT™ may refer to any of these methods. PARENTAL SUPPORT™ is an example of an informatics based method.

In some examples of the present disclosure, improved confidence for an aneuploidy determination can be obtained by determining aneuploidy of a sample using a quantitative non-allelic threshold or cutoff method and for the same sample, determining aneuploidy using a method that determines likelihoods. If the sample is identified having aneuploidy in a chromosome or chromosome segment of interest by a threshold method and the sample is identified as having aneuploidy with high confidence using a likelihood determination for a set of hypothesis, then the sample is identified as a sample having aneuploidy at the chromosome or chromosome segment of interest for one or more target cells in a subject that is the source of the sample. Such methods are further explained and illustrated in U.S. Patent Publication 2018/0173846.

Methods are known in the art for carrying out a non-allelic threshold analysis, especially for NIPT. For example, U.S. Pat. Nos. 7,888,017 and 8,318,430, provide methods for determining fetal aneuploidy by counting the number of reads that map to a suspect chromosome and comparing it to the number of reads that map to a reference chromosome, and using the assumption that an overabundance of reads on the suspect chromosome corresponds to a triploidy in the fetus at that chromosome. Teachings provided therein can be useful in carrying out examples of the present disclosure that involve a depth of sequencing reads and a reference value.

## 7. Methods for Analyzing Genomic Information from Circulating Tumor Cells.

Methods and compositions provided herein improve the detection, diagnosis, staging, screening, treatment, and management of cancer (e.g., breast cancer, bladder cancer, or colorectal cancer) by using liquid biopsy to obtain samples containing circulating tumor cells. Methods provided herein, in illustrative examples analyze mutations associated with cancer in circulating fluids, especially circulating tumor cells (CTCs). The methods provide the advantage of identifying more of the mutations that are found in a tumor and clonal as well as subclonal mutations, in a single test, rather than multiple tests that would be required, if effective at all, that utilize tumor samples.

Accordingly, in one teaching, the present disclosure provides a method for monitoring and detection of early relapse or metastasis of a tumor in a cancer patient, by obtaining one or more circulating cells determined to originate from the tumor, wherein the method comprises the steps of:

a) selecting one or more patient-specific mutations based on mutations identified in a tumor sample of a patient who has been diagnosed with a cancer;

b) longitudinally collecting one or more blood samples from the patient after the patient has been treated with surgery, first-line chemotherapy, and/or adjuvant therapy;

c) generating a first set of amplicons from cellular DNA isolated from one or more circulating cells suspected to be circulating tumor cells obtained from the blood samples obtained from the cancer patient, and a second set of amplicons from cell free DNA, wherein the tumor cells and the normal cells are obtained from each of the blood samples or fractions thereof from the cancer patient, wherein the first set of amplicons and the second set of amplicons are obtained by performing a multiplex amplification reaction of the patient-specific mutations associated with cancer;

d) sequencing the first set of amplicons and the second set of amplicons by next-generation sequencing;

e) determining the origin of the one or more circulating cells based on the sequences of the first set of amplicons, wherein the sequences of the second set of amplicons are used as reference, wherein detection of one or more patient-specific mutations from first set of amplicons generated from the circulating cell determined to originate from the tumor is indicative of early relapse or metastasis of the cancer.

In another teaching, the disclosure herein may provide a method comprising the steps of:

a) longitudinally collecting one or more blood samples from a cancer patient after the patient has been treated with surgery, first-line chemotherapy, and/or adjuvant therapy;

b) generating a first set of amplicons from cellular DNA isolated from one or more circulating cells suspected to be circulating tumor cells obtained from the blood samples obtained from the cancer patient, and a second set of amplicons from cell free DNA, wherein the tumor cells and the normal cells are obtained from each of the blood samples or fractions thereof from the cancer patient, wherein the first set of amplicons and the second set of amplicons are obtained by performing a multiplex amplification reaction of the patient-specific mutations associated with cancer;

c) sequencing the first set of amplicons and the second set of amplicons by next-generation sequencing;

d) determining the origin of the one or more circulating cells based on the sequences of the first set of amplicons, wherein the sequences of the second set of amplicons are used as reference, wherein detection of one or more patient-specific mutations from first set of amplicons generated from the circulating cell determined to originate from the tumor is indicative of early relapse or metastasis of the cancer.

In some examples, the present disclosure may provide a compound for use in the treatment of cancer, wherein the compound is known to be effective in treating the cancer having the one or more patient-specific mutations detected from the blood samples.

The improved methods provided herein allows for accurate analysis and characterization of samples containing very few CTCs. The number of CTCs in the sample analyzed by the present methods may be less than 100 cells, less than 75 cells, less than 50 cells, less than 25 cells, less than 20 cells, less than 15 cells, less than 10 cells, less than 5 cells, or a single cell. By using the methods provided herein, accurate genomic information may be obtained from a single circulating tumor cell, 2 CTCs, 3 CTCs, 4 CTCs, 5 CTCs, 6 CTCs, 7 CTCs, 8 CTCs, 9 CTCs, or 10 CTCs.

In some examples, the method comprises monitoring and detecting early relapse or metastasis of a tumor in a cancer patient by determining the identity of a circulating cell suspected to be a circulating tumor cell based on pre-determined patient-specific mutations. In some examples, the patient specific mutations comprise single nucleotide variants (SNV), copy number variants (CNV), indels, deletions, or gene fusions associated with cancer. In some examples, the presence of at least 2 patient-specific mutations associated with cancer is indicative that the one or more circulating cells are tumor cells. In some examples, the presence of at least 8 patient-specific mutations associated with cancer is indicative that the one or more circulating cells are tumor cells. In some examples, the presence at least 16 patient-specific mutations associated with cancer is indicative that the one or more circulating cells are tumor cells. In some examples, the presence of at least 50 patient-specific mutations associated with cancer is indicative that the one or more circulating cells are tumor cells. In some examples, the presence of at least 100 patient-specific mutations associated with cancer is indicative that the one or more circulating cells are tumor cells. In some examples, the presence of at least 1000 patient-specific mutations associated with cancer is indicative that the one or more circulating cells are tumor cells. In some examples, the presence of at least 5000 patient-specific mutations associated with cancer is indicative that the one or more circulating cells are tumor cells. In some examples, the presence of at least 10000 patient-specific mutations associated with cancer is indicative that the one or more circulating cells are tumor cells. In some examples, the presence of at least 15000 patient-specific mutations associated with cancer is indicative that the one or more circulating cells are tumor cells.

In some examples, the presence of at least 100 to about 1000 patient-specific mutations associated with cancer is indicative that the one or more circulating cells are tumor cells. In some embodiments, the presence of at least 100 to about 1000 patient-specific mutations associated with cancer is indicative that the one or more circulating cells are tumor cells. In some examples, the presence of at least 1000 to about 5000 patient-specific mutations associated with cancer is indicative that the one or more circulating cells are tumor cells. In some examples, the presence of at least 5000 to about 10000 patient-specific mutations associated with cancer is indicative that the one or more circulating cells are tumor cells. In some examples, the presence of at least 10000 to about 15000 patient-specific mutations associated with cancer is indicative that the one or more circulating cells are tumor cells. In some examples, the presence of at least 15000 to about 20000 patient-specific mutations associated with cancer is indicative that the one or more circulating cells are tumor cells. In some examples, the presence of at least 20000 to about 25000 patient-specific mutations associated with cancer is indicative that the one or more circulating cells are tumor cells. In some examples, the presence of at least 50000 patient-specific mutations associated with cancer is indicative that the one or more circulating cells are tumor cells.

In some examples, the presence of at least 5000 patient-specific mutations associated with cancer is indicative that the one or more circulating cells are tumor cells. In some examples, the presence of at least 10000 patient-specific mutations associated with cancer is indicative that the one or more circulating cells are tumor cells. In some examples, the presence of at least 15000 patient-specific mutations associated with cancer is indicative that the one or more circulating cells are tumor cells. In some examples, the presence of at least 25000 patient-specific mutations associated with cancer is indicative that the one or more circulating cells are tumor cells.

In one teaching, multiplex amplification is performed to obtain amplicons that encompasses the patient specific mutations associated with cancer. In some examples, the first set of amplicons and the second set of amplicons are obtained by performing a multiplex amplification reaction of 50 to 1000 patient-specific mutations associated with cancer.

In one teaching, the method for monitoring and detection of early relapse or metastasis of a tumor in a cancer patient comprises separating multiple circulating cells into individual reaction volumes, isolating cellular DNA in each reaction volume, and attaching a sample barcode to the cellular DNA isolated in each reaction volume. In some examples, the cellular DNA is isolated from a single circulating cell suspected to be a tumor cell.

In some examples, the method further comprises performing non-invasive cancer testing using the genotypes of one or more circulating cells determined to be one or more tumor cells.

The methods and compositions can be helpful on their own, or they can be helpful when used along with other methods for detection, diagnosis, staging, screening, treatment, and management of cancer (e.g., breast cancer, bladder cancer, or colorectal cancer), for example to help support the results of these other methods to provide more confidence and/or a definitive result.

The terms "cancer" and "cancerous" refer to or describe the physiological condition in animals that is typically characterized by unregulated cell growth. A "tumor" comprises one or more cancerous cells. There are several main types of cancer. Carcinoma is a cancer that begins in the skin or in tissues that line or cover internal organs. Sarcoma is a cancer that begins in bone, cartilage, fat, muscle, blood vessels, or other connective or supportive tissue. Leukemia is a cancer that starts in blood-forming tissue, such as the bone marrow, and causes large numbers of abnormal blood cells to be produced and enter the blood. Lymphoma and multiple myeloma are cancers that begin in the cells of the immune system. Central nervous system cancers are cancers that begin in the tissues of the brain and spinal cord.

In some examples, the cancer comprises an acute lymphoblastic leukemia; acute myeloid leukemia; adrenocortical carcinoma; AIDS-related cancers; AIDS-related lymphoma; anal cancer; appendix cancer; astrocytomas; atypical teratoid/rhabdoid tumor; basal cell carcinoma; bladder cancer; brain stem glioma; brain tumor (including brain stem glioma, central nervous system atypical teratoid/rhabdoid tumor, central nervous system embryonal tumors, astrocytomas, craniopharyngioma, ependymoblastoma, ependymoma, medulloblastoma, medulloepithelioma, pineal parenchymal tumors of intermediate differentiation, supratentorial primitive neuroectodermal tumors and pineoblastoma); breast cancer; bronchial tumors; Burkitt lymphoma; cancer of unknown primary site; carcinoid tumor; carcinoma of unknown primary site; central nervous system atypical teratoid/rhabdoid tumor; central nervous system embryonal tumors; cervical cancer; childhood cancers; chordoma; chronic lymphocytic leukemia; chronic myelogenous leukemia; chronic myeloproliferative disorders; colon cancer; colorectal cancer; craniopharyngioma; cutaneous T-cell lymphoma; endocrine pancreas islet cell tumors; endometrial cancer; ependymoblastoma; ependymoma; esophageal cancer; esthesioneuroblastoma; Ewing sarcoma; extracranial germ cell tumor; extragonadal germ cell tumor; extrahepatic bile duct cancer; gallbladder cancer; gastric (stomach) cancer; gastrointestinal carcinoid tumor; gastrointestinal stromal cell tumor; gastrointestinal stromal tumor (GIST); gestational trophoblastic tumor; glioma; hairy cell leukemia; head and neck cancer; heart cancer; Hodgkin lymphoma; hypopharyngeal cancer; intraocular melanoma; islet cell tumors; Kaposi sarcoma; kidney cancer; Langerhans cell histiocytosis; laryngeal cancer; lip cancer; liver cancer; malignant fibrous histiocytoma bone cancer; medulloblastoma; medulloepithelioma; melanoma; Merkel cell carcinoma; Merkel cell skin carcinoma; mesothelioma; metastatic squamous neck cancer with occult primary; mouth cancer; multiple endocrine neoplasia syndromes; multiple myeloma; multiple myeloma/plasma cell neoplasm; mycosis fungoides; myelodysplastic syndromes; myeloproliferative neoplasms; nasal cavity cancer; nasopharyngeal cancer; neuroblastoma; Non-Hodgkin lymphoma;

nonmelanoma skin cancer; non-small cell lung cancer; oral cancer; oral cavity cancer; oropharyngeal cancer; osteosarcoma; other brain and spinal cord tumors; ovarian cancer; ovarian epithelial cancer; ovarian germ cell tumor; ovarian low malignant potential tumor; pancreatic cancer; papillomatosis; paranasal sinus cancer; parathyroid cancer; pelvic cancer; penile cancer; pharyngeal cancer; pineal parenchymal tumors of intermediate differentiation; pineoblastoma; pituitary tumor; plasma cell neoplasm/multiple myeloma; pleuropulmonary blastoma; primary central nervous system (CNS) lymphoma; primary hepatocellular liver cancer; prostate cancer; rectal cancer; renal cancer; renal cell (kidney) cancer; renal cell cancer; respiratory tract cancer; retinoblastoma; rhabdomyosarcoma; salivary gland cancer; Sezary syndrome; small cell lung cancer; small intestine cancer; soft tissue sarcoma; squamous cell carcinoma; squamous neck cancer; stomach (gastric) cancer; supratentorial primitive neuroectodermal tumors; T-cell lymphoma; testicular cancer; throat cancer; thymic carcinoma; thymoma; thyroid cancer; transitional cell cancer; transitional cell cancer of the renal pelvis and ureter; trophoblastic tumor; ureter cancer; urethral cancer; uterine cancer; uterine sarcoma; vaginal cancer; vulvar cancer; Waldenstrom macroglobulinemia; or Wilm's tumor.

In another example, provided herein is a method for detecting cancer (e.g., breast cancer, bladder cancer, or colorectal cancer) in a sample of blood or a fraction thereof from an individual, such as an individual suspected of having a cancer, that includes determining the single nucleotide variants present in a sample by determining the single nucleotide variants present in a sample comprising circulating tumor cells and ctDNA sample using a ctDNA SNV amplification/sequencing workflow provided herein. The presence of 1, 2, 3, 4, 5, 6, 7, 8, 9, 10, 11, 12, 13, 14, or 15 SNVs on the low end of the range, and 2, 3, 4, 5, 6, 7, 8, 9, 10, 11, 12, 13, 14, 15, 16, 17, 18, 19, 20, 21, 22, 23, 24, 25, 30, 40, or 50 SNVs on the high end of the range, in the sample at the plurality of single nucleotide loci is indicative of the presence of cancer (e.g., breast cancer, bladder cancer, or colorectal cancer).

In certain examples, the cancer is a stage 1a, 1b, or 2a breast cancer, bladder cancer, or colorectal cancer. In certain examples, the cancer is a stage 1a or 1b breast cancer, bladder cancer, or colorectal cancer. In certain examples, the individual is not subjected to surgery. In certain examples, the individual is not subjected to a biopsy. In certain examples of any of the methods of the present disclosure, before a targeted amplification is performed on CTCs from an individual, data is provided on SNVs that are found in a tumor from the individual. Accordingly, in these examples, a SNV amplification/sequencing reaction is performed on one or more tumor samples from the individual. In these methods, the CTC SNV amplification/sequencing reaction provided herein is still advantageous because it provides a liquid biopsy of clonal and subclonal mutations. Furthermore, as provided herein, clonal mutations can be more unambiguously identified in an individual that has cancer (e.g., breast cancer, bladder cancer, or colorectal cancer), if a high VAF percentage, for example, more than 1, 2, 3, 4, 5, 6, 7, 8, 9, 10% VAF in a ctDNA sample from the individual is determined for an SNV.

In illustrative examples, of any of the methods herein the set of single nucleotide variance loci includes all of the single nucleotide variance loci identified in the TCGA and COSMIC data sets for cancer (e.g., breast cancer, bladder cancer, or colorectal cancer).

In certain examples of any of the methods herein the set of single nucleotide variant loci include 2, 3, 4, 5, 6, 7, 8, 9, 10, 15, 20, 25, 30, 40, 50, 75, 100, 250, 500, 1000, 2500, 5000, or 10,000 single nucleotide variance loci known to be associated with cancer (e.g., breast cancer, bladder cancer, or colorectal cancer) on the low end of the range, and, 5, 6, 7, 8, 9, 10, 15, 20, 25, 30, 40, 50, 75, 100, 250, 500, 1000, 2500, 5000, 10,000, 20,000 and 25,000 on the high end of the range.

In another example, provided herein is a method for supporting a cancer (e.g., breast cancer, bladder cancer, or colorectal cancer) diagnosis for an individual, such as an individual suspected of having cancer (e.g., breast cancer, bladder cancer, or colorectal cancer), from a sample of blood or a fraction thereof from the individual, that includes performing a CTC SNV amplification/sequencing workflow as provided herein, to determine whether one or more single nucleotide variants are present in the plurality of single nucleotide variant loci. In this example, the following elements, statements, guidelines or rules apply: the absence of a single nucleotide variant supports a diagnosis of stage 1a, 1b, or 2a adenocarcinoma, the presence of a single nucleotide variant supports a diagnosis of squamous cell carcinoma or a stage 2b or 3a adenocarcinoma, and/or the presence of ten or more single nucleotide variants supports a diagnosis of squamous cell carcinoma or a stage 2b or 3 adenocarcinomas.

In certain examples, the method further includes determining a treatment plan and/or therapy that targets the one or more clonal single nucleotide variants. In certain examples, subclonal and/or other clonal SNVs are not targeted by therapy. Specific therapies and associated mutations are provided in other sections of this specification and are known in the art. Accordingly, in certain examples, the the present disclosure may provide a compound for use in the treatment of cancer, where the compound is known to be specifically effective in treating cancer (e.g., breast cancer, bladder cancer, or colorectal cancer) having one or more of the determined single nucleotide variants.

In certain examples, methods herein for detecting SNVs can be used to direct a therapeutic regimen. Therapies are available and under development that target specific mutations associated with ADC and SCC (Nature Review Cancer. 14:535-551 (2014). For example, detection of an EGFR mutation at L858R or T790M can be informative for selecting a therapy. Erlotinib, gefitinib, afatinib, AZK9291, CO-1686, and HM61713 are current therapies approved in the U.S. or in clinical trials, that target specific EGFR mutations. In another example, a G12D, G12C, or G12V mutation in KRAS can be used to direct an individual to a therapy of a combination of Selumetinib plus docetaxel. As another example, a mutation of V600E in BRAF can be used to direct a subject to a treatment of Vemurafenib, dabrafenib, and trametinib.

Target genes of the present disclosure in exemplary examples, are cancer-related genes, and in many illustrative examples, cancer-related genes. A cancer-related gene (for example, a cancer-related gene or a bladder cancer-related gene or a colorectal cancer-related gene) refers to a gene associated with an altered risk for a cancer (e.g. breast cancer, bladder cancer, or colorectal cancer) or an altered prognosis for a cancer. Exemplary cancer-related genes that promote cancer include oncogenes; genes that enhance cell proliferation, invasion, or metastasis; genes that inhibit apoptosis; and pro-angiogenesis genes. Cancer-related genes that inhibit cancer include, but are not limited to, tumor suppressor genes; genes that inhibit cell proliferation, invasion, or metastasis; genes that promote apoptosis; and anti-angiogenesis genes.

An example of the mutation detection method begins with the selection of the region of the gene that becomes the target. The region with known mutations is used to develop primers for mPCR-NGS to amplify and detect the mutation.

Methods provided herein can be used to detect virtually any type of mutation, especially mutations known to be associated with cancer and most particularly the methods provided herein are directed to mutations, especially SNVs, associated with cancer, specifically breast cancer, bladder cancer, or colorectal cancer. Exemplary SNVs can be in one or more of the following genes: EGFR, FGFR1, FGFR2, ALK, MET, ROS1, NTRK1, RET, HER2, DDR2, PDGFRA, KRAS, NF1, BRAF, PIK3CA, MEK1, NOTCH1, MLL2, EZH2, TET2, DNMT3A, SOX2, MYC, KEAP1, CDKN2A, NRG1, TP53, LKB1, and PTEN, which have been identified in various lung cancer samples as being mutated, having increased copy numbers, or being fused to other genes and combinations thereof (Non-small-cell lung cancers: a heterogeneous set of diseases. Chen et al. Nat. Rev. Cancer. 2014 Aug 14(8):535-551). In another example, the list of genes is those listed above, where SNVs have been reported, such as in the cited Chen et al. reference.

## 8. Exemplary Examples of Analytical Methods

### 8.1 Exemplary Examples for Detecting Cancer

In certain examples, the analyzing step in the method for determining whether circulating tumor nucleic acids are present, includes analyzing a set of chromosome segments known to exhibit aneuploidy in cancer. In certain examples, the analyzing step in the method for determining whether circulating tumor nucleic acids are present, includes analyzing between 1,000 and 50,000 or between 100 and 1000, polymorphic loci for ploidy. In certain examples, the analyzing step in the method for determining whether circulating tumor nucleic acids are present, includes analyzing between 100 and 1000 single nucleotide variant sites. For example, in these examples the analyzing step can include performing a multiplex PCR to amplify amplicons across the 1000 to 50,000 polymeric loci and the 100 to 1000 single nucleotide variant sites. This multiplex reaction can be set up as a single reaction or as pools of different subset multiplex reactions. The multiplex reaction methods provided herein, such as the massive multiplex PCR disclosed herein provide an exemplary process for carrying out the amplification reaction to help attain improved multiplexing and therefore, sensitivity levels.

In certain examples, the multiplex PCR reaction is carried out under limiting primer conditions for at least 10%, 20%, 25%, 50%, 75%, 90%, 95%, 98%, 99%, or 100% of the reactions. Improved conditions for performing the massive multiplex reaction provided herein can be used.

In certain teachings, the above method for determining whether circulating tumor nucleic acids are present in a sample in an individual, and all examples thereof, can be carried out with a system. The disclosure provides teachings regarding specific functional and structural features to carry out the methods. As a non-limiting example, the system includes the following:

An input processor configured to analyze data from the sample to determine a ploidy at a set of polymorphic loci on a chromosome segment in the individual; and

A modeler configured to determine the level of allelic imbalance present at the polymorphic loci based on the ploidy determination, wherein an allelic imbalance equal to or greater than 0.5% is indicative of the presence of circulating.

### 8.2 Exemplary Examples for Detecting Single Nucleotide Variants

In certain teachings, provided herein are methods for detecting single nucleotide variants in a sample. The improved methods provided herein can achieve limits of detection of 0.015, 0.017, 0.02, 0.05, 0.1, 0.2, 0.3, 0.4 or 0.5 percent SNV in a sample. All the examples for detecting SNVs can be carried out with a system. The disclosure provides teachings regarding specific functional and structural features to carry out the methods. Furthermore, provided herein are examples comprising a nontransitory computer readable medium comprising computer readable code that, when executed by a processing device, causes the processing device to carry out the methods for detecting SNVs provided herein.

Accordingly, provided herein in one example, is a method for determining whether a single nucleotide variant is present at a set of genomic positions in a sample from an individual, the method comprising: for each genomic position, generating an estimate of efficiency and a per cycle error rate for an amplicon spanning that genomic position, using a training data set; receiving observed nucleotide identity information for each genomic position in the sample; determining a set of probabilities of single nucleotide variant percentage resulting from one or more real mutations at each genomic position, by comparing the observed nucleotide identity information at each genomic position to a model of different variant percentages using the estimated amplification efficiency and the per cycle error rate for each genomic position independently; and determining the most-likely real variant percentage and confidence from the set of probabilities for each genomic position.

In illustrative examples of the method for determining whether a single nucleotide variant is present, the estimate of efficiency and the per cycle error rate is generated for a set of amplicons that span the genomic position. For example, 2, 3, 4, 5, 10, 15, 20, 25, 50, 100 or more amplicons can be included that span the genomic position.

In illustrative examples of the method for determining whether a single nucleotide variant is present, the observed nucleotide identity information comprises an observed number of total reads for each genomic position and an observed number of variant allele reads for each genomic position.

In illustrative examples of the method for determining whether a single nucleotide variant is present, the sample is a plasma sample and the single nucleotide variant is present in circulating tumor DNA of the sample. In illustrative examples of the method for determining whether a single nucleotide variant is present, the sample is a plasma sample and the single nucleotide variant is present in circulating fetal DNA of the sample.

In another example provided herein is a method for estimating the percent of single nucleotide variants that are present in a sample from an individual. The method includes the following steps: at a set of genomic positions, generating an estimate of efficiency and a per cycle error rate for one or more amplicon spanning those genomic positions, using a training data set; receiving observed nucleotide identity information for each genomic position in the sample; generating an estimated mean and variance for the total number of molecules, background error molecules and real mutation molecules for a search space comprising an initial percentage of real mutation molecules using the amplification efficiency and the per cycle error rate of the amplicons; and determining the percentage of single nucleotide variants present in the sample resulting from real mutations by determining a most-likely real single nucleotide variant percentage by fitting a distribution using the estimated means and variances to an observed nucleotide identity information in the sample.

The training data set for this example of the present disclosure typically includes samples from one or preferably a group of healthy individuals. In certain illustrative embodiments, the training data set is analyzed on the same day or even on the same run as one or more on-test samples. For example, samples from a group of 2, 3, 4, 5, 10, 15, 20, 25, 30, 36, 48, 96, 100, 192, 200, 250, 500, 1000 or more healthy individuals can be used to generate the training data set. Where data is available for larger number of healthy individuals, e.g. 96 or more, confidence increases for amplification efficiency estimates even if runs are performed in advance of performing the method for on-test samples. The PCR error rate can use nucleic acid sequence information generated not only for the SNV base location, but for the entire amplified region around the SNV, since the error rate is per amplicon. For example, using samples from 50 individuals and sequencing a 20 base pair amplicon around the SNV, error frequency data from 1000 base reads can be used to determine error frequency rate.

Typically, the amplification efficiency is estimating by estimating a mean and standard deviation for amplification efficiency for an amplified segment and then fitting that to a distribution model, such as a binomial distribution or a beta binomial distribution. Error rates are determined for a PCR reaction with a known number of cycles and then a per cycle error rate is estimated.

In certain illustrative examples, estimating the starting molecules of the test data set further includes updating the estimate of the efficiency for the testing data set using the starting number of molecules estimated in step (b) if the observed number of reads is significantly different than the estimated number of reads. Then the estimate can be updated for a new efficiency and/or starting molecules.

The search space used for estimating the total number of molecules, background error molecules and real mutation molecules can include a search space from 0.1%, 0.2%, 0.25%, 0.5%, 1%, 2.5%, 5%, 10%, 15%, 20%, or 25% on the low end and 1%, 2%, 2.5%, 5%, 10%, 12.5%, 15%, 20%, 25%, 50%, 75%, 90%, or 95% on the high end copies of a base at an SNV position being the SNV base. Lower ranges, 0.1%, 0.2%, 0.25%, 0.5%, or 1% on the low end and 1%, 2%, 2.5%, 5%, 10%, 12.5%, or 15% on the high end can be used in illustrative examples for plasma samples where the method is detecting circulating tumor DNA. Higher ranges are used for tumor samples.

A distribution is fit to the number of total error molecules (background error and real mutation) in the total molecules to calculate the likelihood or probability for each possible real mutation in the search space. This distribution could be a binomial distribution or a beta binomial distribution.

The most likely real mutation is determined by determining the most likely real mutation percentage and calculating the confidence using the data from fitting the distribution. As an illustrative example and not intended to limit the clinical interpretation of the methods provided herein, if the mean mutation rate is high then the percent confidence needed to make a positive determination of an SNV is lower. For example, if the mean mutation rate for an SNV in a sample using the most likely hypothesis is 5% and the percent confidence is 99%, then a positive SNV call would be made. On the other hand, for this illustrative example, if the mean mutation rate for an SNV in a sample using the most likely hypothesis is 1% and the percent confidence is 50%, then in certain situations a positive SNV call would not be made. It will be understood that clinical interpretation of the data would be a function of sensitivity, specificity, prevalence rate, and alternative product availability.

In another example, provided herein is a method for detecting one or more single nucleotide variants in a test sample from an individual. The method according to this example, includes the following steps:

determining a median variant allele frequency for a plurality of control samples from each of a plurality of normal individuals, for each single nucleotide variant position in a set of single nucleotide variance positions based on results generated in a sequencing run, to identify selected single nucleotide variant positions having variant median allele frequencies in normal samples below a threshold value and to determine background error for each of the single nucleotide variant positions after removing outlier samples for each of the single nucleotide variant positions; determining an observed depth of read weighted mean and variance for the selected single nucleotide variant positions for the test sample based on data generated in the sequencing run for the test sample; and identifying using a computer, one or more single nucleotide variant positions with a statistically significant depth of read weighted mean compared to the background error for that position, thereby detecting the one or more single nucleotide variants.

In certain examples of this method for detecting one or more SNVs the plurality of control samples comprises at least 25 samples. In certain illustrative examples, the plurality of control samples is at least 5, 10, 15, 20, 25, 50, 75, 100, 200, or 250 samples on the low end and 10, 15, 20, 25, 50, 75, 100, 200, 250, 500, and 1000 samples on the high end.

In certain examples of this method for detecting one or more SNVs, outliers are removed from the data generated in the high throughput sequencing run to calculate the observed depth of read weighted mean and observed variance are determined. In certain examples of this method for detecting one or more SNVs the depth of read for each single nucleotide variant position for the test sample is at least 100 reads.

In certain examples of this method for detecting one or more SNVs the sequencing run comprises a multiplex amplification reaction performed under limited primer reaction conditions. Improved methods for performing multiplex amplification reactions provided herein, are used to perform these examples in illustrative examples.

Not to be limited by theory, methods of the present example utilize a background error model using normal plasma samples, that are sequenced on the same sequencing run as an on-test sample, to account for run-specific artifacts. Noisy positions with normal median variant allele frequencies above a threshold, for example > 0.1%, 0.2%, 0.25%, 0.5% 0.75%, and 1.0%, are removed.

Outlier samples are iteratively removed from the model to account for noise and contamination. For each base substitution of every genomic locus, the depth of read weighted mean and standard deviation of the error are calculated. In certain illustrative examples, samples, such as circulating fetal cell, circulating tumor cell, or cell-free plasma samples, with single nucleotide variant positions with at least a threshold number of reads, for example, at least 2, 3, 4, 5, 6, 7, 8, 9, 10, 15, 20, 25, 50, 100, 250, 500, or 1000 variant reads and a1 Z-score greater than 2.5, 5, 7.5 or 10 against the background error model in certain examples, are counted as a candidate mutation.

In certain examples, a depth of read of greater than 100, 250, 500, 1,000, 2000, 2500, 5000, 10,000, 20,000, 25,0000, 50,000, or 100,000 on the low end of the range and 2000, 2500, 5,000, 7,500, 10,000, 25,000, 50,000, 100,000, 250,000 or 500,000 reads on the high end, is attained in the sequencing run for each single nucleotide variant position in the set of single nucleotide variant positions. Typically, the sequencing run is a high throughput sequencing run. The mean or median values generated for the on-test samples, in illustrative examples are weighted by depth of reads. Therefore, the likelihood that a variant allele determination is real in a sample with 1 variant allele detected in 1000 reads is weighed higher than a sample with 1 variant allele detected in 10,000 reads. Since determinations of a variant allele (i.e. mutation) are not made with 100% confidence, the identified single nucleotide variant can be considered a candidate variant or a candidate mutation.

*8.3 Exemplary Test Statistic for Analysis of Phased Data*

An exemplary test statistic is described below for analysis of phased data from a sample known or suspected of being a mixed sample containing DNA or RNA that originated from two or more cells that are not genetically identical. Let $f$ denote the fraction of DNA or RNA of interest, for example the fraction of DNA or RNA with a CNV of interest, or the fraction of DNA or RNA from cells of interest, such as cancer cells. In some examples for cancer testing, $f$ denotes the fraction of DNA or RNA from cancer cells in a mixture of cancer and normal cells, or $f$ denotes the fraction of cancer cells in a mixture of cancer and normal cells. Note that this refers to the fraction of DNA from cells of interest assuming two copies of DNA are given by each cell of interest. This differs from the DNA fraction from cells of interest at a segment that is deleted or duplicated.

The possible allelic values of each SNP are denoted A and B. AA, AB, BA, and BB are used to denote all possible ordered allele pairs. In some examples, SNPs with ordered alleles AB or BA are analyzed. Let $N_i$ denote the number of sequence reads of the ith SNP, and $A_i$ and $B_i$ denote the number of reads of the ith SNP that indicate allele A and B, respectively. It is assumed:

$$N_i = A_i + B_i.$$

The allele ratio $R_i$ is defined:

$$R_i \triangleq \frac{A_i}{N_i}.$$

Let T denote the number of SNPs targeted.

Without loss of generality, some examples focus on a single chromosome segment. As a matter of further clarity, in this specification the phrase "a first homologous chromosome segment as compared to a second homologous chromosome segment" means a first homolog of a chromosome segment and a second homolog of the chromosome segment. In some such examples, all of the target SNPs are contained in the segment chromosome of interest. In other examples, multiple chromosome segments are analyzed for possible copy number variations.

*MAP Estimation*

This method leverages the knowledge of phasing via ordered alleles to detect the deletion or duplication of the target segment. For each SNP i, define

$$X_i \triangleq \begin{cases} 1 & R_i < 0.5 \text{ and SNP } i \text{ } AB \\ 0 & R_i \geq 0.5 \text{ and SNP } i \text{ } AB \\ 0 & R_i < 0.5 \text{ and SNP } i \text{ } BA \\ 1 & R_i \geq 0.5 \text{ and SNP } i \text{ } BA \end{cases}$$

Then define

$$S \triangleq \sum_{All\ SNPs} X_i.$$

The distributions of the $X_i$ and S under various copy number hypotheses (such as hypotheses for disomy, deletion of the first or second homolog, or duplication of the first or second homolog) are described below.

*Disomy Hypothesis*

Under the hypothesis that the target segment is not deleted or duplicated,

$$X_i = \begin{cases} 0 & wp\ 1 - p\left(\frac{1}{2}, N_i\right) \\ 1 & wp\ p\left(\frac{1}{2}, N_i\right) \end{cases}$$

where

$$p(b, n) \triangleq Pr\left\{X \sim Bino(b, n) \geq \frac{n}{2}\right\}.$$

If we assume a constant depth of read N, this gives us a Binomial distribution S with parameters

$$p\left(\frac{1}{2}, N\right)$$ and T.

*Deletion Hypotheses*

Under the hypothesis that the first homolog is deleted *(i.e., an AB SNP becomes B, and a BA SNP becomes A),* then $R_i$ has a Binomial distribution with parameters $1 - \frac{1}{2-f}$ and T for AB SNPs, and $\frac{1}{2-f}$ and T for BA SNPs. Therefore,

$$X_i = \begin{cases} 0 & wp\ 1 - p\left(\frac{1}{2-f}, N_i\right) \\ 1 & wp\ p\left(\frac{1}{2-f}, N_i\right) \end{cases}$$

If we assume a constant depth of read N, this gives a Binomial distribution S with parameters

$$p\left(\frac{1}{2-f}, N\right)$$ and T.

Under the hypothesis that the second homolog is deleted *(i.e., an AB SNP becomes A, and a BA SNP becomes B),* then $R_i$ has a Binomial distribution with parameters $\frac{1}{2-f}$ and T for AB SNPs, and $1 - \frac{1}{2-f}$ and T for BA SNPs. Therefore,

$$X_i = \begin{cases} 0 & wp\ p\left(\frac{1}{2-f}, N_i\right) \\ 1 & wp\ 1 - p\left(\frac{1}{2-f}, N_i\right) \end{cases}$$

If we assume a constant depth of read N, this gives a Binomial distribution S with parameters

$$1 - p\left(\frac{1}{2-f}, N\right)$$ and T.

*Duplication Hypotheses*

Under the hypothesis that the first homolog is duplicated *(i.e., an AB SNP becomes AAB, and a BA SNP becomes BBA),* then $R_i$ has a Binomial distribution with parameters $\frac{1+f}{2+f}$ and T for AB SNPs, and $1 - \frac{1+f}{2+f}$ and T for BA SNPs. Therefore,

$$X_i = \begin{cases} 0 & wp\ p\left(\frac{1+f}{2+f}, N_i\right) \\ 1 & wp\ 1 - p\left(\frac{1+f}{2+f}, N_i\right) \end{cases}$$

If we assume a constant depth of read N, this gives us a Binomial distribution S with parameters

$$1 - p\left(\frac{1+f}{2+f}, N\right)$$ and T.

Under the hypothesis that the second homolog is duplicated *(i.e., an AB SNP becomes ABB, and a BA SNP becomes BAA),* then $R_i$ has a Binomial distribution with parameters $1 - \frac{1+f}{2+f}$ and T for AB SNPs, and $\frac{1+f}{2+f}$ and T for BA SNPs. Therefore,

$$X_i = \begin{cases} 0 & wp \; 1 - p\left(\frac{1+f}{2+f}, N_i\right) \\ 1 & wp \; p\left(\frac{1+f}{2+f}, N_i\right) \end{cases}$$

If we assume a constant depth of read N, this gives a Binomial distribution S with parameters $p\left(\frac{1+f}{2+f}, N\right)$ and T.

*Classification*

As demonstrated in the sections above, $X_i$ is a binary random variable with

$$Pr\{X_1 = 1\} = \begin{cases} p\left(\frac{1}{2}, N_i\right) & given\ disomy \\ p\left(\frac{1}{2-f}, N_i\right) & homolog\ 1\ deletion \\ 1 - p\left(\frac{1}{2-f}, N_i\right) & homolog\ 2\ deletion \\ 1 - p\left(\frac{1+f}{2+f}, N_i\right) & homolog\ 1\ duplication \\ p\left(\frac{1+f}{2+f}, N_i\right) & homolog\ 2\ duplication \end{cases}$$

This allows one to calculate the probability of the test statistic S under each hypothesis. The probability of each hypothesis given the measured data can be calculated. In some examples, the hypothesis with the greatest probability is selected. If desired, the distribution on S can be simplified by either approximating each $N_i$ with a constant depth of reach N or by truncating the depth of reads to a constant N. This simplification gives

$$S \sim \begin{cases} Bino\left(p\left(\frac{1}{2}, N\right), T\right) & given\ disomy \\ Bino\left(p\left(\frac{1}{2-f}, N\right), T\right) & homolog\ 1\ deletion \\ Bino\left(1 - p\left(\frac{1}{2-f}, N\right), T\right) & homolog\ 2\ deletion \\ Bino\left(1 - p\left(\frac{1+f}{2+f}, N\right), T\right) & homolog\ 1\ duplication \\ Bino\left(p\left(\frac{1+f}{2+f}, N\right), T\right) & homolog\ 2\ duplication \end{cases}$$

The value for *f* can be estimate by selecting the most likely value *off* given the measured data, such as the value of *f* that generates the best data fit using an algorithm *(e.g.,* a search algorithm) such as maximum likelihood estimation, maximum a-posteriori estimation, or Bayesian estimation. In some examples, multiple chromosome segments are analyzed and a value for *f* is estimated based on the data for each segment. If all the target cells have these duplications or deletions, the estimated values for*f*based on data for these different segments are similar. In some examples, *f* is experimentally measured such as by determining the fraction of DNA or RNA from cancer cells based on methylation differences (hypomethylation or hypermethylation) between cancer and non-cancerous DNA or RNA.

*Single Hypothesis Rejection*

The distribution of *S* for the disomy hypothesis does not depend on *f*. Thus, the probability of the measured data can be calculated for the disomy hypothesis without calculating *f*. A single hypothesis rejection test can be used for the null hypothesis of disomy. In some examples, the probability of S under the disomy hypothesis is calculated, and the hypothesis of disomy is rejected if the probability is below a given threshold value (such as less than 1 in 1,000). This indicates that a duplication or deletion of the chromosome segment is present. If desired, the false positive rate can be altered by adjusting the threshold value.

*8.4 Exemplary Methods for Analysis of Phased Data*

Exemplary methods are described below for analysis of data from a sample known or suspected of being a mixed sample containing DNA or RNA that originated from two or more cells that are not genetically identical. In some examples, phased data is used. In some examples, the method involves determining, for each calculated allele ratio, whether the calculated allele ratio is above or below the expected allele ratio and the magnitude of the difference for a particular locus. In some examples, a likelihood distribution is determined for the allele ratio at a locus for a particular hypothesis and the closer the calculated allele ratio is to the center of the likelihood distribution, the more likely the hypothesis is correct. In some examples, the method involves determining the likelihood that a hypothesis is correct for each locus. In some examples, the method involves determining the likelihood that a hypothesis is correct for each locus, and combining the probabilities of that hypothesis for each locus, and the hypothesis with the greatest combined probability is selected. In some examples, the method involves determining the likelihood that a hypothesis is correct for each locus and for each possible ratio of DNA or RNA from the one or more target cells to the total DNA or RNA in the sample. In some examples, a combined probability for each hypothesis is determined by combining the probabilities of that hypothesis for each locus and each possible ratio, and the hypothesis with the greatest combined probability is selected.

In one example, the following hypotheses are considered: $H_{11}$ (all cells are normal), $H_{10}$ (presence of cells with only homolog 1, hence homolog 2 deletion), $H_{01}$ (presence of cells with only homolog 2, hence homolog 1 deletion), $H_{21}$ (presence of cells with homolog 1 duplication), $H_{12}$ (presence of cells with homolog 2 duplication). For a fraction *f* of target cells such as cancer cells or mosaic cells (or the fraction of DNA or RNA from the target cells), the expected allele ratio for heterozygous (*AB* or *BA*) SNPs can be found as follows:

**Equation (1):**

$$r(AB, H_{11}) = r(BA, H_{11}) \quad = \quad 0.5,$$

$$r(AB, H_{10}) = r(BA, H_{01}) \quad = \quad \frac{1}{2 - f},$$

$$r(AB, H_{01}) = r(BA, H_{10}) \quad = \quad \frac{1 - f}{2 - f},$$

$$r(AB, H_{21}) = r(BA, H_{12}) \quad = \quad \frac{1 + f}{2 + f},$$

$$r(AB, H_{12}) = r(BA, H_{21}) \quad = \quad \frac{1}{2 + f}.$$

**Bias, Contamination, and Sequencing Error Correction:**

The observation $D_s$ at the SNP consists of the number of original mapped reads with each allele present, $n_A{}^0$ and $n_B{}^0$. Then, we can find the corrected reads $n_A$ and $n_B$ using the expected bias in the amplification of *A* and B alleles.

Let $c_a$ to denote the ambient contamination (such as contamination from DNA in the air or environment) and $r(c_a)$ to denote the allele ratio for the ambient contaminant (which is taken to be 0.5 initially). Moreover, $c_g$ denotes the genotyped contamination rate (such as the contamination from another sample), and $r(c_g)$ is the allele ratio for the contaminant. Let $s_e$ (*A, B*) and $s_e(B,A)$ denote the sequencing errors for calling one allele a different allele (such as by erroneously detecting an A allele when a B allele is present).

One can find the observed allele ratio $q(r, c_a, r(c_a), c_g, r(c_g), s_e(A,B), s_e(B,A))$ for a given expected allele ratio *r* by correcting for ambient contamination, genotyped contamination, and sequencing error.

Since the contaminant genotypes are unknown, population frequencies can be used to find $P(r(c_g))$. More specifically, let $p$ be the population frequency for one of the alleles (which may be referred to as a reference allele). Then, we have $P(r(c_g) = 0) = (1-p)^2$, $P(r(c_g) = 0) = 2p(1-p)$, and $P(r(cg) = 0) = p^2$. The conditional expectation over $r(c_g)$ can be used to determine the $E[q(r, c_a, r(c_a), c_g, r(c_g), s_e(A,B), s_e(B,A))]$. Note that the ambient and genotyped contamination are determined using the homozygous SNPs, hence they are not affected by the absence or presence of deletions or duplications. Moreover, it is possible to measure the ambient and genotyped contamination using a reference chromosome if desired.

**Likelihood at each SNP:**

The equation below gives the probability of observing $n_A$ and $n_B$ given an allele ratio r:
**Equation (2):**

$$P(n_A, n_B | r) = p_{bino}(n_A; n_A + n_B, r) = \binom{n_A + n_B}{n_A} r^{n_A} (1 - r)^{n_B}.$$

Let $D_s$ denote the data for SNP s. For each hypothesis h $\varepsilon$ { $H_{11}, H_{01}, H_{10}, H_{21}, H_{12}$ }, one can let $r=r(AB,h)$ or $r=r(BA,h)$ in the equation (1) and find the conditional expectation over $r(c_g)$ to determine the observed allele ratio $E[q(r, c_a, r(c_a), c_g, F(cg))]$. Then, letting $r= E[q(r, c_a, r(c_a), c_g, r(c_g), s_e(A,B), s_e(B,A))]$ in equation (2) one can determine $P(D_s | h_f)$.

**Search Algorithm:**

In some examples, SNPs with allele ratios that seem to be outliers are ignored (such as by ignoring or eliminating SNPs with allele ratios that are at least 2 or 3 standard deviations above or below the mean value). Note that an advantage identified for this approach is that in the presence of higher mosaicism percentage, the variability in the allele ratios may be high, hence this ensures that SNPs will not be trimmed due to mosaicism.

Let $F = \{f_1, ...., f_N\}$ denote the search space for the mosaicism percentage (such as the tumor or fetal fraction). One can determine $P(D_s | h,f)$ at each SNP s and $f \varepsilon F$, and combine the likelihood over all SNPs.

The algorithm goes over each $f$ for each hypothesis. Using a search method, one concludes that mosaicism exists if there is a range $F^*$ off where the confidence of the deletion or duplication hypothesis is higher than the confidence of the no deletion and no duplication hypotheses. In some examples, the maximum likelihood estimate for $P(D_s | h,f)$ in $F^*$ is determined. If desired, the conditional expectation over $f \varepsilon F^*$ may be determined. If desired, the confidence for each hypothesis can be determined.

In some examples, a beta binomial distribution is used instead of binomial distribution. In some embodiments, a reference chromosome or chromosome segment is used to determine the sample specific parameters of beta binomial.

*8.5 Exemplary Methods for Detecting Deletions and Duplications Without Phased Data*

In some examples, unphased genetic data is used to determine if there is an overrepresentation of the number of copies of a first homologous chromosome segment as compared to a second homologous chromosome segment in the genome of an individual (such as in the genome of one or more cells or in cfDNA or cfRNA). In some examples, phased genetic data is used but the phasing is ignored. In some examples, the sample of DNA or RNA is a mixed sample of cfDNA or cfRNA from the individual that includes cfDNA or cfRNA from two or more genetically different cells. In some examples, the method utilizes the magnitude of the difference between the calculated allele ratio and the expected allele ratio for each of the loci.

In some examples, the method involves obtaining genetic data at a set of polymorphic loci on the chromosome or chromosome segment in a sample of DNA or RNA from one or more cells from the individual by measuring the quantity of each allele at each locus. In some examples, allele ratios are calculated for the loci that are heterozygous in at least one cell from which the sample was derived. In some examples, the calculated allele ratio for a particular locus is the measured quantity of one of the alleles divided by the total measured quantity of all the alleles for the locus. In some examples, the calculated allele ratio for a particular locus is the measured quantity of one of the alleles (such as the allele on the first homologous chromosome segment) divided by the measured quantity of one or more other alleles (such as the allele on the second homologous chromosome segment) for the locus. The calculated allele ratios and expected allele ratios may be calculated using any of the methods described herein or any standard method (such as any mathematical transformation of the calculated allele ratios or expected allele ratios described herein).

In some examples, a test statistic is calculated based on the magnitude of the difference between the calculated allele ratio and the expected allele ratio for each of the loci. In some examples, the test statistic $\Delta$ is calculated using the

following formula

$$\Delta = \frac{\sum_{All\ Loci}(\delta_i - \mu_i)}{\sqrt{\sum_{All\ Loci}\sigma_i^2}}$$

wherein $\delta_i$ is the magnitude of the difference between the calculated allele ratio and the expected allele ratio for the ith loci;

wherein $\mu_i$ is the mean value of $\delta_i$; and

wherein $\sigma_i^2$ is the standard deviation of $\delta_i$.

For example, we can define $\delta_i$ as follows when the expected allele ratio is 0.5:

$$\delta_i \triangleq \left|\frac{1}{2} - R_i\right|.$$

Values for $\mu_i$ and $\sigma_i$ can be computed using the fact that $R_i$ is a Binomial random variable. In some examples, the standard deviation is assumed to be the same for all the loci. In some examples, the average or weighted average value of the standard deviation or an estimate of the standard deviation is used for the value of $\sigma_i^2$. In some examples, the test statistic is assumed to have a normal distribution. For example, the central limit theorem implies that the distribution of $\Delta$ converges to a standard normal as the number of loci (such as the number of SNPs *T*) grows large.

In some examples, a set of one or more hypotheses specifying the number of copies of the chromosome or chromosome segment in the genome of one or more of the cells are enumerated. In some examples, the hypothesis that is most likely based on the test statistic is selected, thereby determining the number of copies of the chromosome or chromosome segment in the genome of one or more of the cells. In some examples, a hypotheses is selected if the probability that the test statistic belongs to a distribution of the test statistic for that hypothesis is above an upper threshold; one or more of the hypotheses is rejected if the probability that the test statistic belongs to the distribution of the test statistic for that hypothesis is below an lower threshold; or a hypothesis is neither selected nor rejected if the probability that the test statistic belongs to the distribution of the test statistic for that hypothesis is between the lower threshold and the upper threshold, or if the probability is not determined with sufficiently high confidence. In some examples, an upper and/or lower threshold is determined from an empirical distribution, such as a distribution from training data (such as samples with a known copy number, such as diploid samples or samples known to have a particular deletion or duplication). Such an empirical distribution can be used to select a threshold for a single hypothesis rejection test. Note that the test statistic $\Delta$ is independent of S and therefore both can be used independently, if desired.

*8.6 Exemplary Methods for Detecting Deletions and Duplications Using Allele Distributions or Patterns*

This section includes methods for determining if there is an overrepresentation of the number of copies of a first homologous chromosome segment as compared to a second homologous chromosome segment. In some examples, the method involves enumerating (i) a plurality of hypotheses specifying the number of copies of the chromosome or chromosome segment that are present in the genome of one or more cells (such as cancer cells) of the individual or (ii) a plurality of hypotheses specifying the degree of overrepresentation of the number of copies of a first homologous chromosome segment as compared to a second homologous chromosome segment in the genome of one or more cells of the individual. In some examples, the method involves obtaining genetic data from the individual at a plurality of polymorphic loci (such as SNP loci) on the chromosome or chromosome segment. In some examples, a probability distribution of the expected genotypes of the individual for each of the hypotheses is created. In some examples, a data fit between the obtained genetic data of the individual and the probability distribution of the expected genotypes of the individual is calculated. In some examples, one or more hypotheses are ranked according to the data fit, and the hypothesis that is ranked the highest is selected. In some examples, a technique or algorithm, such as a search algorithm, is used for one or more of the following steps: calculating the data fit, ranking the hypotheses, or selecting the hypothesis that is ranked the highest. In some examples, the data fit is a fit to a beta-binomial distribution or a fit to a binomial distribution. In some examples, the technique or algorithm is selected from the group consisting of maximum likelihood estimation, maximum a-posteriori estimation, Bayesian estimation, dynamic estimation (such as dynamic Bayesian estimation), and expectation-maximization estimation. In some examples, the method includes applying the technique or algorithm to the obtained genetic data and the expected genetic data.

In some examples, the method involves enumerating (i) a plurality of hypotheses specifying the number of copies of the chromosome or chromosome segment that are present in the genome of one or more cells (such as cancer cells) of the individual or (ii) a plurality of hypotheses specifying the degree of overrepresentation of the number of copies of a first homologous chromosome segment as compared to a second homologous chromosome segment in the genome of one or more cells of the individual. In some examples, the method involves obtaining genetic data from the individual at a plurality of polymorphic loci (such as SNP loci) on the chromosome or chromosome segment. In some examples, the genetic data includes allele counts for the plurality of polymorphic loci. In some examples, a joint distribution model is created for the expected allele counts at the plurality of polymorphic loci on the chromosome or chromosome segment for each hypothesis. In some examples, a relative probability for one or more of the hypotheses is determined using the joint distribution model and the allele counts measured on the sample, and the hypothesis with the greatest probability is selected.

In some examples, the distribution or pattern of alleles (such as the pattern of calculated allele ratios) is used to determine the presence or absence of a CNV, such as a deletion or duplication. If desired the parental origin of the CNV can be determined based on this pattern.

*8.7 Exemplary Counting Methods/Quantitative Methods*

In some examples, one or more counting methods (also referred to as quantitative methods) are used to detect one or more CNS, such as deletions or duplications of chromosome segments or entire chromosomes. In some examples, one or more counting methods are used to determine whether the overrepresentation of the number of copies of the first homologous chromosome segment is due to a duplication of the first homologous chromosome segment or a deletion of the second homologous chromosome segment. In some examples, one or more counting methods are used to determine the number of extra copies of a chromosome segment or chromosome that is duplicated (such as whether there are 1, 2, 3, 4, or more extra copies). In some examples, one or more counting methods are used to differentiate a sample has many duplications and a smaller tumor fraction from a sample with fewer duplications and a larger tumor fraction. For example, one or more counting methods may be used to differentiate a sample with four extra chromosome copies and a tumor fraction of 10% from a sample with two extra chromosome copies and a tumor fraction of 20%. Exemplary methods are disclosed, e.g. U.S. Publication Nos. 2007/0184467; 2013/0172211; and 2012/0003637; U.S. Patent Nos. 8,467,976; 7,888,017; 8,008,018; 8,296,076; and 8,195,415; U.S. Serial No. 62/008,235, filed June 5, 2014, and U.S. Serial No. 62/032,785, filed August 4, 2014.

In some examples, the value of $f$ (such as tumor or fetal fraction) is used in the CNV determination, such as to compare the observed difference between the amount of two chromosomes or chromosome segments to the difference that would be expected for a particular type of CNV given the value of $f$ (see, *e.g.,* US Publication No 2012/0190020; US Publication No 2012/0190021; US Publication No 2012/0190557; US Publication No 2012/0191358). For example, the difference in the amount of a chromosome segment that is duplicated in a tumor compared to a disomic reference chromosome segment increases as the tumor fraction increases. In some examples, the method includes comparing the relative frequency of a chromosome or chromosome segment of interest to a reference chromosomes or chromosome segment (such as a chromosome or chromosome segment expected or known to be disomic) to the value of $f$ to determine the likelihood of the CNV. For example, the difference in amounts between the first chromosomes or chromosome segment to the reference chromosome or chromosome segment can be compared to what would be expected given the value of $f$ for various possible CNVs (such as one or two extra copies of a chromosome segment of interest).

*8.8 Exemplary Counting Methods/Quantitative Methods Using Reference Samples*

An exemplary quantitative method that uses one or more reference samples is described in U.S. Serial No. 62/008,235, filed June 5, 2014 and U.S. Serial No. 62/032,785, filed August 4, 2014. In some examples, one or more reference samples most likely to not have any CNVs on one or more chromosomes or chromosomes of interest (*e.g.,* a normal sample) are identified by selecting the samples with the highest fraction of tumor DNA, selecting the samples with the z-score closest to zero, selecting the samples where the data fits the hypothesis corresponding to no CNVs with the highest confidence or likelihood, selecting the samples known to be normal, selecting the samples from individuals with the lowest likelihood of having cancer (*e.g.,* having a low age, being a male when screening for breast cancer, having no family history, *etc.*), selecting the samples with the highest input amount of DNA, selecting the samples with the highest signal to noise ratio, selecting samples based on other criteria believed to be correlated to the likelihood of having cancer, or selecting samples using some combination of criteria. Once the reference set is chosen, one can make the assumption that these cases are disomic, and then estimate the per-SNP bias, that is, the experiment-specific amplification and other processing bias for each locus. Then, one can use this experiment-specific bias estimate to correct the bias in the measurements of the chromosome of interest, such as chromosome 21 loci, and for the other chromosome loci as appropriate, for the samples that are not part of the subset where disomy is assumed for chromosome 21. Once the biases have been corrected for in these samples of unknown ploidy, the data for these samples can then be analyzed a second time using the same or a different method to determine whether the individuals are afflicted with trisomy 21. For example, a quantitative method can be used on the remaining samples of unknown ploidy, and a z-score can be calculated using the corrected measured genetic data on chromosome 21. Alternately, as part of the preliminary estimate of the ploidy state of chromosome 21, a tumor fraction for samples from an individual suspected of having cancer can be calculated. The proportion of corrected reads that are expected in the case of a disomy (the disomy hypothesis), and the proportion of corrected reads that are expected in the case of a trisomy (the trisomy hypothesis) can be calculated for a case with that tumor fraction. Alternately, if the tumor fraction was not measured previously, a set of disomy and trisomy hypotheses can be generated for different tumor fractions. For each case, an expected distribution of the proportion of corrected reads can be calculated given expected statistical variation in the selection and measurement of the various DNA loci. The observed corrected proportion of reads can be compared to the distribution of the expected proportion of corrected reads, and a likelihood ratio can be calculated for the disomy and trisomy hypotheses, for each of the samples of unknown ploidy. The ploidy state associated with the hypothesis with the highest calculated likelihood can be selected as the correct ploidy state.

*8.9 Exemplary Reference Chromosomes or Chromosome Segments*

In some examples, any of the methods described herein are also performed on one or more reference chromosomes or chromosomes segments and the results are compared to those for one or more chromosomes or chromosome segments of interest.

In some examples, the reference chromosome or chromosome segment is used as a control for what would be expected for the absence of a CNV. In some examples, the reference is the same chromosome or chromosome segment from one or more different samples known or expected to not have a deletion or duplication in that chromosome or chromosome segment. In some examples, the reference is a different chromosome or chromosome segment from the sample being tested that is expected to be disomic. In some examples, the reference is a different segment from one of the chromosomes of interest in the same sample that is being tested. For example, the reference may be one or more segments outside of the region of a potential deletion or duplication. Having a reference on the same chromosome that is being tested avoids variability between different chromosomes, such as differences in metabolism, apoptosis, histones, inactivation, and/or amplification between chromosomes. Analyzing segments without a CNV on the same chromosome as the one being tested can also be used to determine differences in metabolism, apoptosis, histones, inactivation, and/or amplification between homologs, allowing the level of variability between homologs in the absence of a CNV to be determined for comparison to the results from a potential CNV. In some examples, the magnitude of the difference between the calculated and expected allele ratios for a potential CNV is greater than the corresponding magnitude for the reference, thereby confirming the presence of a CNV.

In some examples, the reference chromosome or chromosome segment is used as a control for what would be expected for the presence of a CNV, such as a particular deletion or duplication of interest. In some examples, the reference is the same chromosome or chromosome segment from one or more different samples known or expected to have a deletion or duplication in that chromosome or chromosome segment. In some examples, the reference is a different chromosome or chromosome segment from the sample being tested that is known or expected to have a CNV. In some examples, the magnitude of the difference between the calculated and expected allele ratios for a potential CNV is similar to (such as not significantly different) than the corresponding magnitude for the reference for the CNV, thereby confirming the presence of a CNV. In some examples, the magnitude of the difference between the calculated and expected allele ratios for a potential CNV is less than (such as significantly less) than the corresponding magnitude for the reference for the CNV, thereby confirming the absence of a CNV. In some examples, one or more loci for which the genotype of a cancer cell (or DNA or RNA from a cancer cell such as cfDNA or cfRNA) differs from the genotype of a noncancerous cell (or DNA or RNA from a noncancerous cell such as cfDNA or cfRNA) is used to determine the tumor fraction. The tumor fraction can be used to determine whether the overrepresentation of the number of copies of the first homologous chromosome segment is due to a duplication of the first homologous chromosome segment or a deletion of the second homologous chromosome segment. The tumor fraction can also be used to determine the number of extra copies of a chromosome segment or chromosome that is duplicated (such as whether there are 1, 2, 3, 4, or more extra copies), such as to differentiate a sample with four extra chromosome copies and a tumor fraction of 10% from a sample with two extra chromosome copies and a tumor fraction of 20%. The tumor fraction can also be used to determine how well the observed data fits the expected data for possible CNVs. In some examples, the degree of overrepresentation of a CNV is used to select a particular therapy or therapeutic regimen for the individual. For example, some therapeutic agents are only effective for at least four, six, or more copies of a chromosome segment.

In some examples, the one or more loci used to determine the tumor fraction are on a reference chromosome or chromosomes segment, such as a chromosome or chromosome segment known or expected to be disomic, a chromosome or chromosome segment that is rarely duplicated or deleted in cancer cells in general or in a particular type of cancer that an individual is known to have or is at increased risk of having, or a chromosome or chromosome segment that is unlikely to be aneuploid (such segment that is expected to lead to cell death if deleted or duplicated). In some examples, any of the methods of the present disclosure are used to confirm that the reference chromosome or chromosome segment is disomic in both the cancer cells and noncancerous cells. In some examples, one or more chromosomes or chromosomes segments for which the confidence for a disomy call is high are used.

Exemplary loci that can be used to determine the tumor fraction include polymorphisms or mutations (such as SNPs) in a cancer cell (or DNA or RNA such as cfDNA or cfRNA from a cancer cell) that aren't present in a noncancerous cell (or DNA or RNA from a noncancerous cell) in the individual. In some examples, the tumor fraction is determined by identifying those polymorphic loci where a cancer cell (or DNA or RNA from a cancer cell) has an allele that is absent in noncancerous cells (or DNA or RNA from a noncancerous cell) in a sample (such as a plasma sample or tumor biopsy) from an individual; and using the amount of the allele unique to the cancer cell at one or more of the identified polymorphic loci to determine the tumor fraction in the sample. In some examples, a noncancerous cell is homozygous for a first allele at the polymorphic locus, and a cancer cell is (i) heterozygous for the first allele and a second allele or (ii) homozygous for a second allele at the polymorphic locus. In some examples, a noncancerous cell is heterozygous for a first allele and a second allele at the polymorphic locus, and a cancer cell is (i) has one or two copies of a third allele at the polymorphic locus. In some examples, the cancer cells are assumed or known to only have one copy of the allele that is not present in the noncancerous cells. For example, if the genotype of the noncancerous cells is AA and the cancer cells is AB and 5% of the signal at that locus in a sample is from the B allele and 95% is from the A allele, then the tumor fraction of the sample is 10%. In some examples, the cancer cells are assumed or known to have two copies of the allele that is not present in the noncancerous cells. For example, if the genotype of the noncancerous cells is AA and the cancer cells is BB and 5% of the signal at that locus in a sample is from the B allele and 95% is from the A allele, the tumor fraction of the sample is 5%. In some examples, multiple loci for which the cancer cells have an allele not in the noncancerous cells are analyzed to determine which of the loci in the cancer cells are heterozygous and which are homozygous. For example, for loci in which the noncancerous cells are AA, if the signal from the B allele is ~5% at some loci and ~10% at some loci, then the cancer cells are assumed to be heterozygous at loci with ~5% B allele, and homozygous at loci with ~10% B allele (indicating the tumor fraction is ~10%).

Exemplary loci that can be used to determine the tumor fraction include loci for which a cancer cell and noncancerous cell have one allele in common (such as loci in which the cancer cell is AB and the noncancerous cell is BB, or the cancer cell is BB and the noncancerous cell is AB). The amount of A signal, the amount of B signal, or the ratio of A to B signal in a mixed sample (containing DNA or RNA from a cancer cell and a noncancerous cell) is compared to the corresponding value for (i) a sample containing DNA or RNA from only cancer cells or (ii) a sample containing DNA or RNA from only noncancerous cells. The difference in values is used to determine the tumor fraction of the mixed sample.

In some examples, loci that can be used to determine the tumor fraction are selected based on the genotype of (i) a sample containing DNA or RNA from only cancer cells, and/or (ii) a sample containing DNA or RNA from only noncancerous cells. In some examples, the loci are selected based on analysis of the mixed sample, such as loci for which the absolute or relative amounts of each allele differs from what would be expected if both the cancer and noncancerous cells have the same genotype at a particular locus. For example, if the cancer and noncancerous cells have the same genotype, the loci would be expected to produce 0% B signal if all the cells are AA, 50% B signal if all the cells are AB, or 100% B signal if all the cells are BB. Other values for the B signal indicate that the genotype of the cancer and noncancerous cells are different at that locus and thus that locus can be used to determine the tumor fraction.

In some examples, the tumor fraction calculated based on the alleles at one or more loci is compared to the tumor fraction calculated using one or more of the counting methods disclosed herein.

*8.10 Exemplary Methods for Detecting a Phenotype or Analyzing Multiple Mutations*

In some examples, the method includes analyzing a sample for a set of mutations associated with a disease or disorder (such as cancer) or an increased risk for a disease or disorder. There are strong correlations between events within classes (such as M or C cancer classes) which can be used to improve the signal to noise ratio of a method and classify tumors into distinct clinical subsets. For example, borderline results for a few mutations (such as a few CNVs) on one or more chromosomes or chromosomes segments considered jointly may be a very strong signal. In some examples, determining the presence or absence of multiple polymorphisms or mutations of interest (such as 2, 3, 4, 5, 8, 10, 12, 15, or more) increases the sensitivity and/or specificity of the determination of the presence or absence of a disease or disorder such as cancer, or an increased risk for with a disease or disorder such as cancer. In some examples, the correlation between events across multiple chromosomes is used to more powerfully look at a signal compared to looking at each of them individually. The design of the method itself can be optimized to best categorize tumors. This may be incredibly useful for early detection and screening--vis-a-vis recurrence where sensitivity to one particular mutation/CNV may be paramount. In some examples, the events are not always correlated but have a probability of being correlated. In some examples, a matrix estimation formulation with a noise covariance matrix that has off diagonal terms is used.

In some examples, the present disclosure features a method for detecting a phenotype (such as a cancer phenotype) in an individual, wherein the phenotype is defined by the presence of at least one of a set of mutations. In some examples, the method includes obtaining DNA or RNA measurements for a sample of DNA or RNA from one or more cells from the individual, wherein one or more of the cells is suspected of having the phenotype; and analyzing the DNA or RNA measurements to determine, for each of the mutations in the set of mutations, the likelihood that at least one of the cells has that mutation. In some examples, the method includes determining that the individual has the phenotype if either (i) for at least one of the mutations, the likelihood that at least one of the cells contains that mutations is greater than a threshold, or (ii) for at least one of the mutations, the likelihood that at least one of the cells has that mutations is less than the threshold, and for a plurality of the mutations, the combined likelihood that at least one of the cells has at least one of the mutations is greater than the threshold. In some examples, one or more cells have a subset or all of the mutations in the set of mutations. In some examples, the subset of mutations is associated with cancer or an increased risk for cancer. In some examples, the set of mutations includes a subset or all of the mutations in the M class of cancer mutations (Ciriello, Nat Genet. 45(10):1127-1133, 2013, doi: 10.1038/ng.2762). In some examples, the set of mutations includes a subset or all of the mutations in the C class of cancer mutations (Ciriello, *supra*). In some examples, the sample includes cell-free DNA or RNA. In some examples, the DNA or RNA measurements include measurements (such as the quantity of each allele at each locus) at a set of polymorphic loci on one or more chromosomes or chromosome segments of interest.

*8.11 Exemplary Combinations of Methods*

To increase the accuracy of the results, two or more methods (such as any of the methods of the present disclosure or any known method) for detecting the presence or absence of a CNV are performed. In some embodiments, one or more methods for analyzing a factor (such as any of the method described herein or any known method) indicative of the presence or absence of a disease or disorder or an increased risk for a disease or disorder are performed.

In some examples, standard mathematical techniques are used to calculate the covariance and/or correlation between two or more methods. Standard mathematical techniques may also be used to determine the combined probability of a particular hypothesis based on two or more tests. Exemplary techniques include meta-analysis, Fisher's combined probability test for independent tests, Brown's method for combining dependent p-values with known covariance, and Kost's method for combining dependent p-values with unknown covariance. In cases where the likelihoods are determined by a first method in a way that is orthogonal, or unrelated, to the way in which a likelihood is determined for a second method, combining the likelihoods is straightforward and can be done by multiplication and normalization, or by using a formula such as:

$$R_{comb} = R_1 R_2 / [R_1 R_2 + (1-R_1)(1-R_2)]$$

$R_{comb}$ is the combined likelihood, and $R_1$ and $R_2$ are the individual likelihoods. For example, if the likelihood of trisomy from method 1 is 90%, and the likelihood of trisomy from method 2 is 95%, then combining the outputs from the two methods allows the clinician to conclude that the fetus is trisomic with a likelihood of (0.90)(0.95) / [(0.90)(0.95) + (1 - 0.90) (1 - 0.95)] = 99.42%. In cases where the first and the second methods are not orthogonal, that is, where there is a correlation between the two methods, the likelihoods can still be combined.

Exemplary methods of analyzing multiple factors or variables are disclosed in U.S. Patent No. 8,024,128 issued on September 20, 2011; U.S. Publication No. 2007/0027636, filed July 31, 2006; and U.S. Publication No. 2007/0178501, filed December 6, 2006).

In various examples, the combined probability of a particular hypothesis or diagnosis is greater than 80, 85, 90, 92, 94, 96, 98, 99, or 99.9%, or is greater than some other threshold value.

*8.12 Exemplary Methods for Phasing Genetic Data*

In some examples, genetic data is phased using the methods described herein or any known method for phasing genetic data (*see, e.g.,* PCT Publ. No. WO2009/105531, filed February 9, 2009, and PCT Publ. No. WO2010/017214, filed August 4, 2009; U.S. Publ. No. 2013/0123120, Nov. 21, 2012; U.S. Publ. No. 2011/ 0033862, filed Oct. 7, 2010; U.S. Publ. No. 2011/0033862, filed August 19, 2010; U.S. Publ. No. 2011/0178719, filed Feb. 3, 2011; U.S. Pat. No. 8,515,679, filed March 17, 2008; U.S. Publ. No. 2007/0184467, filed Nov. 22, 2006; U.S. Publ. No. 2008/0243398, filed March 17, 2008, and U.S. Serial No. 61/994,791, filed May 16, 2014).

In one example, an individual's genetic data is phased using a computer program that uses population based haplotype frequencies to infer the most likely phase, such as HapMap-based phasing. For example, haploid data sets can be deduced directly from diploid data using statistical methods that utilize known haplotype blocks in the general population (such as those created for the public HapMap Project and for the Perlegen Human Haplotype Project). A haplotype block is essentially a series of correlated alleles that occur repeatedly in a variety of populations. Since these haplotype blocks are often ancient and common, they may be used to predict haplotypes from diploid genotypes. Publicly available algorithms that accomplish this task include an imperfect phylogeny approach, Bayesian approaches based on conjugate priors, and priors from population genetics. Some of these algorithms use a hidden Markov model.

In one example, an individual's genetic data is phased using an algorithm that estimates haplotypes from genotype data, such as an algorithm that uses localized haplotype clustering (*see, e.g.*, Browning and Browning, "Rapid and Accurate Haplotype Phasing and Missing-Data Inference for Whole-Genome Association Studies By Use of Localized Haplotype Clustering" Am J Hum Genet. Nov 2007; 81(5): 1084-1097). An exemplary program is Beagle version: 3.3.2 or version 4 (available at the world wide web at hfaculty.washington.edu/browning/beagle/beagle.html).

In one example, an individual's genetic data is phased using an algorithm that estimates haplotypes from genotype data, such as an algorithm that uses the decay of linkage disequilibrium with distance, the order and spacing of genotyped markers, missing-data imputation, recombination rate estimates, or a combination thereof (*see, e.g.,* Stephens and Scheet, "Accounting for Decay of Linkage Disequilibrium in Haplotype Inference and Missing-Data Imputation" Am. J. Hum. Genet. 76:449-462, 2005). An exemplary program is PHASE v.2.1 or v2.1.1. (available at the world wide web at stephenslab.uchicago.edu/software.html).

In one example, an individual's genetic data is phased using an algorithm that estimates haplotypes from population genotype data, such as an algorithm that allows cluster memberships to change continuously along the chromosome according to a hidden Markov model. This approach is flexible, allowing for both "block-like" patterns of linkage disequilibrium and gradual decline in linkage disequilibrium with distance *(see, e.g.,* Scheet and Stephens, "A fast and flexible statistical model for large-scale population genotype data: applications to inferring missing genotypes and haplotypic phase." Am J Hum Genet, 78:629-644, 2006). An exemplary program is fastPHASE (available at the world wide web at stephenslab.uchicago.edu/software.html).

In one example, an individual's genetic data is phased using a genotype imputation method, such as a method that uses one or more of the following reference datasets: HapMap dataset, datasets of controls genotyped on multiple SNP chips, and densely typed samples from the 1,000 Genomes Project. An exemplary approach is a flexible modelling framework that increases accuracy and combines information across multiple reference panels *(see, e.g.,* Howie, Donnelly, and Marchini (2009) "A flexible and accurate genotype imputation method for the next generation of genome-wide association studies." PLoS Genetics 5(6): e1000529, 2009). Exemplary programs are IMPUTE or IMPUTE version 2 (also known as IMPUTE2) (available at the world wide web at mathgen.stats.ox.ac.uk/impute/impute_v2.html).

In one example, an individual's genetic data is phased using an algorithm that infers haplotypes, such as an algorithm that infers haplotypes under the genetic model of coalescence with recombination, such as that developed by Stephens in PHASE v2.1. The major algorithmic improvements rely on the use of binary trees to represent the sets of candidate haplotypes for each individual. These binary tree representations: (1) speed up the computations of posterior probabilities of the haplotypes by avoiding the redundant operations made in PHASE v2.1, and (2) overcome the exponential aspect of the haplotypes inference problem by the smart exploration of the most plausible pathways *(i.e.,* haplotypes) in the binary trees *(see, e.g.,* Delaneau, Coulonges and Zagury, "Shape-IT: new rapid and accurate algorithm for haplotype inference," BMC Bioinformatics 9:540, 2008 doi:10.1186/1471-2105-9-540). An exemplary program is SHAPEIT (available at the world wide web at mathgen.stats.ox.ac.uk/genetics_software/shapeit/shapeit.html).

In one example, an individual's genetic data is phased using an algorithm that estimates haplotypes from population genotype data, such as an algorithm that uses haplotype-fragment frequencies to obtain empirically based probabilities for longer haplotypes. In some embodiments, the algorithm reconstructs haplotypes so that they have maximal local coherence (*see, e.g.,* Eronen, Geerts, and Toivonen, "HaploRec: Efficient and accurate large-scale reconstruction of haplotypes," BMC Bioinformatics 7:542, 2006). An exemplary program is HaploRec, such as HaploRec version 2.3. (available at the world wide web at cs.helsinki.fi/group/genetics/haplotyping.html).

In one example, an individual's genetic data is phased using an algorithm that estimates haplotypes from population genotype data, such as an algorithm that uses a partition-ligation strategy and an expectation-maximization-based algorithm *(see, e.g.,* Qin, Niu, and Liu, "Partition-Ligation-Expectation-Maximization Algorithm for Haplotype Inference with Single-Nucleotide Polymorphisms," Am J Hum Genet. 71(5): 1242-1247, 2002). An exemplary program is PL-EM (available at the world wide web at people.fas.harvard.edu/~junliu/plem/click.html).

In one example, an individual's genetic data is phased using an algorithm that estimates haplotypes from population genotype data, such as an algorithm for simultaneously phasing genotypes into haplotypes and block partitioning. In some examples, an expectation-maximization algorithm is used *(see, e.g.,* Kimmel and Shamir, "GERBIL: Genotype Resolution and Block Identification Using Likelihood," Proceedings of the National Academy of Sciences of the United States of America (PNAS) 102: 158-162, 2005). An exemplary program is GERBIL, which is available as part of the GEVALT version 2 program (available at the world wide web at acgt.cs.tau.ac.il/gevalt).

In one example, an individual's genetic data is phased using an algorithm that estimates haplotypes from population genotype data, such as an algorithm that uses an EM algorithm to calculate ML estimates of haplotype frequencies given genotype measurements which do not specify phase. The algorithm also allows for some genotype measurements to be missing (due, for example, to PCR failure). It also allows multiple imputation of individual haplotypes (*see, e.g.,* Clayton, D. (2002), "SNPHAP: A Program for Estimating Frequencies of Large Haplotypes of SNPs"). An exemplary program is SNPHAP.

In one example, an individual's genetic data is phased using an algorithm that estimates haplotypes from population genotype data, such as an algorithm for haplotype inference based on genotype statistics collected for pairs of SNPs. This software can be used for comparatively accurate phasing of large number of long genome sequences, e.g. obtained from DNA arrays. An exemplary program takes genotype matrix as an input, and outputs the corresponding haplotype matrix *(see, e.g.,* Brinza and Zelikovsky, "2SNP: scalable phasing based on 2-SNP haplotypes," Bioinformatics.22(3):371-3, 2006). An exemplary program is 2SNP (available at the world wide web at alla.cs.gsu.edu/~software/2SNP).

In various examples, an individual's genetic data is phased using data about the probability of chromosomes crossing over at different locations in a chromosome or chromosome segment (such as using recombination data such as may be found in the HapMap database to create a recombination risk score for any interval) to model dependence between polymorphic alleles on the chromosome or chromosome segment. In some examples, allele counts at the polymorphic loci are calculated on a computer based on sequencing data or SNP array data. In some examples, a plurality of hypotheses each pertaining to a different possible state of the chromosome or chromosome segment (such as an overrepresentation of the number of copies of a first homologous chromosome segment as compared to a second homologous chromosome segment in the genome of one or more cells from an individual, a duplication of the first homologous chromosome segment, a deletion of the second homologous chromosome segment, or an equal representation of the first and second homologous chromosome segments) are created (such as creation on a computer); a model (such as a joint distribution model) for the expected allele counts at the polymorphic loci on the chromosome is built (such as building on a computer) for each hypothesis; a relative probability of each of the hypotheses is determined (such as determination on a computer) using the joint distribution model and the allele counts; and the hypothesis with the greatest probability is selected. In some examples, building a joint distribution model for allele counts and the step of determining the relative probability of each hypothesis are done using a method that does not require the use of a reference chromosome.

In some examples, a sample (*e.g.,* a biopsy such as a tumor biopsy, blood sample, plasma sample, serum sample, or another sample likely to contain mostly or only cells, DNA, or RNA with a CNV of interest) from the individual is analyzed to determine the phase for one or more regions that are known or suspected to contain a CNV of interest (such as a deletion or duplication). In some examples, the sample has a high tumor fraction (such as 30, 40, 50, 60, 70, 80, 90, 95, 98, 99, or 100%). In some examples, the sample is a blood sample from a mother pregnant with a fetus. In some examples, the sample blood from a mother pregnant with a fetus contains circulating fetal cells, and/or fetal DNA.

In some examples, the sample has a haplotypic imbalance or any aneuploidy. In some examples, the sample includes any mixture of two types of DNA where the two types have different ratios of the two haplotypes, and share at least one haplotype. In some examples, at least 10; 100; 500; 1,000; 2,000; 3,000; 5,000; 8,000; or 10,000 polymorphic loci are analyzed to determine the phase of alleles at some or all of the loci. In some examples, a sample is from a cell or tissue that was treated to become aneuploidy, such as aneuploidy induced by prolonged cell culture.

In some examples, a large percent or all of the DNA or RNA in the sample has the CNV of interest. In some examples, the ratio of DNA or RNA from the one or more target cells that contain the CNV of interest to the total DNA or RNA in the sample is at least 80, 85, 90, 95, or 100%. For samples with a deletion, only one haplotype is present for the cells (or DNA or RNA) with the deletion. This first haplotype can be determined using standard methods to determine the identity of alleles present in the region of the deletion. In samples that only contain cells (or DNA or RNA) with the deletion, there will only be signal from the first haplotype that is present in those cells. In samples that also contain a small amount of cells (or DNA or RNA) without the deletion (such as a small amount of noncancerous cells), the weak signal from the second haplotype in these cells (or DNA or RNA) can be ignored. The second haplotype that is present in other cells, DNA, or RNA from the individual that lack the deletion can be determined by inference. For example, if the genotype of cells from the individual without the deletion is (AB, AB) and the phased data for the individual indicates that the first haplotype is (A, A); then, the other haplotype can be inferred to be (B, B).

For samples in which both cells (or DNA or RNA) with a deletion and cells (or DNA or RNA) without a deletion are present, the phase can still be determined. For example, plots can be generated in which the x-axis represents the linear position of the individual loci along the chromosome, and the y-axis represents the number of A allele reads as a fraction of the total (A+B) allele reads. In some examples for a deletion, the pattern includes two central bands that represent SNPs for which the individual is heterozygous (top band represents AB from cells without the deletion and A from cells with the deletion, and bottom band represents AB from cells without the deletion and B from cells with the deletion). In some examples, the separation of these two bands increases as the fraction of cells, DNA, or RNA with the deletion increases. Thus, the identity of the A alleles can be used to determine the first haplotype, and the identity of the B alleles can be used to determine the second haplotype.

For samples with a duplication, an extra copy of the haplotype is present for the cells (or DNA or RNA) with duplication. This haplotype of the duplicated region can be determined using standard methods to determine the identity of alleles present at an increased amount in the region of the duplication, or the haplotype of the region that is not duplicated can be determined using standard methods to determine the identity of alleles present at a decreased amount. Once one haplotype is determined, the other haplotype can be determined by inference.

For samples in which both cells (or DNA or RNA) with a duplication and cells (or DNA or RNA) without a duplication are present, the phase can still be determined using a method similar to that described above for deletions. For example, plots can be generated in which the x-axis represents the linear position of the individual loci along the chromosome, and the y-axis represents the number of A allele reads as a fraction of the total (A+B) allele reads. In some examples for a deletion, the pattern includes two central bands that represent SNPs for which the individual is heterozygous (top band represents AB from cells without the duplication and AAB from cells with the duplication, and bottom band represents AB from cells without the duplication and ABB from cells with the duplication). In some examples, the separation of these two bands increases as the fraction of cells, DNA, or RNA with the duplication increases. Thus, the identity of the A alleles can be used to determine the first haplotype, and the identity of the B alleles can be used to determine the second haplotype. In some examples, the phase of one or more CNV region(s) (such as the phase of at least 50, 60, 70, 80, 90, 95, or 100% of the polymorphic loci in the region that were measured) is determined for a sample (such as a tumor biopsy or plasma sample) from an individual known to have cancer and is used for analysis of subsequent samples from the same individual to monitor the progression of the cancer (such as monitoring for remission or reoccurrence of the cancer). In some examples, a sample with a high tumor fraction (such as a tumor biopsy or a plasma sample from an individual with a high tumor load) is used to obtain phased data that is used for analysis of subsequent samples with a lower tumor fraction (such as a plasma sample from an individual undergoing treatment for cancer or in remission).

In some examples, two or more of the methods described herein are used to phase genetic data of an individual. In some examples, both a bioinformatics method (such as using population based haplotype frequencies to infer the most likely phase) and a molecular biology method (such as any of the molecular phasing methods disclosed herein to obtain actual phased data rather than bioinformatics-based inferred phased data) are used. In some examples, phased data from other subjects (such as prior subjects) is used to refine the population data. For example, phased data from other subjects can be added to population data to calculate priors for possible haplotypes for another subject. In some examples, phased data from other subjects (such as prior subjects) is used to calculate priors for possible haplotypes for another subject.

In some examples, probabilistic data may be used. For example, due to the probabilistic nature of the representation of DNA molecules in a sample, as well as various amplification and measurement biases, the relative number of molecules of DNA measured from two different loci, or from different alleles at a given locus, is not always representative of the relative number of molecules in the mixture, or in the individual. If one were trying to determine the genotype of a normal diploid individual at a given locus on an autosomal chromosome by sequencing DNA from the plasma of the individual, one would expect to either observe only one allele (homozygous) or about equal numbers of two alleles (heterozygous). If, at that allele, ten molecules of the A allele were observed, and two molecules of the B allele were observed, it would not be clear if the individual was homozygous at the locus, and the two molecules of the B allele were due to noise or contamination, or if the individual was heterozygous, and the lower number of molecules of the B allele were due to random, statistical variation in the number of molecules of DNA in the plasma, amplification bias, contamination or any number of other causes. In this case, a probability that the individual was homozygous, and a corresponding probability that the individual was heterozygous could be calculated, and these probabilistic genotypes could be used in further calculations.

Note that for a given allele ratio, the likelihood that the ratio closely represents the ratio of the DNA molecules in the individual is greater the greater the number of molecules that are observed. For example, if one were to measure 100 molecules of A and 100 molecules of B, the likelihood that the actual ratio was 50% is considerably greater than if one were to measure 10 molecules of A and 10 molecules of B. In one example, one uses Bayesian theory combined with a detailed model of the data to determine the likelihood that a particular hypothesis is correct given an observation. For example, if one were considering two hypotheses - one that corresponds to a trisomic individual and one that corresponds to a disomic individual - then the probability of the disomic hypothesis being correct would be considerably higher for the case where 100 molecules of each of the two alleles were observed, as compared to the case where 10 molecules of each of the two alleles were observed. As the data becomes noisier due to bias, contamination or some other source of noise, or as the number of observations at a given locus goes down, the probability of the maximum likelihood hypothesis being true given the observed data drops. In practice, it is possible to aggregate probabilities over many loci to increase the confidence with which the maximum likelihood hypothesis may be determined to be the correct hypothesis. In some examples, the probabilities are simply aggregated without regard for recombination. In some examples, the calculations take into account cross-overs.

In an example, probabilistically phased data is used in the determination of copy number variation. In some examples, the probabilistically phased data is population based haplotype block frequency data from a data source such as the HapMap data base. In some examples, the probabilistically phased data is haplotypic data obtained by a molecular method, for example phasing by dilution where individual segments of chromosomes are diluted to a single molecule per reaction, but where, due to stochastic noise the identities of the haplotypes may not be absolutely known. In some examples, the probabilistically phased data is haplotypic data obtained by a molecular method, where the identities of the haplotypes may be known with a high degree of certainty.

Imagine a hypothetical case where a doctor wanted to determine whether or not an individual had some cells in their body which had a deletion at a particular chromosomal segment by measuring the plasma DNA from the individual. The doctor could make use of the knowledge that if all of the cells from which the plasma DNA originated were diploid, and of the same genotype, then for heterozygous loci, the relative number of molecules of DNA observed for each of the two alleles would fall into one distribution that was centered at 50% A allele and 50% B allele. However, if a fraction of the cells from which the plasma DNA originated had a deletion at a particular chromosome segment, then for heterozygous loci, one would expect that the relative number of molecules of DNA observed for each of the two alleles would fall into two distributions, one centered at above 50% A allele for the loci where there was a deletion of the chromosome segment containing the B allele, and one centered at below 50% for the loci where there was a deletion of the chromosome segment containing the A allele. The greater the proportion of the cells from which the plasma DNA originated contained the deletion, the further from 50% these two distributions would be.

In this hypothetical case, imagine a clinician who wants to determine if an individual had a deletion of a chromosomal region in a proportion of cells in the individual's body. The clinician may draw blood from the individual into a vacutainer or other type of blood tube, centrifuge the blood, and isolate the plasma layer. The clinician may isolate the DNA from the plasma, enrich the DNA at the targeted loci, possibly through targeted or other amplification, locus capture techniques, size enrichment, or other enrichment techniques. The clinician may analyze such as by measuring the number of alleles at a set of SNPs, in other words generating allele frequency data, the enriched and/or amplified DNA using an assay such as qPCR, sequencing, a microarray, or other techniques that measure the quantity of DNA in a sample. We will consider data analysis for the case where the clinician amplified the cell-free plasma DNA using a targeted amplification technique, and then sequenced the amplified DNA to give the following exemplary possible data at six SNPs found on a chromosome segment that is indicative of cancer, where the individual was heterozygotic at those SNPs:

SNP 1: 460 reads A allele; 540 reads B allele (46% A)
SNP 2: 530 reads A allele; 470 reads B allele (53% A)
SNP 3: 40 reads A allele; 60 reads B allele (40% A)
SNP 4: 46 reads A allele; 54 reads B allele (46% A)
SNP 5: 520 reads A allele; 480 reads B allele (52% A)
SNP 6: 200 reads A allele; 200 reads B allele (50% A)

From this set of data, it may be difficult to differentiate between the case where the individual is normal, with all cells being disomic, or where the individual may have a cancer, with some portion of cells whose DNA contributed towards the cell-free DNA found in the plasma having a deletion or duplication at the chromosome. For example, the two hypotheses with the maximum likelihood may be that the individual has a deletion at this chromosome segment, with a tumor fraction of 6%, and where the deleted segment of the chromosome has the genotype over the six SNPs of (A, B, A, A, B, B) or (A, B, A, A, B, A). In this representation of the individual's genotype over a set of SNPs, the first letter in the parentheses corresponds to the genotype of the haplotype for SNP 1, the second to SNP 2, etc.

If one were to use a method to determine the haplotype of the individual at that chromosome segment, and were to find that the haplotype for one of the two chromosomes was (A, B, A, A, B, B), this would agree with the maximum likelihood hypothesis, and the calculated likelihood that the individual has a deletion at that segment, and therefore may have cancerous or precancerous cells, would be considerably increased. On the other hand, if the individual were found to have the haplotype (A, A, A, A, A, A), then the likelihood that the individual has a deletion at that chromosome segment would be considerably decreased, and perhaps the likelihood of the no-deletion hypothesis would be higher (the actual likelihood values would depend on other parameters such as the measured noise in the system, among others).

There are many ways to determine the haplotype of the individual, many of which are described elsewhere in this document. A partial list is given here, and is not meant to be exhaustive. One method is a biological method where individual DNA molecules are diluted until approximately one molecule from each chromosomal region is in any given reaction volume, and then methods such as sequencing are used to measure the genotype. Another method is informatically based where population data on various haplotypes coupled with their frequency can be used in a probabilistic manner. Another method is to measure the diploid data of the individual, along with one or a plurality of related individuals who are expected to share haplotype blocks with the individual and to infer the haplotype blocks. Another method would be to take a sample of tissue with a high concentration of the deleted or duplicated segment, and determine the haplotype based on allelic imbalance, for example, genotype measurements from a sample of tumor tissue with a deletion can be used to determine the phased data for that deletion region, and this data can then be used to determine if the cancer has regrown post-resection.

In practice, typically more than 20 SNPs, more than 50 SNPs, more than 100 SNPs, more than 500 SNPs, more than 1,000 SNPs, or more than 5,000 SNPs are measured on a given chromosome segment.

**9. Exemplary Mutations**

Exemplary mutations associated with a disease or disorder such as cancer or an increased risk (such as an above normal level of risk) for a disease or disorder such as cancer include single nucleotide variants (SNVs), multiple nucleotide mutations, deletions (such as deletion of a 2 to 30 million base pair region), duplications, or tandem repeats. In some examples, the mutation is in DNA, such as cfDNA, cell-free mitochondrial DNA (cf mDNA), cell-free DNA that originated from nuclear DNA (cf nDNA), cellular DNA, or mitochondrial DNA. In some examples, the mutation is in RNA, such as cfRNA, cellular RNA, cytoplasmic RNA, coding cytoplasmic RNA, non-coding cytoplasmic RNA, mRNA, miRNA, mitochondrial RNA, rRNA, or tRNA. In some examples, the mutation is present at a higher frequency in subjects with a disease or disorder (such as cancer) than subjects without the disease or disorder (such as cancer). In some examples, the mutation is indicative of cancer, such as a causative mutation. In some examples, the mutation is a driver mutation that has a causative role in the disease or disorder. In some examples, the mutation is not a causative mutation. For example, in some cancers, multiple mutations accumulate but some of them are not causative mutations. Mutations (such as those that are present at a higher frequency in subjects with a disease or disorder than subjects without the disease or disorder) that are not causative can still be useful for diagnosing the disease or disorder. In some examples, the mutation is loss-of-heterozygosity (LOH) at one or more microsatellites.

In some examples, a subject is screened for one of more polymorphisms or mutations that the subject is known to have (e.g., to test for their presence, a change in the amount of cells, DNA, or RNA with these polymorphisms or mutations, or cancer remission or re-occurrence). In some examples, a subject is screened for one of more polymorphisms or mutations that the subject is known to be at risk for (such as a subject who has a relative with the polymorphism or mutation). In some examples, a subject is screened for a panel of polymorphisms or mutations associated with a disease or disorder such as cancer (e.g., at least 5, 10, 50, 100, 200, 300, 500, 750, 1,000, 1,500, 2,000, or 5,000 polymorphisms or mutations).

Many coding variants associated with cancer are described in Abaan et al., "The Exomes of the NCI-60 Panel: A Genomic Resource for Cancer Biology and Systems Pharmacology", Cancer Research, July 15, 2013, and world wide web at dtp.nci.nih.gov/branches/btb/characterizationNCI60.html). The NCI-60 human cancer cell line panel consists of 60 different cell lines representing cancers of the lung, colon, brain, ovary, breast, prostate, and kidney, as well as leukemia and melanoma. The genetic variations that were identified in these cell lines consisted of two types: type I variants that are found in the normal population, and type II variants that are cancer-specific.

Exemplary polymorphisms or mutations (such as deletions or duplications) are in one or more of the following genes: TP53, PTEN, PIK3CA, APC, EGFR, NRAS, NF2, FBXW7, ERBBs, ATAD5, KRAS, BRAF, VEGF, EGFR, HER2, ALK, p53, BRCA, BRCA1, BRCA2, SETD2, LRP1B, PBRM, SPTA1, DNMT3A, ARID1A, GRIN2A, TRRAP, STAG2, EPHA3/5/7, POLE, SYNE1, C20orf80, CSMD1, CTNNB1, ERBB2. FBXW7, KIT, MUC4, ATM, CDH1, DDX11, DDX12, DSPP, EPPK1, FAM186A, GNAS, HRNR, KRTAP4-11, MAP2K4, MLL3, NRAS, RB1, SMAD4, TTN, ABCC9, ACVR1B, ADAM29, ADAMTS19, AGAP10, AKT1, AMBN, AMPD2, ANKRD30A, ANKRD40, APOBR, AR, BIRC6, BMP2, BRAT1, BTNL8, C12orf4, C1QTNF7, C20orf186, CAPRIN2, CBWD1, CCDC30, CCDC93, CD5L, CDC27, CDC42BPA, CDH9, CDKN2A, CHD8, CHEK2, CHRNA9, CIZ1, CLSPN, CNTN6, COL14A1, CREBBP, CROCC, CTSF, CYP1A2, DCLK1, DHDDS, DHX32, DKK2, DLEC1, DNAH14, DNAH5, DNAH9, DNASE1L3, DUSP16, DYNC2H1, ECT2, EFHB, RRN3P2, TRIM49B, TUBB8P5, EPHA7, ERBB3, ERCC6, FAM21A, FAM21C, FCGBP, FGFR2, FLG2, FLT1, FOLR2, FRYL, FSCB, GAB1, GABRA4, GABRP, GH2, GOLGA6L1, GPHB5, GPR32, GPX5, GTF3C3, HECW1, HIST1H3B, HLA-A, HRAS, HS3ST1, HS6ST1, HSPD1, IDH1, JAK2, KDM5B, KIAA0528, KRT15, KRT38, KRTAP21-1, KRTAP4-5, KRTAP4-7, KRTAP5-4, KRTAP5-5, LAMA4, LATS1, LMF1, LPAR4, LPPR4, LRRFIP1, LUM, LYST, MAP2K1, MARCH1, MARCO, MB21D2, MEGF10, MMP16, MORC1, MRE11A, MTMR3, MUC12, MUC17, MUC2, MUC20, NBPF10, NBPF20, NEK1, NFE2L2, NLRP4, NOTCH2, NRK, NUP93, OBSCN, OR11H1, OR2B11, OR2M4, OR4Q3, OR5D13, OR8I2, OXSM, PIK3R1, PPP2R5C, PRAME, PRF1, PRG4, PRPF19, PTH2, PTPRC, PTPRJ, RAC1, RAD50, RBM12, RGPD3, RGS22, ROR1, RP11-671M22.1, RP13-996F3.4, RP1L1, RSBN1L, RYR3, SAMD3, SCN3A, SEC31A, SF1, SF3B1, SLC25A2, SLC44A1, SLC4A11, SMAD2, SPTA1, ST6GAL2, STK11, SZT2, TAF1L, TAX1BP1, TBP, TGFBI, TIF1, TMEM14B, TMEM74, TPTE, TRAPPC8, TRPS1, TXNDC6, USP32, UTP20, VASN, VPS72, WASH3P, WWTR1, XPO1, ZFHX4, ZMIZ1, ZNF167, ZNF436, ZNF492, ZNF598, ZRSR2, ABL1, AKT2, AKT3, ARAF, ARFRP1, ARID2, ASXL1, ATR, ATRX, AURKA, AURKB, AXL, BAP1, BARD1, BCL2, BCL2L2, BCL6, BCOR, BCORL1, BLM, BRIP1, BTK, CARD11, CBFB, CBL, CCND1, CCND2, CCND3, CCNE1, CD79A, CD79B, CDC73, CDK12, CDK4, CDK6, CDK8, CDKN1B, CDKN2B, CDKN2C, CEBPA, CHEK1, CIC, CRKL, CRLF2, CSF1R, CTCF, CTNNA1, DAXX, DDR2, DOT1L, EMSY (C11orf30), EP300, EPHA3, EPHA5, EPHB1, ERBB4, ERG, ESR1, EZH2, FAM123B (WTX), FAM46C, FANCA, FANCC, FANCD2, FANCE, FANCF, FANCG, FANCL, FGF10, FGF14, FGF19, FGF23, FGF3, FGF4, FGF6, FGFR1, FGFR2, FGFR3, FGFR4, FLT3, FLT4, FOXL2, GATA1, GATA2, GATA3, GID4 (C17orf39), GNA11, GNA13, GNAQ, GNAS, GPR124, GSK3B, HGF, IDH1, IDH2, IGF1R, IKBKE, IKZF1, IL7R, INHBA, IRF4, IRS2, JAK1, JAK3, JUN, KAT6A (MYST3), KDM5A, KDM5C, KDM6A, KDR, KEAP1, KLHL6, MAP2K2, MAP2K4, MAP3K1, MCL1, MDM2, MDM4, MED12, MEF2B, MEN1, MET, MITF, MLH1, MLL, MLL2, MPL, MSH2, MSH6, MTOR, MUTYH, MYC, MYCL1, MYCN, MYD88, NF1, NFKBIA, NKX2-1, NOTCH1, NPM1, NRAS, NTRK1, NTRK2, NTRK3, PAK3, PALB2, PAX5, PBRM1, PDGFRA, PDGFRB, PDK1, PIK3CG, PIK3R2, PPP2R1A, PRDM1, PRKAR1A, PRKDC, PTCH1, PTPN11, RAD51, RAF1, RARA, RET, RICTOR, RNF43, RPTOR, RUNX1, SMARCA4, SMARCB1, SMO, SOCS1, SOX10, SOX2, SPEN, SPOP, SRC, STAT4, SUFU, TET2, TGFBR2, TNFAIP3, TNFRSF14, TOP1, TP53, TSC1, TSC2, TSHR, VHL, WISP3, WT1, ZNF217, ZNF703, and combinations thereof (Su et al., J Mol Diagn 2011, 13:74-84; DOI:10.1016/j.jmoldx.2010.11.010; and Abaan et al., "The Exomes of the NCI-60 Panel: A Genomic Resource for Cancer Biology and Systems Pharmacology", Cancer Research, July 15, 2013). In some examples, the duplication is a chromosome 1p ("Chrlp") duplication associated with breast cancer. In some examples, one or more polymorphisms or mutations are in BRAF, such as the V600E mutation. In some examples, one or more polymorphisms or mutations are in K-ras. In some examples, there is a combination of one or more polymorphisms or mutations in K-ras and APC. In some examples, there is a combination of one or more polymorphisms or mutations in K-ras and p53. In some examples, there is a combination of one or more polymorphisms or mutations in APC and p53. In some examples, there is a combination of one or more polymorphisms or mutations in K-ras, APC, and p53. In some examples, there is a combination of one or more polymorphisms or mutations in K-ras and EGFR. Exemplary polymorphisms or mutations are in one or more of the following microRNAs: miR-15a, miR-16-1, miR-23a, miR-23b, miR-24-1, miR-24-2, miR-27a, miR-27b, miR-29b-2, miR-29c, miR-146, miR-155, miR-221, miR-222, and miR-223 (Calin et al. "A microRNA signature associated with prognosis and progression in chronic lymphocytic leukemia." N Engl J Med 353:1793- 801, 2005).

In some examples, the deletion is a deletion of at least 0.01 kb, 0.1 kb, 1 kb, 10 kb, 100 kb, 1 mb, 2 mb, 3 mb, 5 mb, 10 mb, 15 mb, 20 mb, 30 mb, or 40 mb. In some embodiments, the deletion is a deletion of between 1 kb to 40 mb, such as between 1 kb to 100 kb, 100 kb to 1 mb, 1 to 5 mb, 5 to 10 mb, 10 to 15 mb, 15 to 20 mb, 20 to 25 mb, 25 to 30 mb, or 30 to 40 mb, inclusive.

In some examples, the duplication is a duplication of at least 0.01 kb, 0.1 kb, 1 kb, 10 kb, 100 kb, 1 mb, 2 mb, 3 mb, 5 mb, 10 mb, 15 mb, 20 mb, 30 mb, or 40 mb. In some embodiments, the duplication is a duplication of between 1 kb to 40 mb, such as between 1 kb to 100 kb, 100 kb to 1 mb, 1 to 5 mb, 5 to 10 mb, 10 to 15 mb, 15 to 20 mb, 20 to 25 mb, 25 to 30 mb, or 30 to 40 mb, inclusive.

In some examples, the tandem repeat is a repeat of between 2 and 60 nucleotides, such as 2 to 6, 7 to 10, 10 to 20, 20 to 30, 30 to 40, 40 to 50, or 50 to 60 nucleotides, inclusive. In some embodiments, the tandem repeat is a repeat of 2 nucleotides (dinucleotide repeat). In some embodiments, the tandem repeat is a repeat of 3 nucleotides (trinucleotide repeat).

In some examples, the polymorphism or mutation is prognostic. Exemplary prognostic mutations include K-ras mutations, such as K-ras mutations that are indicators of post-operative disease recurrence in colorectal cancer (Ryan et al. " A prospective study of circulating mutant KRAS2 in the serum of patients with colorectal neoplasia: strong prognostic indicator in postoperative follow up," Gut 52:101-108, 2003; and Lecomte T et al. Detection of free-circulating tumor-associated DNA in plasma of colorectal cancer patients and its association with prognosis," Int J Cancer 100:542-548, 2002).

In some examples, the polymorphism or mutation is associated with altered response to a particular treatment (such as increased or decreased efficacy or side-effects). Examples include K-ras mutations are associated with decreased response to EGFR-based treatments in non-small cell lung cancer (Wang et al. "Potential clinical significance of a plasma-based KRAS mutation analysis in patients with advanced non-small cell lung cancer," Clin Canc Res16:1324-1330, 2010).

K-ras is an oncogene that is activated in many cancers. Exemplary K-ras mutations are mutations in codons 12, 13, and 61. K-ras cfDNA mutations have been identified in pancreatic, lung, colorectal, bladder, and gastric cancers (Fleischhacker & Schmidt "Circulating nucleic acids (CNAs) and caner - a survey," Biochim Biophys Acta 1775:181-232, 2007).

p53 is a tumor suppressor that is mutated in many cancers and contributes to tumor progression (Levine & Oren "The first 30 years of p53: growing ever more complex. Nature Rev Cancer," 9:749-758, 2009). Many different codons can be mutated, such as Ser249. p53 cfDNA mutations have been identified in breast, lung, ovarian, bladder, gastric, pancreatic, colorectal, bowel, and hepatocellular cancers (Fleischhacker & Schmidt "Circulating nucleic acids (CNAs) and caner - a survey," Biochim Biophys Acta 1775:181-232, 2007).

BRAF is an oncogene downstream of Ras. BRAF mutations have been identified in glial neoplasm, melanoma, thyroid, and lung cancers (Dias-Santagata et al. BRAF V600E mutations are common in pleomorphic xanthoastrocytoma: diagnostic and therapeutic implications. PLOS ONE 2011;6:e17948, 2011; Shinozaki et al. Utility of circulating B-RAF DNA mutation in serum for monitoring melanoma patients receiving biochemotherapy. Clin Canc Res 13:2068-2074, 2007; and Board et al. Detection of BRAF mutations in the tumor and serum of patients enrolled in the AZD6244 (ARRY-142886) advanced melanoma phase II study. Brit J Canc 2009;101:1724-1730). The BRAF V600E mutation occurs, *e.g.,* in melanoma tumors, and is more common in advanced stages. The V600E mutation has been detected in cfDNA·

EGFR contributes to cell proliferation and is misregulated in many cancers (Downward J. Targeting RAS signalling pathways in cancer therapy. Nature Rev Cancer 3:11-22, 2003; and Levine & Oren "The first 30 years of p53: growing ever more complex. Nature Rev Cancer," 9:749-758, 2009). Exemplary EGFR mutations include those in exons 18-21, which have been identified in lung cancer patients. EGFR cfDNA mutations have been identified in lung cancer patients (Jia et al. "Prediction of epidermal growth factor receptor mutations in the plasma/pleural effusion to efficacy of gefitinib treatment in advanced non-small cell lung cancer," J Canc Res Clin Oncol 2010;136:1341-1347, 2010).

Exemplary polymorphisms or mutations associated with breast cancer include LOH at microsatellites (Kohler et al. "Levels of plasma circulating cell free nuclear and mitochondrial DNA as potential biomarkers for breast tumors," Mol Cancer 8:doi:10.1186/1476-4598-8-105, 2009), p53 mutations (such as mutations in exons 5-8)(Garcia et al. " Extracellular tumor DNA in plasma and overall survival in breast cancer patients," Genes, Chromosomes & Cancer 45:692-701, 2006), HER2 (Sorensen et al. "Circulating HER2 DNA after trastuzumab treatment predicts survival and response in breast cancer," Anticancer Res 30:2463-2468, 2010), PIK3CA, MED1, and GAS6 polymorphisms or mutations (Murtaza et al. "Non-invasive analysis of acquired resistance to cancer therapy by sequencing of plasma DNA," Nature 2013;doi:10.1038/nature12065, 2013).

Increased cfDNA levels and LOH are associated with decreased overall and disease-free survival. p53 mutations (exons 5-8) are associated with decreased overall survival. Decreased circulating HER2 cfDNA levels are associated with a better response to HER2-targeted treatment in HER2-positive breast tumor subjects. An activating mutation in PIK3CA, a truncation of MED1, and a splicing mutation in GAS6 result in resistance to treatment.

Exemplary polymorphisms or mutations associated with colorectal cancer include p53, APC, K-ras, and thymidylate synthase mutations and p16 gene methylation (Wang et al. "Molecular detection of APC, K-ras, and p53 mutations in the serum of colorectal cancer patients as circulating biomarkers," World J Surg 28:721-726, 2004; Ryan et al. "A prospective study of circulating mutant KRAS2 in the serum of patients with colorectal neoplasia: strong prognostic indicator in postoperative follow up," Gut 52:101-108, 2003; Lecomte et al. "Detection of free-circulating tumor-associated DNA in plasma of colorectal cancer patients and its association with prognosis," Int J Cancer 100:542-548, 2002; Schwarzenbach et al. "Molecular analysis of the polymorphisms of thymidylate synthase on cell-free circulating DNA in blood of patients with advanced colorectal carcinoma," Int J Cancer 127:881-888, 2009). Post-operative detection of K-ras mutations in serum is a strong predictor of disease recurrence. Detection of K-ras mutations and p16 gene methylation are associated with decreased survival and increased disease recurrence. Detection of K-ras, APC, and/or p53 mutations is associated with recurrence and/or metastases. Polymorphisms (including LOH, SNPs, variable number tandem repeats, and deletion) in the thymidylate synthase (the target of fluoropyrimidine-based chemotherapies) gene using cfDNA may be associated with treatment response.

Exemplary polymorphisms or mutations associated with lung cancer (such as non-small cell lung cancer) include K-ras (such as mutations in codon 12) and EGFR mutations. Exemplary prognostic mutations include EGFR mutations (exon 19 deletion or exon 21 mutation) associated with increased overall and progression-free survival and K-ras mutations (in codons 12 and 13) are associated with decreased progression-free survival (Jian et al. "Prediction of epidermal growth factor receptor mutations in the plasma/pleural effusion to efficacy of gefitinib treatment in advanced non-small cell lung cancer," J Canc Res Clin Oncol 136:1341-1347, 2010; Wang et al. "Potential clinical significance of a plasma-based KRAS mutation analysis in patients with advanced non-small cell lung cancer," Clin Canc Res 16:1324-1330, 2010). Exemplary polymorphisms or mutations indicative of response to treatment include EGFR mutations (exon 19 deletion or exon 21 mutation) that improve response to treatment and K-ras mutations (codons 12 and 13) that decrease the response to treatment. A resistance-conferring mutation in EFGR has been identified (Murtaza et al. "Non-invasive analysis of acquired resistance to cancer therapy by sequencing of plasma DNA," Nature doi:10.1038/nature12065, 2013).

Exemplary polymorphisms or mutations associated with melanoma (such as uveal melanoma) include those in GNAQ, GNA11, BRAF, and p53. Exemplary GNAQ and GNA11 mutations include R183 and Q209 mutations. Q209 mutations in GNAQ or GNA11 are associated with metastases to bone. BRAF V600E mutations can be detected in patients with metastatic/advanced stage melanoma. BRAF V600E is an indicator of invasive melanoma. The presence of the BRAF V600E mutation after chemotherapy is associated with a non-response to the treatment

Exemplary polymorphisms or mutations associated with pancreatic carcinomas include those in K-ras and p53 (such as p53 Ser249). p53 Ser249 is also associated with hepatitis B infection and hepatocellular carcinoma, as well as ovarian cancer, and non-Hodgkin's lymphoma.

Even polymorphisms or mutations that are present in low frequency in a sample can be detected with the methods of the present disclosure. For example, a polymorphism or mutation that is present at a frequency of 1 in a million can be observed 10 times by performing 10 million sequencing reads. If desired, the number of sequencing reads can be altered depending of the level of sensitivity desired. In some examples, a sample is re-analyzed or another sample from a subject is analyzed using a greater number of sequencing reads to improve the sensitivity. For example, if no or only a small number (such as 1, 2, 3, 4, or 5) polymorphisms or mutations that are associated with cancer or an increased risk for cancer are detected, the sample is re-analyzed or another sample is tested.

In some examples, multiple polymorphisms or mutations are required for cancer or for metastatic cancer. In such cases, screening for multiple polymorphisms or mutations improves the ability to accurately diagnose cancer or metastatic cancer. In some examples when a subject has a subset of multiple polymorphisms or mutations that are required for cancer or for metastatic cancer, the subject can be re-screened later to see if the subject acquires additional mutations.

In some examples in which multiple polymorphisms or mutations are required for cancer or for metastatic cancer, the frequency of each polymorphism or mutation can be compared to see if they occur at similar frequencies. For example, if two mutations required for cancer (denoted "A" and "B"), some cells will have none, some cells with A, some with B, and some with A and B. If A and B are observed at similar frequencies, the subject is more likely to have some cells with both A and B. If observer A and B at dissimilar frequencies, the subject is more likely to have different cell populations.

In some examples in which multiple polymorphisms or mutations are required for cancer or for metastatic cancer, the number or identity of such polymorphisms or mutations that are present in the subject can be used to predict how likely or soon the subject is likely to have the disease or disorder. In some examples in which polymorphisms or mutations tend to occur in a certain order, the subject may be periodically tested to see if the subject has acquired the other polymorphisms or mutations.

In some examples, determining the presence or absence of multiple polymorphisms or mutations (such as 2, 3, 4, 5, 8, 10, 12, 15, or more) increases the sensitivity and/or specificity of the determination of the presence or absence of a disease or disorder such as cancer, or an increased risk for with a disease or disorder such as cancer.

In some examples, the polymorphism(s) or mutation(s) are directly detected. In some examples, the polymorphism(s) or mutation(s) are indirectly detected by detection of one or more sequences (*e.g.*, a polymorphic locus such as a SNP) that are linked to the polymorphism or mutation.

*9.1 Exemplary Nucleic Acid Alterations*

In some examples, there is a change to the integrity of RNA or DNA (such as a change in the size of fragmented cfRNA or cfDNA or a change in nucleosome composition) that is associated with a disease or disorder such as cancer, or an increased risk for a disease or disorder such as cancer. In some examples, there is a change in the methylation pattern RNA or DNA that is associated with a disease or disorder such as cancer, or an increased risk for with a disease or disorder such as cancer (*e.g.*, hypermethylation of tumor suppressor genes). For example, methylation of the CpG islands in the promoter region of tumor-suppressor genes has been suggested to trigger local gene silencing. Aberrant methylation of the p16 tumor suppressor gene occurs in subjects with liver, lung, and breast cancer. Other frequently methylated tumor suppressor genes, including APC, Ras association domain family protein 1A (RASSF1A), glutathione S-transferase P1 (GSTP1), and DAPK, have been detected in various type of cancers, for example nasopharyngeal carcinoma, colorectal cancer, lung cancer, oesophageal cancer, prostate cancer, bladder cancer, melanoma, and acute leukemia. Methylation of certain tumorsuppressor genes, such as p16, has been described as an early event in cancer formation, and thus is useful for early cancer screening.

In some examples, bisulphite conversion or a non-bisulphite based strategy using methylation sensitive restriction enzyme digestion is used to determine the methylation pattern (Hung et al., J Clin Pathol 62:308-313, 2009). On bisulphite conversion, methylated cytosines remain as cytosines while unmethylated cytosines are converted to uracils. Methylation-sensitive restriction enzymes (*e.g.*, BstUI) cleaves unmethylated DNA sequences at specific recognition sites (*e.g.,* 5'-CG $\vee$ CG-3' for BstUI), while methylated sequences remain intact. In some examples, the intact methylated sequences are detected. In some examples, stem-loop primers are used to selectively amplify restriction enzyme-digested unmethylated fragments without co-amplifying the non-enzyme-digested methylated DNA.

*9.2 Exemplary Changes in mRNA Splicing*

In some examples, a change in mRNA splicing is associated with a disease or disorder such as cancer, or an increased risk for a disease or disorder such as cancer. In some examples, the change in mRNA splicing is in one or more of the following nucleic acids associated with cancer or an increased risk for cancer: DNMT3B, BRCA1, KLF6, Ron, or Gemin5. In some examples, the detected mRNA splice variant is associated with a disease or disorder, such as cancer. In some examples, multiple mRNA splice variants are produced by healthy cells (such as non-cancerous cells), but a change in the relative amounts of the mRNA splice variants is associated with a disease or disorder, such as cancer. In some examples, the change in mRNA splicing is due to a change in the mRNA sequence (such as a mutation in a splice site), a change in splicing factor levels, a change in the amount of available splicing factor (such as a decrease in the amount of available splicing factor due to the binding of a splicing factor to a repeat), altered splicing regulation, or the tumor microenvironment.

The splicing reaction is carried out by a multi-protein/RNA complex called the spliceosome (Fackenthal1 and Godley, Disease Models & Mechanisms 1: 37-42, 2008, doi:10.1242/dmm.000331). The spliceosome recognizes intron-exon boundaries and removes intervening introns via two transesterification reactions that result in ligation of two adjacent exons. The fidelity of this reaction must be exquisite, because if the ligation occurs incorrectly, normal protein-encoding potential may be compromised. For example, in cases where exon-skipping preserves the reading frame of the triplet codons specifying the identity and order of amino acids during translation, the alternatively spliced mRNA may specify a protein that lacks crucial amino acid residues. More commonly, exon-skipping will disrupt the translational reading frame, resulting in premature stop codons. These mRNAs are typically degraded by at least 90% through a process known as nonsense-mediated mRNA degradation, which reduces the likelihood that such defective messages will accumulate to generate truncated protein products. If mis-spliced mRNAs escape this pathway, then truncated, mutated, or unstable proteins are produced.

Alternative splicing is a means of expressing several or many different transcripts from the same genomic DNA and results from the inclusion of a subset of the available exons for a particular protein. By excluding one or more exons, certain protein domains may be lost from the encoded protein, which can result in protein function loss or gain. Several types of alternative splicing have been described: exon skipping; alternative 5' or 3' splice sites; mutually exclusive exons; and, much more rarely, intron retention. Others have compared the amount of alternative splicing in cancer versus normal cells using a bioinformatic approach and determined that cancers exhibit lower levels of alternative splicing than normal cells. Furthermore, the distribution of the types of alternative splicing events differed in cancer versus normal cells. Cancer cells demonstrated less exon skipping, but more alternative 5' and 3' splice site selection and intron retention than normal cells. When the phenomenon of exonization (the use of sequences as exons that are used predominantly by other tissues as introns) was examined, genes associated with exonization in cancer cells were preferentially associated with mRNA processing, indicating a direct link between cancer cells and the generation of aberrant mRNA splice forms.

*9.3 Exemplary Changes in DNA or RNA Levels*

In some examples, there is a change in the total amount or concentration of one or more types of DNA (such as cfDNA cf mDNA, cf nDNA, cellular DNA, or mitochondrial DNA) or RNA (cfRNA, cellular RNA, cytoplasmic RNA, coding cytoplasmic RNA, non-coding cytoplasmic RNA, mRNA, miRNA, mitochondrial RNA, rRNA, or tRNA). In some examples, there is a change in the amount or concentration of one or more specific DNA (such as cfDNA cf mDNA, cf nDNA, cellular DNA, or mitochondrial DNA) or RNA (cfRNA, cellular RNA, cytoplasmic RNA, coding cytoplasmic RNA, non-coding cytoplasmic RNA, mRNA, miRNA, mitochondrial RNA, rRNA, or tRNA) molecules. In some examples, one allele is expressed more than another allele of a locus of interest. Exemplary miRNAs are short 20-22 nucleotide RNA molecules that regulate the expression of a gene. In some examples, there is a change in the transcriptome, such as a change in the identity or amount of one or more RNA molecules.

In some examples, an increase in the total amount or concentration of cfDNA or cfRNA is associated with a disease or disorder such as cancer, or an increased risk for a disease or disorder such as cancer. In some examples, the total concentration of a type of DNA (such as cfDNA cf mDNA, cf nDNA, cellular DNA, or mitochondrial DNA) or RNA (cfRNA, cellular RNA, cytoplasmic RNA, coding cytoplasmic RNA, non-coding cytoplasmic RNA, mRNA, miRNA, mitochondrial RNA, rRNA, or tRNA) increases by at least 2, 3, 4, 5, 6, 7, 8, 9, 10-fold, or more compared to the total concentration of that type of DNA or RNA in healthy (such as non-cancerous) subjects. In some examples, a total concentration of cfDNA between 75 to 100 ng/mL, 100 to 150 ng/mL, 150 to 200 ng/mL, 200 to 300 ng/mL, 300 to 400 ng/mL, 400 to 600 ng/mL, 600 to 800 ng/mL, 800 to 1,000 ng/mL, inclusive, or a total concentration of cfDNA of more than 100 ng, mL, such as more than 200, 300, 400, 500, 600, 700, 800, 900, or 1,000 ng/mL is indicative of cancer, an increased risk for cancer, an increased risk of a tumor being malignant rather than benign, a decreased probably of the cancer going into remission, or a worse prognosis for the cancer. In some examples, the amount of a type of DNA (such as cfDNA cf mDNA, cf nDNA, cellular DNA, or mitochondrial DNA) or RNA (cfRNA, cellular RNA, cytoplasmic RNA, coding cytoplasmic RNA, non-coding cytoplasmic RNA, mRNA, miRNA, mitochondrial RNA, rRNA, or tRNA) having one or more polymorphisms/mutations (such as deletions or duplications) associated with a disease or disorder such as cancer or an increased risk for a disease or disorder such as cancer is at least 2, 3, 4, 5, 6, 7, 8, 9, 10, 11, 12, 14, 16, 18, 20, or 25% of the total amount of that type of DNA or RNA. In some examples, at least 2, 3, 4, 5, 6, 7, 8, 9, 10, 11, 12, 14, 16, 18, 20, or 25% of the total amount of a type of DNA (such as cfDNA cf mDNA, cf nDNA, cellular DNA, or mitochondrial DNA) or RNA (cfRNA, cellular RNA, cytoplasmic RNA, coding cytoplasmic RNA, non-coding cytoplasmic RNA, mRNA, miRNA, mitochondrial RNA, rRNA, or tRNA) has a particular polymorphism or mutation (such as a deletion or duplication) associated with a disease or disorder such as cancer or an increased risk for a disease or disorder such as cancer.

In some examples, the cfDNA is encapsulated. In some examples, the cfDNA is not encapsulated.

In some examples, the fraction of tumor DNA out of total DNA (such as fraction of tumor cfDNA out of total cfDNA or fraction of tumor cfDNA with a particular mutation out of total cfDNA) is determined. In some examples, the fraction of tumor DNA may be determined for a plurality of mutations, where the mutations can be single nucleotide variants, copy number variants, differential methylation, or combinations thereof. In some examples, the average tumor fraction calculated for one or a set of mutations with the highest calculated tumor fraction is taken as the actual tumor fraction in the sample. In some examples, the average tumor fraction calculated for all of the mutations is taken as the actual tumor fraction in the sample. In some examples, this tumor fraction is used to stage a cancer (since higher tumor fractions can be associated with more advanced stages of cancer). In some examples, the tumor fraction is used to size a cancer, since larger tumors may be correlated with the fraction of tumor DNA in the plasma. In some examples, the tumor fraction is used to size the proportion of a tumor that is afflicted with a single or plurality of mutations, since there may be a correlation between the measured tumor fraction in a plasma sample and the size of tissue with a given mutation(s) genotype. For example, the size of tissue with a given mutation(s) genotype may be correlated with the fraction of tumor DNA that may be calculated by focusing on that particular mutation(s).

## 10. Exemplary Biochemical Methods and Compositions

### 10.1 Exemplary Samples

In some examples of any of the teachings of the present disclosure, the sample includes cellular and/or extracellular genetic material from cells suspected of having a deletion or duplication, such as cells suspected of being cancerous or circulating cells suspected to be of fetal origin. In some examples, the sample comprises any tissue or bodily fluid suspected of containing cells, DNA, or RNA having a deletion or duplication. The genetic measurements used as part of these methods can be made on any sample comprising DNA or RNA, for example but not limited to, tissue, blood, serum, plasma, urine, hair, tears, saliva, skin, fingernails, feces, bile, lymph, cervical mucus, semen, tumor, fetal or other cells or materials comprising nucleic acids. Samples may include any cell type or DNA or RNA from any cell type may be used (such as cells from any organ or tissue suspected of being cancerous, or neurons). In some examples, the sample includes nuclear and/or mitochondrial DNA. In some examples, the sample is from any of the target individuals disclosed herein. In some examples, the target individual cancer patient.

Exemplary samples include those containing cfDNA or cfRNA. In some examples, cfDNA is available for analysis without requiring the step of lysing cells. Cell-free DNA may be obtained from a variety of tissues, such as tissues that are in liquid form, *e.g.,* blood, plasma, lymph, ascites fluid, or cerebral spinal fluid. In some cases, cfDNA is comprised of DNA derived from fetal cells. In some cases, the cfDNA is isolated from plasma that has been isolated from whole blood that has been centrifuged to remove cellular material. The cfDNA may be a mixture of DNA derived from target cells (such as cancer cells) and non-target cells (such as non-cancer cells).

In some examples, the sample contains or is suspected to contain a mixture of DNA (or RNA), such as mixture of DNA (or RNA) originating from cancer cells and DNA (or RNA) originating from noncancerous (i.e. normal) cells. In some examples, at least 0.5, 1, 3, 5, 7, 10, 15, 20, 30, 40, 50, 60, 70, 80, 90, 92, 94, 95, 96, 98, 99, or 100% of the cells in the sample are cancer cells. In some examples, at least 0.5, 1, 3, 5, 7, 10, 15, 20, 30, 40, 50, 60, 70, 80, 90, 92, 94, 95, 96, 98, 99, or 100% of the DNA (such as cfDNA) or RNA (such as cfRNA) in the sample is from cancer cell(s). In various examples, the percent of cells in the sample that are cancerous cells is between 0.5 to 99%, such as between 1 to 95%, 5 to 95%, 10 to 90%, 5 to 70%, 10 to 70%, 20 to 90%, or 20 to 70%, inclusive. In some examples, the sample is enriched for cancer cells or for DNA or RNA from cancer cells. In some examples in which the sample is enriched for cancer cells, at least 0.5, 1, 2, 3, 4, 5, 6, 7, 10, 15, 20, 30, 40, 50, 60, 70, 80, 90, 92, 94, 95, 96, 98, 99, or 100% of the cells in the enriched sample are cancer cells. In some examples in which the sample is enriched for DNA or RNA from cancer cells, at least 0.5, 1, 2, 3, 4, 5, 6, 7, 10, 15, 20, 30, 40, 50, 60, 70, 80, 90, 92, 94, 95, 96, 98, 99, or 100% of the DNA or RNA in the enriched sample is from cancer cell(s). In some examples, cell sorting (such as Fluorescent Activated Cell Sorting (FACS)) is used to enrich for cancer cells (Barteneva et. al., Biochim Biophys Acta., 1836(1):105-22, Aug 2013. doi: 10.1016/j.bbcan.2013.02.004. Epub 2013 Feb 24, and Ibrahim et al., Adv Biochem Eng Biotechnol. 106:19-39, 2007).

In some examples, the sample is enriched for fetal cells. In some examples in which the sample is enriched for fetal cells, at least 0.5, 1, 2, 3, 4, 5, 6, 7% or more of the cells in the enriched sample are fetal cells. In some examples, the percent of cells in the sample that are fetal cells is between 0.5 to 100%, such as between 1 to 99%, 5 to 95%, 10 to 95%, 10 to 95%, 20 to 90%, or 30 to 70%, inclusive. In some examples, the sample is enriched for fetal DNA. In some examples in which the sample is enriched for fetal DNA, at least 0.5, 1, 2, 3, 4, 5, 6, 7% or more of the DNA in the enriched sample is fetal DNA. In some examples, the percent of DNA in the sample that is fetal DNA is between 0.5 to 100%, such as between 1 to 99%, 5 to 95%, 10 to 95%, 10 to 95%, 20 to 90%, or 30 to 70%, inclusive.

In some examples, the sample includes a single cell or includes DNA and/or RNA from a single cell. In some examples, multiple individual cells *(e.g.,* at least 5, 10, 20, 30, 40, or 50 cells from the same subject or from different subjects) are analyzed in parallel. In some examples, cells from multiple samples from the same individual are combined, which reduces the amount of work compared to analyzing the samples separately. Combining multiple samples can also allow multiple tissues to be tested for cancer simultaneously (which can be used to provide or more thorough screening for cancer or to determine whether cancer may have metastasized to other tissues).

In some examples, the sample contains a single cell or a small number of cells, such as 2, 3, 5, 6, 7, 8, 9, or 10 cells. In some examples, the sample has between 1 to 100, 100 to 500, or 500 to 1,000 cells, inclusive. In some examples, the sample contains 1 to 10 picograms, 10 to 100 picograms, 100 picograms to 1 nanogram, 1 to 10 nanograms, 10 to 100 nanograms, or 100 nanograms to 1 microgram of RNA and/or DNA, inclusive.

In some examples, the sample is embedded in parafilm. In some examples, the sample is preserved with a preservative such as formaldehyde and optionally encased in paraffin, which may cause cross-linking of the DNA such that less of it is available for PCR. In some examples, the sample is a formaldehyde fixed-paraffin embedded (FFPE) sample. In some examples, the sample is a fresh sample (such as a sample obtained with 1 or 2 days of analysis). In some examples, the sample is frozen prior to analysis. In some examples, the sample is a historical sample.

These samples can be used in any of the methods of the present disclosure.

*10.2 Exemplary Sample Preparation Methods*

In some examples, the method includes isolating or purifying the DNA and/or RNA. There are a number of standard procedures known in the art to accomplish such an end. In some examples, the sample may be centrifuged to separate various layers. In some examples, the DNA or RNA may be isolated using filtration. In some examples, the preparation of the DNA or RNA may involve amplification, separation, purification by chromatography, liquid separation, isolation, preferential enrichment, preferential amplification, targeted amplification, or any of a number of other techniques either known in the art or described herein. In some examples for the isolation of DNA, RNase is used to degrade RNA. In some examples for the isolation of RNA, DNase (such as DNase I from Invitrogen, Carlsbad, CA, USA) is used to degrade DNA. In some examples, an RNeasy mini kit (Qiagen), is used to isolate RNA according to the manufacturer's protocol. In some examples, small RNA molecules are isolated using the mirVana PARIS kit (Ambion, Austin, TX, USA) according to the manufacturer's protocol (Gu et al., J. Neurochem. 122:641-649, 2012). The concentration and purity of RNA may optionally be determined using Nanovue (GE Healthcare, Piscataway, NJ, USA), and RNA integrity may optionally be measured by use of the 2100 Bioanalyzer (Agilent Technologies, Santa Clara, CA, USA) (Gu et al., J. Neurochem. 122:641-649, 2012). In some examples, TRIZOL or RNAlater (Ambion) is used to stabilize RNA during storage.

In some examples, universal tagged adaptors are added to make a library. Prior to ligation, sample DNA may be blunt ended, and then a single adenosine base is added to the 3-prime end. Prior to ligation the DNA may be cleaved using a restriction enzyme or some other cleavage method. During ligation the 3-prime adenosine of the sample fragments and the complementary 3-prime tyrosine overhang of adaptor can enhance ligation efficiency. In some examples, adaptor ligation is performed using the ligation kit found in the AGILENT SURESELECT kit. In some examples, the library is amplified using universal primers. In an example, the amplified library is fractionated by size separation or by using products such as AGENCOURT AMPURE beads or other similar methods. In some examples, PCR amplification is used to amplify target loci. In some examples, the amplified DNA is sequenced (such as sequencing using an ILLUMINA IIGAX or HiSeq sequencer). In some examples, the amplified DNA is sequenced from each end of the amplified DNA to reduce sequencing errors. If there is a sequence error in a particular base when sequencing from one end of the amplified DNA, there is less likely to be a sequence error in the complementary base when sequencing from the other side of the amplified DNA (compared to sequencing multiple times from the same end of the amplified DNA).

In some examples, whole genome application (WGA) is used to amplify a nucleic acid sample. There are a number of methods available for WGA: ligation-mediated PCR (LM-PCR), degenerate oligonucleotide primer PCR (DOP-PCR), and multiple displacement amplification (MDA). In LM-PCR, short DNA sequences called adapters are ligated to blunt ends of DNA. These adapters contain universal amplification sequences, which are used to amplify the DNA by PCR. In DOP-PCR, random primers that also contain universal amplification sequences are used in a first round of annealing and PCR. Then, a second round of PCR is used to amplify the sequences further with the universal primer sequences. MDA uses the phi-29 polymerase, which is a highly processive and non-specific enzyme that replicates DNA and has been used for single-cell analysis. In some examples, WGA is not performed.

In some examples, selective amplification or enrichment are used to amplify or enrich target loci. In some examples, the amplification and/or selective enrichment technique may involve PCR such as ligation mediated PCR, fragment capture by hybridization, Molecular Inversion Probes, or other circularizing probes. In some examples, real-time quantitative PCR (RT-qPCR), digital PCR, or emulsion PCR, single allele base extension reaction followed by mass spectrometry are used (Hung et al., J Clin Pathol 62:308-313, 2009). In some examples, capture by hybridization with hybrid capture probes is used to preferentially enrich the DNA. In some examples, methods for amplification or selective enrichment may involve using probes where, upon correct hybridization to the target sequence, the 3-prime end or 5-prime end of a nucleotide probe is separated from the polymorphic site of a polymorphic allele by a small number of nucleotides. This separation reduces preferential amplification of one allele, termed allele bias. This is an improvement over methods that involve using probes where the 3-prime end or 5-prime end of a correctly hybridized probe are directly adjacent to or very near to the polymorphic site of an allele. In an example, probes in which the hybridizing region may or certainly contains a polymorphic site are excluded. Polymorphic sites at the site of hybridization can cause unequal hybridization or inhibit hybridization altogether in some alleles, resulting in preferential amplification of certain alleles. These examples are improvements over other methods that involve targeted amplification and/or selective enrichment in that they better preserve the original allele frequencies of the sample at each polymorphic locus, whether the sample is pure genomic sample from a single individual or mixture of individuals

In some examples, PCR (referred to as mini-PCR) is used to generate very short amplicons (US Application No. 13/683,604, filed Nov. 21, 2012, U.S. Publication No. 2013/0123120, U.S. Application No. 13/300,235, filed Nov. 18, 2011, U.S. Publication No 2012/0270212, filed Nov. 18, 2011, and U.S. Serial No. 61/994,791, filed May 16, 2014). cfDNA (such as necroptically- or apoptotically-released cancer cfDNA) is highly fragmented. For fetal cfDNA, the fragment sizes are distributed in approximately a Gaussian fashion with a mean of 160 bp, a standard deviation of 15 bp, a minimum size of about 100 bp, and a maximum size of about 220 bp. The polymorphic site of one particular target locus may occupy any position from the start to the end among the various fragments originating from that locus. Because cfDNA fragments are short, the likelihood of both primer sites being present the likelihood of a fragment of length $L$ comprising both the forward and reverse primers sites is the ratio of the length of the amplicon to the length of the fragment. Under ideal conditions, assays in which the amplicon is 45, 50, 55, 60, 65, or 70 bp will successfully amplify from 72%, 69%, 66%, 63%, 59%, or 56%, respectively, of available template fragment molecules. In certain examples that relate most preferably to cfDNA from samples of individuals suspected of having cancer, the cfDNA is amplified using primers that yield a maximum amplicon length of 85, 80, 75 or 70 bp, and in certain preferred examples 75 bp, and that have a melting temperature between 50 and 65°C, and in certain preferred examples, between 54-60.5°C. The amplicon length is the distance between the 5-prime ends of the forward and reverse priming sites. Amplicon length that is shorter than typically used by those known in the art may result in more efficient measurements of the desired polymorphic loci by only requiring short sequence reads. In an example, a substantial fraction of the amplicons is less than 100 bp, less than 90 bp, less than 80 bp, less than 70 bp, less than 65 bp, less than 60 bp, less than 55 bp, less than 50 bp, or less than 45 bp.

In some examples, amplification is performed using direct multiplexed PCR, sequential PCR, nested PCR, doubly nested PCR, one-and-a-half sided nested PCR, fully nested PCR, one sided fully nested PCR, one-sided nested PCR, hemi-nested PCR, hemi-nested PCR, triply hemi-nested PCR, semi-nested PCR, one sided semi-nested PCR, reverse semi-nested PCR method, or one-sided PCR, which are described in US Application No. 13/683,604, filed Nov. 21, 2012, U.S. Publication No. 2013/0123120, U.S. Application No. 13/300,235, filed Nov. 18, 2011, U.S. Publication No 2012/0270212, and U.S. Serial No. 61/994,791, filed May 16, 2014. If desired, any of these methods can be used for mini-PCR.

If desired, the extension step of the PCR amplification may be limited from a time standpoint to reduce amplification from fragments longer than 200 nucleotides, 300 nucleotides, 400 nucleotides, 500 nucleotides or 1,000 nucleotides. This may result in the enrichment of fragmented or shorter DNA (such as fetal DNA or DNA from cancer cells that have undergone apoptosis or necrosis) and improvement of test performance.

In some examples, multiplex PCR is used. In some examples, the method of amplifying target loci in a nucleic acid sample involves (i) contacting the nucleic acid sample with a library of primers that simultaneously hybridize to least 100; 200; 500; 750; 1,000; 2,000; 5,000; 7,500; 10,000; 20,000; 25,000; 30,000; 40,000; 50,000; 75,000; or 100,000 different target loci to produce a reaction mixture; and (ii) subjecting the reaction mixture to primer extension reaction conditions (such as PCR conditions) to produce amplified products that include target amplicons. In some examples, at least 50, 60, 70, 80, 90, 95, 96, 97, 98, 99, or 99.5% of the targeted loci are amplified. In various examples, less than 60, 50, 40, 30, 20, 10, 5, 4, 3, 2, 1, 0.5, 0.25, 0.1, or 0.05% of the amplified products are primer dimers. In some examples, the primers are in solution (such as being dissolved in the liquid phase rather than in a solid phase). In some examples, the primers are in solution and are not immobilized on a solid support. In some examples, the primers are not part of a microarray. In some examples, the primers do not include molecular inversion probes (MIPs).

In some examples, two or more (such as 3 or 4) target amplicons (such as amplicons from the miniPCR method disclosed herein) are ligated together and then the ligated products are sequenced. Combining multiple amplicons into a single ligation product increases the efficiency of the subsequent sequencing step. In some examples, the target amplicons are less than 150, 100, 90, 75, or 50 base pairs in length before they are ligated. The selective enrichment and/or amplification may involve tagging each individual molecule with different tags, molecular barcodes, tags for amplification, and/or tags for sequencing. In some examples, the amplified products are analyzed by sequencing (such as by high throughput sequencing) or by hybridization to an array, such as a SNP array, the ILLUMINA INFINIUM array, or the AFFYMETRIX gene chip. In some examples, nanopore sequencing is used, such as the nanopore sequencing technology developed by Genia (see, for example, the world wide web at geniachip.com/technology). In some examples, duplex sequencing is used (Schmitt et al., "Detection of ultra-rare mutations by next-generation sequencing," Proc Natl Acad Sci U S A. 109(36): 14508-14513, 2012). This approach greatly reduces errors by independently tagging and sequencing each of the two strands of a DNA duplex. As the two strands are complementary, true mutations are found at the same position in both strands. In contrast, PCR or sequencing errors result in mutations in only one strand and can thus be discounted as technical error. In some examples, the method entails tagging both strands of duplex DNA with a random, yet complementary double-stranded nucleotide sequence, referred to as a Duplex Tag. Double-stranded tag sequences are incorporated into standard sequencing adapters by first introducing a single-stranded randomized nucleotide sequence into one adapter strand and then extending the opposite strand with a DNA polymerase to yield a complementary, double-stranded tag. Following ligation of tagged adapters to sheared DNA, the individually labeled strands are PCR amplified from asymmetric primer sites on the adapter tails and subjected to paired-end sequencing. In some examples, a sample (such as a DNA or RNA sample) is divided into multiple fractions, such as different wells (e.g., wells of a WaferGen SmartChip). Dividing the sample into different fractions (such as at least 5, 10, 20, 50, 75, 100, 150, 200, or 300 fractions) can increase the sensitivity of the analysis since the percent of molecules with a mutation are higher in some of the wells than in the overall sample. In some examples, each fraction has less than 500, 400, 200, 100, 50, 20, 10, 5, 2, or 1 DNA or RNA molecules. In some examples, the molecules in each fraction are sequenced separately. In some examples, the same barcode (such as a random or non-human sequence) is added to all the molecules in the same fraction (such as by amplification with a primer containing the barcode or by ligation of a barcode), and different barcodes are added to molecules in different fractions. The barcoded molecules can be pooled and sequenced together. In some examples, the molecules are amplified before they are pooled and sequenced, such as by using nested PCR. In some examples, one forward and two reverse primers, or two forward and one reverse primers are used.

In some examples, a mutation (such as an SNV or CNV) that is present in less than 10, 5, 2, 1, 0.5, 0.1, 0.05, 0.01, or 0.005% of the DNA or RNA molecules in a sample (such as a sample of cfDNA or cfRNA) is detected (or is capable of being detected). In some examples, a mutation (such as an SNV or CNV) that is present in less than 1,000, 500, 100, 50, 20, 10, 5, 4, 3, or 2 original DNA or RNA molecules (before amplification) in a sample (such as a sample of cfDNA or cfRNA from, *e.g.,* a blood sample) is detected (or is capable of being detected). In some examples, a mutation (such as an SNV or CNV) that is present in only 1 original DNA or RNA molecule (before amplification) in a sample (such as a sample of cfDNA or cfRNA from, *e.g.,* a blood sample) is detected (or is capable of being detected).

For example, if the limit of detection of a mutation (such as a single nucleotide variant (SNV)) is 0.1%, a mutation present at 0.01% can be detected by dividing the fraction into multiple, fractions such as 100 wells. Most of the wells have no copies of the mutation. For the few wells with the mutation, the mutation is at a much higher percentage of the reads. In one example, there are 20,000 initial copies of DNA from the target locus, and two of those copies include a SNV of interest. If the sample is divided into 100 wells, 98 wells have the SNV, and 2 wells have the SNV at 0.5%. The DNA in each well can be barcoded, amplified, pooled with DNA from the other wells, and sequenced. Wells without the SNV can be used to measure the background amplification/sequencing error rate to determine if the signal from the outlier wells is above the background level of noise.

In some examples, the amplified products are detected using an array, such as an array especially a microarray with probes to one or more chromosomes of interest (*e.g.*, chromosome 13, 18, 21, X, Y, or any combination thereof). It will be understood for example, that a commercially available SNP detection microarray could be used such as, for example, the Illumina (San Diego, CA) GoldenGate, DASL, Infinium, or CytoSNP-12 genotyping assay, or a SNP detection microarray product from Affymetrix, such as the OncoScan microarray.

In some examples involving sequencing, the depth of read is the number of sequencing reads that map to a given locus. The depth of read may be normalized over the total number of reads. In some examples for depth of read of a sample, the depth of read is the average depth of read over the targeted loci. In some examples for the depth of read of a locus, the depth of read is the number of reads measured by the sequencer mapping to that locus. In general, the greater the depth of read of a locus, the closer the ratio of alleles at the locus tend to be to the ratio of alleles in the original sample of DNA. Depth of read can be expressed in variety of different ways, including but not limited to the percentage or proportion. Thus, for example in a highly parallel DNA sequencer such as an Illumina HISEQ, which, *e.g.*, produces a sequence of 1 million clones, the sequencing of one locus 3,000 times results in a depth of read of 3,000 reads at that locus. The proportion of reads at that locus is 3,000 divided by 1 million total reads, or 0.3% of the total reads.

In some examples, allelic data is obtained, wherein the allelic data includes quantitative measurement(s) indicative of the number of copies of a specific allele of a polymorphic locus. In some examples, the allelic data includes quantitative measurement(s) indicative of the number of copies of each of the alleles observed at a polymorphic locus. Typically, quantitative measurements are obtained for all possible alleles of the polymorphic locus of interest. For example, any of the methods discussed in the preceding paragraphs for determining the allele for a SNP or SNV locus, such as for example, microarrays, qPCR, DNA sequencing, such as high throughput DNA sequencing, can be used to generate quantitative measurements of the number of copies of a specific allele of a polymorphic locus. This quantitative measurement is referred to herein as allelic frequency data or measured genetic allelic data. Methods using allelic data are sometimes referred to as quantitative allelic methods; this is in contrast to quantitative methods which exclusively use quantitative data from non-polymorphic loci, or from polymorphic loci but without regard to allelic identity. When the allelic data is measured using high-throughput sequencing, the allelic data typically include the number of reads of each allele mapping to the locus of interest.

In some examples, non-allelic data is obtained, wherein the non-allelic data includes quantitative measurement(s) indicative of the number of copies of a specific locus. The locus may be polymorphic or non-polymorphic. In some examples when the locus is non-polymorphic, the non-allelic data does not contain information about the relative or absolute quantity of the individual alleles that may be present at that locus. Methods using non-allelic data only (that is, quantitative data from non-polymorphic alleles, or quantitative data from polymorphic loci but without regard to the allelic identity of each fragment) are referred to as quantitative methods. Typically, quantitative measurements are obtained for all possible alleles of the polymorphic locus of interest, with one value associated with the measured quantity for all of the alleles at that locus, in total. Non-allelic data for a polymorphic locus may be obtained by summing the quantitative allelic for each allele at that locus. When the allelic data is measured using high-throughput sequencing, the non-allelic data typically includes the number of reads of mapping to the locus of interest. The sequencing measurements could indicate the relative and/or absolute number of each of the alleles present at the locus, and the non-allelic data includes the sum of the reads, regardless of the allelic identity, mapping to the locus. In some examples the same set of sequencing measurements can be used to yield both allelic data and non-allelic data. In some examples, the allelic data is used as part of a method to determine copy number at a chromosome of interest, and the produced non-allelic data can be used as part of a different method to determine copy number at a chromosome of interest. In some examples, the two methods are statistically orthogonal, and are combined to give a more accurate determination of the copy number at the chromosome of interest.

In some examples obtaining genetic data includes (i) acquiring DNA sequence information by laboratory techniques, *e.g.*, by the use of an automated high throughput DNA sequencer, or (ii) acquiring information that had been previously obtained by laboratory techniques, wherein the information is electronically transmitted, *e.g.*, by a computer over the internet or by electronic transfer from the sequencing device.

Additional exemplary sample preparation, amplification, and quantification methods are described in US Application No. 13/683,604, filed Nov. 21, 2012 (U.S. Publication No. 2013/0123120 and U.S. Serial No. 61/994,791, filed May 16, 2014). These methods can be used for analysis of any of the samples disclosed herein.

*10.3 Exemplary Quantification Methods for Cell-free DNA*

If desired, that amount or concentration of cfDNA or cfRNA can be measured using standard methods. In some examples, the amount or concentration of cell-free mitochondrial DNA (cf mDNA) is determined. In some examples, the amount or concentration of cell-free DNA that originated from nuclear DNA (cf nDNA) is determined. In some examples, the amount or concentration of cf mDNA and cf nDNA are determined simultaneously.

In some examples, qPCR is used to measure cf nDNA and/or cfm DNA (Kohler et al. "Levels of plasma circulating cell free nuclear and mitochondrial DNA as potential biomarkers for breast tumors." Mol Cancer 8:105, 2009, 8: doi:10.1186/1476-4598-8-105). For example, one or more loci from cf nDNA (such as Glyceraldehyd-3-phosphat-dehydrogenase, *GAPDH*) and one or more loci from cf mDNA (ATPase 8, *MTATP 8*) can be measured using multiplex qPCR. In some examples, fluorescence-labelled PCR is used to measure cf nDNA and/or cf mDNA (Schwarzenbach et al., "Evaluation of cell-free tumor DNA and RNA in patients with breast cancer and benign breast disease." Mol Biosys 7:2848-2854, 2011). If desired, the normality distribution of the data can be determined using standard methods, such as the Shapiro-Wilk-Test. If desired, cf nDNA and mDNA levels can be compared using standard methods, such as the Mann-Whitney-U-Test. In some examples, cf nDNA and/or mDNA levels are compared with other established prognostic factors using standard methods, such as the Mann-Whitney-U-Test or the Kruskal-Wallis-Test.

*10.4 Exemplary RNA Amplification, Quantification, and Analysis Methods*

Any of the following exemplary methods may be used to amplify and optionally quantify RNA, such as such as cfRNA, cellular RNA, cytoplasmic RNA, coding cytoplasmic RNA, non-coding cytoplasmic RNA, mRNA, miRNA, mitochondrial RNA, rRNA, or tRNA. In some examples, the miRNA is any of the miRNA molecules listed in the miRBase database available at the world wide web at mirbase.org. Exemplary miRNA molecules include miR-509; miR-21, and miR-146a.

In some examples, reverse-transcriptase multiplex ligation-dependent probe amplification (RT-MLPA) is used to amplify RNA. In some examples, each set of hybridizing probes consists of two short synthetic oligonucleotides spanning the SNP and one long oligonucleotide (Li et al., Arch Gynecol Obstet. "Development of noninvasive prenatal diagnosis of trisomy 21 by RT-MLPA with a new set of SNP markers," July 5, 2013, DOI 10.1007/s00404-013-2926-5; Schouten et al. "Relative quantification of 40 nucleic acid sequences by multiplex ligation-dependent probe amplification." Nucleic Acids Res 30: e57, 2002; Deng et al. (2011) "Non-invasive prenatal diagnosis of trisomy 21 by reverse transcriptase multiplex ligation-dependent probe amplification," Clin, Chem. Lab Med. 49:641-646, 2011).

In some examples, RNA is amplified with reverse-transcriptase PCR. In some examples, RNA is amplified with real-time reverse-transcriptase PCR, such as one-step real-time reverse-transcriptase PCR with SYBR GREEN I as previously described (Li et al., Arch Gynecol Obstet. "Development of noninvasive prenatal diagnosis of trisomy 21 by RT-MLPA with a new set of SNP markers," July 5, 2013, DOI 10.1007/s00404-013-2926-5; Lo et al., "Plasma placental RNA allelic ratio permits noninvasive prenatal chromosomal aneuploidy detection," Nat Med 13:218-223, 2007; Tsui et al., Systematic micro-array based identification of placental mRNA in maternal plasma: towards non-invasive prenatal gene expression profiling. J Med Genet 41:461-467, 2004; Gu et al., J. Neurochem. 122:641-649, 2012).

In some examples, a microarray is used to detect RNA. For example, a human miRNA microarray from Agilent Technologies can be used according to the manufacturer's protocol. Briefly, isolated RNA is dephosphorylated and ligated with pCp-Cy3. Labeled RNA is purified and hybridized to miRNA arrays containing probes for human mature miRNAs on the basis of Sanger miRBase release 14.0. The arrays are washed and scanned with use of a microarray scanner (G2565BA, Agilent Technologies). The intensity of each hybridization signal is evaluated by Agilent extraction software v9.5.3. The labeling, hybridization, and scanning may be performed according to the protocols in the Agilent miRNA microarray system (Gu et al., J. Neurochem. 122:641-649, 2012).

In some examples, a TaqMan assay is used to detect RNA. An exemplary assay is the TaqMan Array Human MicroRNA Panel v1.0 (Early Access) (Applied Biosystems), which contains 157 TaqMan MicroRNA Assays, including the respective reverse-transcription primers, PCR primers, and TaqMan probe (Chim et al., "Detection and characterization of placental microRNAs in maternal plasma," Clin Chem. 54(3):482-90, 2008).

If desired, the mRNA splicing pattern of one or more mRNAs can be determined using standard methods (Fackenthal1 and Godley, Disease Models & Mechanisms 1: 37-42, 2008, doi:10.1242/dmm.000331). For example, high-density microarrays and/or high-throughput DNA sequencing can be used to detect mRNA splice variants.

In some examples, whole transcriptome shotgun sequencing or an array is used to measure the transcriptome.

*10.5 Exemplary Amplification Methods*

Improved PCR amplification methods have also been developed that minimize or prevent interference due to the amplification of nearby or adjacent target loci in the same reaction volume (such as part of the sample multiplex PCR reaction that simultaneously amplifies all the target loci). These methods can be used to simultaneously amplify nearby or adjacent target loci, which is faster and cheaper than having to separate nearby target loci into different reaction volumes so that they can be amplified separately to avoid interference.

In some examples, the amplification of target loci is performed using a polymerase (e.g., a DNA polymerase, RNA polymerase, or reverse transcriptase) with low $5' \rightarrow 3'$ exonuclease and/or low strand displacement activity. In some examples, the low level of $5' \rightarrow 3'$ exonuclease reduces or prevents the degradation of a nearby primer (*e.g.*, an unextended primer or a primer that has had one or more nucleotides added to during primer extension). In some examples, the low level of strand displacement activity reduces or prevents the displacement of a nearby primer (*e.g.*, an unextended primer or a primer that has had one or more nucleotides added to it during primer extension). In some examples, target loci that are adjacent to each other (*e.g.,* no bases between the target loci) or nearby (*e.g.*, loci are within 50, 40, 30, 20, 15, 10, 9, 8, 7, 6, 5, 4, 3, 2, or 1 base) are amplified. In some examples, the 3' end of one locus is within 50, 40, 30, 20, 15, 10, 9, 8, 7, 6, 5, 4, 3, 2, or 1 base of the 5' end of next downstream locus.

In some examples, at least 100, 200, 500, 750, 1,000; 2,000; 5,000; 7,500; 10,000; 20,000; 25,000; 30,000; 40,000; 50,000; 75,000; or 100,000 different target loci are amplified, such as by the simultaneous amplification in one reaction volume In some examples, at least 50, 60, 70, 80, 90, 95, 96, 97, 98, 99, or 99.5% of the amplified products are target amplicons. In various examples, the amount of amplified products that are target amplicons is between 50 to 99.5%, such as between 60 to 99%, 70 to 98%, 80 to 98%, 90 to 99.5%, or 95 to 99.5%, inclusive. In some examples, at least 50, 60, 70, 80, 90, 95, 96, 97, 98, 99, or 99.5% of the targeted loci are amplified (*e.g*, amplified at least 5, 10, 20, 30, 50, or 100-fold compared to the amount prior to amplification), such as by the simultaneous amplification in one reaction volume. In various examples, the amount target loci that are amplified (*e.g*, amplified at least 5, 10, 20, 30, 50, or 100-fold compared to the amount prior to amplification) is between 50 to 99.5%, such as between 60 to 99%, 70 to 98%, 80 to 99%, 90 to 99.5%, 95 to 99.9%, or 98 to 99.99% inclusive. In some examples, fewer non-target amplicons are produced, such as fewer amplicons formed from a forward primer from a first primer pair and a reverse primer from a second primer pair. Such undesired non-target amplicons can be produced using prior amplification methods if, *e.g.,* the reverse primer from the first primer pair and/or the forward primer from the second primer pair are degraded and/or displaced.

In some examples, these methods allow longer extension times to be used since the polymerase bound to a primer being extended is less likely to degrade and/or displace a nearby primer (such as the next downstream primer) given the low $5' \rightarrow 3'$ exonuclease and/or low strand displacement activity of the polymerase. In various examples, reaction conditions (such as the extension time and temperature) are used such that the extension rate of the polymerase allows the number of nucleotides that are added to a primer being extended to be equal to or greater than 80, 90, 95, 100, 110, 120, 130, 140, 150, 175, or 200% of the number of nucleotides between the 3' end of the primer binding site and the 5' end of the next downstream primer binding site on the same strand.

In some examples, a DNA polymerase is used produce DNA amplicons using DNA as a template. In some examples, a RNA polymerase is used produce RNA amplicons using DNA as a template. In some examples, a reverse transcriptase is used produce cDNA amplicons using RNA as a template.

In some examples, the low level of $5' \rightarrow 3'$ exonuclease of the polymerase is less than 80, 70, 60, 50, 40, 30, 20, 10, 5, 1, or 0.1% of the activity of the same amount of Thermus aquaticus polymerase ("Taq" polymerase, which is a commonly used DNA polymerase from a thermophilic bacterium, PDB 1BGX, EC 2.7.7.7, Murali et al., "Crystal structure of Taq DNA polymerase in complex with an inhibitory Fab: the Fab is directed against an intermediate in the helix-coil dynamics of the enzyme," Proc. Natl. Acad. Sci. USA 95:12562-12567, 1998) under the same conditions. In some examples, the low level of strand displacement activity of the polymerase is less than 80, 70, 60, 50, 40, 30, 20, 10, 5, 1, or 0.1% of the activity of the same amount of Taq polymerase under the same conditions.

In some examples, the polymerase is a PUSHION DNA polymerase, such as PHUSION High Fidelity DNA polymerase (M0530S, New England BioLabs, Inc.) or PHUSION Hot Start Flex DNA polymerase (M0535S, New England BioLabs, Inc.; Frey and Suppman BioChemica. 2:34-35, 1995; Chester and Marshak Analytical Biochemistry. 209:284-290, 1993). The PHUSION DNA polymerase is a *Pyrococcus-like* enzyme fused with a processivity-enhancing domain. PHUSION DNA polymerase possesses 5'→ 3' polymerase activity and 3'→ 5' exonuclease activity, and generates blunt-ended products. PHUSION DNA polymerase lacks 5'→ 3' exonuclease activity and strand displacement activity.

In some examples, the polymerase is a Q5® DNA Polymerase, such as Q5® High-Fidelity DNA Polymerase (M0491S, New England BioLabs, Inc.) or Q5® Hot Start High-Fidelity DNA Polymerase (M0493S, New England BioLabs, Inc.). Q5® High-Fidelity DNA polymerase is a high-fidelity, thermostable, DNA polymerase with 3'→ 5' exonuclease activity, fused to a processivity-enhancing Sso7d domain. Q5® High-Fidelity DNA polymerase lacks 5'→ 3' exonuclease activity and strand displacement activity.

In some examples, the polymerase is a T4 DNA polymerase (M0203S, New England BioLabs, Inc.; Tabor and Struh. (1989). "DNA-Dependent DNA Polymerases," In Ausebel et al. (Ed.), Current Protocols in Molecular Biology. 3.5.10-3.5.12. New York: John Wiley & Sons, Inc., 1989; Sambrook et al. Molecular Cloning: A Laboratory Manual. (2nd ed.), 5.44-5.47. Cold Spring Harbor: Cold Spring Harbor Laboratory Press, 1989). T4 DNA Polymerase catalyzes the synthesis of DNA in the 5'→ 3' direction and requires the presence of template and primer. This enzyme has a 3'→ 5' exonuclease activity which is much more active than that found in DNA Polymerase I. T4 DNA polymerase lacks 5'→ 3' exonuclease activity and strand displacement activity.

In some examples, the polymerase is a *Sulfolobus* DNA Polymerase IV (M0327S, New England BioLabs, Inc.; (Boudsocq et al. (2001). Nucleic Acids Res., 29:4607-4616, 2001; McDonald, et al. (2006). Nucleic Acids Res., 34:1102-1111, 2006). *Sulfolobus* DNA Polymerase IV is a thermostable Y-family lesion-bypass DNA Polymerase that efficiently synthesizes DNA across a variety of DNA template lesions McDonald, J.P. et al. (2006). Nucleic Acids Res., 34, 1102-1111). *Sulfolobus* DNA Polymerase IV lacks 5'→ 3' exonuclease activity and strand displacement activity.

In some examples, if a primer binds a region with a SNP, the primer may bind and amplify the different alleles with different efficiencies or may only bind and amplify one allele. For subjects who are heterozygous, one of the alleles may not be amplified by the primer. In some examples, a primer is designed for each allele. For example, if there are two alleles *(e.g.,* a biallelic SNP), then two primers can be used to bind the same location of a target locus *(e.g.,* a forward primer to bind the "A" allele and a forward primer to bind the "B" allele). Standard methods, such as the dbSNP database, can be used to determine the location of known SNPs, such as SNP hot spots that have a high heterozygosity rate.

In some examples, the amplicons are similar in size. In some examples, the range of the length of the target amplicons is less than 100, 75, 50, 25, 15, 10, or 5 nucleotides. In some examples (such as the amplification of target loci in fragmented DNA or RNA), the length of the target amplicons is between 50 and 100 nucleotides, such as between 60 and 80 nucleotides, or 60 and 75 nucleotides, inclusive. In some examples (such as the amplification of multiple target loci throughout an exon or gene), the length of the target amplicons is between 100 and 500 nucleotides, such as between 150 and 450 nucleotides, 200 and 400 nucleotides, 200 and 300 nucleotides, or 300 and 400 nucleotides, inclusive.

In some examples, multiple target loci are simultaneously amplified using a primer pair that includes a forward and reverse primer for each target locus to be amplified in that reaction volume. In some examples, one round of PCR is performed with a single primer per target locus, and then a second round of PCR is performed with a primer pair per target locus. For example, the first round of PCR may be performed with a single primer per target locus such that all the primers bind the same strand (such as using a forward primer for each target locus). This allows the PCR to amplify in a linear manner and reduces or eliminates amplification bias between amplicons due to sequence or length differences. In some examples, the amplicons are then amplified using a forward and reverse primer for each target locus.

*10.6 Exemplary Primer Design Methods*

If desired, multiplex PCR may be performed using primers with a decreased likelihood of forming primer dimers. In particular, highly multiplexed PCR can often result in the production of a very high proportion of product DNA that results from unproductive side reactions such as primer dimer formation. In an example, the particular primers that are most likely to cause unproductive side reactions may be removed from the primer library to give a primer library that will result in a greater proportion of amplified DNA that maps to the genome. The step of removing problematic primers, that is, those primers that are particularly likely to firm dimers has unexpectedly enabled extremely high PCR multiplexing levels for subsequent analysis by sequencing.

There are a number of ways to choose primers for a library where the amount of non-mapping primer dimer or other primer mischief products are minimized. Empirical data indicate that a small number of 'bad' primers are responsible for a large amount of non-mapping primer dimer side reactions. Removing these 'bad' primers can increase the percent of sequence reads that map to targeted loci. One way to identify the 'bad' primers is to look at the sequencing data of DNA that was amplified by targeted amplification; those primer dimers that are seen with greatest frequency can be removed to give a primer library that is significantly less likely to result in side product DNA that does not map to the genome. There are also publicly available programs that can calculate the binding energy of various primer combinations, and removing those with the highest binding energy will also give a primer library that is significantly less likely to result in side product DNA that does not map to the genome.

In some examples for selecting primers, an initial library of candidate primers is created by designing one or more primers or primer pairs to candidate target loci. A set of candidate target loci (such as SNPs) can selected based on publically available information about desired parameters for the target loci, such as frequency of the SNPs within a target population or the heterozygosity rate of the SNPs. In one example, the PCR primers may be designed using the Primer3 program (the worldwide web at primer3.sourceforge.net; libprimer3 release 2.2.3). If desired, the primers can be designed to anneal within a particular annealing temperature range, have a particular range of GC contents, have a particular size range, produce target amplicons in a particular size range, and/or have other parameter characteristics. Starting with multiple primers or primer pairs per candidate target locus increases the likelihood that a primer or prime pair will remain in the library for most or all of the target loci. In one example, the selection criteria may require that at least one primer pair per target locus remains in the library. That way, most or all of the target loci will be amplified when using the final primer library. This is desirable for applications such as screening for deletions or duplications at a large number of locations in the genome or screening for a large number of sequences (such as polymorphisms or other mutations) associated with a disease or an increased risk for a disease. If a primer pair from the library would produces a target amplicon that overlaps with a target amplicon produced by another primer pair, one of the primer pairs may be removed from the library to prevent interference.

In some examples, an "undesirability score" (higher score representing least desirability) is calculated (such as calculation on a computer) for most or all of the possible combinations of two primers from a library of candidate primers. In various examples, an undesirability score is calculated for at least 80, 90, 95, 98, 99, or 99.5% of the possible combinations of candidate primers in the library. Each undesirability score is based at least in part on the likelihood of dimer formation between the two candidate primers. If desired, the undesirability score may also be based on one or more other parameters selected from the group consisting of heterozygosity rate of the target locus, disease prevalence associated with a sequence (e.g., a polymorphism) at the target locus, disease penetrance associated with a sequence (e.g., a polymorphism) at the target locus, specificity of the candidate primer for the target locus, size of the candidate primer, melting temperature of the target amplicon, GC content of the target amplicon, amplification efficiency of the target amplicon, size of the target amplicon, and distance from the center of a recombination hotspot. In some examples, the specificity of the candidate primer for the target locus includes the likelihood that the candidate primer will mis-prime by binding and amplifying a locus other than the target locus it was designed to amplify. In some examples, one or more or all the candidate primers that mis-prime are removed from the library. In some examples to increase the number of candidate primers to choose from, candidate primers that may mis-prime are not removed from the library. If multiple factors are considered, the undesirability score may be calculated based on a weighted average of the various parameters. The parameters may be assigned different weights based on their importance for the particular application that the primers will be used for. In some examples, the primer with the highest undesirability score is removed from the library. If the removed primer is a member of a primer pair that hybridizes to one target locus, then the other member of the primer pair may be removed from the library. The process of removing primers may be repeated as desired. In some examples, the selection method is performed until the undesirability scores for the candidate primer combinations remaining in the library are all equal to or below a minimum threshold. In some examples, the selection method is performed until the number of candidate primers remaining in the library is reduced to a desired number.

In various examples, after the undesirability scores are calculated, the candidate primer that is part of the greatest number of combinations of two candidate primers with an undesirability score above a first minimum threshold is removed from the library. This step ignores interactions equal to or below the first minimum threshold since these interactions are less significant. If the removed primer is a member of a primer pair that hybridizes to one target locus, then the other member of the primer pair may be removed from the library. The process of removing primers may be repeated as desired. In some examples, the selection method is performed until the undesirability scores for the candidate primer combinations remaining in the library are all equal to or below the first minimum threshold. If the number of candidate primers remaining in the library is higher than desired, the number of primers may be reduced by decreasing the first minimum threshold to a lower second minimum threshold and repeating the process of removing primers. If the number of candidate primers remaining in the library is lower than desired, the method can be continued by increasing the first minimum threshold to a higher second minimum threshold and repeating the process of removing primers using the original candidate primer library, thereby allowing more of the candidate primers to remain in the library. In some examples, the selection method is performed until the undesirability scores for the candidate primer combinations remaining in the library are all equal to or below the second minimum threshold, or until the number of candidate primers remaining in the library is reduced to a desired number.

If desired, primer pairs that produce a target amplicon that overlaps with a target amplicon produced by another primer pair can be divided into separate amplification reactions. Multiple PCR amplification reactions may be desirable for applications in which it is desirable to analyze all of the candidate target loci (instead of omitting candidate target loci from the analysis due to overlapping target amplicons).

These selection methods minimize the number of candidate primers that have to be removed from the library to achieve the desired reduction in primer dimers. By removing a smaller number of candidate primers from the library, more (or all) of the target loci can be amplified using the resulting primer library.

Multiplexing large numbers of primers imposes considerable constraint on the assays that can be included. Assays that unintentionally interact result in spurious amplification products. The size constraints of miniPCR may result in further constraints. In an example, it is possible to begin with a very large number of potential SNP targets (between about 500 to greater than 1 million) and attempt to design primers to amplify each SNP. Where primers can be designed it is possible to attempt to identify primer pairs likely to form spurious products by evaluating the likelihood of spurious primer duplex formation between all possible pairs of primers using published thermodynamic parameters for DNA duplex formation. Primer interactions may be ranked by a scoring function related to the interaction and primers with the worst interaction scores are eliminated until the number of primers desired is met. In cases where SNPs likely to be heterozygous are most useful, it is possible to also rank the list of assays and select the most heterozygous compatible assays. Experiments have validated that primers with high interaction scores are most likely to form primer dimers. At high multiplexing it is not possible to eliminate all spurious interactions, but it is essential to remove the primers or pairs of primers with the highest interaction scores *in silico* as they can dominate an entire reaction, greatly limiting amplification from intended targets. We have performed this procedure to create multiplex primer sets of up to and in some cases more than 10,000 primers. The improvement due to this procedure is substantial, enabling amplification of more than 80%, more than 90%, more than 95%, more than 98%, and even more than 99% on target products as determined by sequencing of all PCR products, as compared to 10% from a reaction in which the worst primers were not removed. When combined with a partial semi-nested approach as previously described, more than 90%, and even more than 95% of amplicons may map to the targeted sequences.

Note that there are other methods for determining which PCR probes are likely to form dimers. In an example, analysis of a pool of DNA that has been amplified using a non-optimized set of primers may be sufficient to determine problematic primers. For example, analysis may be done using sequencing, and those dimers which are present in the greatest number are determined to be those most likely to form dimers, and may be removed. In an example, the method of primer design may be used in combination with the mini-PCR method described herein.

The use of tags on the primers may reduce amplification and sequencing of primer dimer products. In some examples, the primer contains an internal region that forms a loop structure with a tag. In particular examples, the primers include a 5' region that is specific for a target locus, an internal region that is not specific for the target locus and forms a loop structure, and a 3' region that is specific for the target locus. In some examples, the loop region may lie between two binding regions where the two binding regions are designed to bind to contiguous or neighboring regions of template DNA. In various examples, the length of the 3' region is at least 7 nucleotides. In some examples, the length of the 3' region is between 7 and 20 nucleotides, such as between 7 to 15 nucleotides, or 7 to 10 nucleotides, inclusive. In various examples, the primers include a 5' region that is not specific for a target locus (such as a tag or a universal primer binding site) followed by a region that is specific for a target locus, an internal region that is not specific for the target locus and forms a loop structure, and a 3' region that is specific for the target locus. Tag-primers can be used to shorten necessary target-specific sequences to below 20, below 15, below 12, and even below 10 base pairs. This can be serendipitous with standard primer design when the target sequence is fragmented within the primer binding site or, or it can be designed into the primer design. Advantages of this method include: it increases the number of assays that can be designed for a certain maximal amplicon length, and it shortens the "non-informative" sequencing of primer sequence. It may also be used in combination with internal tagging.

In an example, the relative amount of nonproductive products in the multiplexed targeted PCR amplification can be reduced by raising the annealing temperature. In cases where one is amplifying libraries with the same tag as the target specific primers, the annealing temperature can be increased in comparison to the genomic DNA as the tags will contribute to the primer binding. In some examples reduced primer concentrations are used, optionally along with longer annealing times. In some examples the annealing times may be longer than 3 minutes, longer than 5 minutes, longer than 8 minutes, longer than 10 minutes, longer than 15 minutes, longer than 20 minutes, longer than 30 minutes, longer than 60 minutes, longer than 120 minutes, longer than 240 minutes, longer than 480 minutes, and even longer than 960 minutes. In certain illustrative examples, longer annealing times are used along with reduced primer concentrations. In various embodim examples ents, longer than normal extension times are used, such as greater than 3, 5, 8, 10, or 15 minutes. In some examples, the primer concentrations are as low as 50 nM, 20 nM, 10 nM, 5 nM, 1 nM, and lower than 1 nM. This surprisingly results in robust performance for highly multiplexed reactions, for example 1,000-plex reactions, 2,000-plex reactions, 5,000-plex reactions, 10,000-plex reactions, 20,000-plex reactions, 50,000-plex reactions, and even 100,000-plex reactions. In an example, the amplification uses one, two, three, four or five cycles run with long annealing times, followed by PCR cycles with more usual annealing times with tagged primers.

To select target locations, one may start with a pool of candidate primer pair designs and create a thermodynamic model of potentially adverse interactions between primer pairs, and then use the model to eliminate designs that are incompatible with other the designs in the pool.

In an example, the present disclosure features a method of decreasing the number of target loci (such as loci that may contain a polymorphism or mutation associated with a disease or disorder or an increased risk for a disease or disorder such as cancer) and/or increasing the disease load that is detected (e.g., increasing the number of polymorphisms or mutations that are detected). In some examples, the method includes ranking (such as ranking from highest to lowest) loci by frequency or reoccurrence of a polymorphism or mutation (such as a single nucleotide variation, insertion, or deletion, or any of the other variations described herein) in each locus among subjects with the disease or disorder such as cancer. In some examples, PCR primers are designed to some or all of the loci. During selection of PCR primers for a library of primers, primers to loci that have a higher frequency or reoccurrence (higher ranking loci) are favored over those with a lower frequency or reoccurrence (lower ranking loci). In some examples, this parameter is included as one of the parameters in the calculation of the undesirability scores described herein. If desired, primers (such as primers to high ranking loci) that are incompatible with other designs in the library can be included in a different PCR library/pool. In some examples, multiple libraries/pools (such as 2, 3, 4, 5 or more) are used in separate PCR reactions to enable amplification of all (or a majority) of the loci represented by all the libraries/pools. In some examples, this method is continued until sufficient primers are included in one or more libraries/pools such that the primers, in aggregate, enable the desired disease load to be captured for the disease or disorder (e.g., such as by detection of at least 80, 85, 90, 95, or 99% of the disease load).

*10.7 Exemplary Primer Libraries*

In one teaching, the present disclosure features libraries of primers, such as primers selected from a library of candidate primers using any of the methods of the present disclosure. In some examples, the library includes primers that simultaneously hybridize (or are capable of simultaneously hybridizing) to or that simultaneously amplify (or are capable of simultaneously amplifying) at least 100; 200; 500; 750; 1,000; 2,000; 5,000; 7,500; 10,000; 20,000; 25,000; 30,000; 40,000; 50,000; 75,000; or 100,000 different target loci in one reaction volume. In various examples, the library includes primers that simultaneously amplify (or are capable of simultaneously amplifying) between 100 to 500; 500 to 1,000; 1,000 to 2,000; 2,000 to 5,000; 5,000 to 7,500; 7,500 to 10,000; 10,000 to 20,000; 20,000 to 25,000; 25,000 to 30,000; 30,000 to 40,000; 40,000 to 50,000; 50,000 to 75,000; or 75,000 to 100,000 different target loci in one reaction volume, inclusive. In various examples, the library includes primers that simultaneously amplify (or are capable of simultaneously amplifying) between 1,000 to 100,000 different target loci in one reaction volume, such as between 1,000 to 50,000; 1,000 to 30,000; 1,000 to 20,000; 1,000 to 10,000; 2,000 to 30,000; 2,000 to 20,000; 2,000 to 10,000; 5,000 to 30,000; 5,000 to 20,000; or 5,000 to 10,000 different target loci, inclusive. In some examples, the library includes primers that simultaneously amplify (or are capable of simultaneously amplifying) the target loci in one reaction volume such that less than 60, 40, 30, 20, 10, 5, 4, 3, 2, 1, 0.5, 0.25, 0.1, or 0.5% of the amplified products are primer dimers. The various examples, the amount of amplified products that are primer dimers is between 0.5 to 60%, such as between 0.1 to 40%, 0.1 to 20%, 0.25 to 20%, 0.25 to 10%, 0.5 to 20%, 0.5 to 10%, 1 to 20%, or 1 to 10%, inclusive. In some examples, the primers simultaneously amplify (or are capable of simultaneously amplifying) the target loci in one reaction volume such that at least 50, 60, 70, 80, 90, 95, 96, 97, 98, 99, or 99.5% of the amplified products are target amplicons. In various examples, the amount of amplified products that are target amplicons is between 50 to 99.5%, such as between 60 to 99%, 70 to 98%, 80 to 98%, 90 to 99.5%, or 95 to 99.5%, inclusive. In some examples, the primers simultaneously amplify (or are capable of simultaneously amplifying) the target loci in one reaction volume such that at least 50, 60, 70, 80, 90, 95, 96, 97, 98, 99, or 99.5% of the targeted loci are amplified (e.g, amplified at least 5, 10, 20, 30, 50, or 100-fold compared to the amount prior to amplification). In various examples, the amount target loci that are amplified (e.g, amplified at least 5, 10, 20, 30, 50, or 100-fold compared to the amount prior to amplification) is between 50 to 99.5%, such as between 60 to 99%, 70 to 98%, 80 to 99%, 90 to 99.5%, 95 to 99.9%, or 98 to 99.99% inclusive. In some examples, the library of primers includes at least 100; 200; 500; 750; 1,000; 2,000; 5,000; 7,500; 10,000; 20,000; 25,000; 30,000; 40,000; 50,000; 75,000; or 100,000 primer pairs, wherein each pair of primers includes a forward test primer and a reverse test primer where each pair of test primers hybridize to a target locus. In some examples, the library of primers includes at least 100; 200; 500; 750; 1,000; 2,000; 5,000; 7,500; 10,000; 20,000; 25,000; 30,000; 40,000; 50,000; 75,000; or 100,000 individual primers that each hybridize to a different target locus, wherein the individual primers are not part of primer pairs.

In various examples, the concentration of each primer is less than 100, 75, 50, 25, 20, 10, 5, 2, or 1 nM, or less than 500, 100, 10, or 1 $\mu$M. In various examples, the concentration of each primer is between 1 $\mu$M to 100 nM, such as between 1 $\mu$M to 1 nM, 1 to 75 nM, 2 to 50 nM or 5 to 50 nM, inclusive. In various examples, the GC content of the primers is between 30 to 80%, such as between 40 to 70%, or 50 to 60%, inclusive. In some examples, the range of GC content of the primers is less than 30, 20, 10, or 5%. In some examples, the range of GC content of the primers is between 5 to 30%, such as 5 to 20% or 5 to 10%, inclusive. In some examples, the melting temperature ($T_m$) of the test primers is between 40 to 80 °C, such as 50 to 70 °C, 55 to 65 °C, or 57 to 60.5 °C, inclusive. In some examples, the $T_m$ is calculated using the Primer3 program (libprimer3 release 2.2.3) using the built-in SantaLucia parameters (the world wide web at primer3.sourceforge.net). In some examples, the range of melting temperature of the primers is less than 15, 10, 5, 3, or 1 °C. In some examples, the range of melting temperature of the primers is between 1 to 15 °C, such as between 1 to 10 °C, 1 to 5 °C, or 1 to 3 °C, inclusive. In some examples, the length of the primers is between 15 to 100 nucleotides, such as between 15 to 75 nucleotides, 15 to 40 nucleotides, 17 to 35 nucleotides, 18 to 30 nucleotides, or 20 to 65 nucleotides, inclusive. In some examples, the range of the length of the primers is less than 50, 40, 30, 20, 10, or 5 nucleotides. In some examples, the range of the length of the primers is between 5 to 50 nucleotides, such as 5 to 40 nucleotides, 5 to 20 nucleotides, or 5 to 10 nucleotides, inclusive. In some examples, the length of the target amplicons is between 50 and 100 nucleotides, such as between 60 and 80 nucleotides, or 60 to 75 nucleotides, inclusive. In some examples, the range of the length of the target amplicons is less than 50, 25, 15, 10, or 5 nucleotides. In some examples, the range of the length of the target amplicons is between 5 to 50 nucleotides, such as 5 to 25 nucleotides, 5 to 15 nucleotides, or 5 to 10 nucleotides, inclusive. In some examples, the library does not comprise a microarray. In some examples, the library comprises a microarray.

In some examples, some (such as at least 80, 90, or 95%) or all of the adaptors or primers include one or more linkages between adjacent nucleotides other than a naturally-occurring phosphodiester linkage. Examples of such linkages include phosphoramide, phosphorothioate, and phosphorodithioate linkages. In some examples, some (such as at least 80, 90, or 95%) or all of the adaptors or primers include a thiophosphate (such as a monothiophosphate) between the last 3' nucleotide and the second to last 3' nucleotide. In some examples, some (such as at least 80, 90, or 95%) or all of the adaptors or primers include a thiophosphate (such as a monothiophosphate) between the last 2, 3, 4, or 5 nucleotides at the 3' end. In some examples, some (such as at least 80, 90, or 95%) or all of the adaptors or primers include a thiophosphate (such as a monothiophosphate) between at least 1, 2, 3, 4, or 5 nucleotides out of the last 10 nucleotides at the 3' end. In some examples, such primers are less likely to be cleaved or degraded. In some examples, the primers do not contain an enzyme cleavage site (such as a protease cleavage site).

Additional exemplary multiplex PCR methods and libraries are described in US Application No. 13/683,604, filed Nov. 21, 2012 (U.S. Publication No. 2013/0123120) and U.S. Serial No. 61/994,791, filed May 16, 2014). These methods and libraries can be used for analysis of any of the samples disclosed herein and for use in any of the methods of the present disclosure.

*10.8 Exemplary Primer Libraries for Detection of Recombination*

In some examples, primers in the primer library are designed to determine whether or not recombination occurred at one or more known recombination hotspots (such as crossovers between homologous human chromosomes). Knowing what crossovers occurred between chromosomes allows more accurate phased genetic data to be determined for an individual. Recombination hotspots are local regions of chromosomes in which recombination events tend to be concentrated. Often they are flanked by "coldspots," regions of lower than average frequency of recombination. Recombination hotspots tend to share a similar morphology and are approximately 1 to 2 kilo base pair (kbp) in length. The hotspot distribution is positively correlated with GC content and repetitive element distribution. A partially degenerated 13-mer motif CCNCCNTNNCCNC plays a role in some hotspot activity. It has been shown that the zinc finger protein called PRDM9 binds to this motif and initiates recombination at its location. The average distance between the centers of recombination hot spots is reported to be ~80 kbp. In some examples, the distance between the centers of recombination hot spots ranges between ~3 kb to ~100 kb. Public databases include a large number of known human recombination hotspots, such as the HUMHOT and International HapMap Project databases (see, for example, Nishant et al., "HUMHOT: a database of human meiotic recombination hot spots," Nucleic Acids Research, 34: D25-D28, 2006, Database issue; Mackiewicz et al., "Distribution of Recombination Hotspots in the Human Genome - A Comparison of Computer Simulations with Real Data" PLoS ONE 8(6): e65272, doi:10.1371/journal.pone.0065272; and the world wide web at hapmap.ncbi.nlm.nih.gov/downloads/index.html.en).

In some examples, primers in the primer library are clustered at or near recombination hotspots (such as known human recombination hotspots). In some examples, the corresponding amplicons are used to determine the sequence within or near a recombination hotspot to determine whether or not recombination occurred at that particular hotspot (such as whether the sequence of the amplicon is the sequence expected if a recombination had occurred or the sequence expected if a recombination had not occurred). In some examples, primers are designed to amplify part or all of a recombination hotspot (and optionally sequence flanking a recombination hotspot). In some examples, long read sequencing (such as sequencing using the Moleculo Technology developed by Illumina to sequence up to ~10 kb) or paired end sequencing is used to sequence part or all of a recombination hotspot. Knowledge of whether or not a recombination event occurred can be used to determine which haplotype blocks flank the hotspot. If desired, the presence of particular haplotype blocks can be confirmed using primers specific to regions within the haplotype blocks. In some examples, it is assumed there are no crossovers between known recombination hotspots. In some examples, primers in the primer library are clustered at or near the ends of chromosomes. For example, such primers can be used to determine whether or not a particular arm or section at the end of a chromosome is present. In some examples, primers in the primer library are clustered at or near recombination hotspots and at or near the ends of chromosomes.

In some examples, the primer library includes one or more primers (such as at least 5; 10; 50; 100; 200; 500; 750; 1,000; 2,000; 5,000; 7,500; 10,000; 20,000; 25,000; 30,000; 40,000; or 50,000 different primers or different primer pairs) that are specific for a recombination hotspot (such as a known human recombination hotspot) and/or are specific for a region near a recombination hotspot (such as within 10, 8, 5, 3, 2, 1, or 0.5 kb of the 5' or 3' end of a recombination hotspot). In some examples, at least 1, 5, 10, 20, 40, 60, 80, 100, or 150 different primers (or primer pairs) are specific for the same recombination hotspot, or are specific for the same recombination hotspot or a region near the recombination hotspot. In some examples, at least 1, 5, 10, 20, 40, 60, 80, 100, or 150 different primers (or primer pairs) are specific for a region between recombination hotspots (such as a region unlikely to have undergone recombination); these primers can be used to confirm the presence of haplotype blocks (such as those that would be expected depending on whether or not recombination has occurred). In some examples, at least 10, 20, 30, 40, 50, 60, 70, 80, or 90% of the primers in the primer library are specific for a recombination hotspot and/or are specific for a region near a recombination hotspot (such as within 10, 8, 5, 3, 2, 1, or 0.5 kb of the 5' or 3' end of the recombination hotspot). In some examples, the primer library is used to determine whether or not recombination has occurred at greater than or equal to 5; 10; 50; 100; 200; 500; 750; 1,000; 2,000; 5,000; 7,500; 10,000; 20,000; 25,000; 30,000; 40,000; or 50,000 different recombination hotspots (such as known human recombination hotspots). In some examples, the regions targeted by primers to a recombination hotspot or nearby region are approximately evenly spread out along that portion of the genome. In some examples, at least 1, 5, 10, 20, 40, 60, 80, 100, or 150 different primers (or primer pairs) are specific for a region at or near the end of a chromosome (such as a region within 20, 10, 5, 1, 0.5, 0.1, 0.01, or 0.001 mb from the end of a chromosome). In some examples, at least 10, 20, 30, 40, 50, 60, 70, 80, or 90% of the primers in the primer library are specific for a region at or near the end of a chromosome (such as a region within 20, 10, 5, 1, 0.5, 0.1, 0.01, or 0.001 mb from the end of a chromosome). In some examples, at least 1, 5, 10, 20, 40, 60, 80, 100, or 150 different primers (or primer pairs) are specific for a region within a potential microdeletion in a chromosome. In some examples, at least 10, 20, 30, 40, 50, 60, 70, 80, or 90% of the primers in the primer library are specific for a region within a potential microdeletion in a chromosome. In some examples, at least 10, 20, 30, 40, 50, 60, 70, 80, or 90% of the primers in the primer library are specific for a recombination hotspot, a region near a recombination hotspot, a region at or near the end of a chromosome, or a region within a potential microdeletion in a chromosome.

*10.9 Exemplary Kits*

In one teaching, the present disclosure may feature a kit, such as a kit for amplifying target loci in a nucleic acid sample for detecting deletions and/or duplications of chromosome segments or entire chromosomes using any of the methods described herein). In some examples, the kit can include any of the primer libraries of the present disclosure. In an examples, the kit comprises a plurality of inner forward primers and optionally a plurality of inner reverse primers, and optionally outer forward primers and outer reverse primers, where each of the primers is designed to hybridize to the region of DNA immediately upstream and/or downstream from one of the target sites (*e.g.*, polymorphic sites) on the target chromosome(s) or chromosome segment(s), and optionally additional chromosomes or chromosome segments. In some examples, the kit includes instructions for using the primer library to amplify the target loci, such as for detecting one or more deletions and/or duplications of one or more chromosome segments or entire chromosomes using any of the methods described herein.

In certain examples, kits of the present disclosure provide primer pairs for detecting chromosomal aneuploidy and CNV determination, such as primer pairs for massively multiplex reactions for detecting chromosomal aneuploidy such as CNV (CoNVERGe) (Copy Number Variant Events Revealed Genotypically) and/or SNVs. In these examples, the kits can include between at least 100, 200, 250, 300, 500, 1000, 2000, 2500, 3000, 5000, 10,000, 20,000, 25,000, 28,000, 50,000, or 75,000 and at most 200, 250, 300, 500, 1000, 2000, 2500, 3000, 5000, 10,000, 20,000, 25,000, 28,000, 50,000, 75,000, or 100,000 primer pairs that are shipped together. The primer pairs can be contained in a single vessel, such as a single tube or box, or multiple tubes or boxes. In certain examples, the primer pairs are pre-qualified by a commercial provider and sold together, and in other examples, a customer selects custom gene targets and/or primers and a commercial provider makes and ships the primer pool to the customer neither in one tube or a plurality of tubes. In certain exemplary examples, the kits include primers for detecting both CNVs and SNVs, especially CNVs and SNVs known to be correlated to at least one type of cancer.

Kits for circulating DNA detection according to some examples of the present disclosure, include standards and/or controls for circulating DNA detection. For example, in certain examples, the standards and/or controls are sold and optionally shipped and packaged together with primers used to perform the amplification reactions provided herein, such as primers for performing CoNVERGe. In certain examples, the controls include polynucleotides such as DNA, including isolated genomic DNA that exhibits one or more chromosomal aneuploidies such as CNV and/or includes one or more SNVs. In certain examples, the standards and/or controls are called PlasmArt standards and include polynucleotides having sequence identity to regions of the genome known to exhibit CNV, especially in certain inherited diseases, and in certain disease states such as cancer, as well as a size distribution that reflects that of cfDNA fragments naturally found in plasma. Exemplary methods for making PlasmArt standards are provided in the examples herein. In general, genomic DNA from a source known to include a chromosomal aneuoploidy is isolated, fragmented, purified and size selected.

Accordingly, artificial cfDNA polynucleotide standards and/or controls can be made by spiking isolated polynucleotide samples prepared as summarized above, into DNA samples known not to exhibit a chromosomal aneuploidy and/or SNVs, at concentrations similar to those observed for cfDNA in vivo, such as between, for example, 0.01% and 20%, 0.1 and 15%, or .4 and 10% of DNA in that fluid. These standards/controls can be used as controls for assay design, characterization, development, and/or validation, and as quality control standards during testing, such as cancer testing performed in a CLIA lab and/or as standards included in research use only or diagnostic test kits.

## 11. Additional Examples

### 11.1 Exemplary Databases

The present disclosure also features databases containing one or more results from a method of the present disclosure. For example, the database may include records with any of the following information for one or more subjects: any polymorphisms/mutations (such as CNVs) identified, any known association of the polymorphisms/mutations with a disease or disorder or an increased risk for a disease or disorder, effect of the polymorphisms/mutations on the expression or activity level of the encoded mRNA or protein, fraction of DNA, RNA, or cells associated with a disease or disorder (such as DNA, RNA, or cells having polymorphism/mutation associated with a disease or disorder) out of the total DNA, RNA, or cells in sample, source of sample used to identify the polymorphisms/mutations (such as a blood sample or sample from a particular tissue), number of diseased cells, results from later repeating the test (such as repeating the test to monitor the progression or remission of the disease or disorder), results of other tests for the disease or disorder, type of disease or disorder the subject was diagnosed with, treatment(s) administered, response to such treatment(s), side-effects of such treatment(s), symptoms (such as symptoms associated with the disease or disorder), length and number of remissions, length of survival (such as length of time from initial test until death or length of time from diagnosis until death), cause of death, and combinations thereof.

In some examples, the database includes records with any of the following information for one or more subjects: any polymorphisms/mutations identified, any known association of the polymorphisms/mutations with cancer or an increased risk for cancer, effect of the polymorphisms/mutations on the expression or activity level of the encoded mRNA or protein, fraction of cancerous DNA, RNA or cells out of the total DNA, RNA, or cells in sample, source of sample used to identify the polymorphisms/mutations (such as a blood sample or sample from a particular tissue), number of cancerous cells, size of tumor(s), results from later repeating the test (such as repeating the test to monitor the progression or remission of the cancer), results of other tests for cancer, type of cancer the subject was diagnosed with, treatment(s) administered, response to such treatment(s), side-effects of such treatment(s), symptoms (such as symptoms associated with cancer), length and number of remissions, length of survival (such as length of time from initial test until death or length of time from cancer diagnosis until death), cause of death, and combinations thereof. In some examples, the response to treatment includes any of the following: reducing or stabilizing the size of a tumor (*e.g.,* a benign or cancerous tumor), slowing or preventing an increase in the size of a tumor, reducing or stabilizing the number of tumor cells, increasing the disease-free survival time between the disappearance of a tumor and its reappearance, preventing an initial or subsequent occurrence of a tumor, reducing or stabilizing an adverse symptom associated with a tumor, or combinations thereof. In some examples, the results from one or more other tests for a disease or disorder such as cancer are included, such as results from screening tests, medical imaging, or microscopic examination of a tissue sample.

In one such teaching, the present disclosure features an electronic database including at least 5, 10, $10^2$, $10^3$, $10^4$, $10^5$, $10^6$, $10^7$, $10^8$ or more records. In some examples, the database has records for at least 5, 10, $10^2$, $10^3$, $10^4$, $10^5$, $10^6$, $10^7$, $10^8$ or more different subjects.

In another teaching, the present disclosure may feature a computer including a database of the present disclosure and a user interface. In some examples, the user interface is capable of displaying a portion or all of the information contained in one or more records. In some examples, the user interface is capable of displaying (i) one or more types of cancer that have been identified as containing a polymorphism or mutation whose record is stored in the computer, (ii) one or more polymorphisms or mutations that have been identified in a particular type of cancer whose record is stored in the computer, (iii) prognosis information for a particular type of cancer or a particular a polymorphism or mutation whose record is stored in the computer (iv) one or more compounds or other treatments useful for cancer with a polymorphism or mutation whose record is stored in the computer, (v) one or more compounds that modulate the expression or activity of an mRNA or protein whose record is stored in the computer, and (vi) one or more mRNA molecules or proteins whose expression or activity is modulated by a compound whose record is stored in the computer. The internal components of the computer typically include a processor coupled to a memory. The external components usually include a mass-storage device, *e.g.,* a hard disk drive; user input devices, *e.g.,* a keyboard and a mouse; a display, *e.g.,* a monitor; and optionally, a network link capable of connecting the computer system to other computers to allow sharing of data and processing tasks. Programs may be loaded into the memory of this system during operation.

In another teaching, the present disclosure features a computer-implemented process that includes one or more steps of any of the methods of the present disclosure.

*11.2 Exemplary Risk Factors*

In some examples, the subject is also evaluated for one or more risk factors for a disease or disorder, such as cancer. Exemplary risk factors include family history for the disease or disorder, lifestyle (such as smoking and exposure to carcinogens) and the level of one or more hormones or serum proteins (such as alpha-fetoprotein (AFP) in liver cancer, carcinoembryonic antigen (CEA) in colorectal cancer, or prostate-specific antigen (PSA) in prostate cancer). In some examples, the size and/or number of tumors is measured and use in determining a subject's prognosis or selecting a treatment for the subject.

*11.3 Exemplary Screening Methods*

If desired, the presence or absence of a disease or disorder such cancer can be confirmed, or the disease or disorder such as cancer can be classified using any standard method. For example, a disease or disorder such as cancer can be detected in a number of ways, including the presence of certain signs and symptoms, tumor biopsy, screening tests, or medical imaging (such as a mammogram or an ultrasound). Once a possible cancer is detected, it may be diagnosed by microscopic examination of a tissue sample. In some examples, a subject diagnosed undergoes repeat testing using a method of the present disclosure or known testing for the disease or disorder at multiple time points to monitor the progression of the disease or disorder or the remission or reoccurrence of the disease or disorder.

*11.4 Exemplary Cancers*

Exemplary cancers that can be diagnosed, prognosed, stabilized, treated, prevented, for which a response to treatment can be predicted or monitored using any of the methods of the present disclosure include solid tumors, carcinomas, sarcomas, lymphomas, leukemias, germ cell tumors, or blastomas. In various examples, the cancer is an acute lymphoblastic leukemia, acute myeloid leukemia, adrenocortical carcinoma, AIDS-related cancer, AIDS-related lymphoma, anal cancer, appendix cancer, astrocytoma (such as childhood cerebellar or cerebral astrocytoma), basal-cell carcinoma, bile duct cancer (such as extrahepatic bile duct cancer) bladder cancer, bone tumor (such as osteosarcoma or malignant fibrous histiocytoma), brainstem glioma, brain cancer (such as cerebellar astrocytoma, cerebral astrocytoma/malignant glioma, ependymo, medulloblastoma, supratentorial primitive neuroectodermal tumors, or visual pathway and hypothalamic glioma), glioblastoma, breast cancer, bronchial adenoma or carcinoid, burkitt's lymphoma, carcinoid tumor (such as a childhood or gastrointestinal carcinoid tumor), carcinoma central nervous system lymphoma, cerebellar astrocytoma or malignant glioma (such as childhood cerebellar astrocytoma or malignant glioma), cervical cancer, childhood cancer, chronic lymphocytic leukemia, chronic myelogenous leukemia, chronic myeloproliferative disorders, colon cancer, cutaneous t-cell lymphoma, desmoplastic small round cell tumor, endometrial cancer, ependymoma, esophageal cancer, ewing's sarcoma, tumor in the ewing family of tumors, extracranial germ cell tumor (such as a childhood extracranial germ cell tumor), extragonadal germ cell tumor, eye cancer (such as intraocular melanoma or retinoblastoma eye cancer), gallbladder cancer, gastric cancer, gastrointestinal carcinoid tumor, gastrointestinal stromal tumor, germ cell tumor (such as extracranial, extragonadal, or ovarian germ cell tumor), gestational trophoblastic tumor, glioma (such as brain stem, childhood cerebral astrocytoma, or childhood visual pathway and hypothalamic glioma), gastric carcinoid, hairy cell leukemia, head and neck cancer, heart cancer, hepatocellular (liver) cancer, hodgkin lymphoma, hypopharyngeal cancer, hypothalamic and visual pathway glioma (such as childhood visual pathway glioma), islet cell carcinoma (such as endocrine or pancreas islet cell carcinoma), kaposi sarcoma, kidney cancer, laryngeal cancer, leukemia (such as acute lymphoblastic, acute myeloid, chronic lymphocytic, chronic myelogenous, or hairy cell leukemia), lip or oral cavity cancer, liposarcoma, liver cancer (such as non-small cell or small cell cancer), lung cancer, lymphoma (such as AIDS-related, burkitt, cutaneous T cell, Hodgkin, non-hodgkin, or central nervous system lymphoma), macroglobulinemia (such as waldenström macroglobulinemia, malignant fibrous histiocytoma of bone or osteosarcoma, medulloblastoma (such as childhood medulloblastoma), melanoma, merkel cell carcinoma, mesothelioma (such as adult or childhood mesothelioma), metastatic squamous neck cancer with occult, mouth cancer, multiple endocrine neoplasia syndrome (such as childhood multiple endocrine neoplasia syndrome), multiple myeloma or plasma cell neoplasm. mycosis fungoides, myelodysplastic syndrome, myelodysplastic or myeloproliferative disease, myelogenous leukemia (such as chronic myelogenous leukemia), myeloid leukemia (such as adult acute or childhood acute myeloid leukemia), myeloproliferative disorder (such as chronic myeloproliferative disorder), nasal cavity or paranasal sinus cancer, nasopharyngeal carcinoma, neuroblastoma, oral cancer, oropharyngeal cancer, osteosarcoma or malignant fibrous histiocytoma of bone, ovarian cancer, ovarian epithelial cancer, ovarian germ cell tumor, ovarian low malignant potential tumor, pancreatic cancer (such as islet cell pancreatic cancer), paranasal sinus or nasal cavity cancer, parathyroid cancer, penile cancer, pharyngeal cancer, pheochromocytoma, pineal astrocytoma, pineal germinoma. pineoblastoma or supratentorial primitive neuroectodermal tumor (such as childhood pineoblastoma or supratentorial primitive neuroectodermal tumor), pituitary adenoma, plasma cell neoplasia, pleuropulmonary blastoma, primary central nervous system lymphoma, cancer, rectal cancer, renal cell carcinoma, renal pelvis or ureter cancer (such as renal pelvis or ureter transitional cell cancer, retinoblastoma, rhabdomyosarcoma (such as childhood rhabdomyosarcoma), salivary gland cancer, sarcoma (such as sarcoma in the ewing family of tumors, Kaposi, soft tissue, or uterine sarcoma), sézary syndrome, skin cancer (such as nonmelanoma, melanoma, or merkel cell skin cancer), small intestine cancer, squamous cell carcinoma, supratentorial primitive neuroectodermal tumor (such as childhood supratentorial primitive neuroectodermal tumor), T-cell lymphoma (such as cutaneous T-cell lymphoma), testicular cancer, throat cancer, thymoma (such as childhood thymoma), thymoma or thymic carcinoma, thyroid cancer (such as childhood thyroid cancer), trophoblastic tumor (such as gestational trophoblastic tumor), unknown primary site carcinoma (such as adult or childhood unknown primary site carcinoma), urethral cancer (such as endometrial uterine cancer), uterine sarcoma, vaginal cancer, visual pathway or hypothalamic glioma (such as childhood visual pathway or hypothalamic glioma), vulvar cancer, waldenström macroglobulinemia, or wilms tumor (such as childhood wilms tumor). In various examples, the cancer has metastasized or has not metastasized.

The cancer may or may not be a hormone related or dependent cancer *(e.g.,* an estrogen or androgen related cancer). Benign tumors or malignant tumors may be diagnosed, prognosed, stabilized, treated, or prevented using the methods and/or compositions of the present disclosure.

In some examples, the subject has a cancer syndrome. A cancer syndrome is a genetic disorder in which genetic mutations in one or more genes predispose the affected individuals to the development of cancers and may also cause the early onset of these cancers. Cancer syndromes often show not only a high lifetime risk of developing cancer, but also the development of multiple independent primary tumors. Many of these syndromes are caused by mutations in tumor suppressor genes, genes that are involved in protecting the cell from turning cancerous. Other genes that may be affected are DNA repair genes, oncogenes and genes involved in the production of blood vessels (angiogenesis). Common examples of inherited cancer syndromes are hereditary breast-ovarian cancer syndrome and hereditary non-polyposis colon cancer (Lynch syndrome).

In some examples, a subject with one or more polymorphisms or mutations n K-ras, p53, BRA, EGFR, or HER2 is administered a treatment that targets K-ras, p53, BRA, EGFR, or HER2, respectively.

The methods of the present disclosure can be generally applied to the treatment of malignant or benign tumors of any cell, tissue, or organ type.

*11.5 Exemplary Treatments*

If desired, any treatment for stabilizing, treating, or preventing a disease or disorder such as cancer or an increased risk for a disease or disorder such as cancer can be administered to a subject (*e.g.*, a subject identified as having cancer or an increased risk for cancer using any of the methods of the present disclosure). In various examples, the treatment is a known treatment or combination of treatments for a disease or disorder such as cancer, including but not limited to cytotoxic agents, targeted therapy, immunotherapy, hormonal therapy, radiation therapy, surgical removal of cancerous cells or cells likely to become cancerous, stem cell transplantation, bone marrow transplantation, photodynamic therapy, palliative treatment, or a combination thereof. In some examples, a treatment (such as a preventative medication) is used to prevent, delay, or reduce the severity of a disease or disorder such as cancer in a subject at increased risk for a disease or disorder such as cancer. In some examples, the treatment is surgery, first-line chemotherapy, adjuvant therapy, or neoadjuvant therapy.

In some examples, the targeted therapy is a treatment that targets the cancer's specific genes, proteins, or the tissue environment that contributes to cancer growth and survival. This type of treatment blocks the growth and spread of cancer cells while limiting damage to normal cells, usually leading to fewer side effects than other cancer medications.

One of the more successful approaches has been to target angiogenesis, the new blood vessel growth around a tumor. Targeted therapies such as bevacizumab (Avastin), lenalidomide (Revlimid), sorafenib (Nexavar), sunitinib (Sutent), and thalidomide (Thalomid) interfere with angiogenesis. Another example is the use of a treatment that targets HER2, such as trastuzumab or lapatinib, for cancers that overexpress HER2 (such as some breast cancers). In some examples, a monoclonal antibody is used to block a specific target on the outside of cancer cells. Examples include alemtuzumab (Campath-1H), bevacizumab, cetuximab (Erbitux), panitumumab (Vectibix), pertuzumab (Omnitarg), rituximab (Rituxan), and trastuzumab. In some examples, the monoclonal antibody tositumomab (Bexxar) is used to deliver radiation to the tumor. In some examples, an oral small molecule inhibits a cancer process inside of a cancer cell.

Examples include dasatinib (Sprycel), erlotinib (Tarceva), gefitinib (Iressa), imatinib (Gleevec), lapatinib (Tykerb), nilotinib (Tasigna), sorafenib, sunitinib, and temsirolimus (Torisel). In some examples, a proteasome inhibitor (such as the multiple myeloma drug, bortezomib (Velcade)) interferes with specialized proteins called enzymes that break down other proteins in the cell.

In some examples, immunotherapy is designed to boost the body's natural defenses to fight the cancer. Exemplary types of immunotherapy use materials made either by the body or in a laboratory to bolster, target, or restore immune system function.

In some examples, hormonal therapy treats cancer by lowering the amounts of hormones in the body. Several types of cancer, including some breast and prostate cancers, only grow and spread in the presence of natural chemicals in the body called hormones. In various examples, hormonal therapy is used to treat cancers of the prostate, breast, thyroid, and reproductive system.

In some examples, the treatment includes a stem cell transplant in which diseased bone marrow is replaced by highly specialized cells, called hematopoietic stem cells. Hematopoietic stem cells are found both in the bloodstream and in the bone marrow.

In some examples, the treatment includes photodynamic therapy, which uses special drugs, called photosensitizing agents, along with light to kill cancer cells. The drugs work after they have been activated by certain kinds of light.

In some examples, the treatment includes surgical removal of cancerous cells or cells likely to become cancerous (such as a lumpectomy or a mastectomy). For example, a woman with a breast cancer susceptibility gene mutation (*BRCA1* or *BRCA2* gene mutation) may reduce her risk of breast and ovarian cancer with a risk reducing salpingo-oophorectomy (removal of the fallopian tubes and ovaries) and/or a risk reducing bilateral mastectomy (removal of both breasts). Lasers, which are very powerful, precise beams of light, can be used instead of blades (scalpels) for very careful surgical work, including treating some cancers.

In addition to treatment to slow, stop, or eliminate the cancer (also called disease-directed treatment), an important part of cancer care is relieving a subject's symptoms and side effects, such as pain and nausea. It includes supporting the subject with physical, emotional, and social needs, an approach called palliative or supportive care. People often receive disease-directed therapy and treatment to ease symptoms at the same time.

Exemplary treatments include actinomycin D, adcetris, Adriamycin, aldesleukin, alemtuzumab, alimta, amsidine, amsacrine, anastrozole, aredia, arimidex, aromasin, asparaginase, avastin, bevacizumab, bicalutamide, bleomycin, bondronat, bonefos, bortezomib, busilvex, busulphan, campto, capecitabine, carboplatin, carmustine, casodex, cetuximab, chimax, chlorambucil, cimetidine, cisplatin, cladribine, clodronate, clofarabine, crisantaspase, cyclophos-sphamide, cyproterone acetate, cyprostat, cytarabine, cytoxan, dacarbozine, dactinomycin, dasatinib, daunorubicin, dexamethasone, diethylstilbestrol, docetaxel, doxorubicin, drogenil, emcyt, epirubicin, eposin, Erbitux, erlotinib, estracyte, estramustine, etopophos, etoposide, evoltra, exemestane, fareston, femara, filgrastim, fludara, fludarabine, fluorouracil, flutamide, gefinitib, gemcitabine, gemzar, gleevec, glivec. gonapeptyl depot, goserelin, halaven, herceptin, hycamptin, hydroxycarbamide, ibandronic acid, ibritumomab, idarubicin, ifosfomide, interferon, imatinib mesylate, iressa, irinotecan, jevtana, lanvis, lapatinib, letrozole, leukeran, leuprorelin, leustat, lomustine, mabcampath, mabthera, megace, megestrol, methotrexate, mitozantrone, mitomycin, mutulane, myleran, navelbine, neulasta, neupogen, nexavar, nipent, nolvadex D, novantron, oncovin, paclitaxel, pamidronate, PCV, pemetrexed, pentostatin, perjeta, procarbazine, provenge, prednisolone, prostrap, raltitrexed, rituximab, sprycel, sorafenib, soltamox, streptozocin, stilboestrol, stimuvax, sunitinib, sutent, tabloid, tagamet, tamofen, tamoxifen, tarceva, taxol, taxotere, tegafur with uracil, temodal, temozolomide, thalidomide, thioplex, thiotepa, tioguanine, tomudex, topotecan, toremifene, trastuzumab, tretinoin, treosulfan, triethylenethiophorsphoramide, triptorelin, tyverb, uftoral, velcade, vepesid, vesanoid, vincristine, vinorelbine, xalkori, xeloda, yervoy, zactima, zanosar, zavedos, zevelin, zoladex, zoledronate, zometa zoledronic acid, and zytiga.

In some examples, the cancer is breast cancer and the treatment or compound administered to the individual is one or more of: Abemaciclib, Abraxane (Paclitaxel Albumin-stabilized Nanoparticle Formulation), Ado-Trastuzumab Emtansine, Afinitor (Everolimus), Anastrozole, Aredia (Pamidronate Disodium), Arimidex (Anastrozole), Aromasin (Exemestane), Capecitabine, Cyclophosphamide, Docetaxel, Doxorubicin Hydrochloride, Ellence (Epirubicin Hydrochloride), Epirubicin Hydrochloride, Eribulin Mesylate, Everolimus, Exemestane, 5-FU (Fluorouracil Injection), Fareston (Toremifene), Faslodex (Fulvestrant), Femara (Letrozole), Fluorouracil Injection, Fulvestrant, Gemcitabine Hydrochloride, Gemzar (Gemcitabine Hydrochloride), Goserelin Acetate, Halaven (Eribulin Mesylate), Herceptin (Trastuzumab), Ibrance (Palbociclib), Ixabepilone, Ixempra (Ixabepilone), Kadcyla (Ado-Trastuzumab Emtansine), Kisqali (Ribociclib), Lapatinib Ditosylate, Letrozole, Lynparza (Olaparib), Megestrol Acetate, Methotrexate, Neratinib Maleate, Nerlynx (Neratinib Maleate), Olaparib, Paclitaxel, Paclitaxel Albumin-stabilized Nanoparticle Formulation, Palbociclib, Pamidronate Disodium, Perjeta (Pertuzumab), Pertuzumab, Ribociclib, Tamoxifen Citrate, Taxol (Paclitaxel), Taxotere (Docetaxel), Thiotepa, Toremifene, Trastuzumab, Trexall (Methotrexate), Tykerb (Lapatinib Ditosylate), Verzenio (Abemaciclib), Vinblastine Sulfate, Xeloda (Capecitabine), Zoladex (Goserelin Acetate), Evista (Raloxifene Hydrochloride), Raloxifene Hydrochloride, Tamoxifen Citrate. In some examples, the cancer is breast cancer and the treatment or compound administered to the individual is a combination selected from: Doxorubicin Hydrochloride (Adriamycin) and Cyclophosphamide; Doxorubicin Hydrochloride (Adriamycin), Cyclophosphamide, and Paclitaxel (Taxol); Doxorubicin Hydrochloride (Adriamycin), Cyclophosphamide, and Fluorouracil; Methotrexate, Cyclophosphamide, and Fluorouracil; Epirubicin Hydrochloride, Cyclophosphamide, and Fluorouracil; and Doxorubicin Hydrochloride (Adriamycin), Cyclophosphamide, and Docetaxel (Taxotere).

For subjects that express both a mutant form (*e.g.,* a cancer-related form) and a wild-type form (*e.g.,* a form not associated with cancer) of an mRNA or protein, the therapy preferably inhibits the expression or activity of the mutant form by at least 2, 5, 10, or 20-fold more than it inhibits the expression or activity of the wild-type form. The simultaneous or sequential use of multiple therapeutic agents may greatly reduce the incidence of cancer and reduce the number of treated cancers that become resistant to therapy. In addition, therapeutic agents that are used as part of a combination therapy may require a lower dose to treat cancer than the corresponding dose required when the therapeutic agents are used individually. The low dose of each compound in the combination therapy reduces the severity of potential adverse side-effects from the compounds.

In some examples, a subject identified as having an increased risk of cancer may (using the methods of the present disclosure or any standard method), avoid specific risk factors, or make lifestyle changes to reduce any additional risk of cancer.

In some examples, the polymorphisms, mutations, risk factors, or any combination thereof may be used to select a treatment regimen for the subject. In some examples, a larger dose or greater number of treatments is selected for a subject at greater risk of cancer or with a worse prognosis.

11.5.1 Other Compounds for Inclusion in Individual or Combination Therapies

If desired, additional compounds for stabilizing, treating, or preventing a disease or disorder such as cancer or an increased risk for a disease or disorder such as cancer may be identified from large libraries of both natural product or synthetic (or semi-synthetic) extracts or chemical libraries according to methods known in the art. Those skilled in the field or drug discovery and development will understand that the precise source of test extracts or compounds is not critical to the methods of the present disclosure. Accordingly, virtually any number of chemical extracts or compounds can be screened for their effect on cells from a particular type of cancer or from a particular subject or screened for their effect on the activity or expression of cancer related molecules (such as cancer related molecules known to have altered activity or expression in a particular type of cancer). When a crude extract is found to modulate the activity or expression of a cancer related molecule, further fractionation of the positive lead extract may be performed to isolate chemical constituent responsible for the observed effect using methods known in the art.

11.5.2 Exemplary Assays and Animal Models for the Testing of Therapies

If desired, one or more of the treatment disclosed herein can be tested for their effect on a disease or disorder such as cancer using a cell line (such as a cell line with one or more of the mutations identified in the subject who has been diagnosed with cancer or an increased risk of cancer using the methods of the present disclosure) or an animal model of the disease or disorder, such as a SCID mouse model (Jain et al., Tumor Models In Cancer Research, ed. Teicher, Humana Press Inc., Totowa, N.J., pp. 647-671, 2001). Additionally, there are numerous standard assays and animal models that can be used to determine the efficacy of particular therapies for stabilizing, treating, or preventing a disease or disorder such as cancer or an increased risk for a disease or disorder such as cancer. Therapies can also be tested in standard human clinical trials.

For the selection of a preferred therapy for a particular subject, compounds can be tested for their effect on the expression or activity on one or more genes that are mutated in the subject. For example, the ability of a compound to modulate the expression of particular mRNA molecules or proteins can be detected using standard Northern, Western, or microarray analysis. In some examples, one or more compounds are selected that (i) inhibit the expression or activity of mRNA molecules or proteins that promote cancer that are expressed at a higher than normal level or have a higher than normal level of activity in the subject (such as in a sample from the subject) or (ii) promote the expression or activity of mRNA molecules or proteins that inhibit cancer that are expressed at a lower than normal level or have a lower than normal level of activity in the subject. An individual or combination therapy that (i) modulates the greatest number of mRNA molecules or proteins that have mutations associated with cancer in the subject and (ii) modulates the least number of mRNA molecules or proteins that do not have mutations associated with cancer in the subject. In some examples, the selected individual or combination therapy has high drug efficacy and produces few, if any, adverse side-effects.

As an alternative to the subject-specific analysis described above, DNA chips can be used to compare the expression of mRNA molecules in a particular type of early or late-stage cancer (*e.g.,* breast cancer cells) to the expression in normal tissue (Marrack et al., Current Opinion in Immunology 12, 206-209, 2000; Harkin, Oncologist. 5:501-507, 2000; Pelizzari et al., Nucleic Acids Res. 28(22):4577-4581, 2000). Based on this analysis, an individual or combination therapy for subjects with this type of cancer can be selected to modulate the expression of the mRNA or proteins that have altered expression in this type of cancer.

In addition to being used to select a therapy for a particular subject or group of subjects, expression profiling can be used to monitor the changes in mRNA and/or protein expression that occur during treatment. For example, expression profiling can be used to determine whether the expression of cancer related genes has returned to normal levels. If not, the dose of one or more compounds in the therapy can be altered to either increase or decrease the effect of the therapy on the expression levels of the corresponding cancer related gene(s). In addition, this analysis can be used to determine whether a therapy affects the expression of other genes (*e.g.*, genes that are associated with adverse side-effects). If desired, the dose or composition of the therapy can be altered to prevent or reduce undesired side-effects.

11.5.3 Exemplary Formulations and Methods of Administration

For stabilizing, treating, or preventing a disease or disorder such as cancer or an increased risk for a disease or disorder such as cancer, a composition may be formulated and administered using any method known to those of skill in the art (see, *e.g.,* U.S. Pat. Nos. 8,389,578 and 8,389,557). General techniques for formulation and administration are found in "Remington: The Science and Practice of Pharmacy," 21st Edition, Ed. David Troy, 2006, Lippincott Williams & Wilkins, Philadelphia, Pa.). Liquids, slurries, tablets, capsules, pills, powders, granules, gels, ointments, suppositories, injections, inhalants, and aerosols are examples of such formulations. By way of example, modified or extended release oral formulation can be prepared using additional methods known in the art. For example, a suitable extended release form of an active ingredient may be a matrix tablet or capsule composition. Suitable matrix forming materials include, for example, waxes (*e.g.*, carnauba, bees wax, paraffin wax, ceresine, shellac wax, fatty acids, and fatty alcohols), oils, hardened oils or fats (*e.g.*, hardened rapeseed oil, castor oil, beef tallow, palm oil, and soya bean oil), and polymers (*e.g.*, hydroxypropyl cellulose, polyvinylpyrrolidone, hydroxypropyl methyl cellulose, and polyethylene glycol). Other suitable matrix tabletting materials are microcrystalline cellulose, powdered cellulose, hydroxypropyl cellulose, ethyl cellulose, with other carriers, and fillers. Tablets may also contain granulates, coated powders, or pellets. Tablets may also be multi-layered. Optionally, the finished tablet may be coated or uncoated.

Typical routes of administering such compositions include, without limitation, oral, sublingual, buccal, topical, transdermal, inhalation, parenteral (*e.g.*, subcutaneous, intravenous, intramuscular, intrasternal injection, or infusion techniques), rectal, vaginal, and intranasal. In some examples, the therapy may be administered using an extended release device. Compositions of the present disclosre are formulated so as to allow the active ingredient(s) contained therein to be bioavailable upon administration of the composition. Compositions may take the form of one or more dosage units. Compositions may contain 1, 2, 3, 4, or more active ingredients and may optionally contain 1, 2, 3, 4, or more inactive ingredients.

## 12. Definitions

The following definitions are provided to facilitate understanding of certain terms used throughout this specification.

Technical and scientific terms used herein have the meanings commonly understood by one of ordinary skill in the art, unless otherwise defined. Any suitable materials and/or methodologies known to those of ordinary skill in the art can be utilized in carrying out the methods described herein.

As used in the description of the present disclosre and the appended claims, the singular forms "a", "an" and "the" are used interchangeably and intended to include the plural forms as well and fall within each meaning, unless the context clearly indicates otherwise. Also, as used herein, "and/or" refers to and encompasses any and all possible combinations of one or more of the listed items, as well as the lack of combinations when interpreted in the alternative ("or").

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

"Single Nucleotide Polymorphism (SNP)" refers to a single nucleotide that may differ between the genomes of two members of the same species. The usage of the term should not imply any limit on the frequency with which each variant occurs.

"Sequence" refers to a DNA sequence or a genetic sequence. It may refer to the primary, physical structure of the DNA molecule or strand in an individual. It may refer to the sequence of nucleotides found in that DNA molecule, or the complementary strand to the DNA molecule. It may refer to the information contained in the DNA molecule as its representation in silico.

"Locus" refers to a particular region of interest on the DNA of an individual, which may refer to a SNP, the site of a possible insertion or deletion, or the site of some other relevant genetic variation. Disease-linked SNPs may also refer to disease-linked loci.

"Polymorphic Allele," also "Polymorphic Locus," refers to an allele or locus where the genotype varies between individuals within a given species. Some examples of polymorphic alleles include single nucleotide polymorphisms, short tandem repeats, deletions, duplications, and inversions.

"Polymorphic Site" refers to the specific nucleotides found in a polymorphic region that vary between individuals.

"Allele" refers to the genes that occupy a particular locus.

"Genetic Data" also "Genotypic Data" refers to the data describing teachings of the genome of one or more individuals. It may refer to one or a set of loci, partial or entire sequences, partial or entire chromosomes, or the entire genome. It may refer to the identity of one or a plurality of nucleotides; it may refer to a set of sequential nucleotides, or nucleotides from different locations in the genome, or a combination thereof. Genotypic data is typically in silico, however, it is also possible to consider physical nucleotides in a sequence as chemically encoded genetic data. Genotypic Data may be said to be "on," "of," "at," "from" or "on" the individual(s). Genotypic Data may refer to output measurements from a genotyping platform where those measurements are made on genetic material.

"Genetic Material" also "Genetic Sample" refers to physical matter, such as tissue or blood, from one or more individuals comprising DNA or RNA.

"Confidence" refers to the statistical likelihood that the called SNP, allele, set of alleles, ploidy call, or paternity call is correct.

"Aneuploidy" refers to the state where the wrong number of chromosomes is present in a cell. In the case of a somatic human cell it may refer to the case where a cell does not contain 22 pairs of autosomal chromosomes and one pair of sex chromosomes. In the case of a human gamete, it may refer to the case where a cell does not contain one of each of the 23 chromosomes. In the case of a single chromosome type, it may refer to the case where more or less than two homologous but non-identical chromosome copies are present, or where there are two chromosome copies present that originate from the same parent.

"Chromosome" may refer to a single chromosome copy, meaning a single molecule of DNA of which there are 46 in a normal somatic cell; an example is the maternally derived chromosome 18'. Chromosome may also refer to a chromosome type, of which there are 23 in a normal human somatic cell; an example is chromosome 18'.

"Monosomy" refers to the state where a cell only contains one of a chromosome type.

"Disomy" refers to the state where a cell contains two of a chromosome type.

"Uniparental Disomy" refers to the state where a cell contains two of a chromosome type, and where both chromosomes originate from one parent.

"Trisomy" refers to the state where a cell contains three of a chromosome type.

The State of the Genetic Material or simply "Genetic State" may refer to the identity of a set of SNPs on the DNA, to the phased haplotypes of the genetic material, or to the sequence of the DNA, including insertions, deletions, repeats and mutations. It may also refer to the ploidy state of one or more chromosomes, chromosomal segments, or set of chromosomal segments.

"Establishing the Paternity" or "Determining the Paternity" refers to establishing or determining that an alleged father either is or is not the biological father of a gestating fetus, or determining or establishing the likelihood that an alleged father is the biological father of the fetus.

"Allelic Ratio" refers to the ratio between the amount of each allele at a polymorphic locus that is present in a sample or in an individual. When the sample is measured by sequencing, the allelic ratio may refer to the ratio of sequence reads that map to each allele at the locus. When the sample is measured by an intensity based measurement method, the allele ratio may refer to the ratio of the amounts of each allele present at that locus as estimated by the measurement method.

"Allelic Distribution," or allele count distribution refers to the relative amount of each allele that is present for each locus in a set of loci. An allelic distribution can refer to an individual, to a sample, or to a set of measurements made on a sample. In the context of sequencing, the allelic distribution refers to the number or probable number of reads that map to a particular allele for each allele in a set of polymorphic loci. The allele measurements may be treated probabilistically, that is, the likelihood that a given allele is present for a give sequence read is a fraction between 0 and 1, or they may be treated in a binary fashion, that is, any given read is considered to be exactly zero or one copies of a particular allele.

"Allelic Bias" refers to the degree to which the measured ratio of alleles at a heterozygous locus is different to the ratio that was present in the original sample of DNA. The degree of allelic bias at a particular locus is equal to the observed allelelic ratio at that locus, as measured, divided by the ratio of alleles in the original DNA sample at that locus. Allelic bias may be defined to be greater than one, such that if the calculation of the degree of allelic bias returns a value, x, that is less than 1, then the degree of allelic bias may be restated as 1/x. Allelic bias maybe due to amplification bias, purification bias, or some other phenomenon that affects different alleles differently.

"Primer," also "PCR probe" refers to a single DNA molecule (a DNA oligomer) or a collection of DNA molecules (DNA oligomers) where the DNA molecules are identical, or nearly so, and where the primer contains a region that is designed to hybridize to a targeted polymorphic locus, and may contain a priming sequence designed to allow PCR amplification. A primer may also contain a molecular barcode. A primer may contain a random region that differs for each individual molecule.

"Hybrid Capture Probe" refers to any nucleic acid sequence, possibly modified, that is generated by various methods such as PCR or direct synthesis and intended to be complementary to one strand of a specific target DNA sequence in a sample. The exogenous hybrid capture probes may be added to a prepared sample and hybridized through a denature-reannealing process to form duplexes of exogenous-endogenous fragments. These duplexes may then be physically separated from the sample by various means.

"Sequence Read" refers to data representing a sequence of nucleotide bases that were measured using a clonal sequencing method. Clonal sequencing may produce sequence data representing single, or clones, or clusters of one original DNA molecule. A sequence read may also have associated quality score at each base position of the sequence indicating the probability that nucleotide has been called correctly.

Mapping a sequence read is the process of determining a sequence read's location of origin in the genome sequence of a particular organism. The location of origin of sequence reads is based on similarity of nucleotide sequence of the read and the genome sequence.

"Homozygous" refers to having similar alleles at corresponding chromosomal loci.

"Heterozygous" refers to having dissimilar alleles at corresponding chromosomal loci.

"Heterozygosity Rate" refers to the rate of individuals in the population having heterozygous alleles at a given locus. The heterozygosity rate may also refer to the expected or measured ratio of alleles, at a given locus in an individual, or a sample of DNA.

"Haplotype" refers to a combination of alleles at multiple loci that are typically inherited together on the same chromosome. Haplotype may refer to as few as two loci or to an entire chromosome depending on the number of recombination events that have occurred between a given set of loci. Haplotype can also refer to a set of single nucleotide polymorphisms (SNPs) on a single chromatid that are statistically associated.

"Haplotypic Data," also "Phased Data" or "Ordered Genetic Data," refers to data from a single chromosome in a diploid or polyploid genome, i.e., either the segregated maternal or paternal copy of a chromosome in a diploid genome.

"Phasing" refers to the act of determining the haplotypic genetic data of an individual given unordered, diploid (or polyploid) genetic data. It may refer to the act of determining which of two genes at an allele, for a set of alleles found on one chromosome, are associated with each of the two homologous chromosomes in an individual.

"Phased Data" refers to genetic data where one or more haplotypes have been determined.

"Fetal" refers to of the fetus, or of the region of the placenta that is genetically similar to the fetus. In a pregnant woman, some portion of the placenta is genetically similar to the fetus, and the free floating fetal DNA found in maternal blood may have originated from the portion of the placenta with a genotype that matches the fetus.

"DNA of Fetal Origin" refers to DNA that was originally part of a cell whose genotype was essentially equivalent to that of the fetus. Note that the genetic information in half of the chromosomes in a fetus is inherited from the mother of the fetus; in some examples, the DNA from these maternally inherited chromosomes that came from a fetal cell is considered to be "of fetal origin," and not "of maternal origin."

"DNA of Maternal Origin" refers to DNA that was originally part of a cell whose genotype was essentially equivalent to that of the mother.

"Cell of Fetal Origin" refers to a circulating cell confirmed to have originally been part of the fetus.

"Cell of Maternal Origin" refers to a circulating cell confirmed to be a maternal cell that was not originally a part of the fetus.

"Child" may refer to an embryo, a blastomere, or a fetus. Note that in the presently disclosed examples, the concepts described apply equally well to individuals who are a born child, a fetus, an embryo or a set of cells therefrom. The use of the term child may simply be meant to connote that the individual referred to as the child is the genetic offspring of the parents. "Parent" refers to the genetic mother or father of an individual. An individual typically has two parents, a mother and a father, though this may not necessarily be the case such as in genetic or chromosomal chimerism. "Mother" may refer to the biological mother of an individual, and/or it may refer to the women who is carrying the individual as he/she gestates.

"Parental Context" refers to the genetic state of a given SNP, on each of the two relevant chromosomes for one or both of the two parents of the target.

"Maternal Plasma" refers to the plasma portion of the blood from a female who is pregnant.

"Clinical Decision" refers to any decision to take or not take an action that has an outcome that affects the health or survival of an individual. In the context of prenatal paternity testing, a clinical decision may refer to a decision to abort or not abort a fetus. A clinical decision may also refer to a decision to conduct further testing, or to take actions to prepare for the birth of a child.

"Diagnostic Box" refers to one or a combination of machines designed to perform one or a plurality of teachings of the methods disclosed herein. In an example, the diagnostic box may be placed at a point of patient care. In an example, the diagnostic box may perform targeted amplification followed by sequencing. In an examplesthe diagnostic box may function alone or with the help of a technician.

"Informatics Based Method" or "informatics based approach" refers to a method that relies heavily on statistics to make sense of a large amount of data. In the context of prenatal diagnosis, it refers to a method designed to determine the ploidy state at one or more chromosomes or the allelic state at one or more alleles by statistically inferring the most likely state, rather than by directly physically measuring the state, given a large amount of genetic data, for example from a molecular array or sequencing. In an example of the present disclosure, the informatics based technique may be one disclosed in this patent. In an example of the present disclosure it may be PARENTAL SUPPORT™

Preferential Enrichment of DNA that corresponds to one or a plurality of loci, or preferential enrichment of DNA at one or a plurality of loci, refers to any method that results in the percentage of molecules of DNA in a post-enrichment DNA mixture that correspond to the loci being higher than the percentage of molecules of DNA in the pre-enrichment DNA mixture that correspond to the loci. The method may involve selective amplification of DNA molecules that correspond to the loci. The method may involve removing DNA molecules that do not correspond to the loci.

"Amplification" refers to a method that increases the number of copies of a molecule of DNA.

"Selective Amplification" may refer to a method that increases the number of copies of a particular molecule of DNA, or molecules of DNA that correspond to a particular region of DNA. It may also refer to a method that increases the number of copies of a particular targeted molecule of DNA, or targeted region of DNA more than it increases non-targeted molecules or regions of DNA. Selective amplification may be a method of preferential enrichment.

"Universal Priming Sequence" refers to a DNA sequence that may be appended to a population of target DNA molecules, for example by ligation, PCR, or ligation mediated PCR. Once added to the population of target molecules, primers specific to the universal priming sequences can be used to amplify the target population using a single pair of amplification primers. Universal priming sequences are typically not related to the target sequences.

"Universal Adapters," or "ligation adaptors" or "library tags" are DNA molecules containing a universal priming sequence that can be covalently linked to the 5-prime and 3-prime end of a population of target double stranded DNA molecules. The addition of the adapters provides universal priming sequences to the 5-prime and 3-prime end of the target population from which PCR amplification can take place, amplifying all molecules from the target population, using a single pair of amplification primers.

Targeting refers to a method used to selectively amplify or otherwise preferentially enrich those molecules of DNA that correspond to a set of loci, in a mixture of DNA.

Determining, establishing, and calculating may be used interchangeably.

The following examples are put forth so as to provide those of ordinary skill in the art with a complete disclosure and description of how to use the examples provided herein, and are not intended to limit the scope of the disclosure nor are they intended to represent that the Examples below are all or the only experiments performed. Efforts have been made to ensure accuracy with respect to numbers used (e.g. amounts, temperature, etc.) but some experimental errors and deviations should be accounted for. Unless indicated otherwise, parts are parts by volume, and temperature is in degrees Centigrade. It should be understood that variations in the methods as described can be made without changing the fundamental aspects that the Examples are meant to illustrate.

## WORKING EXAMPLES

### EXAMPLE 1.

The purpose of this example was to develop a strategy for enriching DNA from a small number of cells. In particular, this example demonstrated enrichment of DNA from one single isolated fetal cell.

The model system shown in this example was designed for assessing enrichment efficiency of rare cells from blood, and in particular fetal cell enrichment and isolation. The cell line AG16777 was used for cells of fetal origin (female), the cell line AG16778 was used for cells of maternal origin, and the mixture of DNA from them as cfDNA-like reference.

The output of fetal cell enrichment process was a cell mixture in levels of ones, tens, or hundreds of cells depending on an enrichment platform. A method for assessing fetal cell enrichment efficiency needed to be able to identify fetal cell(s) and accurately estimate fetal fractions using as little as a few cells and even a single cell.

The cell mixture was generated using manual micro-pipetting of AG16777 and AG16778 cells (trisomy 21 daughter/mother pair) individually and mixing the cells at different ratios, (e.g., 100:0, 99:1, 98:2, 97:3, 96:4, 95:5, 94:6, 93:7, 92:8, 91:9, 90:10, 89:11, 88:12, 87:13, 86:14, 85:15, 84:16, 83:17, 82:18, 81:19, 80:20, 79:21, 78:22, 77:23, 76:24, 75:25, 74:26, 73:27, 72:28, 71:29, 70:30, 69:31, 68:32, 67:33, 66:34, 65:35, 64:36, 63:37, 62:38, 61:39, 60:40, 59:41, 58:42, 57:43, 56:44, 55:45, 54:46, 53:47, 52:48, 51:49, 50:50, 49:51, 48:52, 47:53, 46:54, 45:55, 44:56, 43:57, 42:58, 41:59, 40:60, 39:61, 38:62, 37:63, 36:64, 35:65, 34:66, 33:67, 32:68, 31:69, 30:70, 29:71, 28:72, 27:73, 26:74, 25:75, 24:76, 23:77, 22:78, 21:79, 20:80, 19:81, 18:82, 17:83, 16:84, 15:85, 14:86, 13:87, 12:88, 11:89, 10:90, 9:91, 8:92, 7:93, 6:94, 5:95, 4:96, 3:97, 2:98, 1:99, or 0:100).

To prepare the cells for PCR amplification, the cells were lysed using Proteinase K digestion.

The DNA from the target cells was amplified by using highly multiplexed PCR amplification of more than 13,000 single nucleotide polymorphisms (SNPs) selected as target loci. Exemplary workflow and conditions for the multiplex PCR amplification reactions are shown in Figure 6.

The method was also used for analysis of single cell samples. When using single cell isolated from cultured cell lines (AG16777, AG16778) as sample input, the Fetal Cell Workflow was shown to successfully generate hetrate plots comparable to those using standard sample input, therefore allowing the whole chromosomal aneuploidy to be determined using single cell.

Other than its use in fetal cell isolation, the workflow can potentially be used to determine copy number variation (CNV) in tumor setting (Research workflow and data 2014) and the presence of donor cells in organ transplant patients.

Our SNP-based NGS method ran the cell samples without WGA (whole genome amplification) pre-amplification. Omitting WGA makes the present method cost-effective and time-saving compared to WGA dependent conventional methods. Most importantly, omitting WGA avoided PCR bias from the WGA step, reduced errors, and generated more reliable data for genotyping.

## EXAMPLE 2.

The purpose of this example was to show that methods and compositions disclosed herein can be used to analyze single cells.

The cell line AG16777 was used as a fetal cell. Under microscope, each individual cell was picked using microcapillary pipet into a PCR tube contained 5 μL of wash buffer (0.1% Bovine Serum Albumin in PBS buffer). Table 2 below shows samples comprising different number of cells used in this example.

Table 2: Samples used for Example 2

| Sample Name | Number of Fetal Cells |
|:-----------:|:---------------------:|
| 1F | 1 |
| 2F | 2 |
| 4F | 4 |
| 10F | 10 |
| 20F | 20 |

To each of the samples shown in Table 2, 6 μL of lysis buffer (Proteinase K in Arcturus Reconstitution Buffer) was added. The reaction mixture was incubated at 56°C for 60 minutes followed by 95°C for 10 minutes. (Note: This procedure can be carried out in a thermocycler.) The sample mixtures (cell lysate) were cooled down and before they were run through the amplification workflow for fetal cells as described in Example 1. In particular, for the STAR 1 stage, 7.76 μL gDNA STAR1 Primer Mix, 20 μL 4x Master Mix and 0.24 μL 5M TMAC was added to the samples. Then, the reaction mixtures were amplified using the PCR protocol outlined in Table 3 below.

Table 3: PCR reaction for STAR1 stage of the PCR amplification workflow.

| Step | # of Cycles | Temperature | Time |
|------|:-----------:|:-----------:|:----:|
| Hold | 1 | 95°C | 10 min |
| Cycle | 25 | 96°C | 30 sec |
| | | 65°C | 30 min |
| | | 72°C | 30 sec |
| Hold | 1 | 72°C | 2 min |
| Hold | 1 | 4°C | Infinite |

For the STAR2 stage of the PCR workflow, the STAR1 PCR products were diluted by water by 1:200. To form the amplification mixture for this stage, 2uL of the diluted STAR1 product was mixed with 2.94 μL gDNA STAR2 Primer Mix, 2.5 μL 4x Master Mix, 2.5 μL water and 0.06 μL 5M tetramethyl ammonium chloride (TMAC). The STAR2 reaction mixtures were amplified using the PCR protocol outlined in Table 4 below.

Table 4: STAR2 PCR protocol

| Step | # of Cycles | Temperature | Time |
|---|---|---|---|
| Hold | 1 | 95°C | 10 min |
| Cycle | 15 | 95°C | 30 sec |
| | | 65°C | 1 min |
| | | 60°C | 5 min |
| | | 65°C | 5 min |
| | | 72°C | 30 sec |
| Hold | 1 | 72°C | 2 min |
| Hold | 1 | 4°C | Infinite |

For the barcoding stage of the PCR workflow, The STAR2 PCR products were diluted by water by 1:40. To form the amplification mixture for barcoding, 2 μL of the diluted STAR2 product was mixed with 1 μL 10 μM F-BC, 2 μL NS2 Barcode (5μM), 2.5 μL 4x Master Mix and 2.5 μL water were mixed together. The PCR protocol for the barcoding PCR is shown in Table 5 below.

Table 5: PCR protocol for barcoding the STAR2 PCR products.

| Step | # of Cycles | Temperature | Time |
|---|---|---|---|
| Hold | 1 | 95°C | 10 min |
| Cycle | 12 | 95°C | 30 sec |
| | | 70°C | 10 sec |
| | | 60°C | 30 sec |
| | | 65°C | 15 sec |
| | | 72°C | 15 sec |
| Hold | 1 | 72°C | 2 min |
| Hold | 1 | 4°C | Infinite |

The barcoded products were then pooled, purified, and quantitated. The pooled samples were sequenced using next generation sequencing (NGS) technology.

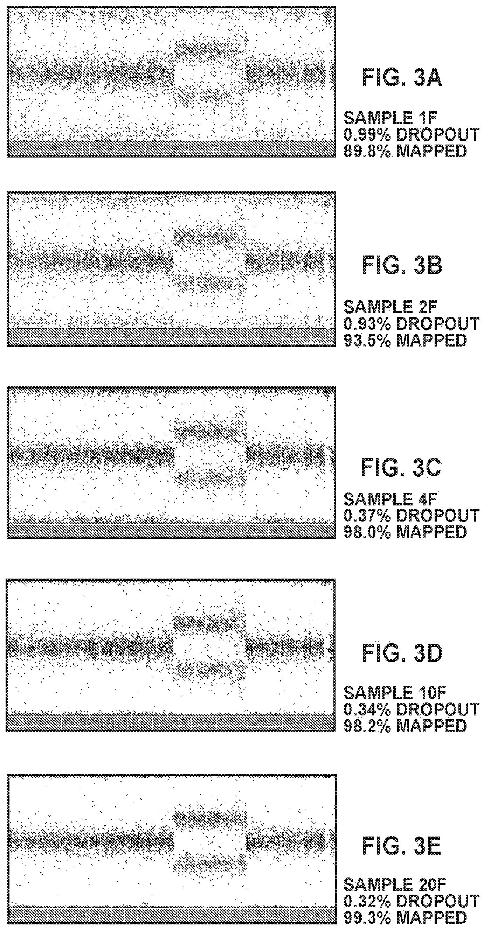

The sequencing data were then analyzed using the data analysis methods described herein to generate Hetrate plots of SNPs of chromosomes 13, 18, 21, and X/Y as shown in Figure 3. Data analysis of copy number variation showed that the trisomy 21 (Down Syndrome) existed in the analyzed cell line. The presence of trisomy 21 was clearly seen even in the sample confirmed to contain only one single cell (Figure 3A). Therefore, this method display superior sensitivity compared to currently used tests based on using cfDNA.

**EXAMPLE 3.**

The purpose of this example was to test the methods of analyzing and characterizing circulating cells disclosed herein on mixtures of cells.

The cell mixture samples were prepared by picking individual fetal cells (AG16777) and individual maternal cells (AG16778) with a microcapillary pipet under a microscope. The individually picked fetal and maternal cells were then mixed in certain ratios as shown in Table 6 below.

Table 6: Cell mixture samples

| Sample Name | Components of the Sample | |
|---|---|---|
| 10F | 10 Fetal Cells | 0 Maternal Cell |
| 9F1M | 9 Fetal Cells | 1 Maternal Cell |
| 7F3M | 7 Fetal Cells | 3 Maternal Cells |

**EP 3 899 030 B1**

(continued)

| Sample Name | Components of the Sample | |
|---|---|---|
| 5F5M | 5 Fetal Cells | 5 Maternal Cells |
| 3F7M | 3 Fetal Cells | 7 Maternal Cells |
| 1F4M | 1 Fetal Cell | 4 Maternal Cells |
| 1F9M | 1 Fetal Cell | 9 Maternal Cells |
| 1F19M | 1 Fetal Cell | 19 Maternal Cells |
| 10M | 0 Fetal Cell | 10 Maternal Cells |
| 10% CF Mix | 10% Child's DNA with Mom's DNA | |

In each sample tube, there cells were suspended in 5 $\mu$L of wash buffer (0.1% Bovine Serum Albumin in PBS buffer). To lyse the cells, 6 $\mu$L of lysis buffer (Proteinase K in Arcturus Reconstitution Buffer) was added to each sample. The reaction mixture was then incubated at 56°C for 60 minutes followed by 95°C for 10 minutes. (Note: This procedure can be carried out in a thermocycler.) The mixture (cell lysate) was cooled down before proceeding to the PCR amplification workflow for fetal cells as described in Example 1.

For the STAR1 stage of the PCR workflow, 7.76 $\mu$L gDNA STAR1 Primer Mix, 20 $\mu$L 4x Master Mix and 0.24 $\mu$L 5M TMAC was added to each sample. The PCR protocol for the STAR1 stage of the PCR workflow is shown in Table 7 below.

Table 7: STAR1 PCR protocol used in Example 3.

| Step | # of Cycles | Temperature | Time |
|---|---|---|---|
| Hold | 1 | 95°C | 10 min |
| Cycle | 25 | 96°C | 30 sec |
| | | 65°C | 30 min |
| | | 72°C | 30 sec |
| Hold | 1 | 72°C | 2 min |
| Hold | 1 | 4°C | Infinite |

For the STAR2 stage of the PCR workflow, the STAR1 PCR products were diluted 1:200 by water. To form the amplification mixture for the STAR2 amplification, 2 $\mu$L of the diluted STAR1 product was mixed with 2.94 $\mu$L gDNA STAR2 Primer Mix, 2.5 $\mu$L 4x Master Mix, 2.5 $\mu$L water, and 0.06 $\mu$L 5M TMAC. The PCR protocol for the STAR2 reaction is shown in Table 8 below.

Table 8: PCR protocol for the STAR2 reaction in Example 3.

| Step | # of Cycles | Temperature | Time |
|---|---|---|---|
| Hold | 1 | 95°C | 10 min |
| Cycle | 15 | 95°C | 30 sec |
| | | 65°C | 1 min |
| | | 60°C | 5 min |
| | | 65°C | 5 min |
| | | 72°C | 30 sec |
| Hold | 1 | 72°C | 2 min |
| Hold | 1 | 4°C | Infinite |

For the barcoding stage of the PCR workflow, the STAR2 PCR products were diluted 1:40 with water. To form the amplification mixture for barcoding, 2 $\mu$L of the diluted STAR2 product was mixed with 1 $\mu$L of 10 $\mu$M F-BC, 2 $\mu$L of NS2

Barcode (5μM), 2.5 μL 4x Master Mix and 2.5 μL water were mixed together. The PCR protocol for the barcoding reaction is shown in Table 9 below.

Table 9: PCR protocol for barcoding the STAR2 PCR products

| Step | # of Cycles | Temperature | Time |
|------|-------------|-------------|------|
| Hold | 1 | 95°C | 10 min |
| Cycle | 12 | 95°C | 30 sec |
| | | 70°C | 10 sec |
| | | 60°C | 30 sec |
| | | 65°C | 15 sec |
| | | 72°C | 15 sec |
| Hold | 1 | 72°C | 2 min |
| Hold | 1 | 4°C | Infinite |

The barcoded products were then pooled, purified, and quantitated. The pooled samples were sequenced using next generation sequencing (NGS) technology.

The sequencing data were then analyzed using the data analysis methods described herein to generate Hetrate plots of SNPs of chromosomes 13, 18, 21, and X/Y as shown in Figures 4. It was known that trisomy 21 existed in the fetal cells analyzed in this example, and trisomy 21 of the fetal cells was confirmed samples 5F5M (5 fetal cells and 5 maternal cells, Figure 4D), 7F3M (7 fetal cells and 3 maternal cells, Figure 4C), 9F1M (9 fetal cells and 1 maternal cell, Figure 4B), and 10F (10 fetal cells, Figure 4A). Accordingly, the methods disclosed herein can analyze mixed samples containing as few as 5 target cells.

The calculation of Fetal Concordance and Maternal Concordance between cfDNA and cell samples, and Fetal purity successfully determined the identity of the selected cells.

Table 10: Results of analysis of sequencing data of the samples in Example 3

| Cell Lysate ID | Fetal Concordance | Maternal Concordance | Fetal Purity |
|----------------|-------------------|----------------------|--------------|
| 10F | 0.991 | 0.435 | 93% |
| 9F1M | 0.991 | 0.493 | 79% |
| 7F3M | 0.990 | 0.479 | 79% |
| 5F5M | 0.985 | 0.952 | 51% |
| 3F7M | 0.616 | 0.996 | 22% |
| 1F4M | 0.155 | 0.997 | 13% |
| 1F9M | 0.166 | 0.997 | 15% |
| 1F19M | 0.006 | 0.999 | 3% |
| 10M | 0.005 | 0.999 | 1% |
| 10% CF Mix | NA | NA | 8% |

**EXAMPLE 4.**

The purpose of this example is to analyze circulating cells isolated from blood samples of pregnant women. This example demonstrates that the disclosed methods can analyze and confirm the identity of individual circulating cells such as fetal cells in maternal blood samples.

A plurality of blood samples (30 mL each) are collected from pregnant women. 5-10 mL plasma fractions are isolated from each blood sample. Cell-free DNA (cfDNA) is extracted from the plasma fractions. Fetal cells and maternal cells are isolated from each blood sample and lysed. 4-6 cells from the same blood sample are lysed to obtain 10 μL of cell lysate. The samples are subjected to the PCR amplification workflow for fetal cells as described in Example 1.

*cfDNA Library Preparation:* The cfDNA libraries are prepared by following the protocols described in Natera's IFU 131100.1 Revision 02 (IFU-10002) using the Library Preparation Kit (Natera, REF 131100).

Next, STAR1 amplificiation of cfDNA is performed by mixing 6.7 µL of the cfDNA library is mixed with 3 µL cfDNA OneStar Primer Mix, 10 µL 4x Master Mix and 0.3 µL 5M TMAC. The STAR1 PCR protocol is shown in Table 11 below.

Table 11: STAR1 PCR protocol for amplification of the cfDNA library in Example 4.

| Step | # of Cycles | Temperature | Time |
|------|-------------|-------------|------|
| Hold | 1 | 95°C | 10 min |
| Cycle | 20 | 95°C | 30 sec |
| | | 64°C | 15 min |
| | | 72°C | 30 sec |
| Hold | 1 | 72°C | 2 min |
| Hold | 1 | 4°C | Infinite |

For barcoding the cfDNA STAR1 products, the STAR1 cfDNA PCR products are diluted 1:400 with water, and 2 µL of the diluted STAR1 cfDNA PCR products are mixed with 1 µL of 10 µM F-BC, 2 µL NS2 Barcode (5µM) and 5 µL 4x Master Mix. The reaction mixtures for barcoding the cfDNA STAR1 products are run with the PCR protocol shown in Table 12 below.

Table 12: PCR protocol for barcoding cfDNA STAR1 products in Example 4.

| Step | # of Cycles | Temperature | Time |
|------|-------------|-------------|------|
| Hold | 1 | 95°C | 10 min |
| Cycle | 12 | 95°C | 30 sec |
| | | 70°C | 10 sec |
| | | 60°C | 30 sec |
| | | 65°C | 15 sec |
| | | 72°C | 15 sec |
| Hold | 1 | 72°C | 2 min |
| Hold | 1 | 4°C | Infinite |

For STAR1 amplification of the cell lysates, 5.82 µL gDNA STAR1 Primer Mix, 15 µL 4x Master Mix, and 0.18 µL 5M TMAC are added to each sample. The STAR1 reaction mixtures are run with the PCR protocol shown in Table 13 below.

Table 13: STAR1 PCR protocol for the cell lysate samples in Example 4.

| Step | # of Cycles | Temperature | Time |
|------|-------------|-------------|------|
| Hold | 1 | 95°C | 10 min |
| Cycle | 25 | 96°C | 30 sec |
| | | 65°C | 30 min |
| | | 72°C | 30 sec |
| Hold | 1 | 72°C | 2 min |
| Hold | 1 | 4°C | Infinite |

For STAR2 amplification of the STAR1 cell lysate PCR products, the STAR1 PCR products of the cell lysates are diluted 1:200 with water, and 2 µL of the diluted STAR1 product is mixed with 2.94 µL gDNA STAR2 Primer Mix, 2.5 µL 4x Master Mix, 2.5 µL water and 0.06 µL 5M TMAC. The STAR2 reaction mixtures are run with the PCR protocol shown in Table 14 below.

Table 14: STAR2 PCR protocol for the cell lysate samples in Example 4.

| Step | # of Cycles | Temperature | Time |
|---|---|---|---|
| Hold | 1 | 95°C | 10 min |
| Cycle | 15 | 95°C | 30 sec |
| | | 65°C | 1 min |
| | | 60°C | 5 min |
| | | 65°C | 5 min |
| | | 72°C | 30 sec |
| Hold | 1 | 72°C | 2 min |
| Hold | 1 | 4°C | Infinite |

For the barcoding of the cell lysate samples, the STAR2 PCR products of the cell lysates are diluted 1:40 with water, and 2 μL of the diluted STAR2 PCR products are mixed with 1 μL 10 μM F-BC, 2 μL NS2 Barcode (5uM), 2.5 μL 4x Master Mix, and 2.5 μL water. The barcoding reaction mixtures are run with the PCR protocol shown in Table 15 below.

Table 15: Barcoding PCR protocol for the cell lysate samples in Example 4.

| Step | # of Cycles | Temperature | Time |
|---|---|---|---|
| Hold | 1 | 95°C | 10 min |
| Cycle | 12 | 95°C | 30 sec |
| | | 70°C | 10 sec |
| | | 60°C | 30 sec |
| | | 65°C | 15 sec |
| | | 72°C | 15 sec |
| Hold | 1 | 72°C | 2 min |
| Hold | 1 | 4°C | Infinite |

The barcoded products are then pooled, purified, and quantitated. The pooled samples are sequenced using next generation sequencing (NGS) technology. Different barcodes are used for each sample. Depending on the total number of samples, the barcoded cfDNA and barcoded cell lysates can be pooled into one tube or two tubes for sequencing.

The sequencing data are analyzed using the data analysis methods described herein to generate Hetrate plots of SNPs of chromosomes 13, 18, 21, and X/Y. Fetal Concordance and Maternal Concordance between cfDNA and cell samples are calculated as described in section 2 herein. The calculation of Fetal Concordance and Maternal Concordance between cfDNA and cell samples, and Fetal purity can successfully determine the identity of the selected cells.

**EXAMPLE 5.**

The purpose of this example is to further test the sensitivity and specificity of the disclosed method to confirm the identity of individual cells by performing an analysis of blinded cell samples from blood.

A plurality of blood samples are collected from pregnant women, from which single fetal cells and single maternal cells are isolated and cfDNA is extracted. Cellular samples are prepared by isolating 2 fetal cells or 2 maternal cells from the same origin. The isolated cells are then lysed to give a plurality of cell-lysate samples. The cfDNA and cell lysate samples are run with the optimized Panorama v2 gDNA protocols with modifications.

*cfDNA Library Preparation:* The cfDNA libraries are prepared by following the protocols described in Natera's IFU 131100.1 Revision 02 (IFU-10002) using the Library Preparation Kit (Natera, REF 131100).

For STAR1 preparation of cfDNA, 6.7 μL of the cfDNA library is mixed with 3 μL cfDNA OneStar Primer Mix, 10 μL 4x Master Mix, and 0.3 μL 5M TMAC. The STAR1 reaction mixtures are run with the PCR protocol shown in Table 16 below.

Table 16: PCR protocol for STAR1 amplification of cfDNA in Example 5.

| Step | # of Cycles | Temperature | Time |
|---|---|---|---|
| Hold | 1 | 95°C | 10 min |
| Cycle | 20 | 95°C | 30 sec |
| | | 64°C | 15 min |
| | | 72°C | 30 sec |
| Hold | 1 | 72°C | 2 min |
| Hold | 1 | 4°C | Infinite |

For barcoding the cfDNA, the OneStar PCR products are diluted 1:400 with water, 2 µL of the diluted STAR1 product is mixed with 1 µL 10uM F-BC, 2 µL NS2 Barcode (5µM), and 5 µL 4x Master Mix. The barcoding reaction mixtures are run with PCR protocol shown in Table 17 below.

Table 17: PCR protocol for barcoding cfDNA STAR2 products in Example 5.

| Step | # of Cycles | Temperature | Time |
|---|---|---|---|
| Hold | 1 | 95°C | 10 min |
| Cycle | 12 | 95°C | 30 sec |
| | | 70°C | 10 sec |
| | | 60°C | 30 sec |
| | | 65°C | 15 sec |
| | | 72°C | 15 sec |
| Hold | 1 | 72°C | 2 min |
| Hold | 1 | 4°C | Infinite |

For STAR1 amplification of cell lysate sample DNA, 5.82 µL gDNA STAR1 Primer Mix, 15 µL 4x Master Mix, and 0.18 µL 5M TMAC are added to the samples. The STAR1 PCR amplification protocol of cell lysate samples is shown in Table 18 below.

Table 18: PCR protocol for STAR1 amplification of cell-lysate samples in Example 5.

| Step | # of Cycles | Temperature | Time |
|---|---|---|---|
| Hold | 1 | 95°C | 10 min |
| Cycle | 25 | 96°C | 30 sec |
| | | 65°C | 30 min |
| | | 72°C | 30 sec |
| Hold | 1 | 72°C | 2 min |
| Hold | 1 | 4°C | Infinite |

For STAR2 amplification of cell-lysate samples, the STAR1 PCR products of the cell lysates are diluted 1:200 with water, and 2 µL of the diluted STAR1 product is mixed with 2.94 µL gDNA STAR2 Primer Mix, 2.5 µL 4x Master Mix, 2.5 µL water, and 0.06 µL 5M TMAC. The STAR2 PCR amplification protocol of cell lysate samples is shown in Table 19 below.

Table 19: PCR protocol for STAR2 amplification of cell-lysate samples in Example 5.

| Step | # of Cycles | Temperature | Time |
|---|---|---|---|
| Hold | 1 | 95°C | 10 min |

(continued)

| Step | # of Cycles | Temperature | Time |
|------|-------------|-------------|------|
| Cycle | 15 | 95°C | 30 sec |
| | | 65°C | 1 min |
| | | 60°C | 5 min |
| | | 65°C | 5 min |
| | | 72°C | 30 sec |
| Hold | 1 | 72°C | 2 min |
| Hold | 1 | 4°C | Infinite |

For barcoding cell-lysate samples, the STAR2 PCR products of the cell lysates are diluted 1:40 with water, and 2 μL of the diluted STAR2 product is mixed with 1 μL 10 μM F-BC, 2 μL NS2 Barcode (5 μM), 2.5 μL 4x Master Mix, and 2.5 μL water. The barcoding PCR amplification protocol of cell lysate samples is shown in Table 20 below.

Table 20: Barcoding PCR protocol for the cell lysate samples in Example 5.

| Step | # of Cycles | Temperature | Time |
|------|-------------|-------------|------|
| Hold | 1 | 95°C | 10 min |
| Cycle | 12 | 95°C | 30 sec |
| | | 70°C | 10 sec |
| | | 60°C | 30 sec |
| | | 65°C | 15 sec |
| | | 72°C | 15 sec |
| Hold | 1 | 72°C | 2 min |
| Hold | 1 | 4°C | Infinite |

The barcoded products are then pooled, purified, and quantitated. The pooled samples are sequenced using next generation sequencing (NGS) technology. After completion of the sequencing, the data are analyzed using the method described herein. The SNPs of chromosomes 13, 18, 21 and X/Y can be visualized in Hetrate Plots. Using the cfDNA as reference, the sequencing data from an unknown cell lysate sample can be characterized. Fetal Concordance and Maternal Concordance between cfDNA and cell lysate samples are calculated using the algorithm described herein, and used to determine the cellular identity of the cell.

## Claims

1.  A method for monitoring and detection of early relapse or metastasis of a tumor in a cancer patient, wherein the method comprises the steps of:

    a) selecting one or more patient-specific mutations based on somatic mutations identified in a tumor sample of a patient who has been diagnosed with a cancer;
    b) longitudinally collecting one or more blood samples from the patient after the patient has been treated with surgery, first-line chemotherapy, and/or adjuvant therapy;
    c) generating a set of amplicons from cellular DNA isolated from one or more circulating cells that are suspected to be circulating tumor cells and are obtained from a blood sample of the patient, wherein the set of amplicons are generated by multiplex amplification of genomic loci encompassing the patient-specific mutations associated with cancer;
    d) sequencing the set of amplicons by next-generation sequencing and determining the origin of the one or more circulating cells based on the presence of one or more patient-specific mutations in the set of amplicons, wherein the detection of one or more circulating tumor cells comprising the one or more patient-specific mutations is indicative of early relapse or metastasis of the cancer.

2. The method of claim 1, wherein the patient-specific mutations comprise single nucleotide variants (SNV), copy number variants (CNV), indels, and/or or gene fusions associated with cancer.

3. The method of claim 1, wherein the set of amplicons are generated by multiplex amplification of genomic loci encompassing at least 8 patient-specific mutations associated with cancer; or wherein the set of amplicons are generated by multiplex amplification of genomic loci encompassing at least 16 patient-specific mutations associated with cancer.

4. The method of claim 1, wherein the presence of at least 2 patient-specific mutations associated with cancer is indicative that the one or more circulating cells are tumor cells; wherein the presence of at least 5 patient-specific mutations associated with cancer is indicative that the one or more circulating cells are tumor cells.

5. The method of claim 1, wherein the detection of at least 2 circulating tumor cells comprising the one or more patient-specific mutations is indicative of early relapse or metastasis of the cancer.

6. The method of claim 1, wherein the method further comprises identifying additional patient-specific mutations associated with cancer from the genotypes of one or more circulating cells determined to be tumor cells.

7. A method for monitoring and detection of early relapse or metastasis of a tumor in a cancer patient, wherein the method comprises the steps of:

a) selecting one or more patient-specific mutations based on somatic mutations identified in a tumor sample of a patient who has been diagnosed with a cancer;
b) longitudinally collecting one or more blood samples from the patient after the patient has been treated with surgery, first-line chemotherapy, and/or adjuvant therapy;
c) generating a first set of amplicons from cell-free DNA isolated from a blood sample of the patient, wherein the first set of amplicons are generated by multiplex amplification of genomic loci encompassing the patient-specific mutations associated with cancer;
d) sequencing the first set of amplicons by next-generation sequencing and detecting the presence of one or more patient-specific mutations in the set of amplicons, wherein the presence of one or more patient-specific mutations is indicative of early relapse or metastasis of cancer;
e) generating a second set of amplicons from cellular DNA isolated from one or more circulating cells obtained from a blood sample of the patient, wherein the second set of amplicons are generated by multiplex amplification of genomic loci encompaissing the patient-specific mutations associated with cancer;
f) sequencing the second set of amplicons by next-generation sequencing and determining the origin of the one or more circulating cells based on the presence of one or more patient-specific mutations in the set of amplicons and by using the cell-free DNA sequences as reference to determine the origin of the cellular DNA and circulating cells, wherein the presence of one or more patient-specific mutations is indicative that the one or more circulating cells are tumor cells; and
g) identifying one or more additional mutations associated with cancer from the sequences of the one or more circulating cells determined to be tumor cells.

8. The method of claim 7, wherein the patient-specific mutations comprise single nucleotide variants (SNV), copy number variants (CNV), indels, and/or or gene fusions associated with cancer.

9. The method of claim 7, wherein the first and/or second set of amplicons are generated by multiplex amplification of genomic loci encompassing at least 8 patient-specific mutations associated with cancer; or wherein the first and/or second set of amplicons are generated by multiplex amplification of genomic loci encompassing at least 16 patient-specific mutations associated with cancer.

10. The method of claim 7, wherein in step (d) the presence of at least 2 or at least 5 patient-specific mutations associated with cancer is indicative of early relapse or metastasis of cancer.

11. The method of claim 7, wherein in step (f) the presence of at least 2 or at least 5 patient-specific mutations associated with cancer is indicative that the one or more circulating cells are tumor cells.

12. The method of claim 1 or 7, wherein the method comprises separating multiple circulating cells into individual reaction volumes, isolating cellular DNA in each reaction volume, and attaching a sample barcode to the cellular DNA isolated in each reaction volume.

13. The method of claim 1 or 7, wherein the cellular DNA is isolated from a single circulating cell suspected to be a tumor cell.

14. The method of claim 1 or 7, wherein the patient suffers from lung cancer, breast cancer, bladder cancer, or colorectal cancer.

15. The method of claim 7, further comprising treating the patient based on the one or more additional mutations associated with cancer.

**Patentansprüche**

1. Verfahren zum Überwachen auf und Nachweisen eines frühen Rezidivs oder einer frühen Metastase eines Tumors bei einem Krebspatienten, wobei das Verfahren die Schritte umfasst:

   a) Auswählen einer oder mehrerer patientenspezifischer Mutationen auf der Basis somatischer Mutationen, die in einer Tumorprobe eines Patienten identifiziert werden, der mit einem Krebs diagnostiziert wurde;
   b) Abnehmen einer oder mehrerer Blutproben von dem Patienten im Längsschnitt, nachdem der Patient durch Chirurgie, First-Line-Chemotherapie und/oder adjuvante Therapie behandelt wurde;
   c) Erzeugen eines Satzes von Amplikons aus zellulärer DNA, die aus einer oder mehreren zirkulierenden Zellen isoliert wird, von denen vermutet wird, dass sie zirkulierende Tumorzellen sind, und aus einer Blutprobe des Patienten erhalten werden, wobei der Satz von Amplikons durch Multiplexamplifikation von Genomloci erzeugt wird, die die patientenspezifischen Mutationen umfassen, die mit Krebs assoziiert sind;
   d) Sequenzieren des Satzes von Amplikons durch Next-Generation-Sequenzierung und Bestimmen des Ursprungs der einen oder der mehreren zirkulierenden Zellen auf der Basis des Vorliegens einer oder mehrerer patientenspezifischer Mutationen in dem Satz von Amplikons, wobei der Nachweis einer oder mehrerer zirkulierender Tumorzellen, die die eine oder die mehreren patientenspezifischen Mutationen umfassen, ein frühes Rezidiv oder eine frühe Metastase des Krebses angibt.

2. Verfahren nach Anspruch 1, wobei die patientenspezifischen Mutationen Einzelnukleotidvarianten (SNV), Kopienzahlvarianten (CNV), Indele und/oder Genfusionen, die mit Krebs assoziiert sind, umfassen.

3. Verfahren nach Anspruch 1, wobei der Satz von Amplikons durch Multiplexamplifikation von Genomloci erzeugt wird, die mindestens 8 patientenspezifische Mutationen umfassen, die mit Krebs assoziiert sind; oder wobei der Satz von Amplikons durch Multiplexamplifikation von Genomloci erzeugt wird, die mindestens 16 patientenspezifische Mutationen umfassen, die mit Krebs assoziiert sind.

4. Verfahren nach Anspruch 1, wobei das Vorliegen von mindestens 2 patientenspezifischen Mutationen, die mit Krebs assoziiert sind, angibt, dass die eine oder die mehreren zirkulierenden Zellen Tumorzellen sind; wobei das Vorliegen von mindestens 5 patientenspezifischen Mutationen, die mit Krebs assoziiert sind, angibt, dass die eine oder die mehreren zirkulierenden Zellen Tumorzellen sind.

5. Verfahren nach Anspruch 1, wobei der Nachweis von mindestens 2 zirkulierenden Tumorzellen, die die eine oder die mehreren patientenspezifischen Mutationen umfassen, ein frühes Rezidiv oder eine frühe Metastase des Krebses angibt.

6. Verfahren nach Anspruch 1, wobei das Verfahren ferner ein Identifizieren zusätzlicher patientenspezifischer Mutationen, die mit Krebs assoziiert sind, aus den Genotypen einer oder mehrerer zirkulierender Zellen, die als Tumorzellen bestimmt werden, umfasst.

7. Verfahren zum Überwachen auf und Nachweisen eines frühen Rezidivs oder einer frühen Metastase eines Tumors bei einem Krebspatienten, wobei das Verfahren die Schritte umfasst:

   a) Auswählen einer oder mehrerer patientenspezifischer Mutationen auf der Basis somatischer Mutationen, die in einer Tumorprobe eines Patienten identifiziert werden, der mit einem Krebs diagnostiziert wurde;
   b) Abnehmen einer oder mehrerer Blutproben von dem Patienten im Längsschnitt, nachdem der Patient durch Chirurgie, First-Line-Chemotherapie und/oder adjuvante Therapie behandelt wurde;

c) Erzeugen eines ersten Satzes von Amplikons aus zellfreier DNA, die aus einer Blutprobe des Patienten isoliert wird, wobei der erste Satz von Amplikons durch Multiplexamplifikation von Genomloci erzeugt wird, die die patientenspezifischen Mutationen umfassen, die mit Krebs assoziiert sind;

d) Sequenzieren des ersten Satzes von Amplikons durch Next-Generation-Sequenzierung und Nachweisen des Vorliegens einer oder mehrerer patientenspezifischer Mutationen in dem Satz von Amplikons, wobei das Vorliegen einer oder mehrerer patientenspezifischer Mutationen ein frühes Rezidiv oder eine frühe Metastase von Krebs angibt;

e) Erzeugen eines zweiten Satzes von Amplikons aus zellulärer DNA, die aus einer oder mehreren zirkulierenden Zellen isoliert wird, die aus einer Blutprobe des Patienten erhalten werden, wobei der zweite Satz von Amplikons durch Multiplexamplifikation von Genomloci erzeugt wird, die die patientenspezifischen Mutationen umfassen, die mit Krebs assoziiert sind;

f) Sequenzieren des zweiten Satzes von Amplikons durch Next-Generation-Sequenzierung und Bestimmen des Ursprungs der einen oder der mehreren zirkulierenden Zellen auf der Basis des Vorliegens einer oder mehrerer patientenspezifischer Mutationen in dem Satz von Amplikons und durch Verwenden der zellfreien DNA-Sequenzen als Referenz zum Bestimmen des Ursprungs der zellulären DNA und der zirkulierenden Zellen, wobei das Vorliegen einer oder mehrerer patientenspezifischer Mutationen angibt, dass die eine oder die mehreren zirkulierenden Zellen Tumorzellen sind; und

g) Identifizieren einer oder mehrerer zusätzlicher Mutationen, die mit Krebs assoziiert sind, aus den Sequenzen der einen oder der mehreren zirkulierenden Zellen, die als Tumorzellen bestimmt wurden.

8. Verfahren nach Anspruch 7, wobei die patientenspezifischen Mutationen Einzelnukleotidvarianten (SNV), Kopienzahlvarianten (CNV), Indele und/oder Genfusionen, die mit Krebs assoziiert sind, umfassen.

9. Verfahren nach Anspruch 7, wobei der erste und/oder der zweite Satz von Amplikons durch Multiplexamplifikation von Genomloci erzeugt werden, die mindestens 8 patientenspezifische Mutationen umfassen, die mit Krebs assoziiert sind; oder wobei der erste und/oder der zweite Satz von Amplikons durch Multiplexamplifikation von Genomloci erzeugt wird, die mindestens 16 patientenspezifische Mutationen umfassen, die mit Krebs assoziiert sind.

10. Verfahren nach Anspruch 7, wobei in Schritt (d) das Vorliegen von mindestens 2 oder mindestens 5 patientenspezifischen Mutationen, die mit Krebs assoziiert sind, ein frühes Rezidiv oder eine frühe Metastase von Krebs angibt.

11. Verfahren nach Anspruch 7, wobei in Schritt (f) das Vorliegen von mindestens 2 oder mindestens 5 patientenspezifischen Mutationen, die mit Krebs assoziiert sind, angibt, dass die eine oder die mehreren zirkulierenden Zellen Tumorzellen sind.

12. Verfahren nach Anspruch 1 oder 7, wobei das Verfahren ein Trennen mehrerer zirkulierender Zellen in einzelne Reaktionsvolumen, ein Isolieren zellulärer DNA in jedem Reaktionsvolumen und ein Anbringen eines Probebarcodes an die zelluläre DNA, die in jedem Reaktionsvolumen isoliert wurde, umfasst.

13. Verfahren nach Anspruch 1 oder 7, wobei die zelluläre DNA von einer einzelnen zirkulierenden Zelle isoliert wird, von der vermutet wird, dass sie eine Tumorzelle ist.

14. Verfahren nach Anspruch 1 oder 7, wobei der Patient an Lungenkrebs, Brustkrebs, Blasenkrebs oder Kolorektalkrebs leidet.

15. Verfahren nach Anspruch 7, ferner umfassend ein Behandeln des Patienten auf der Basis der einen oder der mehreren zusätzlichen Mutationen, die mit Krebs assoziiert sind.

**Revendications**

1. Procédé de surveillance et de détection d'une récidive précoce ou d'une métastase d'une tumeur chez un patient atteint de cancer, dans lequel la méthode comprend les étapes consistant à :

   a) sélectionner une ou plusieurs mutations spécifiques au patient sur la base de mutations somatiques identifiées dans un échantillon de tumeur d'un patient auquel on a diagnostiqué un cancer ;
   b) collecter de manière longitudinale un ou plusieurs échantillons de sang chez le patient après que le patient a été traité par chirurgie, chimiothérapie de première intention et/ou traitement adjuvant ;

c) générer un ensemble d'amplicons de l'ADN cellulaire isolé d'une ou plusieurs cellules circulantes qui sont soupçonnées d'être des cellules tumorales circulantes et sont obtenues à partir d'un échantillon de sang du patient, dans lequel l'ensemble d'amplicons est généré par une amplification multiplex de loci génomiques comprenant les mutations spécifiques au patient associées au cancer ;

d) séquencer l'ensemble d'amplicons par un séquençage de prochaine génération et déterminer l'origine des une ou plusieurs cellules circulantes sur la base de la présence d'une ou plusieurs mutations spécifiques au patient dans l'ensemble d'amplicons, dans lequel la détection d'une ou plusieurs cellules tumorales circulantes comprenant les une ou plusieurs mutations spécifiques au patient est indicative d'une récidive précoce ou de métastases du cancer.

2. Procédé selon la revendication 1, dans lequel les mutations spécifiques au patient comprennent des variants de nucléotides simples (SNV), des variations du nombre de copies (CNV), des indels et/ou des fusions de gènes associées au cancer.

3. Procédé selon la revendication 1, dans lequel l'ensemble d'amplicons sont générés par une amplification multiplex de loci génomiques comprenant au moins 8 mutations spécifiques au patient associées au cancer ; ou dans laquelle l'ensemble d'amplicons est généré par une amplification multiplex de loci génomiques comprenant au moins 16 mutations spécifiques au patient associées au cancer.

4. Procédé selon la revendication 1, dans lequel la présence d'au moins 2 mutations spécifiques au patient associées au cancer est indicative que les une ou plusieurs cellules circulantes sont des cellules tumorales ; dans lequel la présence d'au moins 5 mutations spécifiques au patient associées au cancer est indicative que les une ou plusieurs cellules circulantes sont des cellules tumorales.

5. Procédé selon la revendication 1, dans lequel la détection d'au moins 2 cellules tumorales circulantes comprenant les une ou plusieurs mutations spécifiques au patient est indicative d'une récidive précoce ou de métastases du cancer.

6. Procédé selon la revendication 1, dans lequel le procédé comprend en outre l'identification de mutations spécifiques au patient supplémentaires associées au cancer à partir des génotypes d'une ou plusieurs cellules circulantes déterminées comme étant des cellules tumorales.

7. Procédé de surveillance et de détection d'une récidive précoce ou de métastases d'une tumeur chez un patient atteint d'un cancer, dans lequel le procédé comprend les étapes consistant à :

a) sélectionner une ou plusieurs mutations spécifiques au patient sur la base de mutations somatiques identifiées dans un échantillon de tumeur d'un patient auquel on a diagnostiqué un cancer ;

b) collecter de manière longitudinale un ou plusieurs échantillons de sang chez le patient après que le patient a été traité par chirurgie, chimiothérapie de première intention et/ou traitement adjuvant ;

c) générer un premier ensemble d'amplicons à partir d'ADN sans cellule isolé d'un échantillon de sang du patient, dans lequel le premier ensemble d'amplicons sont générés par une amplification multiplex de loci génomiques comprenant les mutations spécifiques au patient associées au cancer ;

d) séquencer le premier ensemble d'amplicons par un séquençage de prochaine génération et détecter la présence d'une ou plusieurs mutations spécifiques au patient dans l'ensemble d'amplicons, dans lequel la présence d'une ou plusieurs mutations spécifiques au patient est indicative d'une récidive précoce ou de métastases du cancer ;

e) générer un second ensemble d'amplicons à partir d'ADN cellulaire isolé d'une ou plusieurs cellules circulantes obtenues à partir d'un échantillon de sang du patient, dans lequel le second ensemble d'amplicons sont générés par une amplification multiplex de loci génomiques comprenant les mutations spécifiques au patient associées au cancer ;

f) séquencer le second ensemble d'amplicons par un séquençage de prochaine génération et déterminer l'origine des une ou plusieurs cellules circulantes sur la base de la présence d'une ou plusieurs mutations spécifiques au patient dans l'ensemble d'amplicons et en utilisant les séquences d'ADN sans cellule comme référence pour déterminer l'origine de l'ADN cellulaire et des cellules circulantes, dans lequel la présence des une ou plusieurs mutations spécifiques au patient est indicative que les une ou plusieurs cellules circulantes sont des cellules tumorales ; et

g) identifier une ou plusieurs mutations supplémentaires associées au cancer à partir des séquences des une ou plusieurs cellules circulantes déterminées comme étant des cellules tumorales.

8. Procédé selon la revendication 7, dans lequel les mutations spécifiques au patient comprennent des variants de nucléotides simples (SNV), des variations du nombre de copies (CNV), des indels, et/ou des fusions de gènes associées au cancer.

9. Procédé selon la revendication 7, dans lequel le premier et/ou le second ensemble d'amplicons sont générés par amplification multiplex de loci génomiques comprenant au moins 8 mutations spécifiques au patient associées au cancer ; ou dans lequel le premier et/ou le second ensemble d'amplicons sont générés par amplification multiplex de loci génomiques comprenant au moins 16 mutations spécifiques au patient associées au cancer.

10. Procédé selon la revendication 7, dans lequel, à l'étape (d), la présence d'au moins 2 ou d'au moins 5 mutations spécifiques au patient associées au cancer est indicative d'une récidive précoce ou de métastases du cancer.

11. Procédé selon la revendication 7, dans lequel, à l'étape (f), la présence d'au moins 2 ou d'au moins 5 mutations spécifiques au patient associées au cancer est indicative que les une ou plusieurs cellules circulantes sont des cellules tumorales.

12. Procédé selon la revendication 1 ou 7, dans lequel le procédé comprend la séparation de multiples cellules circulantes en volumes de réaction individuels, l'isolement d'ADN cellulaire dans chaque volume de réaction et la fixation d'un code barre d'échantillon à l'ADN cellulaire isolé dans chaque volume de réaction.

13. Procédé selon la revendication 1 ou 7, dans lequel l'ADN cellulaire est isolé d'une cellule circulante unique soupçonnée d'être une cellule tumorale.

14. Procédé selon la revendication 1 ou 7, dans lequel le patient souffre d'un cancer du poumon, d'un cancer du sein, d'un cancer de la vessie ou d'un cancer colorectal.

15. Procédé selon la revendication 7, comprenant en outre le traitement du patient sur la base des une ou plusieurs mutations supplémentaires associées au cancer.

FIG. 1

EP 3 899 030 B1

FIG. 2

EP 3 899 030 B1

**FIG. 3A**

SAMPLE 1F
0.99% DROPOUT
89.8% MAPPED

**FIG. 3B**

SAMPLE 2F
0.93% DROPOUT
93.5% MAPPED

**FIG. 3C**

SAMPLE 4F
0.37% DROPOUT
98.0% MAPPED

**FIG. 3D**

SAMPLE 10F
0.34% DROPOUT
98.2% MAPPED

**FIG. 3E**

SAMPLE 20F
0.32% DROPOUT
99.3% MAPPED

SAMPLE 10F
FIG. 4A

SAMPLE 9F1M
FIG. 4B

SAMPLE 7F3M
FIG. 4C

SAMPLE 5F5M
FIG. 4D

SAMPLE 3F7M
FIG. 4E

SAMPLE 1F4M
FIG. 4F

SAMPLE 1F9M
FIG. 4G

SAMPLE 1F19M
FIG. 4H

SAMPLE 10M
FIG. 4I

SAMPLE 10% CF MIX
FIG. 4J

| Protocol | Fetal Cell Workflow | | | | | | Panorama v2 gDNA Workkflow | | | | | |
|---|---|---|---|---|---|---|---|---|---|---|---|---|
| Target Set | More than 13,000 SNPs (Panorama v2 targets) | | | | | | More than 13,000 SNPs (Panorama v2 targets) | | | | | |
| Sample Source | Cultured cells or isolated cells | | | | | | Extracted gDNA | | | | | |
| Sample Prep | Arcturus PicoPure DNA Kit | | | | | | Qiagen QIAamp DNA Mini Kit | | | | | |
| Input Amount | 1 - 20 cells | | | | | | 3 - 60 ng gDNA | | | | | |
| STAR1 | STAR1 Components | uL/sample | | STAR1 cycle | | | STAR1 Components | uL/sample | | STAR1 cycle | | |
| | gDNA STAR1 Primer Mix | 7.76 | Step | # of Cycles | Temperature | Time | gDNA STAR1 Primer Mix | 1.94 | Step | # of Cycles | Temperature | Time |
| | 4X MasterMix | 20 | Hold | 1 | 95°C | 10 min | 4X MasterMix | 5 | Hold | 1 | 95°C | 10 min |
| | TMAC (5M) | 0.24 | Cycle | 25 | 96°C | 30 sec | TMAC (5M) | 0.06 | Cycle | 15 | 96°C | 30 sec |
| | cell lysate | 12 | | | 65°C | 30 min | gDNA | 3 | | | 65°C | 1 min |
| | Total | 40 | | | 72°C | 30 sec | Total | 10 | | | 58°C | 6 min |
| | | | Hold | 1 | 72°C | 2 min | | | | | 60°C | 8 min |
| | | | Hold | 1 | 4°C | Infinite | | | | | 65°C | 4 min |
| | | | | | | | | | | | 72°C | 30 sec |
| | | | | | | | | | Hold | 1 | 72°C | 2 min |
| | | | | | | | | | Hold | 1 | 4°C | Infinite |
| STAR2 | STAR2 Components | uL/sample | | STAR2 cycle | | | STAR2 Components | uL/sample | | STAR2 cycle | | |
| | gDNA STAR2 Primer Mix | 2.94 | Step | # of Cycles | Temperature | Time | gDNA STAR2 Primer Mix | 2.94 | Step | # of Cycles | Temperature | Time |
| | 4X MasterMix | 2.5 | Hold | 1 | 95°C | 10 min | 4X MasterMix | 5 | Hold | 1 | 95°C | 10 min |
| | TMAC (5M) | 0.06 | Cycle | 15 | 95°C | 30 sec | TMAC (5M) | 0.06 | Cycle | 15 | 95°C | 30 sec |
| | STAR1 Diltion (1:200) | 2 | | | 65°C | 1 min | STAR1 Diltion (1:200) | 2 | | | 65°C | 1 min |
| | H2O | 2.5 | | | 60°C | 5 min | Total | 10 | | | 60°C | 5 min |
| | Total | 10 | | | 65°C | 5 min | | | | | 65°C | 5 min |
| | | | | | 72°C | 30 sec | | | | | 72°C | 30 sec |
| | | | Hold | 1 | 72°C | 2 min | | | Hold | 1 | 72°C | 2 min |
| | | | Hold | 1 | 4°C | Infinite | | | Hold | 1 | 4°C | Infinite |
| Barcoding | Barcoding Components | uL/sample | | Barcoding cycle | | | Barcoding Components | uL/sample | | Barcoding cycle | | |
| | F-BC (10 uM) | 1 | Step | # of Cycles | Temperature | Time | F-BC (10 uM) | 1 | Step | # of Cycles | Temperature | Time |
| | 4X MasterMix | 2.5 | Hold | 1 | 95°C | 10 min | 4X MasterMix | 5 | Hold | 1 | 95°C | 10 min |
| | H2O | 2.5 | Cycle | 12 | 95°C | 30 sec | NS2 barcode (5 uM) | 2 | Cycle | 12 | 95°C | 30 sec |
| | NS2 barcode (5 uM) | 2 | | | 70°C | 10 sec | STAR2 Dilution (1:40) | 2 | | | 70°C | 10 sec |
| | STAR2 Dilution (1:40) | 2 | | | 60°C | 30 sec | Total | 10 | | | 60°C | 30 sec |
| | Total | 10 | | | 65°C | 15 sec | | | | | 65°C | 15 sec |
| | | | | | 72°C | 15 sec | | | | | 72°C | 15 sec |
| | | | Hold | 1 | 72°C | 2 min | | | Hold | 1 | 72°C | 2 min |
| | | | Hold | 1 | 4°C | Infinite | | | Hold | 1 | 4°C | Infinite |

## FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62780724
- US 62797518
- US 91854415
- US 20130172211 A1
- US 8008018 B
- US 8467976 B2
- US 20120003637 A1
- US 20180173846 A
- US 20180298439 A
- US 603406
- US 20070184467 A
- US 076348
- US 20080243398 A
- US 110685
- US 0952730 W
- WO 2010017214 A
- US 10050824 W
- WO 2011041485 A
- US 2011037018 W
- WO 2011146632 A
- US 201161506 W
- US 7888017 B
- US 8318430 B
- US 20130172211 A
- US 20120003637 A
- US 8467976 B
- US 8296076 B
- US 8195415 B
- US 62008235
- US 62032785
- US 20120190020 A
- US 20120190021 A
- US 20120190557 A
- US 20120191358 A
- US 8024128 B
- US 20070027636 A
- US 20070178501 A
- WO 2009105531 A
- US 20130123120 A
- US 20110033862 A
- US 20110178719 A
- US 8515679 B
- US 61994791
- US 13683604 B
- US 30023511
- US 20120270212 A
- US 8389578 B
- US 8389557 B

**Non-patent literature cited in the description**

- **ALIX-PANABIERES** ; **PANTEL**. *Clin. Chem.*, 2013, vol. 59 (1), 110-118
- **KALNINA et al.** *World J Gastroenterol.*, 07 November 2015, vol. 21 (41), 11636-11653
- **JIANG et al.** 112. *Proc Natl Acad Sci USA*, E1317-E1325
- **HAMAKAWA et al.** *Br J Cancer.*, 2015, vol. 112, 352-356
- **TAKIYA et al.** An empirical approach for thermal stability (Tm) prediction of PNA/DNA duplexes. *Nucleic Acids Symp Ser (Oxf)*, 2004 (48), 131-2
- **H. MAMON et al.** Preferential Amplification of Apoptotic DNA from Plasma: Potential for Enhancing Detection of Minor DNA Alterations in Circulating DNA. *Clinical Chemistry*, 2008, vol. 54, 9
- **A. SIKORA**. Detection of increased amounts of cell-free fetal DNA with short PCR amplicons. *Clin Chem*, January 2010, vol. 56 (1), 136-8
- **WANG HY** ; **LUO M** ; **TERESHCHENKO IV** ; **FRIKKER DM** ; **CUI X** ; **LI JY** ; **HU G** ; **CHU Y** ; **AZARO MA** ; **LIN Y**. *enome Res.*, February 2005, vol. 15 (2), 276-83
- Department of Molecular Genetics, Microbiology and Immunology/The Cancer Institute of New Jersey. *Robert Wood Johnson Medical School*
- **LI H** ; **WANG HY** ; **CUI X** ; **LUO M** ; **HU G** ; **GREENAWALT DM** ; **TERESHCHENKO IV** ; **LI JY** ; **CHU Y** ; **GAO R**. *Methods Mol Biol.*, 2007, 396
- **VARLEY KE** ; **MITRA RD**. *Genome Res.*, 10 October 2008, vol. 18 (11), 1844-50
- *Nature Review Cancer.*, 2014, vol. 14, 535-551
- **CHEN et al.** *Nat. Rev. Cancer.*, 14 August 2014 (8), 535-551
- **CIRIELLO**. *Nat Genet.*, 2013, vol. 45 (10), 1127-1133

- **BROWNING** ; **BROWNING**. Rapid and Accurate Haplotype Phasing and Missing-Data Inference for Whole-Genome Association Studies By Use of Localized Haplotype Clustering. *Am J Hum Genet.*, November 2007, vol. 81 (5), 1084-1097
- **STEPHENS** ; **SCHEET**. Accounting for Decay of Linkage Disequilibrium in Haplotype Inference and Missing-Data Imputation. *Am. J. Hum. Genet.*, 2005, vol. 76, 449-462
- **SCHEET** ; **STEPHENS**. A fast and flexible statistical model for large-scale population genotype data: applications to inferring missing genotypes and haplotypic phase. *Am J Hum Genet*, 2006, vol. 78, 629-644
- **HOWIE** ; **DONNELLY** ; **MARCHINI**. A flexible and accurate genotype imputation method for the next generation of genome-wide association studies. *PLoS Genetics*, 2009, vol. 5 (6), e1000529
- **DELANEAU** ; **COULONGES** ; **ZAGURY**. Shape-IT: new rapid and accurate algorithm for haplotype inference. *BMC Bioinformatics*, 2008, vol. 9, 540
- **ERONEN** ; **GEERTS** ; **TOIVONEN**. HaploRec: Efficient and accurate large-scale reconstruction of haplotypes. *BMC Bioinformatics*, 2006, vol. 7, 542
- **QIN** ; **NIU** ; **LIU**. Partition-Ligation-Expectation-Maximization Algorithm for Haplotype Inference with Single-Nucleotide Polymorphisms. *Am J Hum Genet.*, 2002, vol. 71 (5), 1242-1247
- **KIMMEL** ; **SHAMIR**. GERBIL: Genotype Resolution and Block Identification Using Likelihood. *Proceedings of the National Academy of Sciences of the United States of America (PNAS)*, 2005, vol. 102, 158-162
- **CLAYTON, D.** *SNPHAP: A Program for Estimating Frequencies of Large Haplotypes of SNPs*, 2002
- **BRINZA** ; **ZELIKOVSKY**. 2SNP: scalable phasing based on 2-SNP haplotypes. *Bioinformatics*, 2006, vol. 22 (3), 371-3
- **ABAAN et al.** The Exomes of the NCI-60 Panel: A Genomic Resource for Cancer Biology and Systems Pharmacology. *Cancer Research*, 15 July 2013
- **SU et al.** *J Mol Diagn*, 2011, vol. 13, 74-84
- **CALIN et al.** A microRNA signature associated with prognosis and progression in chronic lymphocytic leukemia. *N Engl J Med*, 2005, vol. 353, 1793-801
- **RYAN et al.** A prospective study of circulating mutant KRAS2 in the serum of patients with colorectal neoplasia: strong prognostic indicator in postoperative follow up. *Gut*, 2003, vol. 52, 101-108
- **LECOMTE T et al.** Detection of free-circulating tumor-associated DNA in plasma of colorectal cancer patients and its association with prognosis. *Int J Cancer*, 2002, vol. 100, 542-548
- **WANG et al.** Potential clinical significance of a plasma-based KRAS mutation analysis in patients with advanced non-small cell lung cancer. *Clin Canc Res*, 2010, vol. 16, 1324-1330
- **FLEISCHHACKER** ; **SCHMIDT**. Circulating nucleic acids (CNAs) and caner - a survey. *Biochim Biophys Acta*, 2007, vol. 1775, 181-232
- **LEVINE** ; **OREN**. The first 30 years of p53: growing ever more complex. *Nature Rev Cancer*, 2009, vol. 9, 749-758
- **DIAS-SANTAGATA et al.** BRAF V600E mutations are common in pleomorphic xanthoastrocytoma: diagnostic and therapeutic implications. *PLOS ONE*, 2011, vol. 6, e17948
- **SHINOZAKI et al.** Utility of circulating B-RAF DNA mutation in serum for monitoring melanoma patients receiving biochemotherapy. *Clin Canc Res*, 2007, vol. 13, 2068-2074
- **BOARD et al.** Detection of BRAF mutations in the tumor and serum of patients enrolled in the AZD6244 (ARRY-142886) advanced melanoma phase II study. *Brit J Canc*, 2009, vol. 101, 1724-1730
- **DOWNWARD J.** Targeting RAS signalling pathways in cancer therapy. *Nature Rev Cancer*, 2003, vol. 3, 11-22
- **JIA et al.** Prediction of epidermal growth factor receptor mutations in the plasma/pleural effusion to efficacy of gefitinib treatment in advanced non-small cell lung cancer. *J Canc Res Clin Oncol*, 2010, vol. 136, 1341-1347
- **KOHLER et al.** Levels of plasma circulating cell free nuclear and mitochondrial DNA as potential biomarkers for breast tumors. *Mol Cancer*, 2009, vol. 8, 53
- **GARCIA et al.** Extracellular tumor DNA in plasma and overall survival in breast cancer patients. *Genes, Chromosomes & Cancer*, 2006, vol. 45, 692-701
- **SORENSEN et al.** Circulating HER2 DNA after trastuzumab treatment predicts survival and response in breast cancer. *Anticancer Res*, 2010, vol. 30, 2463-2468
- **MURTAZA et al.** Non-invasive analysis of acquired resistance to cancer therapy by sequencing of plasma DNA. *Nature*, 2013, vol. 2013
- **WANG et al.** Molecular detection of APC, K-ras, and p53 mutations in the serum of colorectal cancer patients as circulating biomarkers. *World J Surg*, 2004, vol. 28, 721-726
- **LECOMTE et al.** Detection of free-circulating tumor-associated DNA in plasma of colorectal cancer patients and its association with prognosis. *Int J Cancer*, 2002, vol. 100, 542-548

- **SCHWARZENBACH et al.** Molecular analysis of the polymorphisms of thymidylate synthase on cell-free circulating DNA in blood of patients with advanced colorectal carcinoma. *Int J Cancer*, 2009, vol. 127, 881-888
- **JIAN et al.** Prediction of epidermal growth factor receptor mutations in the plasma/pleural effusion to efficacy of gefitinib treatment in advanced non-small cell lung cancer. *J Canc Res Clin Oncol*, 2010, vol. 136, 1341-1347
- **MURTAZA et al.** Non-invasive analysis of acquired resistance to cancer therapy by sequencing of plasma DNA. *Nature*, 2013
- **HUNG et al.** *J Clin Pathol*, 2009, vol. 62, 308-313
- **FACKENTHAL1** ; **GODLEY**. *Disease Models & Mechanisms*, 2008, vol. 1, 37-42
- **BARTENEVA**. *Biochim Biophys Acta*, 24 February 2013, vol. 1836 (1), 105-22
- **IBRAHIM et al.** *Adv Biochem Eng Biotechnol.*, 2007, vol. 106, 19-39
- **GU et al.** *J. Neurochem.*, 2012, vol. 122, 641-649
- **SCHMITT et al.** Detection of ultra-rare mutations by next-generation sequencing. *Proc Natl Acad Sci U S A.*, 2012, vol. 109 (36), 14508-14513
- **KOHLER et al.** Levels of plasma circulating cell free nuclear and mitochondrial DNA as potential biomarkers for breast tumors. *Mol Cancer*, 2009, vol. 8 (105), 8
- **SCHWARZENBACH et al.** Evaluation of cell-free tumor DNA and RNA in patients with breast cancer and benign breast disease.. *Mol Biosys*, 2011, vol. 7, 2848-2854
- **LI et al.** Development of noninvasive prenatal diagnosis of trisomy 21 by RT-MLPA with a new set of SNP markers. *Arch Gynecol Obstet.*, 05 July 2013
- **SCHOUTEN et al.** Relative quantification of 40 nucleic acid sequences by multiplex ligation-dependent probe amplification.. *Nucleic Acids Res*, 2002, vol. 30, e57
- **DENG et al.** Non-invasive prenatal diagnosis of trisomy 21 by reverse transcriptase multiplex ligation-dependent probe amplification. *Clin, Chem. Lab Med.*, 2011, vol. 49, 641-646
- **LO et al.** Plasma placental RNA allelic ratio permits noninvasive prenatal chromosomal aneuploidy detection. *Nat Med*, 2007, vol. 13, 218-223
- **TSUI et al.** Systematic micro-array based identification of placental mRNA in maternal plasma: towards non-invasive prenatal gene expression profiling. *J Med Genet*, 2004, vol. 41, 461-467
- **CHIM et al.** Detection and characterization of placental microRNAs in maternal plasma. *Clin Chem.*, 2008, vol. 54 (3), 482-90
- **MURALI et al.** , "Crystal structure of Taq DNA polymerase in complex with an inhibitory Fab: the Fab is directed against an intermediate in the helix-coil dynamics of the enzyme. *Proc. Natl. Acad. Sci. USA*, 1998, vol. 95, 12562-12567
- **FREY** ; **SUPPMAN**. *BioChemica.*, 1995, vol. 2, 34-35
- **CHESTER** ; **MARSHAK**. *Analytical Biochemistry*, 1993, vol. 209, 284-290
- DNA-Dependent DNA Polymerases. **TABOR** ; **STRUH et al.** Current Protocols in Molecular Biology. John Wiley & Sons, Inc., 1989, 3.5.10-3.5.12
- **SAMBROOK et al.** Molecular Cloning: A Laboratory Manual.. Cold Spring Harbor: Cold Spring Harbor Laboratory Press, 1989, 5.44-5.47
- **BOUDSOCQ et al.** *Nucleic Acids Res.*, 2001, vol. 29, 4607-4616
- **MCDONALD et al.** *Nucleic Acids Res.*, 2006, vol. 34, 1102-1111
- **MCDONALD, J.P. et al.** *Nucleic Acids Res.*, 2006, vol. 34, 1102-1111
- **NISHANT et al.** HUMHOT: a database of human meiotic recombination hot spots. *Nucleic Acids Research*, 2006, vol. 34, D25-D28
- **MACKIEWICZ et al.** Distribution of Recombination Hotspots in the Human Genome - A Comparison of Computer Simulations with Real Data. *PLoS ONE*, vol. 8 (6), e65272
- **JAIN et al.** Tumor Models In Cancer Research. Humana Press Inc, 2001, 647-671
- **MARRACK et al.** *Current Opinion in Immunology*, 2000, vol. 12, 206-209
- **HARKIN**. *Oncologist*, 2000, vol. 5, 501-507
- **PELIZZARI et al.** *Nucleic Acids Res.*, 2000, vol. 28 (22), 4577-4581
- Remington: The Science and Practice of Pharmacy. Lippincott Williams & Wilkins, 2006